(19) **Europäisches Patentamt / European Patent Office / Office européen des brevets**

(11) **EP 3 059 306 A1**

(12) **EUROPEAN PATENT APPLICATION**

(43) Date of publication:
**24.08.2016 Bulletin 2016/34**

(51) Int Cl.:
**C12N 5/04** (2006.01)  **C12N 15/82** (2006.01)
**C12N 15/90** (2006.01)  **A01H 5/00** (2006.01)
**C12N 9/10** (2006.01)  **C12N 9/24** (2006.01)

(21) Application number: **16151745.3**

(22) Date of filing: **12.01.2006**

(84) Designated Contracting States:
**AT BE BG CH CY CZ DE DK EE ES FI FR GB GR HU IE IS IT LI LT LU LV MC NL PL PT RO SE SI SK TR**

(30) Priority: **12.01.2005  US 643717 P**

(62) Document number(s) of the earlier application(s) in accordance with Art. 76 EPC:
**12184430.2 / 2 584 033**
**12152296.5 / 2 489 726**
**06718079.4 / 1 850 656**

(71) Applicant: **Monsanto Technology LLC**
**St. Louis, MO 63167 (US)**

(72) Inventor: **ABAD, Mark**
**St. Louis, MO Missouri 63167 (US)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

Remarks:
•The complete document including Reference Tables and the Sequence Listing can be downloaded from the EPO website
•This application was filed on 18.01.2016 as a divisional application to the application mentioned under INID code 62.
•Claims filed after the date of filing of the application/after the date of receipt of the divisional application (Rule 68(4) EPC).

(54) **GENES AND USES FOR PLANT IMPROVEMENT**

(57)    This invention provides transgenic plant cells with recombinant DNA for expression of proteins that are useful for imparting enhanced agronomic trait(s) to transgenic crop plants. This invention also provides transgenic plants and progeny seed comprising the transgenic plant cells where the plants are selected for having an enhanced trait selected from the group of traits consisting of enhanced water use efficiency, enhanced cold tolerance, increased yield, enhanced nitrogen use efficiency, enhanced seed protein and enhanced seed oil. Also disclosed are methods for manufacturing transgenic seed and plants with enhanced traits.

EP 3 059 306 A1

**Description**

**CROSS REFERENCE TO RELATED APPLICATIONS**

This application claims benefit under 35USC § 119(e) of United States provisional application Serial No. 60/642,717, filed 01/12/2005, herein incorporated by reference.

**INCOPORATION OF SEQUENCE LISTING**

Two copies of the sequence listing (Copy 1 and Copy 2) and a computer readable form (CRF) of the sequence listing, all on CD-ROMs, each containing the file named 38-21(53629)B_seqListing.txt, which is 59,207,680 bytes (measured in MS-WINDOWS) and was created on January 11, 2006, are herein incorporated by reference.

**INCORPORATION OF COMPUTER PROGRAM LISTING**

Computer Program Listing folders hmmer-2.3.2 and 158pfamDir are contained on a compact disc and is hereby incorporated herein by reference in their entirety. Folder hmmer-2.3.2 contains the source code and other associated file for implementing the HMMer software for Pfam analysis. Folder 158pfamDir contains 158 Pfam Hidden Markov Models. Both folders were created on the disk on January 11, 2006, having a total size of 16,027,648 bytes (measured in MS-WINDOWS).

**FIELD OF THE INVENTION**

Disclosed herein are inventions in the field of plant genetics and developmental biology. More specifically, the present inventions provide transgenic seeds for crops, wherein the genome of said seed comprises recombinant DNA, the expression of which results in the production of transgenic plants that have improved trait(s).

**BACKGROUND OF THE INVENTION**

Transgenic plants with improved traits such as improved yield, environmental stress tolerance, pest resistance, herbicide tolerance, modified seed compositions, and the like are desired by both farmers and consumers. Although considerable efforts in plant breeding have provided significant gains in desired traits, the ability to introduce specific DNA into plant genomes provides further opportunities for generation of plants with improved and/or unique traits. The ability to develop transgenic plants with improved traits depends in part on the identification of genes that are useful in recombinant DNA constructs for production of transformed plants with improved properties.

**SUMMARY OF THE INVENTION (NEW)**

This invention provides recombinant DNA for expression of proteins that impart enhanced agronomic traits in transgenic plants. Recombinant DNA in this invention is provided in a construct comprising a promoter that is functional in plant cells and that is operably linked to DNA that encodes a protein having at least one amino acid domain in a sequence that exceeds the Pfam gathering cutoff for amino acid sequence alignment with a protein domain family identified by a Pfam name in the group of Pfam domain names identified in Table 17. In more specific embodiments of the invention plant cells are provided which express a protein having amino acid sequence with at least 90% identity to a consensus amino acid sequence in the group of consensus amino acid sequences consisting of the consensus amino acid sequence constructed for SEQ ID NO: 205 and homo logs thereof listed in Table 2 through the consensus amino acid sequence constructed for SEQ ID NO:408 and homo logs thereof listed in Table 2. Amino acid sequences of homologs are SEQ ID NO:409 through 19247. In even more specific embodiments of the invention the protein expressed in plant cells is a protein selected from the group of proteins identified in Table 1 by annotation to a related protein in Genbank and alternatively identified in Table 16 by identification of protein domain family. An exemplary plant cell of this invention has recombinant DNA that encodes a protein identified by the Pdam name "RNA_pol_L".

Other aspects of the invention are specifically directed to transgenic plant cells comprising the recombinant DNA of the invention, transgenic plants comprising a plurality of such plant cells, progeny transgenic seed, embryo and transgenic pollen from such plants. Such plant cells are selected from a population of transgenic plants regenerated from plant cells transformed with recombinant DNA and that express the protein by screening transgenic plants in the population for an enhanced trait as compared to control plants that do not have said recombinant DNA, where the enhanced trait is enhanced water use efficiency, enhanced cold tolerance, enhanced heat tolerance, enhanced shade tolerance, enhanced tolerance to salt exposure, increased yield, enhanced nitrogen use efficiency, enhanced seed protein and enhanced seed oil. Thus, this invention provides transgenic plants and seeds with cells having recombinant DNA that impart at least one of those enhanced traits to the plants or seeds.

In yet another aspect of the invention the plant cells, plants, seeds, embryo and pollen further comprise DNA expressing a protein that provides tolerance from exposure to an herbicide applied at levels that are lethal to a wild type of said plant cell. Such tolerance is especially useful not only as an advantageous trait in such plants but is also useful in a selection step in the methods of the invention. In aspects of the invention the agent of such herbicide is a glyphosate, dicamba, or glufosinate compound.

Yet other aspects of the invention provide transgenic plants which are homozygous for the recombinant DNA and transgenic seed of the invention from corn, soybean, cotton, canola, alfalfa, wheat or rice plants. In other important embodiments for practice of various aspects of the invention in Argentina the recombinant DNA is provided in plant cells derived from corn lines that that are and maintain resistance to the Mal de Rio Cuarto virus or the *Puccina sorghi* fungus or both.

This invention also provides methods for manufacturing non-natural, transgenic seed that can be used to produce a crop of transgenic plants with an enhanced trait resulting from expression of stably-integrated, recombinant DNA for expressing a protein having at least one domain of amino acids in a sequence that exceeds the Pfam gathering cutoff for amino acid sequence alignment with a protein domain family identified by a Pfam name in the group of Pfam names identified in Table 17. More specifically the method comprises (a) screening a population of plants for an enhanced trait and recombinant DNA, where individual plants in the population can exhibit the trait at a level less than, essentially the same as or greater than the level that the trait is exhibited in control plants which do not express the recombinant DNA, (b) selecting from the population one or more plants that exhibit the trait at a level greater than the level that said trait is exhibited in control plants, (c) verifying that the recombinant DNA is stably integrated in said selected plants, (d) analyzing tissue of a selected plant to determine the production of a protein having the function of a protein encoded by nucleotides in a sequence of one of SEQ ID NO:1-204; and (e) collecting seed from a selected plant. In one aspect of the invention the plants in the population further comprise DNA expressing a protein that provides tolerance to exposure to an herbicide applied at levels that are lethal to wild type plant cells and the selecting is effected by treating the population with the herbicide, e.g. a glyphosate, dicamba, or glufosinate compound. In another aspect of the invention the plants are selected by identifying plants with the enhanced trait. The methods are especially useful for manufacturing corn, soybean, cotton, alfalfa, wheat or rice seed selected as having one of the enhanced traits described above.

Another aspect of the invention provides a method of producing hybrid corn seed comprising acquiring hybrid corn seed from a herbicide tolerant corn plant which also has stably-integrated, recombinant DNA comprising a promoter that is (a) functional in plant cells and (b) is operably linked to DNA that encodes a protein having at least one domain of amino acids in a sequence that exceeds the Pfam gathering cutoff for amino acid sequence alignment with a protein domain family identified by a Pfam name in the group of Pfam names identified in Table 17. The methods further comprise producing corn plants from said hybrid corn seed, where a fraction of the plants produced from said hybrid corn seed is homozygous for said recombinant DNA, a fraction of the plants produced from said hybrid corn seed is hemizygous for said recombinant DNA, and a fraction of the plants produced from said hybrid corn seed has none of said recombinant DNA; selecting corn plants which are homozygous and hemizygous for said recombinant DNA by treating with an herbicide; collecting seed from herbicide-treated-surviving corn plants and planting said seed to produce further progeny corn plants; repeating the selecting and collecting steps at least once to produce an inbred corn line; and crossing the inbred corn line with a second corn line to produce hybrid seed.

Another aspect of the invention provides a method of selecting a plant comprising plant cells of the invention by using an immunoreactive antibody to detect the presence of protein expressed by recombinant DNA in seed or plant tissue. Yet another aspect of the invention provides anti-counterfeit milled seed having, as an indication of origin, a plant cells of this invention with unique recombinant DNA.

Another aspect of the invention provides plant cells having recombinant DNA for suppressing the expression of DNA identified in Table 1 and Table 16. More specific aspects of the invention provide plant cells having recombinant DNA for suppressing the expression of a protein having the function in a plant of the protein with amino acid sequence of SEQ ID NO: 213, 215, 218, 222, 258, 269, 275, 334, 361, 368, and 407 or the corresponding Pfam identified in Table 16, i.e. Catalase, Bromdomain, FTCD_N, MatE, DPBB_1, tRNA-synt_2b, Sugar_tr and MFS_1, DUF6 and DUF250, LEA_4, MIP and DUF231, respectively. Such suppression can be effected by any of a number of ways known in the art, e.g. anti-sense suppression, sense co-suppression, RNAi or knockout.

Still other specific aspects of this invention relate to growing transgenic plants with enhanced water use efficiency or enhanced nitrogen use efficiency. For instance, this invention provides methods of growing a corn, cotton or soybean crop without irrigation water comprising planting seed having plant cells of the invention which are selected for enhanced water use efficiency. Alternatively methods comprise applying reduced irrigation water, e.g. providing up to 300 millimeters of ground water during the production of a corn crop. This invention also provides methods of growing a corn, cotton or soybean crop without added nitrogen fertilizer comprising planting seed having plant cells of the invention which are selected for enhanced nitrogen use efficiency.

The various aspects of this invention are especially useful for transgenic plant cells in seeds and transgenic plants having any of the above-described enhanced traits in crop plants such as corn (maize), soybean, cotton, canola (rape), wheat, sunflower, sorghum, alfalfa, barley, millet, rice, tobacco, fruit and vegetable crops, and turfgrass.

The invention also comprises recombinant DNA constructs of the DNA useful for imparting enhanced traits in plants having thee cells of this invention.

## BRIEF DESCRIPTION OF THE DRAWINGS

Figure 1 is an alignment of amino acid sequences.
Figures 2 and 3 are plasmid maps.

## DETAILED DESCRIPTION OF THE INVENTION

In the attached sequence listing:

SEQ ID NO: 1-204 are DNA sequence of "genes" used in the recombinant DNA imparting an enhanced trait in plant cells;
SEQ ID NO:205-408 are amino acid sequence of the cognate protein of those "genes";
SEQ ID NO:409-19247 are amino acid sequence of homologous proteins;
SEQ ID NO:19248 is a consensus amino acid sequence.
SEQ ID NO: 19249 is a DNA sequence of a plasmid vector useful for corn transformation; and
SEQ ID NO: 19250 is a DNA sequence of a plasmid vector useful for soybean transformation.

As used herein, "gene" means DNA including chromosomal DNA, plasmid DNA, cDNA, synthetic DNA, or other DNA that is transcribed to RNA, e.g. mRNA that encodes a protein or a protein fragment or anti-sense RNA or dsRNA for suppression of expression of a target gene and its cognate protein.

"Transgenic plant cell" means a plant cell produced as an original transformation event, cells in plants regenerated from the original transformation, cells in progeny plants and seeds, and cells in plants and seed from later generations or crosses of progeny plants and seeds, where such plant cells have recombinant DNA in their genome resulting from the original transformation.

"Recombinant DNA" means genetically engineered polynucleotide produced from endogenous and/or exogenous elements generally arranged as a transcription unit. Recombinant DNA may comprise DNA segments obtained from different sources, or DNA segments obtained from the same source, but which have been manipulated to join DNA segments which do not naturally exist in the joined form. A recombinant polynucleotide may exist outside of the cell, for example as a PCR fragment, or integrated into a genome, such as a plant genome.

"Trait" means to a physiological, morphological, biochemical, or physical characteristic of a plant or particular plant material or cell. In some instances, this characteristic is visible to the human eye, such as seed or plant size, or can be measured by biochemical techniques, such as detecting the protein, starch, or oil content of seed or leaves, or by observation of a metabolic or physiological process, e.g. by measuring uptake of carbon dioxide, or by the observation of the expression level of a gene or genes, e.g., by employing Northern analysis, RT-PCR, microarray gene expression assays, or reporter gene expression systems, or by agricultural observations such as stress tolerance, yield, or pathogen tolerance. An "enhanced trait" as used in describing the aspects of this invention includes enhanced water use efficiency, enhanced cold tolerance, enhanced heat tolerance, enhanced shade tolerance, enhanced tolerance to salt exposure, increased yield, enhanced nitrogen use efficiency, enhanced seed protein and enhanced seed oil.

As used herein, "control plant" is a plant without trait-improving recombinant DNA. A control plant is used to measure and compare trait improvement in a transgenic plant with such trait-improving recombinant DNA. A suitable control plant may be a non-transgenic plant of the parental line used to generate a transgenic plant herein. Alternatively, a control plant may be a transgenic plant that comprises an empty vector or marker gene, but does not contain the recombinant DNA that produces the trait improvement. A control plant may also be a negative segregant progeny of hemizygous transgenic plant. In certain demonstrations of trait improvement, the use of a limited number of control plants can cause a wide variation in the control dataset. In analyzing trait data during screening to discover DNA useful in the plant cells of this invention it is useful to minimize the effect of the variation within the control dataset, i.e. a "reference" which is a trimmed mean of all data from both transgenic and control plants grown under the same conditions and at the same developmental stage. The trimmed mean is calculated by eliminating a specific percentage, i.e. 20%, of the smallest and largest observation from the data set and then calculating the average of the remaining observation. Many transgenic plants comprising transgenic plant cells containing the recombinant DNA identified herein as imparting an enhanced trait will not exhibit an enhanced agronomic trait. The transgenic plants and seeds comprising the transgenic plant cells and having enhanced agronomic traits of this invention are identified by screening a population of transgenic plants and/or seeds for the members of the population having the enhanced trait. Screens for transgenic plant cells in crop plants are described more particularly in the examples below. In some cases, the trait enhancement can be measured quantitatively. For example, the trait enhancement can be at least a 2% desirable difference in an observed trait, at least a 5% desirable difference, at least about a 10% desirable difference, at least about a 20% desirable difference, at least about a 30% desirable difference, at least about a 50% desirable difference, at least about a 70% desirable difference, or at least about a 100% difference, or an even greater desirable difference. In other cases, the trait enhancement is measured qualitatively.

Many agronomic traits can affect "yield", including without limitation, plant height, pod number, pod position on the plant, number of internodes, incidence of pod shatter, grain size, efficiency of nodulation and nitrogen fixation, efficiency of nutrient assimilation, resistance to biotic and abiotic stress, carbon assimilation, plant architecture, resistance to lodging, percent seed germination, seedling vigor, and juvenile traits. Other traits that can affect yield include, efficiency of germination (including germination in stressed conditions), growth rate (including growth rate in stressed conditions), ear number, seed number per ear, seed size, composition of seed (starch, oil, protein) and characteristics of seed fill. Also of interest is the generation of transgenic plants that demonstrate desirable phenotypic properties that may or may not confer an increase in overall plant yield. Such properties include enhanced plant morphology, plant physiology or improved components of the mature seed harvested from the transgenic plant.

"Stress condition" refers to the condition unfavorable for a plant, which adversely affect plant metabolism, growth and/or development. A plant under the stress condition typically shows reduced germination rate, retarded growth and development, reduced photosynthesis rate, and eventually leading to reduction in yield. Specifically, "water deficit stress" used herein preferably refers to the sub-optimal conditions for water and humidity needed for normal growth of natural plants. Relative water content (RWC) can be used as a physiological measure of plant water deficit. It measures the effect of osmotic adjustment in plant water status, when a plant is under stressed conditions. Conditions which may result in water deficit stress include heat, drought, high salinity and PEG induced osmotic stress. "Cold stress" used herein preferably refers to the exposure of a plant to a temperatures below (two or more degrees Celsius below) those normal for a particular species or particular strain of plant. "Low nitrogen availability stress" used herein preferably refers to a plant growth condition with 50% of the conventional nitrogen inputs. "Shade stress" used herein preferably refers to limited light availability that triggers the shade avoidance response in plant. Plants are subject to shade stress when localized at lower part of the canopy, or in close proximity of neighboring vegetation. Shade stress may become exacerbated when the planting density exceeds the average prevailing density for a particular plant species. The average prevailing densities per acre of a few examples of crop plants in the USA in the year 2000 were: wheat 1,000,000-1,500,000; rice 650,000-900,000; soybean 150,000-200,000, canola 260,000-350,000, sunflower 17,000-23,000 and cotton 28,000-55,000 plants per acre (Cheikh, et al., (2003) U.S. Patent Application No..20030101479).

"Increased yield" of a transgenic plant of the present invention may be evidenced and measured in a number of ways, including test weight, seed number per plant, seed weight, seed number per unit area (i.e. seeds, or weight of seeds, per acre), bushels per acre, tons per acre, tons per acre, kilo per hectare. For example, maize yield may be measured as production of shelled corn kernels per unit of production area, e.g. in bushels per acre or metric tons per hectare, often reported on a moisture adjusted basis, e.g. at 15.5 % moisture. Increased yield may result from improved utilization of key biochemical compounds, such as nitrogen, phosphorous and carbohydrate, or from improved tolerance to environmental stresses, such as cold, heat, drought, salt, and attack by pests or pathogens. Trait-enhancing recombinant DNA may also be used to provide transgenic plants having improved growth and development, and ultimately increased yield, as the result of modified expression of plant growth regulators or modification of cell cycle or photosynthesis pathways.

"Expression" means transcription of DNA to produce RNA. The resulting RNA may be without limitation mRNA encoding a protein, antisense RNA that is complementary to an mRNA encoding a protein, or an RNA transcript comprising a combination of sense and antisense gene regions, such as for use in RNAi technology. Expression as used herein may also refer to production of encoded protein from mRNA.

"Promoter" means a region of DNA that is upstream from the start of transcription and is involved in recognition and binding of RNA polymerase and other proteins to initiate transcription. A "plant promoter" is a promoter capable of initiating transcription in plant cells whether or not its origin is a plant cell. Exemplary plant promoters include, but are not limited to, those that are obtained from plants, plant viruses, and bacteria which comprise genes expressed in plant cells such Agrobacterium or Rhizobium. Examples of promoters under developmental control include promoters that preferentially initiate transcription in certain tissues, such as leaves, roots, or seeds. Such promoters are referred to as "tissue preferred". Promoters which initiate transcription only in certain tissues are referred to as "tissue specific". A "cell type" specific promoter primarily drives expression in certain cell types in one or more organs, for example, vascular cells in roots or leaves. An "inducible" or "repressible" promoter is a promoter which is under environmental control.

Examples of environmental conditions that may effect transcription by inducible promoters include anaerobic conditions, or certain chemicals, or the presence of light. Tissue specific, tissue preferred, cell type specific, and inducible promoters constitute the class of "non-constitutive" promoters. A "constitutive" promoter is a promoter which is active under most conditions. As used herein, "antisense orientation" includes reference to a polynucleotide sequence that is operably linked to a promoter in an orientation where the antisense strand is transcribed. The antisense strand is sufficiently complementary to an endogenous transcription product such that translation of the endogenous transcription product is often inhibited. "Operably linked" refers to the association of two or more nucleic acid fragments on a single nucleic acid fragment so that the function of one is affected by the other. For example, a promoter is operably linked with a coding sequence when it is capable of affecting the expression of that coding sequence (i.e., that the coding sequence is under the transcriptional control of the promoter). Coding sequences can be operably linked to regulatory sequences in sense or antisense orientation.

"Consensus amino acid sequence" means an artificial, amino acid sequence of conserved parts of the proteins encoded by homologous genes, e.g. as determined by a CLUSTALW alignment of amino acid sequence of homolog proteins or a group of proteins having identified by the gathering cutoff for a Pfam protein domain family.

Homologous genes are genes which encode homologous proteins with the same or similar biological function or having the same Pfam protein domain family. Homologous genes may be generated by the event of speciation (see ortholog) or by the event of genetic duplication (see paralog). "Orthologs" refer to a set of homologous genes in different species that evolved from a common ancestral gene by specification. Normally, orthologs retain the same function in the course of evolution; and "paralogs" refer to a set of homologous genes in the same species that have diverged from each other as a consequence of genetic duplication. Thus, homologous genes can be from the same or a different organism. As used herein, "homolog" means a protein that performs the same biological function as a second protein including those identified by sequence identity search.

"Percent identity" refers to the extent to which two optimally aligned DNA or protein segments are invariant throughout a window of alignment of components, e.g. nucleotide sequence or amino acid sequence. An "identity fraction" for aligned segments of a test sequence and a reference sequence is the number of identical components which are shared by sequences of the two aligned segments divided by the total number of sequence components in the reference segment over a window of alignment which is the smaller of the full test sequence or the full reference sequence. "Percent identity" ("% identity") is the identity fraction times 100. "% identity to a consensus amino acid sequence" is 100 times the identity fraction in a window of alignment of an amino acid sequence of a test protein optimally aligned to consensus amino acid sequence of this invention.

"Arabidopsis" means plants of *Arabidopsis thaliana.*

As used herein "Pfam" refers to a large collection of multiple sequence alignments and hidden Markov models covering many common protein families, e.g. Pfam version 18.0 (August 2005) contains alignments and models for 7973 protein families and is based on the Swissprot 47.0 and SP-TrEMBL 30.0 protein sequence databases. See S.R. Eddy, "Profile Hidden Markov Models", Bioinformatics 14:755-763, 1998. Pfam is currently maintained and updated by a Pfam Consortium. The alignments represent some evolutionary conserved structure that has implications for the protein's function. Profile hidden Markov models (profile HMMs) built from the Pfam alignments are useful for automatically recognizing that a new protein belongs to an existing protein family even if the homology by alignment appears to be low. Once one DNA is identified as encoding a protein which imparts an enhanced trait when expressed in transgenic plants, other DNA encoding proteins in the same protein family are identified by querying the amino acid sequence of protein encoded by candidate DNA against the Hidden Markov Model which characterizes the Pfam domain using HMMER software, a current version of which is provided in the appended computer listing. Candidate proteins meeting the gathering cutoff for the alignment of a particular Pfam are in the protein family and have cognate DNA that is useful in constructing recombinant DNA for the use in the plant cells of this invention. Hidden Markov Model databases for use with HMMER software in identifying DNA expressing protein in a common Pfam for recombinant DNA in the plant cells of this invention are also included in the appended computer listing. The HMMER software and Pfam databases are version 18.0 and were used to identify known domains in the proteins corresponding to amino acid sequence of SEQ ID NO:205 through SEQ ID NO:408. All DNA encoding proteins that have scores higher than the gathering cutoff disclosed in Table 27 by Pfam analysis disclosed herein can be used in recombinant DNA of the plant cells of this invention, e.g. for selecting transgenic plants having enhanced agronomic traits. The relevant Pfams for use in this invention, as more specifically disclosed below, are 2-oxoacid_dh, ADH_N, ADH_zinc_N, AP2, AUX_IAA, Aa_trans, Abhydrolase_1, Acyl_transf_1, Aldedh, Aldo_ket_red, Alpha-amylase, Aminotran_1_2, Aminotran_3, Ammonium_transp, Arm, Asn_synthase, BAG, BSD, Beta_elim_lyase, Biotin_lipoyl, Brix, Bromodomain, C1_4, CTP_transf_2, Catalase, CcmH, Chal_sti_synt_C, Cyclin_C, Cyclin_N, Cys_Met_Meta_PP, DAO, DIM1, DPBB_1, DRMBL, DUF167, DUF231, DUF250, DUF6, DUF783, DUF962, E2F_TDP, E3_binding, EBP, Enolase_C, Enolase_N, F420_oxidored, FAD_binding_2, FA_desaturase, FKBP_C, FTCD_N, Fe_bilin_red, Fer4, GAF, GATase_2, GIDA, GSHPx, Gpi16, HGTP_anticodon, HI0933_like, HLH, HMG_CoA_synt, HWE_HK, Ham1p_like, HhH-GPD, Homeobox, Hpt, Iso_dh, K-box, LEA_4, LRRNT_2, LRR_1, Ldh_1_C, Ldh_1_N, Lectin_legA, Lectin_legB, Lipase_GDSL, MFS_1, MIP, MatE, Metalloenzyme, Methyltransf_11, Methyltransf_12, Molybdop_Fe4S4, Molybdopterin, Molydop_binding, Mov34, MtN3_slv, Myb_DNA-binding, NAD_Gly3P_dh_N, NAD_binding_2, NIR_SIR, NIR_SIR_ferr, NPH3, NTP_transferase, Nuc_sug_transp, PA, PAR1, PFK, PGI, PGK, PGM_PMM_I, PGM_PMM_II, PGM_PMM_III, PGM_PMM_IV, PP2C, PTR2, Peptidase_C26, Phi_1, Phytochrome, Pkinase, Pkinase_Tyr, Pollen_allerg_1, Pribosyltran, Proteasome, Pyr_redox, Pyr_redox_2, Pyr_redox_dim, RNA_pol_L, RNA_pol_Rpb6, RRM_1, RRN3, Radical_SAM, Ras, Response_reg, Rhodanese, Ribosomal_S8e, Rieske, SAC3_GANP, SBDS, SET, SRF-TF, SURF5, Skp1, Skp1_POZ, Ssl1, Sterol_desat, Sugar_tr, TCP, ThiF, Transaldolase, UQ_con, Ubie_methyltran, WD40, WRKY, adh_short, bZIP_1, bZIP_2, cNMP_binding, iPGM_N, p450, tRNA-synt_2b, ubiquitin, zf-A20, zf-AN1, zf-B_box, zf-C2H2, zf-C3HC4, zf-CCCH, the databases for which are included in the appended computer program listing.

## Recombinant DNA constructs

This invention provides recombinant DNA constructs comprising one or more of the genes disclosed herein for imparting one or more enhanced traits to transgenic plants and seeds. Such constructs also typically comprise a promoter operatively linked to said polynucleotide to provide for expression in a target plant. Other construct components may include additional regulatory elements, such as 5' or 3' untranslated regions (such as polyadenylation sites), intron regions, and transit or signal peptides. Such recombinant DNA constructs can be assembled using methods known to those of ordinary skill in the art.

Recombinant constructs prepared in accordance with this invention generally includes a 3' untranslated DNA region (UTR) that typically contains a polyadenylation sequence following the polynucleotide coding region. Examples of useful 3' UTRs include those from the nopaline synthase gene of *Agrobacterium tumefaciens (nos),* a gene encoding the small subunit of a ribulose-1,5-bisphosphate carboxylase-oxygenase (rbcS), and the T7 transcript of *Agrobacterium tumefaciens* and those 3' UTR elements disclosed in the following examples. Constructs and vectors may also include a transit peptide for targeting of a gene target to a plant organelle, particularly to a chloroplast, leucoplast or other plastid organelle. For descriptions of the use of chloroplast transit peptides, see U.S. Patent 5, 188,642 and U.S. Patent No. 5,728,925, incorporated herein by reference.

Table1 provides a list of genes that can be used in recombinant DNA for imparting an enhanced trait in the transgenic plant cells, plants and seeds of this invention. In screens of recombinant DNA expressed in a model plant the recombinant DNA was shown to be associated with enhanced traits. The cognate protein was used to identify homologs for constructing a consensus amino acid sequence for each cognate protein and for identifying the characterizing Pfams. With reference to Table 1:

"NUC SEQ ID" refers to a SEQ ID NO. for particular DNA sequence in the Sequence Listing;

"PEP SEQ ID" refers to a SEQ ID NO. in the Sequence Listing for the amino acid sequence of a protein cognate to a particular DNA;

"construct_id" refers to an arbitrary number used to identify a particular recombinant DNA construct comprising the particular DNA;

"gene" refers to an arbitrary name used to identify the particular DNA;

"orientation" refers to the orientation of the particular DNA in a recombinant DNA construct relative to the promoter; and

"species name" refers to the organism from which the particular DNA was derived.

Table 1

| NUC SEQ ID | PEP SEQ ID | construct_id | Gene | orientation | Species Name |
|---|---|---|---|---|---|
| 1 | 205 | 12360 | CGPG1018 | SENSE | Arabidopsis thaliana |
| 2 | 206 | 12030 | CGPG1063 | SENSE | Arabidopsis thaliana |
| 3 | 207 | 15210 | CGPG1124 | SENSE | Arabidopsis thaliana |
| 4 | 208 | 17465 | CGPG1178 | SENSE | Arabidopsis thaliana |
| 5 | 209 | 13222 | CGPG1259 | SENSE | Arabidopsis thaliana |
| 6 | 210 | 12919 | CGPG1290 | SENSE | Arabidopsis thaliana |
| 7 | 211 | 12927 | CGPG1300 | SENSE | Arabidopsis thaliana |

(continued)

| NUC SEQ ID | PEP SEQ ID | construct_id | Gene | orientation | Species Name |
|---|---|---|---|---|---|
| 8 | 212 | 14841 | CGPG1572 | SENSE | Arabidopsis thaliana |
| 9 | 213 | 13478 | CGPG1616 | ANTI-SENSE | Arabidopsis thaliana |
| 10 | 214 | 17309 | CGPG1840 | SENSE | Arabidopsis thaliana |
| 11 | 215 | 19116 | CGPG1861 | ANTI-SENSE | Arabidopsis thaliana |
| 12 | 216 | 14837 | CGPG1882 | SENSE | Arabidopsis thaliana |
| 13 | 217 | 71253 | CGPG195 | SENSE | Arabidopsis thaliana |
| 14 | 218 | 16004 | CGPG2061 | ANTI-SENSE | Arabidopsis thaliana |
| 15 | 219 | 72712 | CGPG2256 | SENSE | Arabidopsis thaliana |
| 16 | 220 | 71546 | CGPG2305 | SENSE | Arabidopsis thaliana |
| 17 | 221 | 70109 | CGPG2368 | SENSE | Saccharomyces cerevisiae |
| 18 | 222 | 10335 | CGPG246 | ANTI-SENSE | Arabidopsis thaliana |
| 19 | 223 | 17919 | CGPG2780 | SENSE | Arabidopsis thaliana |
| 20 | 224 | 71148 | CGPG3225 | SENSE | Arabidopsis thaliana |
| 21 | 225 | 19647 | CGPG3229 | SENSE | Arabidopsis thaliana |
| 22 | 226 | 18844 | CGPG3371 | SENSE | Arabidopsis thaliana |
| 23 | 227 | 19525 | CGPG3548 | SENSE | Arabidopsis thaliana |
| 24 | 228 | 19617 | CGPG3573 | SENSE | Arabidopsis thaliana |
| 25 | 229 | 19750 | CGPG3913 | SENSE | Saccharomyces cerevisiae |
| 26 | 230 | 19964 | CGPG3922 | SENSE | Glycine max |
| 27 | 231 | 19814 | CGPG3934 | SENSE | Glycine max |
| 28 | 232 | 19720 | CGPG3954 | SENSE | Glycine max |
| 29 | 233 | 19845 | CGPG3956 | SENSE | Glycine max |
| 30 | 234 | 71072 | CGPG3963 | SENSE | Glycine max |
| 31 | 235 | 19949 | CGPG4010 | SENSE | Glycine max |
| 32 | 236 | 19902 | CGPG4016 | SENSE | Glycine max |
| 33 | 237 | 19981 | CGPG4019 | SENSE | Glycine max |
| 34 | 238 | 19757 | CGPG4037 | SENSE | Glycine max |
| 35 | 239 | 19850 | CGPG4056 | SENSE | Glycine max |
| 36 | 240 | 19942 | CGPG4115 | SENSE | Glycine max |
| 37 | 241 | 19787 | CGPG4124 | SENSE | Glycine max |
| 38 | 242 | 19801 | CGPG4137 | SENSE | Glycine max |
| 39 | 243 | 19705 | CGPG4156 | SENSE | Glycine max |
| 40 | 244 | 19737 | CGPG4175 | SENSE | Glycine max |
| 41 | 245 | 19812 | CGPG4184 | SENSE | Glycine max |
| 42 | 246 | 71556 | CGPG4298 | SENSE | Arabidopsis thaliana |
| 43 | 247 | 71330 | CGPG4457 | SENSE | Arabidopsis thaliana |
| 44 | 248 | 71332 | CGPG4460 | SENSE | Arabidopsis thaliana |

(continued)

| NUC SEQ ID | PEP SEQ ID | construct_id | Gene | orientation | Species Name |
|---|---|---|---|---|---|
| 45 | 249 | 71571 | CGPG4493 | SENSE | Arabidopsis thaliana |
| 46 | 250 | 70677 | CGPG4558 | SENSE | Arabidopsis thaliana |
| 47 | 251 | 71689 | CGPG4661 | SENSE | Glycine max |
| 48 | 252 | 73685 | CGPG4807 | SENSE | Arabidopsis thaliana |
| 49 | 253 | 72636 | CGPG4858 | SENSE | Arabidopsis thaliana |
| 50 | 254 | 73651 | CGPG4869 | SENSE | Arabidopsis thaliana |
| 51 | 255 | 73342 | CGPG4876 | SENSE | Arabidopsis thaliana |
| 52 | 256 | 73271 | CGPG5011 | SENSE | Arabidopsis thaliana |
| 53 | 257 | 73282 | CGPG5071 | SENSE | Arabidopsis thaliana |
| 54 | 258 | 10903 | CGPG514 | ANTI-SENSE | Arabidopsis thaliana |
| 55 | 259 | 73913 | CGPG5337 | SENSE | Glycine max |
| 56 | 260 | 74305 | CGPG5400 | SENSE | Arabidopsis thaliana |
| 57 | 261 | 74306 | CGPG5402 | SENSE | Arabidopsis thaliana |
| 58 | 262 | 73769 | CGPG5430 | SENSE | Arabidopsis thaliana |
| 59 | 263 | 74237 | CGPG5448 | SENSE | Arabidopsis thaliana |
| 60 | 264 | 74244 | CGPG5466 | SENSE | Arabidopsis thaliana |
| 61 | 265 | 74256 | CGPG5491 | SENSE | Arabidopsis thaliana |
| 62 | 266 | 72714 | CGPG5513 | SENSE | Saccharomyces cerevisiae |
| 63 | 267 | 16014 | CGPG552 | SENSE | Arabidopsis thaliana |
| 64 | 268 | 72751 | CGPG5524 | SENSE | Saccharomyces cerevisiae |
| 65 | 269 | 10814 | CGPG554 | ANTI-SENSE | Arabidopsis thaliana |
| 66 | 270 | 72743 | CGPG5555 | SENSE | Saccharomyces cerevisiae |
| 67 | 271 | 72721 | CGPG5569 | SENSE | Saccharomyces cerevisiae |
| 68 | 272 | 72959 | CGPG5608 | SENSE | Glycine max |
| 69 | 273 | 72984 | CGPG5620 | SENSE | Arabidopsis thaliana |
| 70 | 274 | 73061 | CGPG5675 | SENSE | Rhodopseudomonas palustris |
| 71 | 275 | 10180 | CGPG57 | ANTI-SENSE | Arabidopsis thaliana |
| 72 | 276 | 73029 | CGPG5737 | SENSE | Saccharomyces cerevisiae |
| 73 | 277 | 11145 | CGPG574 | SENSE | Arabidopsis thaliana |
| 74 | 278 | 72920 | CGPG5763 | SENSE | Saccharomyces cerevisiae |
| 75 | 279 | 72957 | CGPG5788 | SENSE | Saccharomyces cerevisiae |
| 76 | 280 | 73044 | CGPG5808 | SENSE | Glycine max |
| 77 | 281 | 74323 | CGPG5873 | SENSE | Arabidopsis thaliana |
| 78 | 282 | 74327 | CGPG5901 | SENSE | Arabidopsis thaliana |
| 79 | 283 | 74329 | CGPG5903 | SENSE | Arabidopsis thaliana |
| 80 | 284 | 74340 | CGPG5907 | SENSE | Arabidopsis thaliana |
| 81 | 285 | 74341 | CGPG5911 | SENSE | Arabidopsis thaliana |

(continued)

| NUC SEQ ID | PEP SEQ ID | construct_id | Gene | orientation | Species Name |
|---|---|---|---|---|---|
| 82 | 286 | 74345 | CGPG5932 | SENSE | Arabidopsis thaliana |
| 83 | 287 | 74616 | CGPG6017 | SENSE | Arabidopsis thaliana |
| 84 | 288 | 74604 | CGPG6019 | SENSE | Arabidopsis thaliana |
| 85 | 289 | 74608 | CGPG6043 | SENSE | Arabidopsis thaliana |
| 86 | 290 | 74609 | CGPG6044 | SENSE | Arabidopsis thaliana |
| 87 | 291 | 74366 | CGPG6073 | SENSE | Arabidopsis thaliana |
| 88 | 292 | 74383 | CGPG6098 | SENSE | Arabidopsis thaliana |
| 89 | 293 | 74374 | CGPG6100 | SENSE | Arabidopsis thaliana |
| 90 | 294 | 74618 | CGPG6117 | SENSE | Arabidopsis thaliana |
| 91 | 295 | 74619 | CGPG6118 | SENSE | Arabidopsis thaliana |
| 92 | 296 | 74622 | CGPG6124 | SENSE | Arabidopsis thaliana |
| 93 | 297 | 74628 | CGPG6131 | SENSE | Arabidopsis thaliana |
| 94 | 298 | 74631 | CGPG6139 | SENSE | Arabidopsis thaliana |
| 95 | 299 | 74669 | CGPG6140 | SENSE | Arabidopsis thaliana |
| 96 | 300 | 74670 | CGPG6145 | SENSE | Arabidopsis thaliana |
| 97 | 301 | 74647 | CGPG6163 | SENSE | Arabidopsis thaliana |
| 98 | 302 | 74385 | CGPG6310 | SENSE | Arabidopsis thaliana |
| 99 | 303 | 74386 | CGPG6330 | SENSE | Arabidopsis thaliana |
| 100 | 304 | 74387 | CGPG6331 | SENSE | Arabidopsis thaliana |
| 101 | 305 | 74685 | CGPG6362 | SENSE | Arabidopsis thaliana |
| 102 | 306 | 73487 | CGPG6386 | SENSE | Sinorhizobium meliloti |
| 103 | 307 | 73476 | CGPG6393 | SENSE | Bacillus subtilis subsp. subtilis str. 168 |
| 104 | 308 | 73465 | CGPG6400 | SENSE | Escherichia coli K12 |
| 105 | 309 | 73418 | CGPG6404 | SENSE | Nostoc punctiforme PCC 73102 |
| 106 | 310 | 73480 | CGPG6425 | SENSE | Sinorhizobium meliloti |
| 107 | 311 | 73482 | CGPG6438 | SENSE | Agrobacterium tumefaciens str. C58 |
| 108 | 312 | 73513 | CGPG6457 | SENSE | Bacillus subtilis subsp. subtilis str. 168 |
| 109 | 313 | 73550 | CGPG6468 | SENSE | Bacillus halodurans |
| 110 | 314 | 73527 | CGPG6474 | SENSE | Desulfitobacterium hafniense |
| 111 | 315 | 73516 | CGPG6481 | SENSE | Xenorhabdus nematophila |
| 112 | 316 | 73530 | CGPG6498 | SENSE | Escherichia coli K12 |
| 113 | 317 | 73534 | CGPG6527 | SENSE | Bacillus subtilis subsp. subtilis str. 168 |
| 114 | 318 | 74125 | CGPG6544 | SENSE | Pseudomonas fluorescens PfO-1 |
| 115 | 319 | 74161 | CGPG6547 | SENSE | Escherichia coli K12 |
| 116 | 320 | 74126 | CGPG6552 | SENSE | Escherichia coli K12 |
| 117 | 321 | 74115 | CGPG6559 | SENSE | Escherichia coli K12 |
| 118 | 322 | 74127 | CGPG6560 | SENSE | Escherichia coli K12 |

(continued)

| NUC SEQ ID | PEP SEQ ID | construct_id | Gene | orientation | Species Name |
|---|---|---|---|---|---|
| 119 | 323 | 74128 | CGPG6568 | SENSE | Escherichia coli K12 |
| 120 | 324 | 74165 | CGPG6579 | SENSE | Escherichia coli K12 |
| 121 | 325 | 74106 | CGPG6582 | SENSE | Pseudomonas fluorescens PfO-1 |
| 122 | 326 | 74130 | CGPG6584 | SENSE | Pseudomonas syringae pv. tomato str. DC3000 |
| 123 | 327 | 74132 | CGPG6600 | SENSE | Xenorhabdus nematophila |
| 124 | 328 | 74144 | CGPG6601 | SENSE | Xenorhabdus nematophila |
| 125 | 329 | 74402 | CGPG6663 | SENSE | Synechocystis sp. |
| 126 | 330 | 74476 | CGPG6685 | SENSE | Bacillus halodurans |
| 127 | 331 | 74417 | CGPG6688 | SENSE | Bacillus halodurans |
| 128 | 332 | 74453 | CGPG6691 | SENSE | Bacillus subtilis subsp. subtilis str. 168 |
| 129 | 333 | 74418 | CGPG6696 | SENSE | Escherichia coli K12 |
| 130 | 334 | 10150 | CGPG67 | ANTI-SENSE | Arabidopsis thaliana |
| 131 | 335 | 74503 | CGPG6767 | SENSE | Escherichia coli K12 |
| 132 | 336 | 74504 | CGPG6775 | SENSE | Agrobacterium tumefaciens |
| 133 | 337 | 74528 | CGPG6777 | SENSE | Bacillus halodurans |
| 134 | 338 | 74588 | CGPG6782 | SENSE | Escherichia coli K12 |
| 135 | 339 | 74541 | CGPG6786 | SENSE | Escherichia coli K12 |
| 136 | 340 | 74553 | CGPG6787 | SENSE | Nostoc punctiforme PCC 73102 |
| 137 | 341 | 74554 | CGPG6795 | SENSE | Pseudomonas syringae pv. tomato str. DC3000 |
| 138 | 342 | 74578 | CGPG6797 | SENSE | Synechocystis sp. PCC 6803 |
| 139 | 343 | 74590 | CGPG6798 | SENSE | Xenorhabdus nematophila |
| 140 | 344 | 75834 | CGPG6820 | SENSE | Arabidopsis thaliana |
| 141 | 345 | 75835 | CGPG6822 | SENSE | Arabidopsis thaliana |
| 142 | 346 | 18020 | CGPG684 | SENSE | Arabidopsis thaliana |
| 143 | 347 | 75850 | CGPG6893 | SENSE | Arabidopsis thaliana |
| 144 | 348 | 75861 | CGPG6937 | SENSE | Arabidopsis thaliana |
| 145 | 349 | 75875 | CGPG6992 | SENSE | Arabidopsis thaliana |
| 146 | 350 | 74548 | CGPG712 | SENSE | Arabidopsis thaliana |
| 147 | 351 | 74880 | CGPG7357 | SENSE | Glycine max |
| 148 | 352 | 74903 | CGPG7407 | SENSE | Zea mays |
| 149 | 353 | 74915 | CGPG7408 | SENSE | Zea mays |
| 150 | 354 | 74927 | CGPG7409 | SENSE | Zea mays |
| 151 | 355 | 74951 | CGPG7411 | SENSE | Glycine max |
| 152 | 356 | 74940 | CGPG7418 | SENSE | Zea mays |
| 153 | 357 | 74977 | CGPG7429 | SENSE | Synechocystis sp. |
| 154 | 358 | 74954 | CGPG7435 | SENSE | Xanthomonas campestris |

(continued)

| NUC SEQ ID | PEP SEQ ID | construct_id | Gene | orientation | Species Name |
|---|---|---|---|---|---|
| 155 | 359 | 74907 | CGPG7439 | SENSE | Synechocystis sp. |
| 156 | 360 | 74919 | CGPG7440 | SENSE | Synechocystis sp. |
| 157 | 361 | 11735 | CGPG745 | ANTI-SENSE | Arabidopsis thaliana |
| 158 | 362 | 74980 | CGPG7453 | SENSE | Synechocystis sp. |
| 159 | 363 | 74993 | CGPG7462 | SENSE | Bacillus halodurans |
| 160 | 364 | 75337 | CGPG7469 | SENSE | Saccharomyces cerevisiae |
| 161 | 365 | 75339 | CGPG7485 | SENSE | Zea mays |
| 162 | 366 | 75316 | CGPG7491 | SENSE | Glycine max |
| 163 | 367 | 75352 | CGPG7494 | SENSE | Zea mays |
| 164 | 368 | 12189 | CGPG752 | ANTI-SENSE | Arabidopsis thaliana |
| 165 | 369 | 75321 | CGPG7531 | SENSE | Zea mays |
| 166 | 370 | 75358 | CGPG7542 | SENSE | Zea mays |
| 167 | 371 | 75312 | CGPG7554 | SENSE | Zea mays |
| 168 | 372 | 75463 | CGPG7583 | SENSE | Zea mays |
| 169 | 373 | 75475 | CGPG7584 | SENSE | Zea mays |
| 170 | 374 | 75440 | CGPG7589 | SENSE | Glycine max |
| 171 | 375 | 75488 | CGPG7593 | SENSE | Glycine max |
| 172 | 376 | 75418 | CGPG7603 | SENSE | Glycine max |
| 173 | 377 | 75419 | CGPG7611 | SENSE | Zea mays |
| 174 | 378 | 75431 | CGPG7612 | SENSE | Glycine max |
| 175 | 379 | 75455 | CGPG7614 | SENSE | Glycine max |
| 176 | 380 | 75491 | CGPG7617 | SENSE | Zea mays |
| 177 | 381 | 75456 | CGPG7622 | SENSE | Glycine max |
| 178 | 382 | 75480 | CGPG7624 | SENSE | Glycine max |
| 179 | 383 | 75492 | CGPG7625 | SENSE | Zea mays |
| 180 | 384 | 75409 | CGPG7626 | SENSE | Zea mays |
| 181 | 385 | 75424 | CGPG7651 | SENSE | Zea mays |
| 182 | 386 | 75550 | CGPG7676 | SENSE | Glycine max |
| 183 | 387 | 75575 | CGPG7686 | SENSE | Glycine max |
| 184 | 388 | 75528 | CGPG7690 | SENSE | Glycine max |
| 185 | 389 | 75564 | CGPG7693 | SENSE | Glycine max |
| 186 | 390 | 75553 | CGPG7700 | SENSE | Glycine max |
| 187 | 391 | 75506 | CGPG7704 | SENSE | Glycine max |
| 188 | 392 | 75554 | CGPG7708 | SENSE | Glycine max |
| 189 | 393 | 75590 | CGPG7711 | SENSE | Glycine max |
| 190 | 394 | 75543 | CGPG7715 | SENSE | Glycine max |
| 191 | 395 | 75567 | CGPG7717 | SENSE | Glycine max |

(continued)

| NUC SEQ ID | PEP SEQ ID | construct_id | Gene | orientation | Species Name |
|---|---|---|---|---|---|
| 192 | 396 | 75544 | CGPG7723 | SENSE | Glycine max |
| 193 | 397 | 75556 | CGPG7724 | SENSE | Glycine max |
| 194 | 398 | 75546 | CGPG7739 | SENSE | Glycine max |
| 195 | 399 | 75558 | CGPG7740 | SENSE | Glycine max |
| 196 | 400 | 75571 | CGPG7749 | SENSE | Glycine max |
| 197 | 401 | 75583 | CGPG7750 | SENSE | Glycine max |
| 198 | 402 | 75536 | CGPG7754 | SENSE | Zea mays |
| 199 | 403 | 75991 | CGPG8266 | SENSE | Chloroflexus aurantiacus |
| 200 | 404 | 75909 | CGPG8275 | SENSE | Chloroflexus aurantiacus |
| 201 | 405 | 12824 | CGPG885 | SENSE | Arabidopsis thaliana |
| 202 | 406 | 18026 | CGPG894 | SENSE | Arabidopsis thaliana |
| 203 | 407 | 11810 | CGPG899 | ANTI-SENSE | Arabidopsis thaliana |
| 204 | 408 | 72418 | CGPG968 | SENSE | Arabidopsis thaliana |

Recombinant DNA

Exemplary DNA for use in the present invention to improve traits in plants are provided herein as SEQ ID NO:1 through SEQ ID NO:204, as well as the homo logs of such DNA molecules. A subset of the exemplary DNA includes fragments of the disclosed full polynucleotides consisting of oligonucleotides of at least 15, preferably at least 16 or 17, more preferably at least 18 or 19, and even more preferably at least 20 or more, consecutive nucleotides. Such oligonucleotides are fragments of the larger molecules having a sequence selected from the group consisting of SEQ ID NO:1 through SEQ ID NO:204, and find use, for example as probes and primers for detection of the polynucleotides of the present invention.

Also of interest in the present invention are variants of the DNA provided herein. Such variants may be naturally occurring, including DNA from homologous genes from the same or a different species, or may be non-natural variants, for example DNA synthesized using chemical synthesis methods, or generated using recombinant DNA techniques. Degeneracy of the genetic code provides the possibility to substitute at least one base of the protein encoding sequence of a gene with a different base without causing the amino acid sequence of the polypeptide produced from the gene to be changed. Hence, a DNA useful in the present invention may have any base sequence that has been changed from the sequences provided herein by substitution in accordance with degeneracy of the genetic code.

Homologs of the genes providing DNA of demonstrated as useful in improving traits in model plants disclosed herein will generally demonstrate significant identity with the DNA provided herein. DNA is substantially identical to a reference DNA if, when the sequences of the polynucleotides are optimally aligned there is about 60% nucleotide equivalence; more preferably 70%; more preferably 80% equivalence; more preferably 85% equivalence; more preferably 90%; more preferably 95%; and/or more preferably 98% or 99% equivalence over a comparison window. A comparison window is preferably at least 50-100 nucleotides, and more preferably is the entire length of the polynucleotide provided herein. Optimal alignment of sequences for aligning a comparison window may be conducted by algorithms; preferably by computerized implementations of these algorithms (for example, the Wisconsin Genetics Software Package Release 7.0-10.0, Genetics Computer Group, 575 Science Dr., Madison, WI). The reference polynucleotide may be a full-length molecule or a portion of a longer molecule. Preferentially, the window of comparison for determining polynucleotide identity of protein encoding sequences is the entire coding region.

Proteins useful for imparting improved traits are entire proteins or at least a sufficient portion of the entire protein to impart the relevant biological activity of the protein. The term "protein" also includes molecules consisting of one or more polypeptide chains. Thus, a protein useful in the present invention may constitute an entire protein having the desired biological activity, or may constitute a portion of an oligomeric protein having multiple polypeptide chains. Proteins useful for generation of transgenic plants having improved traits include the proteins with an amino acid sequence provided herein as SEQ ID NO: 205 through SEQ ID NO: 408, as well as homologs of such proteins.

Homologs of the proteins useful in the present invention may be identified by comparison of the amino acid sequence of the protein to amino acid sequences of proteins from the same or different plant sources, e.g. manually or by using known homology-based search algorithms such as those commonly known and referred to as BLAST, FASTA, and Smith-Waterman. As used herein, a homolog is a protein from the same or a different organism that performs the same biological function as the polypeptide to which it is compared. An orthologous relation between two organisms is not necessarily manifest as a one-to-one correspondence between two genes, because a gene can be duplicated or deleted after organism phylogenetic separation, such as speciation. For a given protein, there may be no ortholog or more than one ortholog. Other complicating factors include alternatively spliced transcripts from the same gene, limited gene identification, redundant copies of the same gene with different sequence lengths or corrected sequence. A local sequence alignment program, e.g. BLAST, can be used to search a database of sequences to find similar sequences, and the summary Expectation value (E-value) used to measure the sequence base similarity. As a protein hit with the best E-value for a particular organism may not necessarily be an ortholog or the only ortholog, a reciprocal BLAST search is used in the present invention to filter hit sequences with significant E-values for ortholog identification. The reciprocal BLAST entails search of the significant hits against a database of amino acid sequences from the base organism that are similar to the sequence of the query protein. A hit is a likely ortholog, when the reciprocal BLAST's best hit is the query protein itself or a protein encoded by a duplicated gene after speciation. Thus, homolog is used herein to described proteins that are assumed to have functional similarity by inference from sequence base similarity. The relationship of homologs with amino acid sequences of SEQ ID NO:409 to 19247 to the proteins with amino acid sequences of SEQ ID NO:206 to 408 is found in the listing of Table 2.

A further aspect of the invention comprises functional homolog proteins which differ in one or more amino acids from those of a trait-improving protein disclosed herein as the result of one or more of the well-known conservative amino acid substitutions, e.g. valine is a conservative substitute for alanine and threonine is a conservative substitute for serine. Conservative substitutions for an amino acid within the native sequence can be selected from other members of a class to which the naturally occurring amino acid belongs. Representative amino acids within these various classes include, but are not limited to: (1) acidic (negatively charged) amino acids such as aspartic acid and glutamic acid; (2) basic (positively charged) amino acids such as arginine, histidine, and lysine; (3) neutral polar amino acids such as glycine, serine, threonine, cysteine, tyrosine, asparagine, and glutamine; and (4) neutral nonpolar (hydrophobic) amino acids such as alanine, leucine, isoleucine, valine, proline, phenylalanine, tryptophan, and methionine. Conserved substitutes for an amino acid within a native amino acid sequence can be selected from other members of the group to which the naturally occurring amino acid belongs. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfur-containing side chains is cysteine and methionine. Naturally conservative amino acids substitution groups are: valine-leucine, valine-isoleucine, phenylalanine-tyrosine, lysine-arginine, alanine-valine, aspartic acid-glutamic acid, and asparagine-glutamine. A further aspect of the invention comprises proteins that differ in one or more amino acids from those of a described protein sequence as the result of deletion or insertion of one or more amino acids in a native sequence.

Homologs of the trait-improving proteins disclosed provided herein will generally demonstrate significant sequence identity. Of particular interest are proteins having at least 50% sequence identity, more preferably at least about 70% sequence identity or higher, e.g. at least about 80% sequence identity with an amino acid sequence of SEQ ID NO: 205 through SEQ ID NO: 408. Of course useful proteins also include those with higher identity, e.g. 90% to 99% identity. Identity of protein homologs is determined by optimally aligning the amino acid sequence of a putative protein homolog with a defined amino acid sequence and by calculating the percentage of identical and conservatively substituted amino acids over the window of comparison. The window of comparison for determining identity can be the entire amino acid sequence disclosed herein, e.g. the full sequence of any of SEQ ID NO:205 through SEQ ID NO:408.

Genes that are homologous to each other can be grouped into families and included in multiple sequence alignments. Then a consensus sequence for each group can be derived. This analysis enables the derivation of conserved and class- (family) specific residues or motifs that are functionally important. These conserved residues and motifs can be further validated with 3D protein structure if available. The consensus sequence can be used to define the full scope of the invention, e.g. to identify proteins with a homolog relationship. Thus, the present invention contemplates that protein homologs include proteins with an amino acid sequence that has at least 90% identity to such a consensus amino acid sequence sequences.

In particular embodiments, the inventors contemplate the use of antibodies, either monoclonal or polyclonal which bind to the proteins disclosed herein. Means for preparing and characterizing antibodies are well known in the art (See, e.g., Antibodies: A Laboratory Manual, Cold Spring Harbor Laboratory, 1988; incorporated herein by reference). The methods for generating monoclonal antibodies (mAbs) generally begin along the same lines as those for preparing polyclonal antibodies. Briefly, a polyclonal antibody is prepared by immunizing an animal with an immunogenic composition in accordance with the present invention and collecting antisera from that immunized animal. A wide range of animal species can be used for the production of antisera. Typically the animal used for production of anti-antisera is a rabbit, a mouse, a rat, a hamster, a guinea pig or a *goat.* Because of the relatively large blood volume of rabbits, a rabbit is a preferred choice for production of polyclonal antibodies.

mAbs may be readily prepared through use of well-known techniques, such as those exemplified in U.S. Pat. No. 4,196,265, incorporated herein by reference. Typically, this technique involves immunizing a suitable animal with a selected immunogen composition, e.g., a purified or partially purified antifungal protein, polypeptide or peptide. The immunizing composition is administered in a manner effective to stimulate antibody producing cells. Rodents such as mice and rats are preferred animals, however, the use of rabbit, sheep, or frog cells is also possible. The use of rats may provide certain advantages (Goding, 1986, pp. 60-61), but mice are preferred, with the BALB/c mouse being most preferred as this is most routinely used and generally gives a higher percentage of stable fusions.

Following immunization, somatic cells with the potential for producing antibodies, specifically B lymphocytes (B cells), are selected for use in the mAb generating protocol.The antibody-producing B lymphocytes from the immunized animal are then fused with cells of an immortal myeloma cell, generally one of the same species as the animal that was immunized to establish a population of hybridomas from which specific hybridomas are selected. The selected hybridomas would then be serially diluted and cloned into individual antibody-producing cell lines, which clones can then be propagated indefinitely to provide mAbs.

Promoters

Numerous promoters that are active in plant cells have been described in the literature. These include promoters present in plant genomes as well as promoters from other sources, including nopaline synthase (NOS) promoter and octopine synthase (OCS) promoters carried on tumor-inducing plasmids of *Agrobacterium tumefaciens,* caulimovirus promoters such as the cauliflower mosaic virus or figwort mosaic virus promoters. For instance, see U.S. Patents No. 5,858,742 and 5,322,938 which disclose versions of the constitutive promoter derived from cauliflower mosaic virus (CaMV35S), US Patent No. 5,378,619 which discloses a Figwort Mosaic Virus (FMV) 35S promoter, U.S. Patent 6,437,217 which discloses a maize RS81 promoter, U.S. Patent 5,641,876 which discloses a rice actin promoter, U.S. Patent 6,426,446 which discloses a maize RS324 promoter, U.S. Patent 6,429,362 which discloses a maize PR-1 promoter, U.S. Patent 6,232,526 which discloses a maize A3 promoter, U.S. Patent 6,177,611 which discloses constitutive maize promoters, U.S. Patent 6,433,252 which discloses a maize L3 oleosin promoter, U.S. Patent 6,429,357 which discloses a rice actin 2 promoter and intron, U.S. Patent 5,837,848 which discloses a root specific promoter, U.S. Patent 6,084,089 which discloses cold inducible promoters, U.S. Patent 6,294,714 which discloses light inducible promoters, U.S. Patent 6,140,078 which discloses salt inducible promoters, U.S. Patent 6,252,138 which discloses pathogen inducible promoters, U.S. Patent 6,175,060 which discloses phosphorus deficiency inducible promoters, U.S. Patent Application Publication 2002/0192813A1 which discloses 5', 3' and intron elements useful in the design of effective plant expression vectors, U.S. patent application Serial No. 09/078,972 which discloses a coixin promoter, U.S. patent application Serial No. 09/757,089 which discloses a maize chloroplast aldolase promoter, and U.S. patent application Serial No. 10/739,565 which discloses water-deficit inducible promoters, all of which are incorporated herein by reference. These and numerous other promoters that function in plant cells are known to those skilled in the art and available for use in recombinant polynucleotides of the present invention to provide for expression of desired genes in transgenic plant cells.

Furthermore, the promoters may be altered to contain multiple "enhancer sequences" to assist in elevating gene expression. Such enhancers are known in the art. By including an enhancer sequence with such constructs, the expression of the selected protein may be enhanced. These enhancers often are found 5' to the start of transcription in a promoter that functions in eukaryotic cells, but can often be inserted in the forward or reverse orientation 5' or 3' to the coding sequence. In some instances, these 5' enhancing elements are introns. Deemed to be particularly useful as enhancers are the 5' introns of the rice actin 1 and rice actin 2 genes. Examples of other enhancers that can be used in accordance with the invention include elements from the CaMV 35S promoter, octopine synthase genes, the maize alcohol dehydrogenase gene, the maize shrunken 1 gene and promoters from non-plant eukaryotes.

In some aspects of the invention it is preferred that the promoter element in the DNA construct be capable of causing sufficient expression to result in the production of an effective amount of a polypeptide in water deficit conditions. Such promoters can be identified and isolated from the regulatory region of plant genes that are over expressed in water deficit conditions. Specific water-deficit-inducible promoters for use in this invention are derived from the 5' regulatory region of genes identified as a heat shock protein 17.5 gene *(HSP17.5),* an HVA22 gene *(HVA22),* a Rab17 gene and a cinnamic acid 4-hydroxylase (CA4H) gene *(CA4H)* of *Zea maize.* Such water-deficit-inducible promoters are disclosed in U.S. application Serial No.10/739,565, incorporated herein by reference.

In other aspects of the invention, sufficient expression in plant seed tissues is desired to effect improvements in seed composition. Exemplary promoters for use for seed composition modification include promoters from seed genes such as napin (U.S. Patent 5,420,034), maize L3 oleosin (U.S. Patent 6,433,252), zein Z27 (Russell et al. (1997) Transgenic Res. 6(2):157-166), globulin 1 (Belanger et al (1991) Genetics 129:863-872), glutelin 1 (Russell (1997) *supra*), and peroxiredoxin antioxidant (Per1) (Stacy et al. (1996) PlantMol Biol. 31(6):1205-1216).

In still other aspects of the invention, preferential expression in plant green tissues is desired. Promoters of interest for such uses include those from genes such as SSU (Fischhoff et al. (1992) Plant Mol Biol. 20:81-93), aldolase and pyruvate orthophosphate dikinase (PPDK) (Taniguchi et al. (2000) Plant Cell Physiol. 41(1):42-48).

Gene overexpression

"Gene overexpression" used herein in reference to a polynucleotide or polypeptide indicates that the expression level of a target protein, in a transgenic plant or in a host cell of the transgenic plant, exceeds levels of expression in a non-transgenic plant. In a preferred embodiment of the present invention, a recombinant DNA construct comprises the polynucleotide of interest in the sense orientation relative to the promoter to achieve gene overexpression, which is identified as such in Table 1.

Gene suppression

Gene suppression includes any of the well-known methods for suppressing transcription of a gene or the accumulation of the mRNA corresponding to that gene thereby preventing translation of the transcript into protein. Posttranscriptional gene suppression is mediated by transcription of integrated recombinant DNA to form double-stranded RNA (dsRNA) having homology to a gene targeted for suppression. This formation of dsRNA most commonly results from transcription of an integrated inverted repeat of the target gene, and is a common feature of gene suppression methods known as anti-sense suppression, co-suppression, RNA interference (RNAi) and knockout, e.g. by mutagenesis. Transcriptional suppression can be mediated by a transcribed dsRNA having homology to a promoter DNA sequence to effect what is called promoter *trans* suppression.

More particularly, posttranscriptional gene suppression by inserting a recombinant DNA construct with anti-sense oriented DNA to regulate gene expression in plant cells is disclosed in U.S. Patent 5,107,065 (Shewmaker et al.) and US Patent 5,759,829 (Shewmaker et al.). Transgenic plants transformed using such anti-sense oriented DNA constructs for gene suppression can comprise integrated DNA arranged as an inverted repeats that result from insertion of the DNA construct into plants by *Agrobacterium*-mediated transformation, as disclosed by Redenbaugh et al. in "Safety Assessment of Genetically Engineered Flavr Savr™ Tomato, CRC Press, Inc. (1992). Inverted repeat insertions can comprises a part or all of the T-DNA construct, e.g. an inverted repeat of a complete transcription unit or an invetred repeat of transcription terminator sequence. Screening for inserted DNA comprising inverted repeat elements can improve the efficiency of identifying transformation events effective for gene silencing whether the transformation construct is a simple anti-sense DNA construct which must be inserted in multiple copies or a complex inverted repeat DNA construct (e.g. an RNAi construct) which can be inserted as a single copy.

Posttranscriptional gene suppression by inserting a recombinant DNA construct with sense-oriented DNA to regulate gene expression in plants is disclosed in U.S. Patent 5,283,184 (Jorgensen et al.) and U.S. Patent 5,231,020 (Jorgensen et al.). Inserted T-DNA providing gene suppression in plants transformed with such sense constructs by *Agrobacterium* is organized predominately in inverted repeat structures, as disclosed by Jorgensen et al., Mol. Gen. Genet., 207:471-477 (1987). See also Stam et al., The Plant Journal,, 12(1), 63-82 (1997) who used segregation studies to support Jorgensen's finding that gene silencing is mediated by multimeric transgene T-DNA loci in which the T-DNAs are arranged in inverted repeats. Screening for inserted DNA comprising inverted repeat elements can improve the gene silencing efficiency when transforming with simple sense-orientated DNA constructs. Gene silencing efficiency can also be improved by screening for single insertion events when transforming with an RNAi construct containing inverted repeat elements

As disclosed by Redenbaugh *et al.* gene suppression can be achieved by inserting into a plant genome recombinant DNA that transcribes dsRNA. Such a DNA insert can be transcribed to an RNA element having the 3' region as a double stranded RNA. RNAi constructs are also disclosed in EP 0426195 A1 (Goldbach et al. - 1991) where recombinant DNA constructs for transcription into hairpin dsRNA for providing transgenic plants with resistance to tobacco spotted wilt virus. Double-stranded RNAs were also disclosed in WO 94/01550 (Agrawal et al.) where anti-sense RNA was stabilized with a self-complementary 3' segment. Agrawal *et al.* referred to U.S. Patent 5,107,065 for using such self-stablized anti-sense RNAs for regulating gene expression in plant cells; see International Publication No. 94/01550. Other double-stranded hairpin-forming elements in transcribed RNA are disclosed in International Publication No. 98/05770 (Werner et al.) where the anti-sense RNA is stabilized by hairpin forming repeats ofpoly(CG) nucleotides. See also U.S. Patent Application Publication No. 2003/0175965 A1 (Lowe et al.) which discloses gene suppression using and RNAi construct comprising a gene coding sequence preceded by inverted repeats of 5'UTR. See also U.S. Patent Application Publication No. 2002/0048814 A1 (Oeller) where RNAi constructs are transcribed to sense or anti-sense RNA which is stabilized by a poly(T)-poly(A) tail. See also U.S. Patent Application Publication No. 2003/0018993 A1 (Gutterson et al.) where sense or anti-sense RNA is stabilized by an inverted repeat of a of the 3' untranslated region of the NOS gene. See also U.S. Patent Application Publication No. 2003/0036197 A1 (Glassman et al.) where RNA having homology to a target is stabilized by two complementary RNA regions.

Gene silencing can also be effected by transcribing RNA from both a sense and an anti-sense oriented DNA, e.g. as disclosed by Shewmaker et al. in U.S. Patent 5,107,065 where in Example 1 a binary vector was prepared with both sense and anti-sense *aroA* genes. See also U.S. Patent 6,326,193 where gene targeted DNA is operably linked to opposing promoters.

Gene silencing can also be affected by transcribing from contiguous sense and anti-sense DNA. In this regard see Sijen et al., The Plant Cell, Vol. 8, 2277-2294 (1996) discloses the use of constructs carrying inverted repeats of a cowpea mosaic virus gene in transgenic plants to mediate virus resistance. Such constructs for posttranscriptional gene suppression in plants by double-stranded RNA are also disclosed in International Publication No. WO 99/53050 (Waterhouse et al.), International Publication No. WO 99/49029 (Graham et al.), U.S. Patent Application No.10/465,800 (Fillatti), U.S. Patent 6,506,559 (Fire et al.). See also U.S. Application Serial No. 10/393,347 (Shewmaker et al.) that discloses constructs and methods for simultaneously expressing one or more recombinant genes while simultaneously suppressing one or more native genes in a transgenic plant. See also U.S. Patent 6,448,473 (Mitsky et al.) that discloses multi-gene suppression vectors for use in plants. All of the above-described patents, applications and international publications disclosing materials and methods for posttranscriptional gene suppression in plants are incorporated herein by reference. Transcriptional suppression such as promoter *trans* suppression can be affected by a expressing a DNA construct comprising a promoter operably linked to inverted repeats of promoter DNA for a target gene. Constructs useful for such gene suppression mediated by promoter *trans* suppression are disclosed by Mette et al., The EMBO Journal, Vol. 18, No. 1, pp. 241-148, 1999 and by Mette et al., The EMBO Journal, Vol. 19, No. 19, pp. 5194-5201-148, 2000, both of which are incorporated herein by reference.

Suppression can also be achieved by insertion mutations created by transposable elements may also prevent gene function. For example, in many dicot plants, transformation with the T-DNA of *Agrobacterium* may be readily achieved and large numbers of transformants can be rapidly obtained. Also, some species have lines with active transposable elements that can efficiently be used for the generation of large numbers of insertion mutations, while some other species lack such options. Mutant plants produced by *Agrobacterium* or transposon mutagenesis and having altered expression of a polypeptide of interest can be identified using the polynucleotides of the present invention. For example, a large population of mutated plants may be screened with polynucleotides encoding the polypeptide of interest to detect mutated plants having an insertion in the gene encoding the polypeptide of interest.

Gene stacking

The present invention also contemplates that the trait-improving recombinant DNA provided herein can be used in combination with other recombinant DNA to create plants with a multiple desired traits. The combinations generated can include multiple copies of any one or more of the recombinant DNA constructs. These stacked combinations can be created by any method, including but not limited to cross breeding of transgenic plants, or multiple genetic transformation.

**Plant Cell Transformation Methods**

Numerous methods for transforming plant cells with recombinant DNA are known in the art and may be used in the present invention. Two commonly used methods for plant transformation are *Agrobacterium*-mediated transformation and microprojectile bombardment. Microprojectile bombardment methods are illustrated in U.S. Patents 5,015,580 (soybean); 5,550,318 (corn); 5,538,880 (corn); 5,914,451 (soybean); 6,160,208 (corn); 6,399,861 (corn) and 6,153,812 (wheat) and *Agrobacterium*-mediated transformation is described in U.S. Patents 5,159,135 (cotton); 5,824,877 (soybean); 5,591,616 (corn); and 6,384,301 (soybean), all of which are incorporated herein by reference. For *Agrobacterium tumefaciens* based plant transformation system, additional elements present on transformation constructs will include T-DNA left and right border sequences to facilitate incorporation of the recombinant polynucleotide into the plant genome.

In general it is useful to introduce recombinant DNA randomly, i.e. at a non-specific location, in the genome of a target plant line. In special cases it may be useful to target recombinant DNA insertion in order to achieve site-specific integration, for example to replace an existing gene in the genome, to use an existing promoter in the plant genome, or to insert a recombinant polynucleotide at a predetermined site known to be active for gene expression. Several site specific recombination systems exist which are known to function implants include cre-lox as disclosed in U.S. Patent 4,959,317 and FLP-FRT as disclosed in U.S. Patent 5,527,695, both incorporated herein by reference.

Transformation methods of this invention are preferably practiced in tissue culture on media and in a controlled environment. "Media" refers to the numerous nutrient mixtures that are used to grow cells *in vitro*, that is, outside of the intact living organism. Recipient cell targets include, but are not limited to, meristem cells, callus, immature embryos and gametic cells such as microspores, pollen, sperm and egg cells. It is contemplated that any cell from which a fertile plant may be regenerated is useful as a recipient cell. Callus may be initiated from tissue sources including, but not limited to, immature embryos, seedling apical meristems, microspores and the like. Cells capable of proliferating as callus are also recipient cells for genetic transformation. Practical transformation methods and materials for making transgenic plants of this invention, for example various media and recipient target cells, transformation of immature embryo cells and subsequent regeneration of fertile transgenic plants are disclosed in U.S. Patents 6,194,636 and 6,232,526, which are incorporated herein by reference.

The seeds of transgenic plants can be harvested from fertile transgenic plants and be used to grow progeny generations of transformed plants of this invention including hybrid plants line for selection of plants having an enhanced trait. In addition to direct transformation of a plant with a recombinant DNA, transgenic plants can be prepared by crossing a first plant having a recombinant DNA with a second plant lacking the DNA. For example, recombinant DNA can be introduced into first plant line that is amenable to transformation to produce a transgenic plant which can be crossed with a second plant line to introgress the recombinant DNA into the second plant line. A transgenic plant with recombinant DNA providing an enhanced trait, e.g. enhanced yield, can be crossed with transgenic plant line having other recombinant DNA that confers another trait, for example herbicide resistance or pest resistance, to produce progeny plants having recombinant DNA that confers both traits. Typically, in such breeding for combining traits the transgenic plant donating the additional trait is a male line and the transgenic plant carrying the base traits is the female line. The progeny of this cross will segregate such that some of the plants will carry the DNA for both parental traits and some will carry DNA for one parental trait; such plants can be identified by markers associated with parental recombinant DNA, e.g. marker identification by analysis for recombinant DNA or, in the case where a selectable marker is linked to the recombinant, by application of the selecting agent such as a herbicide for use with a herbicide tolerance marker, or by selection for the enhanced trait. Progeny plants carrying DNA for both parental traits can be crossed back into the female parent line multiple times, for example usually 6 to 8 generations, to produce a progeny plant with substantially the same genotype as one original transgenic parental line but for the recombinant DNA of the other transgenic parental line

In the practice of transformation DNA is typically introduced into only a small percentage of target plant cells in any one transformation experiment. Marker genes are used to provide an efficient system for identification of those cells that are stably transformed by receiving and integrating a transgenic DNA construct into their genomes. Preferred marker genes provide selective markers which confer resistance to a selective agent, such as an antibiotic or herbicide. Any of the herbicides to which plants of this invention may be resistant are useful agents for selective markers. Potentially transformed cells are exposed to the selective agent. In the population of surviving cells will be those cells where, generally, the resistance-conferring gene is integrated and expressed at sufficient levels to permit cell survival. Cells may be tested further to confirm stable integration of the exogenous DNA. Commonly used selective marker genes include those conferring resistance to antibiotics such as kanamycin and paromomycin (*nptII*), hygromycin B (*aph IV*) and gentamycin (*aac3* and *aac*C4) or resistance to herbicides such as glufosinate (*bar* or *pat)* and glyphosate (*aroA* or EPSPS). Examples of such selectable are illustrated in U.S. Patents 5,550,318; 5,633,435; 5,780,708 and 6,118,047, all of which are incorporated herein by reference. Selectable markers which provide an ability to visually identify transformants can also be employed, for example, a gene expressing a colored or fluorescent protein such as a luciferase or green fluorescent protein (GFP) or a gene expressing a *beta*-glucuronidase or *uid*A gene (GUS) for which various chromogenic substrates are known.

Plant cells that survive exposure to the selective agent, or plant cells that have been scored positive in a screening assay, may be cultured in regeneration media and allowed to mature into plants. Developing plantlets regenerated from transformed plant cells can be transferred to plant growth mix, and hardened off, for example, in an environmentally controlled chamber at about 85% relative humidity, 600 ppm $CO_2$, and 25-250 microeinsteins $m^{-2}$ $s^{-1}$ of light, prior to transfer to a greenhouse or growth chamber for maturation. Plants are regenerated from about 6 weeks to 10 months after a transformant is identified, depending on the initial tissue. Plants may be pollinated using conventional plant breeding methods known to those of skill in the art and seed produced, for example self-pollination is commonly used with transgenic corn. The regenerated transformed plant or its progeny seed or plants can be tested for expression of the recombinant DNA and selected for the presence of enhanced agronomic trait.

## Transgenic Plants and Seeds

Transgenic plants derived from the plant cells of this invention are grown to generate transgenic plants having an enhanced trait as compared to a control plant and produce transgenic seed and haploid pollen of this invention. Such plants with enhanced traits are identified by selection of transformed plants or progeny seed for the enhanced trait. For efficiency a selection method is designed to evaluate multiple transgenic plants (events) comprising the recombinant DNA , for example multiple plants from 2 to 20 or more transgenic events. Transgenic plants grown from transgenic seed provided herein demonstrate improved agronomic traits that contribute to increased yield or other trait that provides increased plant value, including, for example, improved seed quality. Of particular interest are plants having enhanced water use efficiency, enhanced cold tolerance, increased yield, enhanced nitrogen use efficiency, enhanced seed protein and enhanced seed oil.

### Discovery of Trait-improving Recombinant DNA

To identify recombinant DNA that imparts an enhanced trait to plants, *Arabidopsis* ce;;s were transformed with a candidate recombinant DNA construct and screened for an improved trait. A two-step screening process was employed which comprised two passes of trait characterization to ensure that the trait modification was dependent on expression of the recombinant DNA, but not due to the chromosomal location of the integration of the transgene. Twelve independent transgenic lines for each recombinant DNA construct were established and assayed for the transgene expression levels. Five transgenic lines with high transgene expression levels were used in the first pass screen to evaluate the transgene's function in T2 transgenic plants. Subsequently, three transgenic events, which had been shown to have one or more improved traits, were further evaluated in the second pass screen to confirm the transgene's ability to impart an improved trait. The following Table 3 summarizes the improved traits that have been confirmed as provided by a recombinant DNA construct.

In particular, Table3 reports "PEP SEQ ID" which is the amino acid sequence of the protein cognate to the DNA in the recombinant DNA construct corresponding to a protein sequence of a SEQ ID NO. in the Sequence Listing. "annotation" refers to a description of the top hit protein obtained from an amino acid sequence query of each PEP SEQ ID NO to GenBank database of the National Center for Biotechnology Information (ncbi). More particularly, "gi" is the GenBank ID number for the top BLAST hit. The components of "annotation" are "e-value" which provides the expectation value for the BLAST hit; "% id" which refers to the percentage of identically matched amino acid residues along the length of the portion of the sequences which is aligned by BLAST between the sequence of interest provided herein and the hit sequence in GenBank; "GenBank ID" which provides a reference number for the top BLAST hit in GenBank; and " description" which refers to the description of that top BLAST hit.

"traits" identify by two letter codes the confirmed improvement in a transgenic plant provided by the recombinant DNA. The codes for improved traits are:

"CK" which indicates cold tolerance improvement identified under a cold shock tolerance screen;
"CS" which indicates cold tolerance improvement identified by a cold germination tolerance screen;
"DS" which indicates drought tolerance improvement identified by a soil drought stress tolerance screen;
"PEG" which indicates osmotic stress tolerance improvement identified by a PEG induced osmotic stress tolerance screen;
"HS" which indicates heat stress tolerance improvement identified by a heat stress tolerance screen;
"SS" which indicates high salinity stress tolerance improvement identified by a salt stress tolerance screen;
"LN" which indicates nitrogen use efficiency improvement identified by a limited nitrogen tolerance screen;
"LL" which indicates attenuated shade avoidance response identified by a shade tolerance screen under a low light condition;
"PP" which indicates improved growth and development at early stages identified by an early plant growth and development screen;
"SP" which indicates improved growth and development at late stages identified by a late plant growth and development screen provided herein.

Table 3

| PEP SEQ ID | annotation | | | | Traits | | | | | |
|---|---|---|---|---|---|---|---|---|---|---|
| | e-value | % id | GenBank ID | description | | | | | | |
| 205 | 5.00E-34 | 85 | gi\|21281159\| | gb\|AAD48981.1\| contains similarity to Solanum lycopersicum (tomato) wound induced protein | HS | | | | | |
| 206 | 1.00E-66 | 76 | gi\|21553397\| | gb\|AAM62490.1\|putative zinc finger protein | LN | | | | | |
| 207 | 1.00E-118 | 93 | gi\|7573441\| | ref\|NP_191871.1\| cyclin family protein | HS | | | | | |
| 208 | 0 | 93 | gi\|22136838\| | ref\|NP_566299.1\| GPI transamidase component Gpi16 subunit family protein | CK | CS | | | | |
| 209 | 1.00E-171 | 97 | gi\|15028251\| | ref\|NP_566244.1\| transmembrane protein, putative | CK | | | | | |
| 210 | 2.00E-57 | 98 | gi\|37202020\| | emb\|CAB81279.1\| putative protein | HS | PP | PEG | | | |
| 211 | 8.00E-37 | 100 | gi\|26451972\| | dbj\|BAC43077.1\|unknown protein | PP | SS | HS | | | |
| 212 | 0 | 86 | gi\|42562765\| | ref\|NP_175971.3\|transcription factor-related | CK | | | | | |
| 213 | 0 | 100 | gi\|21280989\| | gb\|AAM44902.1\|putative catalase | PP | SP | | | | |
| 214 | 0 | 89 | gi\|22087278\| | gb\|AAC97995.1\| Similar to gb\|Z30094 basic transcripion factor 2, 44 kD subunit from Homo sapiens. | LN | PP | HS | | | |
| 215 | 0 | 77 | gi\|12324313\| | gb\|AAD55662.1\| Highly similar to non intermediate filament IFA binding protein | DS | | | | | |
| 216 | 0 | 100 | gi\|21537362\| | gb\|AAM61703.1\|protein kinase-like protein | CK | | | | | |
| 217 | 0 | 87 | gi\|4678332\| | emb\|CAB41143.1\|putative peptide transporter | CK | | | | | |
| 218 | 1.00E-144 | 86 | gi\|14532890\| | gb\|AAK64127.1\|unknown protein | PEG | | | | | |
| 219 | 1.00E-145 | 95 | gi\|3242721\| | gb\|AAC23773.1\|putative acetone-cyanohydrin lyase | DS | | | | | |
| 220 | 0 | 84 | gi\|15293165\| | gb\|AAK93693.1\|putative 3-methyladenine DNA glycosylase | CK | PEG | CS | HS | | PP |
| 221 | 1.00E-178 | 79 | gi\|6321581\| | ref\|NP_011658.1\|Btn2p [Saccharomyces cerevisiae] | PP | | | | | |
| 222 | 0 | 98 | gi\|30387605\| | ref\|NP_178499.2\| MATE efflux family protein | LN | | | | | |
| 223 | 1.00E-147 | 90 | gi\|9758099\| | ref\|NP_198887.1\| zinc finger (C2H2 type) family protein | CS | | | | | |
| 224 | 1.00E-139 | 95 | gi\|23505833\| | gb\|AAN28776.1\|At3g51780/O RF3 | CK | | | | | |

| PEP SEQ ID | annotation | | | | Traits | | | | | |
|---|---|---|---|---|---|---|---|---|---|---|
| | e-value | % id | GenBank ID | description | | | | | | |
| 225 | 0 | 100 | gi|6729028| | gb|AAF27024.1|putative nodulin [Arabidopsis thaliana] ref|NP_187169.1| GDSL-motif lipase/hydrolase family protein | CS | | | | |
| 226 | 0 | 100 | gi|21436143| | gb|AAM51318.1|unknown protein | CS | | | | |
| 227 | 0 | 97 | gi|31711910| | gb|AAM64944.1| betaine aldehyde dehydrogenase, putative | PP | | | | |
| 228 | 1.00E-128 | 80 | gi|21554268| | ref|NP_171616.1| 33 kDa ribonucleoprotein, chloroplast, putative / RNA-binding protein cp33, putative | PEG | SS | | | |
| 229 | 0 | 95 | gi|6321563| | emb|CAA58159.1| glutamic-dependent asparagine synthase | CK | CS | SS | | |
| 230 | 1.00E-132 | 57 | gi|25403213| | pir||A86468probable zinc finger protein | PP | PEG | | | |
| 231 | 2.00E-54 | 52 | gi|20127115| | gb|AAM10965.1|putative bHLH transcription factor [Arabidopsis thaliana] | CK | | | | |
| 232 | 1.00E-160 | 72 | gi|18401370| | ref|NP_566566.1|protein phosphatase 2C family protein | CK | HS | PEG | CS | |
| 233 | 1.00E-33 | 45 | gi|21593875| | gb|AAM65842.1|putative RING-H2 zinc finger protein | PP | HS | | | |
| 234 | 2.00E-34 | 51 | gi|55419648| | gb|AAV51937.1|AP2/EREBP transcription factor ERF-2 | CK | | | | |
| 235 | 2.00E-73 | 59 | gi|3955021| | emb|CAA09367.1|HB2 homeodomain protein | CK | CS | | | |
| 236 | 1.00E-51 | 58 | gi|4689366| | gb|AAD27870.1|BRH1 RING finger protein | HS | PP | PEG | | |
| 237 | 1.00E-149 | 76 | gi|52354283| | gb|AAM14913.1| putative malonyl-CoA:Acyl carrier protein transacylase | HS | | | | |
| 238 | 1.00E-154 | 85 | gi|20260680| | gb|AAM13238.1|putative NADPH-dependent mannose 6-phosphate reductase | PP | PEG | | | |
| 239 | 2.00E-89 | 64 | gi|21593394| | ref|NP_567300.1| short-chain dehydrogenase/ reductase (SDR) family protein | HS | | | | |
| 240 | 0 | 82 | gi|42795470| | gb|AAS46245.1|HMG-CoA synthase 2 | CK | HS | SS | | |
| 241 | 1.00E-113 | 83 | gi|20385588| | gb|AAM21344.1|MADS-box protein 4 | SP | | | | |
| 242 | 1.00E-135 | 89 | gi|21280889| | ref|NP_196254.1| ribosomal protein S8e family protein | SS | HS | | | |
| 243 | 5.00E-28 | 43 | gi|4567308| | gb|AAD23719.1|putative RING zinc finger protein | PEG | | | | |
| 244 | 3.00E-35 | 33 | gi|25352200| | pir||T52379zinc finger protein ZPT3-3 | PEG | | | | |
| 245 | 0 | 83 | gi|10800918| | emb|CAC12995.1|putative AUX1-like permease | CK | PEG | CS | | |

EP 3 059 306 A1

| PEP SEQ ID | annotation | | | | Traits | | | | | |
|---|---|---|---|---|---|---|---|---|---|---|
| | e-value | % id | GenBank ID | description | | | | | | |
| 246 | 3.00E-61 | 87 | gi\|21554008\| | gb\|AAM63089.1\|cold-regulated protein cor15b precursor | CS | HS | PP | | | |
| 247 | 0 | 100 | gi\|25406415\| | pir\|\|D96781cytochrome P450, probable, 64213-66051 | PEG | | | | | |
| 248 | 0 | 92 | gi\|3885330\| | gb\|AAC77858.1\|putative cytochrome P450 | PP | | | | | |
| 249 | 1.00E-159 | 100 | gi\|21593400\| | gb\|AAM65367.1\|phi-1-like protein | LN | LL | | | | |
| 250 | 1.00E-89 | 76 | gi\|45825151\| | ref\|NP_200258.2\| zinc finger (B-box type) family protein | DS | | | | | |
| 251 | 1.00E-144 | 72 | gi\|3643085\| | gb\|AAC36698.1\|protein phosphatase-2C; PP2C | PP | CK | | | | |
| 252 | 2.00E-90 | 91 | gi\|21537084\| | gb\|AAM61425.1\|unknown | CK | PEG | | | | |
| 253 | 2.00E-51 | 100 | gi\|28393947\| | ref\|NP_174092.1\| glycine-rich protein | HS | PP | | | | |
| 254 | 0 | 91 | gi\|23197704\| | ref\|NP_565909.1\| radical SAM domain-containing protein | PEG | | | | | |
| 255 | 0 | 93 | gi\|4902476\| | emb\|CAB43520.1\|MAP kinase | CK | CS | | | | |
| 256 | 1.00E-100 | 72 | gi\|34146806\| | gb\|AAC34217.1\| putative alcohol dehydrogenase | CS | | | | | |
| 257 | 2.00E-87 | 82 | gi\|3548814\| | gb\|AAC34486.1\|E3 ubiquitin ligase SCF complex subunit SKP1/ASK1 (At19), | CK | LL | CS | | | |
| 258 | 1.00E-113 | 81 | gi\|7939553\| | dbj\|BAA95756.1\|expansin-like protein | PP | HS | | | | |
| 259 | 1.00E-163 | 52 | gi\|13506810\| | gb\|AAK28345.1\|receptor-like protein kinase 3 | CK | PP | | | | |
| 260 | 1.00E-163 | 68 | gi\|12597803\| | gb\|AAG60115.1\|hypothetical protein | PP | | | | | |
| 261 | 1.00E-161 | 100 | gi\|20258881\| | gb\|AAM14112.1\|putative ubiquinone/menaquinone biosynthesis methyltransferase | LL | | | | | |
| 262 | 1.00E-133 | 92 | gi\|12597757\| | ref\|NP_176849.1\| nodulin MtN3 family protein | SS | PEG | | | | |
| 263 | 0 | 95 | gi\|6911875\| | sp\|P53780\|METC_ARATH Cystathionine beta-lyase, chloroplast precursor (CBL) (Beta-cystathionase) (Cysteine lyase) | PP | SS | | | | |
| 264 | 1.00E-100 | 100 | gi\|21389647\| | gb\|AAM48022.1 \|photoassimila te-responsive protein PAR-1 b-like protein | SP | DS | | | | |
| 265 | 0 | 100 | gi\|25402889\| | ref\|NP_173376.1\| very-long-chain fatty acid condensing enzyme, putative | PEG | PP | | | | |

| PEP SEQ ID | annotation | | | | Traits | | | | | |
|---|---|---|---|---|---|---|---|---|---|---|
| | e-value | % id | GenBank ID | description | | | | | | |
| 266 | 0 | 88 | gi\|6324999\| | sp\|P20438\|CG12_YEAST G1/S-specific cyclin CLN2 gb\|AAA65725.1\| cyclin 2 | DS | | | | | |
| 267 | 0 | 100 | gi\|3218550\| | dbj\|BAA28775.1\|Cdk-activating kinase 1At | LL | | | | | |
| 268 | 1.00E-169 | 100 | gi\|6321880\| | ref\|NP_011956.1\|Nucleolar protein involved in the assembly of the large ribosomal subunit; contains a sigma(70)-like motif, which is thought to bind RNA | SP | | | | | |
| 269 | 0 | 89 | gi\|21436315\| | gb\|AAM51327.1\|putative histidyl-tRNA synthetase | CK | HS | CS | | | |
| 270 | 1.00E-150 | 100 | gi\|14318575\| | ref\|NP_116708.1\|20S proteasome beta-type subunit | PP | PEG | SS | | | |
| 271 | 0 | 99 | gi\|6325396\| | pir\|\|S69027 ammonium transport protein MEP3 | LL | DS | | | | |
| 272 | 5.00E-76 | 72 | gi\|7442240\| | sp\|O24543\|AX2E_PHAAU Auxin-induced protein 22E (Indole-3-acetic acid induced protein ARG14) | DS | | | | | |
| 273 | 0 | 94 | gi\|9759247\| | dbj\|BAB09771.1\|serine/threoni ne protein kinase-like protein | LL | | | | | |
| 274 | 0 | 97 | gi\|39647867\| | emb\|CAE26387.1\|phosphogly cerate kinase | CK | DS | CS | | | |
| 275 | 0 | 89 | gi\|9758468\| | dbj\|BAB08997.1\|monosacchar ide transporter | PP | PEG | | | | |
| 276 | 1.00E-66 | 80 | gi\|6319966\| | ref\|NP_010046.1\|Phosphorela y intermediate protein, phosphorylated by the plasma membrane sensor Sln1p in response to osmotic stress and then in turn phosphorylates the response regulators Ssk1p in the cytosol and Skn7p in the nucleus | HS | | | | | |
| 277 | 2.00E-88 | 83 | gi\|21536637\| | ref\|NP_565524.1\| stress enhanced protein 2 (SEP2) | DS | LN | | | | |
| 278 | 0 | 90 | gi\|6322724\| | ref\|NP_012797.1\|Required for transcription of rDNA by RNA Polymerase I; DNA-independent RNA Polymerase I transcription factor | SS | | | | | |
| 279 | 0 | 94 | gi\|6320705\| | pir\|\|S69555 myo-inositol transport protein ITR1 - yeast) | HS | SS | PP | | | |
| 280 | 5.00E-24 | 49 | gi\|25453551\| | pir\|\|T52011 ethylene responsive element binding factor 3 | LL | | | | | |
| 281 | 1.00E-150 | 100 | gi\|4580468\| | gb\|AAD24392.1\|putative cAMP-dependent protein kinase | PP | | | | | |
| 282 | 1.00E-169 | 91 | gi\|21436057\| | ref\|NP_193037.1\| oxidoreductase, zinc-binding dehydrogenase family protein | LL | SS | | | | |

| PEP SEQ ID | annotation | | | | Traits | | | | | |
|---|---|---|---|---|---|---|---|---|---|---|
| | e-value | % id | GenBank ID | description | | | | | | |
| 283 | 1.00E-165 | 83 | gi\|21280925\| | gb\|AAM44967.1\|putative cinnamyl alcohol dehydrogenase | HS | | | | | |
| 284 | 0 | 100 | gi\|20334800\| | ref\|NP_568453.1\| alcohol dehydrogenase, putative [Arabidopsis thaliana] | PEG | | | | | |
| 285 | 0 | 100 | gi\|22136298\| | gb\|AAM91227.1\|alcohol dehydrogenase | PP | | | | | |
| 286 | 1.00E-144 | 89 | gi\|20259173\| | sp\|004202\|IF35_ARATH Eukaryotic translation initiation factor 3 subunit 5 (eIF-3 epsilon) (eIF3 p32 subunit) (eIF3f) | CS | SS | CK | | | |
| 287 | 6.00E-74 | 96 | gi\|21593170\| | ref\|NP_196239.1\| RNA-binding protein, putative | PP | | | | | |
| 288 | 1.00E-112 | 80 | gi\|9759521\| | dbj\|BAB10987.1\|nuclear cap-binding protein; CBP20 | PP | | | | | |
| 289 | 1.00E-177 | 95 | gi\|12083276\| | gb\|AAG48797.1\|putative delta 9 desaturase | CS | PEG | | | | |
| 290 | 1.00E-111 | 94 | gi\|12083264\| | gb\|AAG48791.1\|putative GTP-binding protein RAB11D | LN | SS | PP | | | |
| 291 | 1.00E-134 | 91 | gi\|23505935\| | gb\|AAP86673.1\| 26S proteasome subunit RPN12 | HS | SS | | | | |
| 292 | 3.00E-53 | 86 | gi\|26452894\| | dbj\|BAC43525.1\|putative DNA-directed RNA polymerase 14 kDa subunit AtRPAC14 | CK | HS | PP | SS | PEG | CS |
| 293 | 5.00E-94 | 100 | gi\|21554412\| | gb\|AAM63517.1\|probable glutathione peroxidase At2g31570 | CK | CS | PEG | | PP | |
| 294 | 1.00E-116 | 92 | gi\|6143884\| | ref\|NP_187617.1\| immunophilin, putative / FKBP-type peptidyl-prolyl cis-trans isomerase, putative | SP | CK | | | | |
| 295 | 1.00E-114 | 100 | gi\|6671929\| | gb\|AAF23189.1\|putative GTP-binding protein (ATFP8) [Arabidopsis thaliana] | PP | | | | | |
| 296 | 1.00E-161 | 92 | gi\|7670024\| | ref\|NP_566563.1\| ubiquitin-conjugating enzyme, putative | HS | PP | SS | | | |
| 297 | 1.00E-132 | 95 | gi\|21554045\| | gb\|AAM63126.1\|20S proteasome subunit PAC1 | SS | | | | | |
| 298 | 1.00E-111 | 94 | gi\|21593047\| | gb\|AAM64996.1\|GTP-binding protein Rab11 | PP | | | | | |
| 299 | 0 | 93 | gi\|23308437\| | ref\|NP_190336.1\| malate dehydrogenase [NAD], chloroplast (MDH) | SS | HS | | | | |
| 300 | 3.00E-94 | 80 | gi\|6562282\| | emb\|CAB62652.1\|rac-like GTP binding protein Arac11 | PEG | | | | | |
| 301 | 0 | 100 | gi\|21554607\| | gb\|AAM63631.1\|ubiquitin activating enzyme-like protein | SS | | | | | |
| 302 | 5.00E-26 | 81 | gi\|15217910\| | ref\|NP_173453.1\|homeobox-leucine zipper protein-related | PP | | | | | |

(continued)

| PEP SEQ ID | annotation | | | | Traits | | | | | |
|---|---|---|---|---|---|---|---|---|---|---|
| | e-value | % id | GenBank ID | description | | | | | | |
| 303 | 1.00E-129 | 86 | gi\|23505995\| | ref\|NP_177122.2\| acid phosphatase, putative [Arabidopsis thaliana] | SP | PP | | | | |
| 304 | 1.00E-157 | 100 | gi\|28827628\| | gb\|AA050658.1\|putative C-4 sterol methyloxidase | PP | | | | | |
| 305 | 9.00E-19 | 100 | gi\|21592539\| | ref\|NP_565794.1\| hydroxyproline-rich glycoprotein family protein [Arabidopsis thaliana] | CS | SS | CK | | | |
| 306 | 0 | 97 | gi\|15965196\| | ref\|NP_385549.1\|PROBABLE ENOLASE PROTEIN | SP | LL | | | | |
| 307 | 1.00E-153 | 100 | gi\|16080137\| | pir\|\|A69990 UTP-glucose-1-phosphate uridylyltransferase homolog ytdA | LL | | | | | |
| 308 | 1.00E-147 | 98 | gi\|26246388\| | ref\|NP_752427.1\|Pyrroline-5-carboxylate reductase | LN | | | | | |
| 309 | 1.00E-135 | 94 | gi\|23126946\| | ref\|ZP_00108826.1\|COG0345 : Pyrroline-5-carboxylate reductase | PP | | | | | |
| 310 | 0 | 100 | gi\|16263079\| | ref\|NP_435872.1\|probable alcohol | PEG | | | | | |
| 311 | 0 | 99 | gi\|15888903\| | pir\|\|H97551 probable aminotransferase aatc | LL | | | | | |
| 312 | 0 | 90 | gi\|1608058\| | ref\|NP_390984.1\|glycine betaine aldehyde dehydrogenase | CS | CK | | | | |
| 313 | 1.00E-141 | 94 | gi\|15614866\| | ref\|NP_243169.1\|UTP-glucose-1-phosphate uridylyltransferas | PP | | | | | |
| 314 | 0 | 99 | gi\|23111329\| | ref\|ZP_00097007.1\|COG0205 : 6-phosphofructokinase | CS | PP | CK | | | |
| 315 | 1.00E-150 | 87 | gi\|37526393\| | ref\|NP_929737.1\|UTP-glucose-1-phosphate uridylyltransferase (UDP-glucose pyrophosphorylase) (UDPGP) | PEG | SS | | | | |
| 316 | 0 | 99 | gi\|16128895\| | ref\|NP_415448.1\|aspartate aminotransferase | PP | | | | | |
| 317 | 0 | 97 | gi\|16080149\| | ref\|NP_390975.1\|glucose-1-phosphate adenylyltransferase | DS | | | | | |
| 318 | 0 | 96 | gi\|48732455\| | ref\|ZP_00266198.1\|COG1012 : NAD-dependent aldehyde dehydrogenases | HS | PP | | | | |
| 319 | 0 | 97 | gi\|16129263\| | ref\|NP_415818.1\|4-aminobutyrate aminotransferase | SS | PP | | | | |
| 320 | 0 | 99 | gi\|16128583\| | ref\|NP_415133.1\|putative PLP-dependent aminotransferase | PP | | | | | |
| 321 | 0 | 99 | gi\|30063716\| | ref\|NP_837887.1\|putative aminotransferase | CS | CK | | | | |
| 322 | 0 | 99 | gi\|16130084\| | ref\|NP_416651.1\|bifunctional: putative glutamate synthase (N-terminal); putative oxidoreductase (C-terminal) | SS | PP | | | | |
| 323 | 0 | 94 | gi\|16128664\| | ref\|NP_415214.1\|phosphogluc omutase | SP | PP | | | | |

| PEP SEQ ID | annotation | | | | Traits | | | | | |
|---|---|---|---|---|---|---|---|---|---|---|
| | e-value | % id | GenBank ID | description | | | | | | |
| 324 | 0 | 96 | gi\|49176307\| | ref\|NP_417544.3\|probable ornithine aminotransferase [Escherichia coli K12] (EC 2.6.1.13) | CS | CK | | | | |
| 325 | 1.00E-175 | 80 | gi\|48729503\| | ref\|ZP_00263253.1\|COG0508 : Pyruvate/2-oxoglutarate dehydrogenase complex, dihydrolipoamide acyltransferase (E2) component, and related enzymes | CK | PP | | | | |
| 326 | 1.00E-173 | 80 | gi\|28869402\| | ref\|NP_792021.1\|2-oxoglutarate dehydrogenase, E2 component, dihydrolipoamide succinyltransferase | CK | PP | | | | |
| 327 | 1.00E-161 | 90 | gi\|37524574\| | ref\|NP_927918.1\|Transaldolas eB | PP | | | | | |
| 328 | 0 | 82 | gi\|37525385\| | ref\|NP_928729.1\|Dihydrolipoa mide succinyltransferase component of 2-oxoglutarate dehydrogenase complex (E2) | LN | PP | | | | |
| 329 | 3.00E-55 | 86 | gi\|16332334\| | ref\|NP_443062.1\|hypothetical protein slr0607 | CS | PP | HS | | | |
| 330 | 0 | 100 | gi\|15614388\| | ref\|NP_242691.1\|acetoin dehydrogenase E3 component | LL | | | | | |
| 331 | 0 | 99 | gi\|15615327\| | ref\|NP_243630.1\|dihydrolipoa mide dehydrogenase | LN | | | | | |
| 332 | 0 | 97 | gi\|16078525\| | ref\|NP_389344.1\|dihydrolipoa mide dehydrogenase E3 subunit of both pyruvate dehydrogenase and 2-oxoglutarate dehydrogenase complexes | HS | DS | | | | |
| 333 | 0 | 94 | gi\|15800431\| | ref\|NP_286443.1\|2-oxoglutarate dehydrogenase | CS | CK | | | | |
| 334 | 0 | 89 | gi\|22136798\| | gb\|AAM91743.1\|putative phosphate/phosphoenolpyruv ate translocator precursor protein | LN | | | | | |
| 335 | 0 | 100 | gi\|49176098\| | ref\|NP_415825.3\|putative polysaccharide hydrolase | HS | SS | | | | |
| 336 | 0 | 96 | gi\|15889444\| | ref\|NP_355125.1\|AGR_C_392 7p [Agrobacterium tumefaciens str. C58] ref\|NP_532838.1\| bacteriophytochrome protein | CK | HS | PP | PEG | CS | SS |
| 337 | 0 | 95 | gi\|15613173\| | ref\|NP_241476.1\|sulfite reductase (NADPH) | PP | CK | CS | PEG | | |
| 338 | 0 | 100 | gi\|30062749\| | ref\|NP_836920.1\|nitrate reductase 1, beta subunit | LN | | | | | |
| 339 | 0 | 100 | gi\|15804618\| | ref\|NP_290659.1\|glucosephos phate isomerase | PP | CS | | | | |
| 340 | 0 | 100 | gi\|23125493\| | ref\|ZP_00107424.1\|COG0243 : Anaerobic dehydrogenases, typically selenocysteine-containing | PP | PEG | HS | | | |

| PEP SEQ ID | annotation | | | | Traits | | | | | |
|---|---|---|---|---|---|---|---|---|---|---|
| | e-value | % id | GenBank ID | description | | | | | | |
| 341 | 0 | 98 | gi\|28868179\| | ref\|NP_790798.1\|glucose-6-phosphate isomerase | CK | PP | SP | PEG | CS | |
| 342 | 0 | 96 | gi\|16329427\| | ref\|NP_440155.1\|isocitrate dehydrogenase (NADP+) | CK | CS | | | | |
| 343 | 0 | 85 | gi\|37524705\| | ref\|NP_928049.1\|sulfite reductase [NADPH] hemoprotein beta-component (SIR-HP) | DS | PP | PEG | | | |
| 344 | 1.00E-121 | 95 | gi\|6728966\| | gb\|AAF26964.1\|unknown protein | LN | | | | | |
| 345 | 0 | 96 | gi\|6714417\| | gb\|AAF26105.1\|unknown protein | LN | | | | | |
| 346 | 0 | 78 | gi\|22136866\| | ref\|NP_177343.2\| protease-associated zinc finger (C3HC4-type RING finger) family protein | CS | | | | | |
| 347 | 0 | 92 | gi\|51971567\| | ref\|NP_850943.1\| glutamine amidotransferase-related | CK | HS | CS | | | |
| 348 | 2.00E-65 | 88 | gi\|26450572\| | dbj\|BAC42398.1\|unknown protein [Arabidopsis thaliana] | CK | CS | | | | |
| 349 | 1.00E-115 | 100 | gi\|6730712\| | gb\|AAF27107.1\|Unknown protein | CK | CS | | | | |
| 350 | 0 | 100 | gi\|20465757\| | gb\|AAM20367.1\|putative cyclin protein | PP | | | | | |
| 351 | 8.00E-90 | 56 | gi\|23297314\| | ref\|NP_849559.1\| WRKY family transcription factor [Arabidopsis thaliana] | CK | CS | | | | |
| 352 | 1.00E-171 | 83 | gi\|50940357\| | ref\|XP_479706.1\|putative Shwachman-Bodian-Diamond syndrome protein | PP | HS | SS | | | |
| 353 | 2.00E-46 | 92 | gi\|50929801\| | ref\|XP_474428.1\|OSJNBa007 0M12.6 | CK | PP | | | | |
| 354 | 7.00E-54 | 92 | gi\|5042333\| | emb\|CAB44664.1\|BETL4 protein | PP | | | | | |
| 355 | 1.00E-56 | 34 | gi\|42563228\| | ref\|NP_565108.2\|zinc finger (CCCH-type) family protein | HS | SS | PEG | | | |
| 356 | 4.00E-61 | 72 | gi\|51970440\| | dbj\|BAD43912.1\|hypothetical protein | CK | PP | | | | |
| 357 | 7.00E-91 | 99 | gi\|16331395\| | ref\|NP_442123.1\|hypothetical protein slr0013 | SS | | | | | |
| 358 | 0 | 99 | gi\|21229841\| | ref\|NP_635758.1\|vanillate O-demethylase oxygenase subunit | CS | CK | | | | |
| 359 | 1.00E-142 | 100 | gi\|16331855\| | sp\|Q55891\|PCYA_SYNY3 Phycocyanobilin:ferredoxin oxidoreductase | CK | PP | CS | | | |
| 360 | 0 | 100 | gi\|16331872\| | ref\|NP_442600.1\|hypothetical protein slr0304 | SP | CK | | | | |

(continued)

| PEP SEQ ID | annotation | | | | Traits | | | | | |
|---|---|---|---|---|---|---|---|---|---|---|
| | e-value | % id | GenBank ID | description | | | | | | |
| 361 | 3.00E-86 | 87 | gi\|21593344\| | gb\|AAM65293.1\|putative cold-regulated protein ref\|NP_178469.1\| late embryogenesis abundant domain-containing protein / LEA domain-containing protein | LN | | | | | |
| 362 | 8.00E-91 | 100 | gi\|16330328\| | sp\|P73690\|Y51L_SYNY3 Ycf51-like protein dbj\|BAA17736.1\| ORF_ID:sll1702~hypothetical protein | HS | | | | | |
| 363 | 5.00E-87 | 100 | gi\|15612647\| | ref\|NP_240950.1\|hypoxanthin e-guanine phosphoribosyltransferase | PEG | | SS | | | |
| 364 | 0 | 83 | gi\|6323679\| | sp\|P23748\|MPIP_YEAST M-phase inducer phosphatase (Mitosis initiation protein MIH1) (Mitotic inducer homolog) | CS | LL | PP | CK | HS | SS |
| 365 | 2.00E-33 | 90 | gi\|34898476\| | ref\|NP_910584.1\|EST AU082567(S21715) corresponds to a region of the predicted gene.~Similar to S.tuberosum ubiquinol cytochrome c reductase. (X79275) | PP | | | | | |
| 366 | 6.00E-54 | 81 | gi\|21592528\| | gb\|AAM64477.1\|ring-box protein-like | HS | SS | | | | |
| 367 | 0 | 76 | gi\|34910110\| | dbj\|BAB92553.1\| DNA cross-link repair 1B-like protein | CK | HS | | | | |
| 368 | 1.00E-156 | 100 | gi\|21436267\| | gb\|AAM51272.1\|putative nodulin-26 protein | LN | | | | | |
| 369 | 1.00E-100 | 81 | gi\|50900588\| | ref\|XP_462727.1\|putative phenylalkylamine binding protein sp\|Q9FTZ2\|EBP_ORYSA Probable 3-beta-hydroxysteroid-delta(8),delta(7)-isomerase (Cholestenol delta-isomerase) (Delta8-delta7 sterol isomerase) (D8-D7 sterol isomerase) dbj\|BAB92148.1\| putative C-8,7 sterol isomerase | LN | | | | | |
| 370 | 4.00E-39 | 46 | gi\|25361093\| | pir\|\|T00967hypothetical protein At2g26340 | HS | | | | | |
| 371 | 2.00E-78 | 89 | gi\|50906887\| | ref\|XP_464932.1\|cytochrome c biogenesis protein-like | LL | PP | | | | |
| 372 | 1.00E-22 | 98 | gi\|50899510\| | ref\|XP_50543.1\|unknown protein | CK | LL | CS | PP | SS | |
| 373 | 5.00E-79 | 85 | gi\|50934647\| | ref\|XP_476851.1\|bifunctional phosphopantetheine adenylyl transferase dephospho CoA kinase-like protein | CK | CS | | | | |
| 374 | 8.00E-56 | 50 | gi\|38257027\| | dbj\|BAD01556.1\|ERF-like protein | PP | | | | | |
| 375 | 1.00E-64 | 55 | gi\|18423944\| | ref\|NP_568850.1\|basic helix-loop-helix (bHLH) family protein | PP | | | | | |
| 376 | 1.00E-39 | 42 | gi\|42567912\| | ref\|NP_568344.2\|myb family transcription factor | SP | PEG | | | | |

EP 3 059 306 A1

(continued)

| PEP SEQ ID | annotation | | | | Traits | | | | |
|---|---|---|---|---|---|---|---|---|---|
| | e-value | % id | GenBank ID | description | | | | | |
| 377 | 6.00E-89 | 84 | gi\|37535020\| | ref\|NP_921812.1\|putative HAM-1-like protein | LN | | | | |
| 378 | 2.00E-49 | 59 | gi\|27804371\| | gb\|AA022987.1\|MADS-box transcription factor CDM104 | LL | SS | | | |
| 379 | 2.00E-68 | 60 | gi\|22137112\| | emb\|CAB72174.1\| responce reactor 4 [Arabidopsis thaliana] | CK | SS | | | |
| 380 | 0 | 89 | gi\|50910245\| | ref\|XP_466611.1\|putative PLRR-4 polymorphic leucine-rich repeat protein | CS | CK | | | |
| 381 | 2.00E-30 | 44 | gi\|17933450\| | gb\|AAK70215.1\|MADS-box protein | CS | CK | PEG | | |
| 382 | 1.00E-93 | 54 | gi\|20502508\| | dbj\|BAB91414.1\|E2F-like repressor E2L3 | CK | PEG | CS | | |
| 383 | 2.00E-38 | 58 | gi\|50909627\| | ref\|XP_466302.1\|unknown protein | PEG | | | | |
| 384 | 3.00E-41 | 63 | gi\|50399946\| | gb\|AAT76334.1\|putative DNA-directed RNA polymerase II subunit | HS | | | | |
| 385 | 4.00E-59 | 68 | gi\|37535924\| | ref\|NP_922264.1\|unknown protein | LN | | | | |
| 386 | 8.00E-57 | 58 | gi\|50944571\| | ref\|XP_481813.1\|transfactor-like | HS | | | | |
| 387 | 3.00E-73 | 42 | gi\|53792319\| | dbj\|BAD53026.1\|putative ring finger protein 1 | LN | | | | |
| 388 | 9.00E-93 | 47 | gi\|25054862\| | ref\|NP_850517.1\| transcription factor, putative / zinc finger (C3HC4 type RING finger) family protein | LN | | | | |
| 389 | 7.00E-91 | 66 | gi\|20269059\| | emb\|CAC84710.1\|aux/IAA protein | PEG | | | | |
| 390 | 2.00E-72 | 45 | gi\|26450026\| | ref\|NP_172358.1\| myb family transcription factor (MYB60) | LL | LN | | | |
| 391 | 1.00E-80 | 46 | gi\|50946213\| | ref\|XP_482634.1\|AP2/EREBP transcription factor-like protein | LN | | | | |
| 392 | 8.00E-54 | 43 | gi\|29824137\| | ref\|NP_189337.1\| TCP family transcription factor, putative [Arabidopsis thaliana] | CK | CS | | | |
| 393 | 0 | 99 | gi\|558543\| | emb\|CAA85320.1\|C-terminal zinc-finger | HS | PP | PEG | | |
| 394 | 1.00E-124 | 62 | gi\|20259301\| | ref\|NP_566010.1\| SET domain-containing protein (ASHH3) | PP | SS | | | |
| 395 | 4.00E-54 | 65 | gi\|21553740\| | gb\|AAM62833.1\|putative zinc finger protein | PP | PEG | CS | | |
| 396 | 2.00E-54 | 40 | gi\|20465561\| | ref\|NP_974448.1\| zinc finger (C3HC4-type RING finger) family protein | LL | LN | | | |
| 397 | 1.00E-112 | 65 | gi\|51557078\| | gb\|AAU06309.1\|MYB transcription factor | PP | LN | | | |
| 398 | 0 | 82 | gi\|15148926\| | gb\|AAK84890.1\|TGA-type basic leucine zipper protein TGA2.2 | LN | | | | |

EP 3 059 306 A1

29

| PEP SEQ ID | annotation | | | | Traits | | | | | |
|---|---|---|---|---|---|---|---|---|---|---|
| | e-value | % id | GenBank ID | description | | | | | | |
| 399 | 6.00E-78 | 47 | gi\|28558782\| | gb\|AA045753.1\|RING/C3HC4 /PHD zinc finger-like protein | LN | | | | | |
| 400 | 0 | 67 | gi\|42565068\| | ref\|NP_1887 43.3\|transducin family protein / WD-40 repeat family protein | HS | | | | | |
| 401 | 7.00E-29 | 46 | gi\|38638682\| | ref\|NP_177307.1\| AP2 domain-containing transcription factor, putative | CS | LL | LN | | | |
| 402 | 0 | 84 | gi\|50904461\| | ref\|XP_463719.1\|P0466H10.2 7 | PEG | | | | | |
| 403 | 0 | 88 | gi\|53796982\| | ref\|ZP_00357872.1\|COG0160 : 4-aminobutyrate aminotransferase and related aminotransferases | SP | CS | | | | |
| 404 | 0 | 94 | gi\|53796007\| | ref\|ZP_00357032.1\|COG0696 : Phosphoglyromutase | PP | SS | PEG | HS | | |
| 405 | 0 | 93 | gi\|30697938\| | ref\|NP_201207.2\|expressed protein | DS | HS | | | | |
| 406 | 0 | 100 | gi\|13878095\| | gb\|AAK44125.1\|unknown protein | DS | CS | | | | |
| 407 | 0 | 97 | gi\|12324320\| | gb\|AAG52129.1\|hypothetical protein; 63994-65574 | LL | | | | | |
| 408 | 0 | 84 | gi\|22136086\| | gb\|AAM91121.1\|photorecepto r-interacting protein-like | CS | PP | | | | |

**Trait Improvement Screens**

DS- Improvement of drought tolerance identified by a soil drought stress tolerance screen: Drought or water deficit conditions impose mainly osmotic stress on plants. Plants are particularly vulnerable to drought during the flowering stage. The drought condition in the screening process disclosed in Example 1B started from the flowering time and was sustained to the end of harvesting. The present invention provides recombinant DNA that can improve the plant survival rate under such sustained drought condition. Exemplary recombinant DNA for conferring such drought tolerance are identified as such in Table 3. Such recombinant DNA may find particular use in generating transgenic plants that are tolerant to the drought condition imposed during flowering time and in other stages of the plant life cycle. As demonstrated from the model plant screen, in some embodiments of transgenic plants with trait-improving recombinant DNA grown under such sustained drought condition can also have increased total seed weight per plant in addition to the increased survival rate within a transgenic population, providing a higher yield potential as compared to control plants.

PEG-Improvement of drought tolerance identified by PEG induced osmotic stress tolerance screen: Various drought levels can be artificially induced by using various concentrations of polyethylene glycol (PEG) to produce different osmotic potentials (Pilon-Smits et al. (1995) Plant Physiol. 107:125-130). Several physiological characteristics have been reported as being reliable indications for selection of plants possessing drought tolerance. These characteristics include the rate of seed germination and seedling growth. The traits can be assayed relatively easily by measuring the growth rate of seedling in PEG solution. Thus, a PEG-induced osmotic stress tolerance screen is a useful surrogate for drought tolerance screen. As demonstrated from the model plant screen, embodiments of transgenic plants with trait-improving recombinant DNA identified in the PEG-induced osmotic stress tolerance screen can survive better drought conditions providing a higher yield potential as compared to control plants.

SS-Improvement of drought tolerance identified by high salinity stress tolerance screen: Three different factors are responsible for salt damages: (1) osmotic effects, (2) disturbances in the mineralization process, (3) toxic effects caused by the salt ions, e.g. inactivation of enzymes. While the first factor of salt stress results in the wilting of the plants that is similar to drought effect, the ionic aspect of salt stress is clearly distinct from drought. The present invention provides genes that help plants to maintain biomass, root growth, and/or plant development in high salinity conditions, which are identified as such in Table 3. Since osmotic effect is one of the major components of salt stress, which is common to the drought stress, trait-improving recombinant DNA identified in a high salinity stress tolerance screen can also provide transgenic crops with improved drought tolerance. As demonstrated from the model plant screen, embodiments of transgenic plants with trait-improving recombinant DNA identified in a high salinity stress tolerance screen can survive better drought conditions and/or high salinity conditions providing a higher yield potential as compared to control plants.

HS-Improvement of drought tolerance identified by heat stress tolerance screen: Heat and drought stress often occur simultaneously, limiting plant growth. Heat stress can cause the reduction in photosynthesis rate, inhibition of leaf growth and osmotic potential in plants. Thus, genes identified by the present invention as heat stress tolerance conferring genes may also impart improved drought tolerance to plants. As demonstrated from the model plant screen, embodiments of transgenic plants with trait-improving recombinant DNA identified in a heat stress tolerance screen can survive better heat stress conditions and/or drought conditions providing a higher yield potential as compared to control plants.

CK and CS-Improvement of tolerance to cold stress: Low temperature may immediately result in mechanical constraints, changes in activities of macromolecules, and reduced osmotic potential. In the present invention, two screening conditions, i.e. cold shock tolerance screen (CK) and cold germination tolerance screen (CS), were set up to look for transgenic plants that display visual growth advantage at lower temperature. In cold germination tolerance screen, the transgenic *Arabidopsis* plants were exposed to a constant temperature of 8°C from planting until day 28 post plating. The trait-improving recombinant DNA identified by such screen are particular useful for the production of transgenic plant that can germinate more robustly in a cold temperature as compared to the wild type plants. In cold shock tolerance screen, the transgenic plants were first grown under the normal growth temperature of 22°C until day 8 post plating, and subsequently were placed under 8°C until day 28 post plating. As demonstrated from the model plant screen, embodiments of transgenic plants with trait-improving recombinant DNA identified in a cold shock stress tolerance screen and/or a cold germination stress tolerance screen can survive better cold conditions providing a higher yield potential as compared to control plants.

Improvement of tolerance to multiple stresses: Different kinds of stresses often lead to identical or similar reaction in the plants. Genes that are activated or inactivated as a reaction to stress can either act directly in a way the genetic product reduces a specific stress, or they can act indirectly by activating other specific stress genes. By manipulating the activity of such regulatory genes, i.e. multiple stress tolerance genes, the plant can be enabled to react to different kinds of stresses. For examples, PEP SEQ ID NO: 229 and PEP SEQ ID NO: 372 can be used to improve both salt stress tolerance and cold stress tolerance in plants. Of particular interest, plants transformed with PEP SEQ ID NO: 364 can resist heat stress, salt stress and cold stress. In addition to these multiple stress tolerance genes, the stress tolerance conferring genes provided by the present invention may be used in combinations to generate transgenic plants that can resist multiple stress conditions.

PP-Improvement of early plant growth and development: It has been known in the art that to minimize the impact of disease on crop profitability, it is important to start the season with healthy vigorous plants. This means avoiding seed and seedling diseases, leading to increased nutrient uptake and increased yield potential. Traditionally early planting and applying fertilizer are the methods used for promoting early seedling vigor. In early development stage, plant embryos establish only the basic root-shoot axis, a cotyledon storage organ(s), and stem cell populations, called the root and shoot apical meristems, that continuously generate new organs throughout post-embryonic development. "Early growth and development" used herein encompasses the stages of seed imbibition through the early vegetative phase. The present invention provides genes that are useful to produce transgenic plants that have advantages in one or more processes including, but not limited to, germination, seedling vigor, root growth and root morphology under non-stressed conditions. The transgenic plants starting from a more robust seedling are less susceptible to the fungal and bacterial pathogens that attach germinating seeds and seedling. Furthermore, seedlings with advantage in root growth are more resistant to drought stress due to extensive and deeper root architecture. Therefore, it can be recognized by those skilled in the art that genes conferring the growth advantage in early stages to plants may also be used to generate transgenic plants that are more resistant to various stress conditions due to improved early plant development. The present invention provides such exemplary recombinant DNA that confer both the stress tolerance and growth advantages to plants, identified as such in Table 3, e.g. PEP SEQ ID NO: 372 which can improve the plant early growth and development, and impart salt and cold tolerance to plants. As demonstrated from the model plant screen, embodiments of transgenic plants with trait-improving recombinant DNA identified in the early plant development screen can grow better under non-stress conditions and/or stress conditions providing a higher yield potential as compared to control plants.

SP-Improvement of late plant growth and development: "Late growth and development" used herein encompasses the stages of leaf development, flower production, and seed maturity. In certain embodiments, transgenic plants produced using genes that confer growth advantages to plants provided by the present invention, identified as such in Table 3, exhibit at least one phenotypic characteristics including, but not limited to, increased rosette radius, increased rosette dry weight, seed dry weight, silique dry weight, and silique length. On one hand, the rosette radius and rosette dry weight are used as the indexes of photosynthesis capacity, and thereby plant source strength and yield potential of a plant. On the other hand, the seed dry weight, silique dry weight and silique length are used as the indexes for plant sink strength, which are considered as the direct determinants of yield. As demonstrated from the model plant screen, embodiments of transgenic plants with trait-improving recombinant DNA identified in the late development screen can grow better and/or have improved development during leaf development and seed maturation providing a higher yield potential as compared to control plants.

LL-Improvement of tolerance to shade stress identified in a low light screen: The effects of light on plant development are especially prominent at the seedling stage. Under normal light conditions with unobstructed direct light, a plant seeding develops according to a characteristic photomorphogenic pattern, in which plants have open and expanded cotyledons and short hypocotyls. Then the plant's energy is devoted to cotyledon and leaf development while longitudinal extension growth is minimized. Under low light condition where light quality and intensity are reduced by shading, obstruction or high population density, a seedling displays a shade-avoidance pattern, in which the seedling displays a reduced cotyledon expansion, and hypocotyls extension is greatly increased. As the result, a plant under low light condition increases significantly its stem length at the expanse of leaf, seed or fruit and storage organ development, thereby adversely affecting of yield. The present invention provides recombinant DNA that enable plants to have an attenuated shade avoidance response so that the source of plant can be contributed to reproductive growth efficiently, resulting higher yield as compared to the wild type plants. As demonstrated from the model plant screen, embodiments of transgenic plants with trait-improving recombinant DNA identified in a shade stress tolerance screen can have attenuated shade response under shade conditions providing a higher yield potential as compared to control plants. The transgenic plants generated by the present invention may be suitable for a higher density planting, thereby resulting increased yield per unit area.

LN-Improvement of tolerance to low nitrogen availability stress

Nitrogen is a key factor in plant growth and crop yield. The metabolism, growth and development of plants are profoundly affected by their nitrogen supply. Restricted nitrogen supply alters shoot to root ratio, root development, activity of enzymes of primary metabolism and the rate of senescence (death) of older leaves. All field crops have a fundamental dependence on inorganic nitrogenous fertilizer. Since fertilizer is rapidly depleted from most soil types, it must be supplied to growing crops two or three times during the growing season. Enhanced nitrogen use efficiency by plants should enable crops cultivated under low nitrogen availability stress condition resulted from low fertilizer input or poor soil quality.

According to the present invention, transgenic plants generated using the recombinant nucleotides, which confer enhanced nitrogen use efficiency, identified as such in Table 3, exhibit one or more desirable traits including, but not limited to, increased seedling weight, increased number of green leaves, increased number of rosette leaves, increased root length and advanced flower bud formation. One skilled in the art may recognize that the transgenic plants provided by the present invention with enhanced nitrogen use efficiency may also have altered amino acid or protein compositions, increased yield and/or better seed quality. The transgenic plants of the present invention may be productively cultivated under nitrogen nutrient deficient conditions, i.e. nitrogen-poor soils and low nitrogen fertilizer inputs, that would cause the growth of wild type plants to cease or to be so diminished as to make the wild type plants practically useless. The transgenic plants also may be advantageously used to achieve earlier maturing, faster growing, and/or higher yielding crops and/or produce more nutritious foods and animal feedstocks when cultivated using nitrogen non-limiting growth conditions.

Stacked Traits: The present invention also encompasses transgenic plants with stacked engineered traits, e.g. a crop having an improved phenotype resulting from expression of a trait-improving recombinant DNA, in combination with herbicide and/or pest resistance traits. For example, genes of the current invention can be stacked with other traits of agronomic interest, such as a trait providing herbicide resistance, for example a RoundUp Ready trait, or insect resistance, such as using a gene from *Bacillus thuringensis* to provide resistance against lepidopteran, coliopteran, homopteran, hemiopteran, and other insects. Herbicides for which resistance is useful in a plant include glyphosate herbicides, phosphinothricin herbicides, oxynil herbicides, imidazolinone herbicides, dinitroaniline herbicides, pyridine herbicides, sulfonylurea herbicides, bialaphos herbicides, sulfonamide herbicides and gluphosinate herbicides. To illustrate that the production of transgenic plants with herbicide resistance is a capability of those of ordinary skill in the art, reference is made to U.S. patent application publications 2003/0106096A1 and 2002/0112260A1 and U.S. Patents 5,034,322; 5,776,760, 6,107,549 and 6,376,754, all of which are incorporated herein by reference. To illustrate that the production of transgenic plants with pest resistance is a capability of those of ordinary skill in the art reference is made to U.S. Patents 5,250,515 and 5,880,275 which disclose plants expressing an endotoxin of *Bacillus thuringiensis* bacteria, to U.S. Patent 6,506,599 which discloses control of invertebrates which feed on transgenic plants which express dsRNA for suppressing a target gene in the invertebrate, to U.S. Patent 5,986,175 which discloses the control of viral pests by transgenic plants which express viral replicase, and to U.S. Patent Application Publication 2003/0150017 A1 which discloses control of pests by a transgenic plant which express a dsRNA targeted to suppressing a gene in the pest, all of which are incorporated herein by reference.

Once one recombinant DNA has been identified as conferring an improved trait of interest in transgenic *Arabidopsis* plants, several methods are available for using the sequence of that recombinant DNA and knowledge about the protein it encodes to identify homologs of that sequence from the same plant or different plant species or other organisms, e.g. bacteria and yeast. Thus, in one aspect, the invention provides methods for identifying a homologous gene with a DNA sequence homologous to any of SEQ ID NO: 1 through SEQ ID NO: 204, or a homologous protein with an amino acid sequence homologous to any of SEQ ID NO: 205 through SEQ ID NO: 408. In another aspect, the present invention provides the protein sequences of identified homologs for a sequence listed as SEQ ID NO: 205 through SEQ ID NO: 408. In yet another aspect, the present invention also includes linking or associating one or more desired traits, or gene function with a homolog sequence provided herein.

The trait-improving recombinant DNA and methods of using such trait-improving recombinant DNA for generating transgenic plants with improved traits provided by the present invention are not limited to any particular plant species. Indeed, the plants according to the present invention may be of any plant species, i.e., may be monocotyledonous or dicotyledonous. Preferably, they will be agricultural useful plants, i.e., plants cultivated by man for purposes of food production or technical, particularly industrial applications. Of particular interest in the present invention are corn and soybean plants. The recombinant DNA constructs optimized for soybean transformation and recombinant DNA constructs optimized for corn transformation are provided by the present invention. Other plants of interest in the present invention for production of transgenic plants having improved traits include, without limitation, cotton, canola, wheat, sunflower, sorghum, alfalfa, barley, millet, rice, tobacco, fruit and vegetable crops, and turfgrass.

In certain embodiments, the present invention contemplates to use an orthologous gene in generating the transgenic plants with similarly improved traits as the transgenic *Arabidopsis* counterpart. Improved physiological properties in transgenic plants of the present invention may be confirmed in responses to stress conditions, for example in assays using imposed stress conditions to detect improved responses to drought stress, nitrogen deficiency, cold growing conditions, or alternatively, under naturally present stress conditions, for example under field conditions. Biomass measures may be made on greenhouse or field grown plants and may include such measurements as plant height, stem diameter, root and shoot dry weights, and, for corn plants, ear length and diameter.

Trait data on morphological changes may be collected by visual observation during the process of plant regeneration as well as in regenerated plants transferred to soil. Such trait data includes characteristics such as normal plants, bushy plants, taller plants, thicker stalks, narrow leaves, striped leaves, knotted phenotype, chlorosis, albino, anthocyanin production, or altered tassels, ears or roots. Other enhanced traits may be identified by measurements taken under field conditions, such as days to pollen shed, days to silking, leaf extension rate, chlorophyll content, leaf temperature, stand, seedling vigor, internode length, plant height, leaf number, leaf area, tillering, brace roots, stay green, stalk lodging, root lodging, plant health, barreness/prolificacy, green snap, and pest resistance. In addition, trait characteristics of harvested grain may be confirmed, including number of kernels per row on the ear, number of rows of kernels on the ear, kernel abortion, kernel weight, kernel size, kernel density and physical grain quality.

To confirm hybrid yield in transgenic corn plants expressing genes of the present invention, it may be desirable to test hybrids over multiple years at multiple locations in a geographical location where maize is conventionally grown, e.g. in Iowa, Illinois or other locations in the midwestern United States, under "normal" field conditions as well as under stress conditions, e.g. under drought or population density stress.

Transgenic plants can be used to provide plant parts according to the invention for regeneration or tissue culture of cells or tissues containing the constructs described herein. Plant parts for these purposes can include leaves, stems, roots, flowers, tissues, epicotyl, meristems, hypocotyls, cotyledons, pollen, ovaries, cells and protoplasts, or any other portion of the plant which can be used to regenerate additional transgenic plants, cells, protoplasts or tissue culture. Seeds of transgenic plants are provided by this invention can be used to propagate more plants containing the trait-improving recombinant DNA constructs of this invention. These descendants are intended to be included in the scope of this invention if they contain a trait-improving recombinant DNA construct of this invention, whether or not these plants are selfed or crossed with different varieties of plants.

The various aspects of the invention are illustrated by means of the following examples which are in no way intended to limit the full breath and scope of claims.

**Example 1.**

This example illustrates the identification of recombinant DNA that confers improved trait(s) to plants

A large set of genes of interest were cloned from a genomic or cDNA library using primers specific to sequences upstream and downstream of the coding region. Transformation vectors were prepared to constitutively transcribe DNA in either sense orientation (for enhanced protein expression) or anti-sense orientation (for endogenous gene suppression) under the control of an enhanced Cauliflower Mosaic Virus 35S promoter. The transformation vectors also contain a *bar* gene as a selectable marker for resistance to glufosinate herbicide. The transformation of *Arabidopsis* plants was carried out using the vacuum infiltration method known in the art (Bethtold et al. Methods Mol. Biol. 82:259-66, 1998). Seeds harvested from the plants, named as T1 seeds, were subsequently grown in a glufosinate-containing selective medium to select for plants which were actually transformed and which produced T2 transgenic seed. The plants and seeds were screened for an enhanced trait or a surrogate for an enhanced trait.

**Soil Drought tolerance screen**

This screen identified genes for recombinant DNA that imparts enhanced water use efficiency as shown in *Arabidopsis* plants transformed with recombinant DNA that wilt less rapidly and/or produce higher seed yield when grown in soil under drought conditions

T2 seeds were sown in flats filled with Metro/Mix® 200 (The Scotts® Company, USA). Humidity domes were added to each flat and flats were assigned locations and placed in climate-controlled growth chambers. Plants were grown under a temperature regime of 22°C at day and 20°C at night, with a photoperiod of 16 hours and average light intensity of 170 $\mu$mol/m$^2$/s. After the first true leaves appeared, humidity domes were removed. The plants were sprayed with glufosinate herbicide and put back in the growth chamber for 3 additional days. Flats were watered for 1 hour the week following the herbicide treatment. Watering was continued every seven days until the flower bud primordia became apparent, at which time plants were watered for the last time.

To identify drought tolerant plants, plants were evaluated for wilting response and seed yield. Beginning ten days after the last watering, plants were examined daily until 4 plants/line had wilted. In the next six days, plants were monitored for wilting response. Five drought scores were assigned according to the visual inspection of the phenotypes: 1 for healthy, 2 for dark green, 3 for wilting, 4 severe wilting, and 5 for dead. A score of 3 or higher was considered as wilted.

At the end of this assay, seed yield measured as seed weight per plant under the drought condition was characterized for the transgenic plants and their controls and analyzed as a quantitative response according to example 1M.

Two approaches were used for statistical analysis on the wilting response. First, the risk score was analyzed for wilting phenotype and treated as a qualitative response according to the example 1L. Alternatively, the survival analysis was carried out in which the proportions of wilted and non-wilted transgenic and control plants were compared over each of the six days under scoring and an overall log rank test was performed to compare the two survival curves using S-PLUS statistical software (S-PLUS 6, Guide to statistics, Insightful, Seattle, WA, USA). Table 4 provides a list of recombinant DNA constructs that improve drought tolerance in transgenic plants.

Table 4

| Pep SEQ ID | Construct_ id | Orientation | Wilt Response Risk score | | | Seed Weight/plant | | | Survival Analysis of wilt response | | |
|---|---|---|---|---|---|---|---|---|---|---|---|
| | | | RS mean | p-value | c | delta | p-value | c | diff time to wilting | p-value | c |
| 215 | 19116 | ANTI-SENSE | 0 | 0.795 | S | -0.341 | 0.795 | / | 0.52 | 0.077 | T |
| 250 | 70677 | SENSE | 0.045 | 0.982 | S | -5.73 | 0.982 | / | 0.14 | 0.07 | T |
| 219 | 72712 | SENSE | 0.002 | 0.986 | S | -2.822 | 0.986 | / | 0.64 | 0.242 | / |
| 266 | 72714 | SENSE | 0.01 | 0 | S | 1.253 | 0 | S | 0.14 | 0.059 | T |
| 271 | 72721 | SENSE | 0.05 | 0.834 | T | -0.129 | 0.834 | / | 0 | 1 | / |
| 272 | 72959 | SENSE | 0.001 | 0.172 | S | 0.341 | 0.172 | / | 0.42 | 0.32 | / |
| 317 | 73534 | SENSE | 0.006 | 0.732 | S | -0.195 | 0.732 | / | -0.14 | 0.724 | / |
| 264 | 74244 | SENSE | 0.012 | 0.984 | S | -0.512 | 0.984 | / | 0.13 | 0.54 | / |
| 332 | 74453 | SENSE | 0.655 | 0.001 | / | 0.886 | 0.001 | S | -0.15 | 0.468 | / |

S: represents that the transgenic plants showed statistically significant trait improvement as compared to the reference (p<0.05, p value, of the delta of a quantitative response or of the risk score of a qualitative response, is the probability that the observed difference between the transgenic plants and the reference occur by chance)
T: represents that the transgenic plants showed a trend of trait improvement as compared to the reference with p<0.2
/: represents the transgenic plants didn't show any alteration or had unfavorable change in traits examined compared to the reference in the current dataset.

**Heat stress tolerance screen**

Under high temperatures, *Arabidopsis* seedlings become chlorotic and root growth is inhibited. This screen identified genes for recombinant DNA that imparts enhanced heat tolerance as shown in *Arabidopsis* plants transformed with the gene of interest that are more resistant to heat stress based on primarily their seedling weight and root growth under high temperature.

T2 seeds were plated on 1/2 X MS salts, 1/% phytagel, with 10 μg/ml BASTA (7 per plate with 2 control seeds; 9 seeds total per plate). Plates were placed at 4°C for 3 days to stratify seeds. Plates were then incubated at room temperature for 3 hours and then held vertically for 11 additional days at temperature of 34°C at day and 20°C at night. Photoperiod was 16 h. Average light intensity was ~140 μmol/m$^2$/s. After 14 days of growth, plants were scored for glufosinate resistance, root length, final growth stage, visual color, and seedling fresh weight. A photograph of the whole plate was taken on day 14.

The seedling weight and root length were analyzed as quantitative responses according to example 1M. The final grow stage at day 14 was scored as success if 50% of the plants had reached 3 rosette leaves and size of leaves are greater than 1mm (Boyes et al. (2001) The Plant Cell 13, 1499-1510). The growth stage data was analyzed as a qualitative response according to example 1L.Table 5 provides a list of recombinant DNA constructs that improve heat tolerance in transgenic plants.

Table 5

| Pep SEQ ID | Construct_ id | Orientation | seedling weight | | | Root Length | | | growth stage | | |
|---|---|---|---|---|---|---|---|---|---|---|---|
| | | | delta | p-value | c | delta | p-value | c | RS mean | p-value | c |
| 258 | 10903 | ANTI-SENSE | 1.26 | 0 | S | 0.131 | 0.053 | T | 0.86 | 0.051 | T |
| 205 | 12360 | SENSE | 1.305 | 0 | S | 0.142 | 0.052 | T | 0.307 | 0.043 | S |
| 405 | 12824 | SENSE | 1.309 | 0 | S | 0.25 | 0.002 | S | 0.63 | 0.063 | T |
| 211 | 12927 | SENSE | 1.527 | 0 | S | 0.268 | 0.013 | S | 0.803 | 0.018 | S |
| 207 | 15210 | SENSE | 1.189 | 0 | S | 0.121 | 0.029 | S | 0.636 | 0.079 | T |
| 214 | 17309 | SENSE | 1.29 | 0 | S | 0.176 | 0.01 | S | 0.581 | 0.044 | S |
| 242 | 19801 | SENSE | 1.156 | 0 | S | 0.148 | 0.041 | S | 0.413 | 0.141 | T |
| 233 | 19845 | SENSE | 1.242 | 0 | S | 0.148 | 0.051 | T | 0.479 | 0.016 | S |
| 239 | 19850 | SENSE | 1.234 | 0 | S | 0.217 | 0.015 | S | 1.883 | 0.001 | S |
| 237 | 19981 | SENSE | 1.406 | 0 | S | 0.36 | 0 | S | 2.199 | 0 | S |
| 220 | 71546 | SENSE | 1.496 | 0 | S | 0.24 | 0.016 | S | 0.313 | 0.052 | T |
| 246 | 71556 | SENSE | 1.394 | 0 | S | 0.26 | 0.015 | S | 0.194 | 0.076 | T |
| 276 | 73029 | SENSE | 0.88 | 0.002 | S | 0.115 | 0.061 | T | 0.092 | 0.105 | T |
| 318 | 74125 | SENSE | 0.988 | 0 | S | 0.171 | 0.058 | T | 0.206 | 0.053 | T |
| 283 | 74329 | SENSE | 1.19 | 0 | S | 0.124 | 0.08 | T | 0.043 | 0.239 | / |
| 329 | 74402 | SENSE | 1.407 | 0 | S | 0.291 | 0.019 | S | 0.181 | 0.087 | T |
| 335 | 74503 | SENSE | 1.159 | 0 | S | 0.171 | 0.019 | S | 0.206 | 0.02 | S |
| 340 | 74553 | SENSE | 1.141 | 0 | S | 0.184 | 0.02 | S | 0.704 | 0.03 | S |
| 299 | 74669 | SENSE | 1.105 | 0 | S | 0.133 | 0.049 | S | 0.051 | 0.362 | / |
| 352 | 74903 | SENSE | 1.167 | 0 | S | 0.168 | 0.096 | T | 0.836 | 0.041 | S |
| 362 | 74980 | SENSE | 1.084 | 0 | S | 0.165 | 0.027 | S | 1.07 | 0.033 | S |
| 364 | 75337 | SENSE | 1.695 | 0 | S | 0.216 | 0.009 | S | 0.369 | 0.072 | T |
| 367 | 75352 | SENSE | 1.342 | 0 | S | 0.198 | 0.003 | S | 0.311 | 0.053 | T |
| 370 | 75358 | SENSE | 1.314 | 0 | S | 0.131 | 0.034 | S | 0.012 | 0.365 | / |

(continued)

| Pep SEQ ID | Construct_ id | Orientation | seedling weight | | | Root Length | | | growth stage | | |
|---|---|---|---|---|---|---|---|---|---|---|---|
| | | | delta | p-value | c | delta | p-value | c | RS mean | p-value | c |
| 384 | 75409 | SENSE | 1.264 | 0 | S | 0.172 | 0.041 | S | 0.716 | 0.039 | S |
| 386 | 75550 | SENSE | 1.117 | 0 | S | 0.18 | 0.032 | S | 0.384 | 0.074 | T |
| 400 | 75571 | SENSE | 1.182 | 0 | S | 0.185 | 0.042 | S | 0.496 | 0.088 | T |
| 404 | 75909 | SENSE | 1.264 | 0 | S | 0.237 | 0.021 | S | -0.01 | 1 | / |

S: represents the transgenic plants showed statistically significant trait improvement as compared to the reference (p<0.05) T: represents the transgenic plants showed a trend of trait improvement as compared to the reference with p<0.2

/: represents the transgenic plants didn't show any alteration or had unfavorable change in traits examined compared to the reference in the current dataset

**Salt stress tolerance screen**

This screen identified genes for recombinant DNA that imparts enhanced salt tolerance, a surrogate for enhanced water use efficiency, as shown in *Arabidopsis* plants transformed with the gene of interest that are tolerant to high levels of salt based on their rate of development, root growth and chlorophyll accumulation under high salt conditions.

T2 seeds were plated on glufosinate selection plates containing 90 mM NaCl and grown under standard light and temperature conditions. All seedlings used in the experiment were grown at a temperature of 22°C at day and 20°C at night, a 16-hour photoperiod, an average light intensity of approximately 120 umol/m$^2$. On day 11, plants were measured for primary root length. After 3 more days of growth (day 14), plants were scored for transgenic status, primary root length, growth stage, visual color, and the seedlings were pooled for fresh weight measurement. A photograph of the whole plate was also taken on day 14.

The seedling weight and root length were analyzed as quantitative responses according to example 1M. The final growth stage at day 14 was scored as success if 50% of the plants reached 3 rosette leaves and size of leaves are greater than 1mm (Boyes, D.C. et. al. (2001), The Plant Cell 13, 1499/1510). The growth stage data was analyzed as a qualitative response according to example 1L. Table 6 provides a list of recombinant DNA constructs that improve high salinity tolerance in transgenic plants

Table 6

| Pep SEQ ID | Construct id | Orientation | Seedling Weight at day14 | | | Root Length at day 11 | | | Root Length at day 14 | | | Growth Stage | | |
|---|---|---|---|---|---|---|---|---|---|---|---|---|---|---|
| | | | delta | p-value | c | delta | p-value | c | delta | p-value | c | RS mean | p-value | c |
| 228 | 19617 | SENSE | 0.662 | 0.023 | S | 0.211 | 0.138 | T | 0.019 | 0.845 | / | 0.467 | 0.06 | T |
| 229 | 19750 | SENSE | 0.794 | 0.001 | S | 0.324 | 0.035 | S | 0.192 | 0.001 | S | 3.409 | 0.001 | S |
| 240 | 19942 | SENSE | 0.496 | 0.022 | S | 0.123 | 0.294 | / | 0.103 | 0.208 | / | 1.302 | 0.103 | T |
| 270 | 72743 | SENSE | 0.522 | 0.043 | S | 0.018 | 0.856 | / | 0.194 | 0.004 | S | 0.651 | 0.055 | T |
| 278 | 72920 | SENSE | 0.377 | 0.033 | S | 0.251 | 0.001 | S | 0.133 | 0.004 | S | 0.191 | 0.233 | / |
| 315 | 73516 | SENSE | 0.41 | 0.005 | S | 0.079 | 0.292 | / | 0.034 | 0.372 | / | 1.252 | 0.053 | T |
| 263 | 74237 | SENSE | 0.711 | 0.002 | S | 0.18 | 0.135 | T | 0.187 | 0.066 | T | 2.74 | 0.01 | s |
| 282 | 74327 | SENSE | 0.554 | 0.006 | S | 0.169 | 0.056 | T | 0.162 | 0.005 | S | 1.469 | 0.005 | S |
| 291 | 74366 | SENSE | 0.623 | 0.008 | S | 0.125 | 0.228 | / | 0.108 | 0.248 | / | 0.695 | 0.062 | T |
| 335 | 74503 | SENSE | 0.666 | 0.002 | S | 0.258 | 0.027 | S | 0.103 | 0.221 | / | 2.431 | 0.01 | S |
| 336 | 74504 | SENSE | 0.976 | 0.001 | S | 0.356 | 0.013 | S | 0.261 | 0 | S | 3.178 | 0.001 | s |
| 296 | 74622 | SENSE | 1.092 | 0.003 | S | 0.261 | 0.005 | S | 0.217 | 0.013 | S | 1.457 | 0.065 | T |
| 297 | 74628 | SENSE | 0.423 | 0.057 | T | -0.062 | 0.725 | / | 0.226 | 0.02 | S | 0.087 | 0.31 | / |
| 301 | 74647 | SENSE | 0.161 | 0.442 | / | -0.083 | 0.569 | / | 0.208 | 0.001 | S | 0.573 | 0.043 | S |
| 352 | 74903 | SENSE | 0.446 | 0.009 | S | 0.001 | 0.99 | / | 0.116 | 0.034 | S | 1.234 | 0.05 | T |
| 357 | 74977 | SENSE | 0.496 | 0.019 | S | 0.2 | 0.012 | S | 0.198 | 0 | S | 0.764 | 0.043 | S |
| 363 | 74993 | SENSE | 0.379 | 0.017 | S | 0.242 | 0.075 | T | 0.108 | 0.07 | T | 0.163 | 0.319 | / |
| 366 | 75316 | SENSE | 0.475 | 0.078 | T | 0.287 | 0.002 | S | 0.178 | 0.011 | S | 4 | 0 | S |
| 364 | 75337 | SENSE | 0.934 | 0.004 | S | 0.217 | 0.124 | T | 0.314 | 0.002 | S | 1.831 | 0.013 | S |
| 378 | 75431 | SENSE | 0.429 | 0.083 | T | 0.076 | 0.397 | / | 0.096 | 0.271 | / | 0.522 | 0.121 | T |
| 379 | 75455 | SENSE | 0.286 | 0.281 | / | 0.314 | 0.001 | S | 0.221 | 0.006 | s | 0.396 | 0.095 | T |
| 372 | 75463 | SENSE | 0.601 | 0.011 | S | 0.141 | 0.221 | / | 0.163 | 0.049 | S | 1.252 | 0.058 | T |

(continued)

| Pep SEQ ID | Construct id | Orientation | Seedling Weight at day14 | | | Root Length at day 11 | | | Root Length at day 14 | | | Growth Stage | | |
|---|---|---|---|---|---|---|---|---|---|---|---|---|---|---|
| | | | delta | p-value | c | delta | p-value | c | delta | p-value | c | RS mean | p-vallue | c |
| 394 | 75543 | SENSE | 0.396 | 0.042 | S | 0.136 | 0.054 | T | 0.088 | 0.192 | T | 1.426 | 0.078 | T |
| S: represents the transgenic plants showed statistically significant trait improvement as compared to the reference (p<0.05)<br>T: represents the transgenic plants showed a trend of trait improvement as compared to the reference with p<0.2<br>/: represents the transgenic plants didn't show any alteration or had unfavorable change in traits examined compared to the reference in the current dataset | | | | | | | | | | | | | | |

**Polyethylene Glycol (PEG) induced osmotic stress tolerance screen**

There are numerous factors, which can influence seed germination and subsequent seedling growth, one being the availability of water. Genes, which can directly affect the success rate of germination and early seedling growth, are potentially useful agronomic traits for improving the germination and growth of crop plants under drought stress. This screen identified genes for recombinant DNA that imparts enhance osmotic stress tolerance, a surrogate for enhanced water use efficiency, as shown in *Arabidopsis* seed when PEG was used to induce osmotic stress on germinating transgenic lines of seeds.

T2 seeds were plated on BASTA selection plates containing 3% PEG and grown under standard light and temperature conditions. Seeds were plated on each plate containing 3% PEG,1/2 X MS salts, 1% phytagel, and 10 μg/ml glufosinate. Plates were placed at 4°C for 3 days to stratify seeds. On day 11, plants were measured for primary root length. After 3 more days of growth, i.e. at day 14, plants were scored for transgenic status, primary root length, growth stage, visual color, and the seedlings were pooled for fresh weight measurement. A photograph of the whole plate was taken on day14.

Seedling weight and root length were analyzed as quantitative responses according to example 1M. The final growth stage at day 14 was scored as success or failure based on whether the plants reached 3 rosette leaves and size of leaves are greater than 1mm. The growth stage data was analyzed as a qualitative response according to example 1L.Table 7 provides a list of recombinant DNA constructs that improve osmotic stress tolerance in transgenic plants.

Table 7

| Pep SEQ ID | Gene | Orientation | Seedling Weight at day14 | | | Root Length at day 11 | | | Root Length at day 14 | | | Growth Stage | | |
|---|---|---|---|---|---|---|---|---|---|---|---|---|---|---|
| | | | delta | p-value | c | delta | p-value | c | delta | p-value | c | RS mean | p-value | c |
| 275 | 10180 | ANTI-SENSE | 0.322 | 0.024 | S | 0.154 | 0.112 | T | 0.077 | 0.233 | / | 2.679 | 0.014 | S |
| 210 | 12919 | SENSE | 0.523 | 0.001 | S | 0.15 | 0.104 | T | 0.134 | 0.211 | / | 1.067 | 0.084 | T |
| 218 | 16004 | ANTI-SENSE | 0.58 | 0.005 | S | 0.109 | 0.465 | / | 0.137 | 0.048 | S | 0.96 | 0.183 | T |
| 243 | 19705 | SENSE | 0.415 | 0.098 | T | 0.225 | 0.014 | S | 0.185 | 0.104 | T | 2.87 | 0.009 | S |
| 244 | 19737 | SENSE | 0.71 | 0.065 | T | 0.163 | 0.454 | / | 0.127 | 0.477 | / | 2.147 | 0.036 | S |
| 238 | 19757 | SENSE | 0.876 | 0.006 | S | 0.095 | 0.477 | / | 0.173 | 0.016 | S | 2.298 | 0.019 | S |
| 236 | 19902 | SENSE | 0.635 | 0.012 | S | 0.233 | 0.044 | S | 0.1 | 0.104 | T | 2.314 | 0.016 | S |
| 230 | 19964 | SENSE | 0.421 | 0.002 | S | 0.313 | 0.027 | S | 0.388 | 0.004 | S | 2.051 | 0.038 | S |
| 247 | 71330 | SENSE | 0.35 | 0.003 | S | -0.09 | 0.453 | / | 0.137 | 0.189 | T | 3.292 | 0.003 | S |
| 220 | 71546 | SENSE | 0.892 | 0.003 | S | 0.32 | 0.005 | S | 0.266 | 0.028 | S | 3.207 | 0.005 | S |
| 310 | 73480 | SENSE | 0.257 | 0.053 | T | 0.12 | 0.058 | T | 0.069 | 0.487 | / | 3.231 | 0.004 | S |
| 254 | 73651 | SENSE | 0.581 | 0.04 | S | 0.053 | 0.73 | / | 0.034 | 0.609 | / | 2.992 | 0.004 | S |
| 252 | 73685 | SENSE | 0.467 | 0.024 | S | 0.137 | 0.171 | T | 0.006 | 0.933 | / | 1.556 | 0.026 | S |
| 262 | 73769 | SENSE | 0.532 | 0.005 | S | 0.243 | 0.073 | T | 0.043 | 0.7 | / | 2.939 | 0.02 | S |
| 284 | 74340 | SENSE | 0.56 | 0.003 | S | 0.258 | 0.049 | S | 0.237 | 0.036 | S | 3.541 | 0 | S |
| 293 | 74374 | SENSE | 0.587 | 0.081 | T | 0.361 | 0.011 | S | 0.251 | 0.009 | S | 3.001 | 0.003 | S |
| 336 | 74504 | SENSE | 0.801 | 0.003 | S | 0.185 | 0.021 | S | 0.111 | 0.021 | S | 4 | 0 | S |
| 337 | 74528 | SENSE | 0.552 | 0.026 | S | 0.107 | 0.338 | / | 0.112 | 0.418 | / | 4 | 0 | S |
| 341 | 74554 | SENSE | 0.726 | 0.001 | S | 0.337 | 0.012 | S | 0.224 | 0.065 | T | 3.462 | 0.001 | S |
| 343 | 74590 | SENSE | 0.511 | 0.034 | S | 0.067 | 0.671 | / | 0.052 | 0.753 | / | 2.479 | 0.029 | S |
| 289 | 74608 | SENSE | 0.448 | 0.043 | S | 0.175 | 0.298 | / | 0.241 | 0.109 | T | 2.617 | 0.016 | S |
| 300 | 74670 | SENSE | 0.855 | 0 | S | 0.154 | 0.059 | T | 0.063 | 0.447 | / | 2.331 | 0.013 | S |
| 355 | 74951 | SENSE | 0.558 | 0.009 | S | 0.488 | 0.003 | S | 0.517 | 0.001 | s | 3.302 | 0.003 | S |
| 363 | 74993 | SENSE | 0.677 | 0 | S | 0.147 | 0 | S | 0.028 | 0.499 | / | 3.211 | 0.005 | S |

| Pep SEQ ID | Gene | Orientation | Seedling Weight at day14 | | | Root Length at day 11 | | | Root Length at day 14 | | | Growth Stage | | |
|---|---|---|---|---|---|---|---|---|---|---|---|---|---|---|
| | | | delta | p-value | c | delta | p-value | c | delta | p-value | c | RS mean | p-value | c |
| 376 | 75418 | SENSE | 0.339 | 0.06 | T | 0.023 | 0.763 | / | 0.263 | 0.016 | S | 0.811 | 0.246 | / |
| 381 | 75456 | SENSE | 0.43 | 0.044 | S | 0.23 | 0.03 | S | 0.198 | 0.003 | S | 2.674 | 0.004 | S |
| 383 | 75492 | SENSE | 0.434 | 0.018 | S | 0.073 | 0.024 | S | 0.09 | 0.093 | T | 0.955 | 0.186 | T |
| 402 | 75536 | SENSE | 0.214 | 0.066 | T | 0.146 | 0.015 | S | 0.221 | 0.025 | S | -1.33 | 0.996 | / |
| 389 | 75564 | SENSE | 0.407 | 0.043 | S | 0.053 | 0.623 | / | 0.074 | 0.569 | / | 1.839 | 0.058 | T |
| 395 | 75567 | SENSE | 0.44 | 0.02 | S | 0.077 | 0.522 | / | 0.107 | 0.291 | / | 2.4 | 0.034 | S |
| 393 | 75590 | SENSE | 0.431 | 0.006 | S | 0.21 | 0.022 | S | 0.195 | 0.014 | S | 2.848 | 0.006 | S |

S: represents the transgenic plants showed statistically significant trait improvement as compared to the reference (p<0.05) T: represents the transgenic plants showed a trend of trait improvement compared to the reference with p<0.2

/: represents the transgenic plants didn't show any alteration or had unfavorable change in traits examined compared to the reference in the current dataset

EP 3 059 306 A1

**Cold shock tolerance screen**

This screen identified genes for recombinant DNA that imparts enhanced sold tolerance as shown in *Arabidopsis* plants transformed with the genes of interest that are more tolerant to cold stress subjected during day 8 to day 28 after seed planting. During these crucial early stages, seedling growth and leaf area increase were measured to assess tolerance when *Arabidopsis* seedlings were exposed to low temperatures. Using this screen, genetic alterations can be found that enable plants to germinate and grow better than wild type plants under sudden exposure to low temperatures.

Eleven seedlings from T2 seeds of each transgenic line plus one control line were plated together on a plate containing ½ X Gamborg Salts with 0.8 Phytagel™, 1% Phytagel, and 0.3% Sucrose. Plates were then oriented horizontally and stratified for three days at 4°C. At day three, plates were removed from stratification and exposed to standard conditions (16 hr photoperiod, 22°C at day and 20°C at night) until day 8. At day eight, plates were removed from standard conditions and exposed to cold shock conditions (24 hr photoperiod, 8°C at both day and night) until the final day of the assay, i.e. day 28. Rosette areas were measured at day 8 and day 28, which were analyzed as quantitative responses according to example 1M. Table 8 provides a list of recombinant nucleotides that improve cold shock stress tolerance in plants.

Table 8

| Pep SEQ ID | Construct_id | Orientation | rosette area at day 8 | | | rosette area at day 28 | | | difference in rosette area between day 28 and day 8 | | |
|---|---|---|---|---|---|---|---|---|---|---|---|
| | | | Delta | p-value | c | delta | p-value | c | delta | p-value | c |
| 209 | 13222 | SENSE | -0.562 | 0.978 | / | 0.396 | 0.074 | T | 0.581 | 0.058 | T |
| 216 | 14837 | SENSE | -0.08 | 0.661 | / | 1.007 | 0.002 | S | 1.154 | 0.002 | S |
| 212 | 14841 | SENSE | 0.662 | 0.023 | S | 1.026 | 0.001 | S | 1.088 | 0.004 | S |
| 231 | 19814 | SENSE | 0.38 | 0.008 | S | 0.328 | 0.027 | S | 0.55 | 0.01 | S |
| 234 | 71072 | SENSE | -0.054 | 0.58 | / | 0.736 | 0.005 | S | 0.662 | 0.027 | S |
| 224 | 71148 | SENSE | -0.43 | 0.899 | / | 0.189 | 0.14 | T | 0.643 | 0.02 | S |
| 217 | 71253 | SENSE | 0.318 | 0.004 | S | 0.442 | 0.019 | S | 0.54 | 0.009 | S |
| 251 | 71689 | SENSE | -0.566 | 0.907 | / | 0.62 | 0.01 | S | 0.879 | 0.043 | S |
| 312 | 73513 | SENSE | 0.48 | 0.009 | S | 0.61 | 0.022 | S | 0.439 | 0.074 | T |
| 314 | 73527 | SENSE | 0.499 | 0.034 | S | 0.9 | 0.001 | S | 1.087 | 0.001 | s |
| 321 | 74115 | SENSE | 0.254 | 0.17 | T | 0.896 | 0.002 | S | 1.029 | 0.002 | S |
| 324 | 74165 | SENSE | 0.142 | 0.28 | / | 0.293 | 0.115 | T | 0.61 | 0.018 | S |
| 286 | 74345 | SENSE | -0.109 | 0.746 | / | 0.819 | 0.004 | S | 1.148 | 0.009 | S |
| 333 | 74418 | SENSE | 0.421 | 0.002 | S | 1.012 | 0.003 | S | 0.565 | 0.038 | S |
| 337 | 74528 | SENSE | 0.395 | 0.004 | S | 0.544 | 0.018 | S | 0.725 | 0.009 | S |
| 294 | 74618 | SENSE | 0.274 | 0.003 | S | 0.781 | 0.011 | S | 0.941 | 0.033 | S |
| 305 | 74685 | SENSE | -0.238 | 0.923 | / | 0.758 | 0.001 | S | 1.076 | 0 | S |
| 360 | 74919 | SENSE | 0.221 | 0.214 | / | 0.358 | 0.053 | T | 0.388 | 0.108 | T |
| 356 | 74940 | SENSE | 0.28 | 0.143 | T | 0.903 | 0.001 | S | 0.952 | 0.002 | S |
| 358 | 74954 | SENSE | 0.197 | 0.176 | T | 0.602 | 0.013 | S | 0.712 | 0.024 | S |
| 364 | 75337 | SENSE | 0.669 | 0.002 | S | 0.695 | 0.002 | S | 0.603 | 0.014 | S |
| 381 | 75456 | SENSE | 0.413 | 0.034 | S | 0.895 | 0.002 | S | 0.998 | 0 | S |

(continued)

| Pep SEQ ID | Construct_id | Orientation | rosette area at day 8 | | | rosette area at day 28 | | | difference in rosette area between day 28 and day 8 | | |
|---|---|---|---|---|---|---|---|---|---|---|---|
| | | | Delta | p-value | c | delta | p-value | c | delta | p-value | c |
| 380 | 75491 | SENSE | 0.324 | 0.047 | S | 0.914 | 0.001 | S | 1.11 | 0.001 | S |

S: represents the transgenic plants showed statistically significant trait improvement as compared to the reference (p<0.05) T: represents the transgenic plants showed a trend of trait improvement compared to the reference with p<0.2

/: represents the transgenic plants didn't show any alteration or had unfavorable change in traits examined compared to the reference in the current dataset.

**Cold germination tolerance screen**

This screen identified genes for recombinant DNA that imparts enhanced cold tolerance as shown in *Arabidopsis* plants transformed with the genes of interests are resistant to cold stress based on their rate of development, root growth and chlorophyll accumulation under low temperature conditions.

T2 seeds were plated and all seedlings used in the experiment were grown at 8°C. Seeds were first surface disinfested using chlorine gas and then seeded on assay plates containing an aqueous solution of 1/2 X Gamborg's B/5 Basal Salt Mixture (Sigma/Aldrich Corp., St. Louis, MO, USA G/5788), 1% Phytagel™ (Sigma-Aldrich, P-8169), and 10 ug/ml glufosinate with the final pH adjusted to 5.8 using KOH. Test plates were held vertically for 28 days at a constant temperature of 8°C, a photoperiod of 16 hr, and average light intensity of approximately 100 umol/m$^2$/s. At 28 days post plating, root length was measured, growth stage was observed, the visual color was assessed, and a whole plate photograph was taken.

The root length at day 28 was analyzed as a quantitative response according to example 1M. The growth stage at day 7 was analyzed as a qualitative response according to example 1L.Table 9 provides a list of recombinant DNA constructs that improve cold stress tolerance in transgenic plants.

Table 9

| Pep SEQ ID | Construct_id | Orientation | Root Length at day 28 | | | Growth Stage at day 28 | | |
|---|---|---|---|---|---|---|---|---|
| | | | delta | p-value | c | RS mean | p-value | c |
| 269 | 10814 | ANTI-SENSE | 0.218 | 0.009 | S | 3.148 | 0.007 | S |
| 208 | 17465 | SENSE | 0.269 | 0.027 | S | 3.246 | 0.004 | S |
| 223 | 17919 | SENSE | 0.366 | 0.002 | S | 4 | 0 | S |
| 346 | 18020 | SENSE | 0.112 | 0.105 | T | 3.016 | 0.014 | S |
| 406 | 18026 | SENSE | 0.407 | 0.002 | S | 2.111 | 0.039 | S |
| 226 | 18844 | SENSE | 0.464 | 0.012 | S | 2.927 | 0.005 | S |
| 225 | 19647 | SENSE | 0.479 | 0.002 | S | 3.129 | 0.008 | S |
| 232 | 19720 | SENSE | 0.295 | 0.003 | S | 4 | 0 | S |
| 229 | 19750 | SENSE | 0.224 | 0.006 | S | 3.161 | 0.006 | S |
| 245 | 19812 | SENSE | 0.261 | 0.002 | S | 3.022 | 0.014 | S |
| 235 | 19949 | SENSE | 0.226 | 0 | S | 3.093 | 0.01 | S |
| 220 | 71546 | SENSE | 0.3 | 0.001 | S | 3.181 | 0.006 | S |
| 274 | 73061 | SENSE | 0.306 | 0.002 | S | 3.447 | 0.001 | S |
| 256 | 73271 | SENSE | 0.062 | 0.209 | / | 4 | 0 | S |
| 257 | 73282 | SENSE | 0.216 | 0.023 | S | 4 | 0 | S |
| 255 | 73342 | SENSE | 0.366 | 0 | S | 4 | 0 | S |
| 292 | 74383 | SENSE | 0.182 | 0.019 | S | 4 | 0 | S |
| 336 | 74504 | SENSE | 0.368 | 0.001 | S | 4 | 0 | S |
| 337 | 74528 | SENSE | 0.202 | 0 | S | 4 | 0 | S |
| 339 | 74541 | SENSE | 0.338 | 0.002 | S | 4 | 0 | S |
| 341 | 74554 | SENSE | 0.239 | 0.001 | S | 4 | 0 | S |
| 342 | 74578 | SENSE | 0.53 | 0 | S | 4 | 0 | S |
| 289 | 74608 | SENSE | 0.261 | 0.003 | S | 4 | 0 | S |
| 351 | 74880 | SENSE | 0.21 | 0.04 | S | 2.977 | 0.017 | S |
| 359 | 74907 | SENSE | 0.253 | 0.004 | S | 1.951 | 0.096 | T |
| 372 | 75463 | SENSE | 0.467 | 0.002 | S | 4 | 0 | S |

(continued)

| Pep SEQ ID | Construct_id | Orientation | Root Length at day 28 | | | Growth Stage at day 28 | | |
|---|---|---|---|---|---|---|---|---|
| | | | delta | p-value | c | RS mean | p-value | c |
| 373 | 75475 | SENSE | 0.307 | 0.009 | S | 3.496 | 0 | S |
| 382 | 75480 | SENSE | 0.268 | 0.012 | S | 2.955 | 0.018 | S |
| 392 | 75554 | SENSE | 0.149 | 0.039 | S | 4 | 0 | S |
| 395 | 75567 | SENSE | 0.236 | 0.003 | S | 4 | 0 | S |
| 347 | 75850 | SENSE | 0.616 | 0 | S | 3.053 | 0.002 | S |
| 348 | 75861 | SENSE | 0.272 | 0.001 | S | 4 | 0 | S |
| 349 | 75875 | SENSE | 0.138 | 0.066 | T | 4 | 0 | S |
| 403 | 75991 | SENSE | 0.038 | 0.296 | / | 2.667 | 0.051 | T |

S: represents the transgenic plants showed statistically significant trait improvement as compared to the reference ($p < 0.05$)

T: represents the transgenic plants showed a trend of trait improvement as compared to the reference with $p < 0.2$

/: represents the transgenic plants didn't show any alteration or had unfavorable change in traits examined compared to the reference in the current dataset

**Shade tolerance screen**

Plants undergo a characteristic morphological response in shade that includes the elongation of the petiole, a change in the leaf angle, and a reduction in chlorophyll content. While these changes may confer a competitive advantage to individuals, in a monoculture the shade avoidance response is thought to reduce the overall biomass of the population. Thus, genetic alterations that prevent the shade avoidance response may be associated with higher yields. Genes that favor growth under low light conditions may also promote yield, as inadequate light levels frequently limit yield. This screen identified genes for recombinant DNA that imparts enhanced shade tolerance in *Arabidopsis* plants that show an attenuated shade avoidance response and/or grow better than control plants under low light intensity. Of particular interest, we were looking for plants that didn't extend their petiole length, had an increase in seedling weight relative to the reference and had leaves that were more close to parallel with the plate surface.

T2 seeds were plated on glufosinate selection plates with ½ MS medium. Seeds were sown on 1/2 X MS salts, 1% Phytagel, 10 ug/ml BASTA. Plants were grown on vertical plates at a temperature of 22°C at day, 20°C at night and under low light (approximately 30 uE/m$^2$/s, far/red ratio (655/665/725/735) ~0.35 using PLAQ lights with GAM color filter #680). Twenty-three days after seedlings were sown, measurements were recorded including seedling status, number of rosette leaves, status of flower bud, petiole leaf angle, petiole length, and pooled fresh weights. A digital image of the whole plate was taken on the measurement day. Seedling weight and petiole length were analyzed as quantitative responses according to example 1M. The number of rosette leaves, flowering bud formation and leaf angel were analyzed as qualitative responses according to example 1L.

Table 10 provides a list of recombinant DNA constructs that improve shade tolerance in plants

Table 10

| Pep SEQ ID | Construct_id | Orientation | Petiole length at day 23 | | | seedling weight day 23 | | | Leaf angle at day 23 | | | Number of rosette leaves at day 23 | | |
|---|---|---|---|---|---|---|---|---|---|---|---|---|---|---|
| | | | delta | p-value | c | delta | p-value | c | RS mean | p-value | c | RS mean | p-value | c |
| 407 | 11810 | ANTI-SENSE | -0.138 | 0.03 | S | 0.158 | 0.245 | / | 0.246 | 0.296 | / | -0.1 | 0.822 | / |
| 267 | 16014 | SENSE | -0.267 | 0.012 | S | -1.129 | 0.015 | / | 0.042 | 0.425 | / | 0.773 | 0.209 | / |
| 249 | 71571 | SENSE | -0.548 | 0.005 | S | -0.212 | 0.177 | / | 0.325 | 0.224 | / | 0.611 | 0.22 | / |
| 273 | 72984 | SENSE | -0.219 | 0.064 | T | -0.126 | 0.178 | / | 0.12 | 0.315 | / | 0.476 | 0.28 | / |
| 280 | 73044 | SENSE | -0.722 | 0.083 | T | -0.572 | 0.173 | / | -0.032 | 1 | / | -1.35 | 0.992 | / |
| 307 | 73476 | SENSE | -0.01 | 0.905 | / | 0.103 | 0.354 | / | 0.739 | 0.177 | T | 1.896 | 0.053 | T |
| 311 | 73482 | SENSE | -0.394 | 0.107 | T | -0.095 | 0.611 | / | 0.045 | 0.375 | / | 0.252 | 0.376 | / |
| 306 | 73487 | SENSE | 0.064 | 0.457 | / | -0.131 | 0.583 | / | 1.656 | 0.051 | T | 1.131 | 0.15 | T |
| 261 | 74306 | SENSE | -1.107 | 0.13 | T | -1.924 | 0.116 | / | 0.291 | 0.261 | / | -0.103 | 0.591 | / |
| 282 | 74327 | SENSE | 0.029 | 0.777 | / | 0.105 | 0.762 | / | 0.666 | 0.12 | T | 1.252 | 0.116 | T |
| 330 | 74476 | SENSE | -0.092 | 0.072 | T | -0.613 | 0.107 | / | 0.271 | 0.246 | / | -0.026 | 0.511 | / |

S: represents the transgenic plants showed statistically significant trait improvement as compared to the reference (p<0.05) T: represents the transgenic plants showed a trend of trait improvement as compared to the reference with p<0.2

/: represents the transgenic plants didn't show any alteration or had unfavorable change in traits examined compared to the reference in the current dataset.

**Early plant growth and development screen**

This screen identified genes for recombinant DNA that imparts enhanced early plant growth and development, a surrogate for increased yield, as shown in *Arabidopsis* plants examined in a plate based phenotypic analysis platform for the rapid detection of phenotypes that are evident during the first two weeks of growth. In this screen, we were looking for genes that confer advantages in the processes of germination, seedling vigor, root growth and root morphology under non-stressed growth conditions to plants. The transgenic plants with advantages in seedling growth and development were determined by the seedling weight and root length at day 14 after seed planting.

T2 seeds were plated on glufosinate selection plates and grown under standard conditions (~100 uE/m$^2$/s, 16 h photoperiod, 22°C at day, 20°C at night). Seeds were stratified for 3 days at 4°C. Seedlings were grown vertically (at a temperature of 22°C at day 20°C at night). Observations were taken on day 10 and day 14. Both seedling weight and root length at day 14 were analyzed as quantitative responses according to example 1M.

Table 11 provides a list recombinant DNA constructs that improve early plant growth and development.

Table 11

| Pep SEQ ID | Construct_id | Orientation | Root Length at day 10 | | | Root Length at day 14 | | | Seedling Weight | | |
|---|---|---|---|---|---|---|---|---|---|---|---|
| | | | delta | p-value | c | delta | p-value | c | delta | p-value | c |
| 213 | 13478 | ANTI-SENSE | 0.242 | 0.062 | T | 0.159 | 0.075 | T | 0.603 | 0.013 | S |
| 227 | 19525 | SENSE | 0.249 | 0.183 | T | 0.245 | 0.001 | S | 0.385 | 0.062 | T |
| 221 | 70109 | SENSE | 0.282 | 0.001 | S | 0.23 | 0.001 | S | 0.564 | 0 | S |
| 248 | 71332 | SENSE | 0.071 | 0.548 | / | 0.093 | 0.17 | T | 0.419 | 0.009 | S |
| 220 | 71546 | SENSE | 0.307 | 0.057 | T | 0.231 | 0.013 | S | 0.561 | 0.006 | S |
| 246 | 71556 | SENSE | 0.408 | 0.008 | S | 0.291 | 0.011 | S | 0.328 | 0.263 | / |
| 408 | 72418 | SENSE | 0.144 | 0.063 | T | 0.17 | 0 | S | 0.46 | 0.006 | S |
| 253 | 72636 | SENSE | 0.486 | 0.026 | S | 0.339 | 0 | S | 0.705 | 0.006 | S |
| 279 | 72957 | SENSE | 0.228 | 0.053 | T | 0.158 | 0.089 | T | 0.371 | 0.08 | T |
| 309 | 73418 | SENSE | 0.242 | 0.082 | T | 0.148 | 0.089 | T | 0.212 | 0.085 | T |
| 316 | 73530 | SENSE | 0.239 | 0.023 | S | 0.17 | 0.003 | S | 0.442 | 0.001 | S |
| 313 | 73550 | SENSE | 0.181 | 0.046 | S | 0.113 | 0.016 | S | 0.304 | 0.045 | S |
| 259 | 73913 | SENSE | 0.243 | 0.069 | T | 0.091 | 0.26 | / | 0.452 | 0.015 | S |
| 325 | 74106 | SENSE | 0.257 | 0.003 | S | 0.192 | 0.015 | S | 0.34 | 0.052 | T |
| 318 | 74125 | SENSE | 0.104 | 0.549 | / | 0.139 | 0.048 | S | 0.524 | 0.002 | S |
| 320 | 74126 | SENSE | 0.149 | 0.082 | T | 0.065 | 0.284 | / | 0.354 | 0.127 | T |
| 322 | 74127 | SENSE | 0.381 | 0 | S | 0.212 | 0.006 | S | 0.63 | 0.002 | S |
| 323 | 74128 | SENSE | 0.213 | 0.091 | T | 0.12 | 0.161 | T | 0.541 | 0.002 | S |
| 326 | 74130 | SENSE | 0.14 | 0.002 | S | 0.144 | 0.001 | S | 0.003 | 0.955 | / |
| 327 | 74132 | SENSE | 0.134 | 0.275 | / | 0.144 | 0.049 | S | 0.336 | 0.025 | S |
| 328 | 74144 | SENSE | 0.137 | 0.217 | / | 0.134 | 0.065 | T | 0.492 | 0.012 | S |
| 319 | 74161 | SENSE | 0.205 | 0.02 | S | 0.131 | 0.018 | S | 0.459 | 0.035 | S |
| 263 | 74237 | SENSE | 0.253 | 0 | S | 0.189 | 0.002 | S | 0.408 | 0.013 | S |
| 265 | 74256 | SENSE | 0.28 | 0.04 | S | 0.202 | 0.012 | S | 0.461 | 0.034 | S |
| 260 | 74305 | SENSE | 0.184 | 0.063 | T | 0.131 | 0.026 | S | 0.257 | 0.121 | T |
| 281 | 74323 | SENSE | 0.13 | 0.08 | T | 0.067 | 0.054 | T | 0.426 | 0.005 | S |

(continued)

| Pep SEQ ID | Construct_id | Orientation | Root Length at day 10 | | | Root Length at day 14 | | | Seedling Weight | | |
|---|---|---|---|---|---|---|---|---|---|---|---|
| | | | delta | p-value | c | delta | p-value | c | delta | p-value | c |
| 285 | 74341 | SENSE | 0.185 | 0.044 | S | 0.07 | 0.106 | T | 0.144 | 0.191 | T |
| 293 | 74374 | SENSE | 0.103 | 0.187 | T | 0.171 | 0.008 | S | 0.518 | 0.023 | S |
| 302 | 74385 | SENSE | 0.051 | 0.461 | / | 0.043 | 0.693 | / | 0.214 | 0.098 | T |
| 303 | 74386 | SENSE | 0.211 | 0.12 | T | 0.136 | 0.092 | T | 0.081 | 0.616 | / |
| 304 | 74387 | SENSE | 0.11 | 0.528 | / | 0.17 | 0.052 | T | 0.378 | 0.027 | S |
| 350 | 74548 | SENSE | 0.169 | 0.041 | S | 0.09 | 0.046 | S | 0.336 | 0.08 | T |
| 288 | 74604 | SENSE | 0.157 | 0.231 | / | 0.181 | 0.014 | S | 0.586 | 0.001 | S |
| 290 | 74609 | SENSE | 0.24 | 0.029 | S | 0.102 | 0.227 | / | 0.457 | 0.014 | S |
| 287 | 74616 | SENSE | 0.192 | 0.047 | S | 0.137 | 0.125 | T | 0.286 | 0.204 | / |
| 295 | 74619 | SENSE | 0.181 | 0.415 | / | 0.153 | 0.149 | T | 0.448 | 0.056 | T |
| 296 | 74622 | SENSE | 0.083 | 0.481 | / | 0.071 | 0.331 | / | 0.311 | 0.076 | T |
| 298 | 74631 | SENSE | 0.203 | 0.02 | S | 0.115 | 0.019 | S | 0.049 | 0.633 | / |
| 353 | 74915 | SENSE | 0.202 | 0.004 | S | 0.136 | 0.027 | S | 0.409 | 0.012 | S |
| 354 | 74927 | SENSE | 0.103 | 0.148 | T | 0.117 | 0.021 | S | 0.319 | 0.056 | T |
| 356 | 74940 | SENSE | 0.127 | 0.001 | S | 0.117 | 0.049 | S | 0.344 | 0.028 | S |
| 371 | 75312 | SENSE | 0.191 | 0.055 | T | 0.155 | 0.006 | S | 0.526 | 0.001 | S |
| 365 | 75339 | SENSE | 0.168 | 0.029 | S | 0.031 | 0.704 | / | 0.21 | 0.208 | / |
| 374 | 75440 | SENSE | 0.15 | 0.037 | S | 0.059 | 0.566 | / | 0.039 | 0.923 | / |
| 372 | 75463 | SENSE | 0.392 | 0.006 | S | 0.284 | 0.011 | S | 0.432 | 0.15 | T |
| 375 | 75488 | SENSE | 0.101 | 0.289 | / | 0.083 | 0.113 | T | 0.444 | 0.003 | S |

S: represents the transgenic plants showed statistically significant trait improvement as compared to the reference (p<0.05)
T: represents the transgenic plants showed a trend of trait improvement as compared to the reference with p<0.2
/: represents the transgenic plants didn't show any alteration or had unfavorable change in traits examined compared to the reference in the current dataset

**Late plant growth and development screen**

This screen identified genes for recombinant DNA that imparts enhanced late plant growth and development, a surrogate for increased yield, as shown in *Arabidopsis* plants examined in a soil based phenotypic platform to identify genes that confer advantages in the processes of leaf development, flowering production and seed maturity to plants.

*Arabidopsis* plants were grown on a commercial potting mixture (Metro Mix 360, Scotts Co., Marysville, OH) consisting of 30-40% medium grade horticultural vermiculite, 35-55% sphagnum peat moss, 10-20% processed bark ash, 1-15% pine bark and a starter nutrient charge. Soil was supplemented with Osmocote time-release fertilizer at a rate of 30 mg/ft$^3$. T2 seeds were imbibed in 1% agarose solution for 3 days at 4 °C and then sown at a density of ~5 per 2 ½" pot. Thirty-two pots were ordered in a 4 by 8 grid in standard greenhouse flat. Plants were grown in environmentally controlled rooms under a 16 h day length with an average light intensity of ~200 μmoles/m$^2$/s. Day and night temperature set points were 22 °C and 20 °C, respectively. Humidity was maintained at 65%. Plants were watered by sub-irrigation every two days on average until mid-flowering, at which point the plants were watered daily until flowering was complete.

Application of the herbicide glufosinate was performed to select T2 individuals containing the target transgene. A single application of glufosinate was applied when the first true leaves were visible. Each pot was thinned to leave a single glufosinate-resistant seedling ~3 days after the selection was applied.

The rosette radius was measured at day 25. The silique length was measured at day 40. The plant parts were harvested at day 49 for dry weight measurements if flowering production was stopped. Otherwise, the dry weights of rosette and silique were carried out at day 53. The seeds were harvested at day 58. All measurements were analyzed as quantitative responses according to example 1M.

Table 12 provides a list of recombinant DNA constructs that improve late plant growth and development.

Table 12

| Pep SEQ ID | Construct_id | Rosette Dry Weight | | | Rosette Radius | | | Seed Dry Weight | | | Silique Dry Weight | | | Silique Length | | |
|---|---|---|---|---|---|---|---|---|---|---|---|---|---|---|---|---|
| | | delta | p-value | c | delta | p-value | c | Delta | p-value | c | delta | p-value | c | delta | p-value | c |
| 213 | 13478 | 0.054 | 0.365 | / | 0.217 | 0.013 | S | 0.052 | 0.347 | / | 0.293 | 0.084 | T | 0.079 | 0.021 | S |
| 241 | 19787 | 0.233 | 0.052 | T | 0.067 | 0.022 | S | 0.66 | 0.019 | S | -0.02 | 0.75 | / | 0.06 | 0.006 | S |
| 268 | 72751 | 0.287 | 0.012 | S | -0.141 | 0.714 | / | -0.991 | 0.973 | / | 0.094 | 0.099 | T | -0.024 | 0.675 | / |

S: represents the transgenic plants showed statistically significant trait improvement as compared to the reference (p<0.05) T: represents the transgenic plants showed a trend of trait improvement compared to the reference with p<0.2

/: represents the transgenic plants didn't show any alteration or had unfavorable change in traits examined compared to the reference in the current dataset

**Limited nitrogen tolerance screen**

Under low nitrogen conditions, *Arabidopsis* seedlings become chlorotic and have less biomass. This screen identified genes for recombinant DNA that imparts enhanced nitrogen use efficiency as shown in *Arabidopsis* plants transformed with the gene of interest that are altered in their ability to accumulate biomass and/or retain chlorophyll under low nitrogen condition.

T2 seeds were plated on glufosinate selection plates containing 0.5x N-Free Hoagland's T 0.1 mM $NH_4NO_3$ T 0.1 % sucrose T1% phytagel media and grown under standard light and temperature conditions. At 12 days of growth, plants were scored for seedling status (i.e. viable or non-viable) and root length. After 21 days of growth, plants were scored for BASTA resistance, visual color, seedling weight, number of green leaves, number of rosette leaves, root length and formation of flowering buds. A photograph of each plant was also taken at this time point.

The seedling weight and root length were analyzed as quantitative responses according to example 1M. The number green leaves, the number of rosette leaves and the flowerbud formation were analyzed as qualitative responses according to example 1L. The leaf color raw data were collected on each plant as the percentages of five color elements (Green, DarkGreen, LightGreen, RedPurple, YellowChlorotic) using a computer imaging system. A statistical logistic regression model was developed to predict an overall value based on five colors for each plant.

Table 13 provides a list of recombinant DNA constructs that improve low nitrogen availability tolerance in plants.

Table 13

| Pep SEQ ID | Construct id | Number of green leaves | | | leaf color | | | Root Length | | | Rosette Weight | | |
|---|---|---|---|---|---|---|---|---|---|---|---|---|---|
| | | RS mean | p-value | c | delta | p-value | c | RS mean | p-value | c | delta | p-value | c |
| 334 | 10150 | 1.496 | 0.007 | S | 0.974 | 0.007 | S | -0.285 | 0.022 | / | -0.024 | 0.572 | / |
| 222 | 10335 | 0.993 | 0.014 | S | 0.953 | 0.01 | S | -0.359 | 0 | / | -0.057 | 0.122 | / |
| 277 | 11145 | 0.368 | 0.41 | / | 1.059 | 0.004 | S | -0.141 | 0.211 | / | -0.086 | 0.143 | / |
| 361 | 11735 | 0.522 | 0.554 | / | 1.132 | 0.003 | S | -0.134 | 0.147 | / | 0.082 | 0.001 | S |
| 206 | 12030 | 0.57 | 0.321 | / | 0.944 | 0.027 | S | -0.28 | 0.005 | / | -0.031 | 0.545 | / |
| 368 | 12189 | 0.134 | 0.791 | / | 0.76 | 0.034 | S | -0.147 | 0.024 | / | -0.082 | 0.099 | / |
| 308 | 73465 | 0.591 | 0.195 | T | 0.586 | 0.061 | T | -0.03 | 0.631 | / | -0.04 | 0.249 | / |
| 328 | 74144 | 0.845 | 0.069 | T | 1.021 | 0.003 | S | 0.181 | 0.04 | S | 0.028 | 0.481 | / |
| 331 | 74417 | 1.051 | 0.021 | S | 1.4 | 0.001 | s | -0.155 | 0.019 | / | -0.071 | 0.16 | / |
| 338 | 74588 | 0.484 | 0.31 | / | 1.059 | 0.01 | S | -0.101 | 0.167 | / | -0.051 | 0.608 | / |
| 369 | 75321 | 0.607 | 0.041 | S | 0.317 | 0.423 | / | -0.095 | 0.238 | / | 0.056 | 0.021 | S |
| 377 | 75419 | -0.779 | 0.198 | / | 0.635 | 0.019 | S | -0.116 | 0.024 | / | 0.009 | 0.795 | / |
| 385 | 75424 | -0.354 | 0.537 | / | 1.489 | 0 | S | -0.245 | 0.003 | / | -0.062 | 0.165 | / |
| 391 | 75506 | 0.969 | 0 | S | 0.588 | 0.049 | S | 0.081 | 0.108 | T | -0.026 | 0.545 | / |
| 388 | 75528 | -0.026 | 0.932 | / | 0.229 | 0.594 | / | -0.042 | 0.534 | / | 0.111 | 0.058 | T |
| 396 | 75544 | 0.197 | 0.369 | / | 1.033 | 0.006 | S | -0.283 | 0.034 | / | -0.134 | 0.021 | / |
| 398 | 75546 | 0.577 | 0.001 | S | 1.108 | 0.002 | S | -0.321 | 0.006 | / | 0.01 | 0.765 | / |
| 390 | 75553 | -0.182 | 0.643 | / | 0.425 | 0.058 | T | -0.104 | 0.12 | / | -0.096 | 0.104 | / |
| 397 | 75556 | 0.766 | 0.01 | S | -0.544 | 0.179 | / | 0 | 1 | / | 0.271 | 0 | S |
| 399 | 75558 | 0.343 | 0.406 | / | 0.861 | 0.008 | S | -0.102 | 0.035 | / | -0.106 | 0.003 | / |
| 387 | 75575 | 0.087 | 0.83 | / | 1.147 | 0.001 | S | -0.117 | 0.093 | / | -0.03 | 0.325 | / |
| 401 | 75583 | -0.621 | 0.399 | / | -0.166 | 0.595 | / | -0.01 | 0.906 | / | 0.162 | 0.002 | S |
| 344 | 75834 | 0.425 | 0.038 | S | 0.589 | 0.03 | S | -0.067 | 0.343 | / | 0.028 | 0.429 | / |

| Pep SEQ ID | Construct id | Number of green leaves | | | leaf color | | | Root Length | | | Rosette Weight | | |
|---|---|---|---|---|---|---|---|---|---|---|---|---|---|
| | | RS mean | p-value | c | delta | p-value | c | RS mean | p-value | c | delta | p-value | c |
| 345 | 75835 | 0.486 | 0.266 | / | 0.228 | 0.499 | / | -0.16 | 0.026 | / | 0.159 | 0 | S |

S: represents the transgenic plants showed statistically significant trait improvement as compared to the reference (p<0.05) T: represents the transgenic plants showed a trend of trait improvement compared than the reference with p<0.2

/: represents the transgenic plants didn't show any alteration or had unfavorable change in traits examined compared to the reference in the current dataset

EP 3 059 306 A1

**Statistic analysis for qualitative responses**

Table 14 provides a list of responses that were analyzed as qualitative responses

Table 14

| response | screen | categories (success vs. failure) |
|---|---|---|
| wilting response Risk Score | Soil drought tolerance screen | non-wilted vs. wilted |
| growth stage at day 14 | heat stress tolerance screen | 50% of plants reach stage1.03 vs. not |
| growth stage at day 14 | salt stress tolerance screen | 50% of plants reach stage1.03 vs. not |
| growth stage at day 14 | PEG induced osmotic stress tolerance screen | 50% of plants reach stage1.03 vs. not |
| growth stage at day 7 | cold germination tolerance screen | 50% of plants reach stage 0.5 vs. not |
| number of rosette leaves at day 23 | Shade tolerance screen | 5 leaves appeared vs. not |
| flower bud formation at day 23 | Shade tolerance screen | flower buds appear vs. not |
| leaf angle at day 23 | Shade tolerance screen | >60 degree vs. <60 degree |
| number of green leaves at day 21 | limited nitrogen tolerance screen | 6 or 7 leaves appeared vs. not |
| number of rosette leaves at day 21 | limited nitrogen tolerance screen | 6 or 7 leaves appeared vs. not |
| Flower bud formation at day 21 | limited nitrogen tolerance screen | flower buds appear vs. not |

Plants were grouped into transgenic and reference groups and were scored as success or failure according to Table 16. First, the risk (R) was calculated, which is the proportion of plants that were scored as of failure plants within the group. Then the relative risk (RR) was calculated as the ratio of R (transgenic) to R (reference). Risk score (RS) was calculated as $-\log_2 RR$. Subsequently the risk scores from multiple events for each transgene of interest were evaluated for statistical significance by t-test using S-PLUS statistical software (S-PLUS 6, Guide to statistics, Insightful, Seattle, WA, USA). RS with a value greater than 0 indicates that the transgenic plants perform better than the reference. RS with a value less than 0 indicates that the transgenic plants perform worse than the reference. The RS with a value equal to 0 indicates that the performance of the transgenic plants and the reference don't show any difference.

**Statistic analysis for quantitative responses**

Table 15 provides a list of responses that were analyzed as quantitative responses.

Table 15

| response | screen |
|---|---|
| seed yield | Soil drought stress tolerance screen |
| seedling weight at day 14 | heat stress tolerance screen |
| root length at day 14 | heat stress tolerance screen |
| seedling weight at day 14 | salt stress tolerance screen |
| root length at day 14 | salt stress tolerance screen |
| root length at day 11 | salt stress tolerance screen |
| seedling weight at day 14 | PEG induced osmotic stress tolerance screen |
| root length at day 11 | PEG induced osmotic stress tolerance screen |
| root length at day 14 | PEG induced osmotic stress tolerance screen |
| rosette area at day 8 | cold shock tolerance screen |

(continued)

| response | screen |
|---|---|
| rosette area at day28 | cold shock tolerance screen |
| difference in rosette area from day 8 to day 28 | cold shock tolerance screen |
| root length at day 28 | cold germination tolerance screen |
| seedling weight at day 23 | Shade tolerance screen |
| petiole length at day 23 | Shade tolerance screen |
| root length at day 14 | Early plant growth and development screen |
| Seedling weight at day14 | Early plant growth and development screen |
| Rosette dry weight at day 53 | Late plant growth and development screen |
| rosette radius at day 25 | Late plant growth and development screen |
| seed dry weight at day 58 | Late plant growth and development screen |
| silique dry weight at day 53 | Late plant growth and development screen |
| silique length at day 40 | Late plant growth and development screen |
| Seedling weight at day 21 | Limited nitrogen tolerance screen |
| Root length at day 21 | Limited nitrogen tolerance screen |

The measurements (M) of each plant were transformed by $\log_2$ calculation. The Delta was calculated as $\log_2 M$(transgenic)- $\log_2 M$(reference). Subsequently the mean delta from multiple events of the transgene of interest was evaluated for statistical significance by t-test using S-PLUS statistical software (S-PLUS 6, Guide to statistics, Insightful, Seattle, WA, USA). The Delta with a value greater than 0 indicates that the transgenic plants perform better than the reference. The Delta with a value less than 0 indicates that the transgenic plants perform worse than the reference. The Delta with a value equal to 0 indicates that the performance of the transgenic plants and the reference don't show any difference.

**Example 2**

This example illustrates the identification of homologs of the cognate proteins of the genes identified as imparting an enhanced trait.

A BLAST searchable "All Protein Database" was constructed of known protein sequences using a proprietary sequence database and the National Center for Biotechnology Information (NCBI) non-redundant amino acid database (nr.aa). For each organism from which a DNA sequence provided herein was obtained, an "Organism Protein Database" was constructed of known protein sequences of the organism; the Organism Protein Database is a subset of the All Protein Database based on the NCBI taxonomy ID for the organism.

The All Protein Database was queried using amino acid sequence of cognate protein for gene DNA used in trait-improving recombinant DNA, i.e. sequences of SEQ ID NO: 205 through SEQ ID NO: 408 using "blastp" with E-value cutoff of 1e-8. Up to 1000 top hits were kept, and separated by organism names. For each organism other than that of the query sequence, a list was kept for hits from the query organism itself with a more significant E-value than the best hit of the organism. The list contains likely duplicated genes, and is referred to as the Core List. Another list was kept for all the hits from each organism, sorted by E-value, and referred to as the Hit List.

The Organism Protein Database was queried using amino acid sequences of SEQ ID NO: 205 through SEQ ID NO: 408 using "blastp" with E-value cutoff of 1e-4. Up to 1000 top hits were kept. A BLAST searchable database was constructed based on these hits, and is referred to as "SubDB". SubDB was queried with each sequence in the Hit List using "blastp" with E-value cutoff of 1e-8. The hit with the best E-value was compared with the Core List from the corresponding organism. The hit is deemed a likely ortholog if it belongs to the Core List, otherwise it is deemed not a likely ortholog and there is no further search of sequences in the Hit List for the same organism. Likely orthologs from a large number of distinct organisms were identified and are reported by amino acid sequences of SEQ ID NO: 409 to SEQ ID NO: 19247. These orthologs are reported in Tables 2 as homo logs to the proteins cognate to genes used in trait-improving recombinant DNA.

**Table 2**

SEQ ID NO:  homolog SEQ ID NOs

| | | | | | | | | | | | |
|---|---|---|---|---|---|---|---|---|---|---|---|
| 206: 9461 | 19072 | 14856 | 5315 | 7726 | 11678 | 8466 | 4567 | 9541 | 4039 | 2736 | 826 |
| 4434 | 18755 | 7163 | 19021 | 16621 | 18594 | | | | | | |
| 207:11732 | 5088 | 14017 | 14658 | 8659 | 17828 | 14150 | 11838 | 7420 | 577 | 13978 | 3089 |
| 9378 | 8177 | 5473 | 4151 | 15455 | 3299 | 3397 | 17568 | 12473 | 2946 | 17428 | 3126 |
| 15408 | 3997 | 18806 | 10067 | 3246 | 19105 | | | | | | |
| 208:11715 | 12490 | 18003 | 15562 | 5946 | 18565 | 5550 | 13362 | 1866 | 4207 | 6966 | 6785 |
| 16958 | 17740 | 12495 | 9774 | 3632 | 7059 | 937 | 10643 | 12198 | 6865 | 13806 | 1023 |
| 12708 | 14552 | 16746 | 18201 | 16712 | 7735 | | | | | | |
| 209: 3105 | 15418 | 1368 | 15360 | 5654 | 7902 | 3038 | 16108 | 14416 | 16444 | 10078 | 17851 |
| 12848 | 15183 | 12997 | 18090 | 18617 | 1312 | 16630 | 4080 | 4082 | 7689 | 19205 | 17262 |
| 13305 | 5780 | 10544 | 14798 | 16363 | 16166 | 17737 | | | | | |
| 210:17539 | 13894 | 15187 | 11374 | 17968 | 9384 | 7645 | 13991 | 5852 | | | |
| 211:16101 | 13052 | 9940 | 10298 | 9899 | 15964 | 10104 | 18651 | 6853 | 12619 | 1551 | 4764 |
| 684 | 13219 | 15864 | 9975 | 3261 | 4237 | 2710 | 18739 | 15477 | 7248 | 582 | 15025 |
| 212:11577 | 10930 | 12130 | 13904 | 13619 | 10462 | 9686 | 10796 | 9854 | 12277 | 14708 | 4822 |
| 437 | 6953 | 7206 | 9936 | 2948 | 7987 | 13119 | 2409 | | | | |
| 213:11281 | 4744 | 9418 | 8607 | 3777 | 4299 | 2607 | 2319 | 7251 | 7279 | 9577 | 14778 |
| 18625 | 6286 | 4491 | 14417 | 1831 | 7825 | 8703 | 3214 | 17914 | 10229 | 11951 | 17625 |
| 970 | 966 | 7114 | 17802 | 17749 | 17813 | 17788 | 17783 | 17787 | 17765 | 17808 | 17806 |
| 17764 | 17726 | 17746 | 17810 | 17834 | 17732 | 17745 | 17767 | 17728 | 17730 | 17744 | 17747 |
| 17781 | 2457 | 15330 | 15328 | 7187 | 1588 | 17923 | 1156 | 5341 | 16738 | 11147 | 11237 |
| 10534 | 13932 | 6444 | 2913 | 14896 | 13736 | 10170 | 11594 | 17581 | 1429 | 12223 | 7798 |
| 16703 | 10946 | 10966 | 10944 | 10961 | 10934 | 11706 | 11679 | 11677 | 11704 | 3305 | 1863 |
| 17474 | 15572 | 4795 | 6459 | 794 | 8522 | 15354 | 13706 | 8632 | 5186 | 8156 | 7182 |
| 1057 | 12383 | 18370 | 18371 | 12218 | 14763 | 6085 | 9619 | 6084 | 3613 | 11617 | 15462 |
| 4013 | 14623 | 13880 | 3030 | 1260 | 16762 | 11244 | 3082 | 9189 | 11858 | 14221 | 19035 |
| 2439 | 4014 | 1840 | 8134 | 16791 | 14100 | 8244 | 8347 | 15753 | 6777 | 10859 | 2830 |
| 16405 | 8205 | 13954 | 11632 | 18494 | 11891 | 2663 | 1366 | 18247 | 15522 | 10282 | 14126 |
| 10940 | 8802 | 12556 | 997 | 3391 | 4295 | 7548 | 5823 | 17340 | 5932 | 2459 | 10562 |
| 14935 | 17104 | 15135 | 2045 | 2256 | 14248 | 15748 | 638 | 11436 | 10293 | 14019 | 12831 |
| 7142 | 6143 | 5949 | 14114 | 17359 | 10556 | 13121 | 2699 | 12459 | 3820 | 10865 | 15441 |
| 5257 | 8413 | 14080 | 10255 | 12573 | 772 | 6606 | 3642 | 8804 | 3457 | 1348 | 16022 |
| 7874 | 5815 | 15304 | 598 | 3258 | 3259 | 1401 | 4197 | 4837 | 5770 | 9990 | 11860 |
| 5998 | 13653 | 19240 | 18528 | 798 | 5714 | 7362 | 5197 | 13400 | 6454 | 4954 | 587 |
| 15889 | 1391 | 1208 | 2577 | 2564 | 4704 | 4774 | 5289 | 11843 | 15452 | 3750 | 18789 |
| 950 | 13589 | 16248 | 18822 | 15416 | 9549 | 8091 | 2839 | 2840 | 16123 | 8406 | 8410 |
| 15495 | 1886 | 15489 | 18634 | 15405 | 8526 | 8546 | 8530 | 11309 | 10415 | 12157 | 12164 |
| 12160 | 12156 | 18974 | 9416 | 10400 | 9468 | 6180 | 6183 | 17850 | 11085 | 6361 | 1846 |
| 9141 | 7414 | 12282 | 2648 | 18663 | 16836 | 2696 | 12925 | 19084 | 7529 | 7271 | 9600 |
| 16269 | 8179 | 15909 | 8971 | 14368 | 11123 | 15003 | 7928 | 18412 | 13507 | 8354 | 2167 |
| 8528 | 8545 | 13622 | 11823 | 5877 | 9383 | 18047 | 12323 | 7126 | 13778 | 15775 | 6818 |
| 14349 | 1843 | 1841 | 5467 | 18619 | 2511 | 13117 | 12441 | 18799 | 4847 | 4844 | 4846 |
| 1482 | 1029 | 13661 | 9950 | 7913 | 18555 | 13411 | 12291 | 15118 | 13667 | 11824 | 17679 |
| 15097 | 13368 | 4611 | 15383 | 5294 | 5297 | 5295 | 10809 | 6024 | 13660 | 13664 | 18078 |
| 18063 | 18075 | 18080 | 18064 | 18061 | 2901 | 2896 | 11461 | 9567 | 2623 | 15619 | 13681 |
| 16483 | 19066 | 18430 | 14575 | 17264 | 14824 | 16907 | 3361 | 3539 | 9690 | 9562 | 13782 |
| 14476 | 16298 | 672 | 4789 | 18071 | 16409 | 11810 | 9328 | 15142 | 16658 | 19113 | 15890 |
| 11168 | 14282 | 7337 | 2737 | 18782 | 2977 | 6021 | 6936 | 15780 | 15779 | 14381 | 3119 |
| 8277 | 5647 | 3188 | 5391 | 2197 | 8529 | 16229 | 4706 | 11648 | 18333 | 12351 | 10590 |

(continued)

| SEQ ID NO: | homolog SEQ ID NOs | | | | | | | | | |
|---|---|---|---|---|---|---|---|---|---|---|
| 4964 | 12869 | 12571 | 17461 | 15151 | 2108 | 12256 | 13689 | 6178 | 11145 | 10127 | 14620 |
| 521 | 12494 | 9664 | 13352 | 18819 | 17275 | 16855 | 6094 | 16051 | 1861 | 1543 | 17278 |
| 3184 | 3785 | 7977 | 10869 | 14020 | 1073 | 8390 | 13292 | 3685 | 12532 | 3942 | 3338 |
| 7643 | 5224 | 7941 | 17062 | 4374 | 17281 | 15560 | 18682 | 6397 | 9485 | 11017 | 18260 |
| 11733 | 7788 | 6676 | 7371 | 15931 | 2677 | 1855 | 7550 | 12942 | 16195 | 2434 | 13586 |
| 14216 | 2671 | 18036 | 5925 | 11102 | 2387 | 3968 | 15553 | 12886 | 12211 | 6446 | 13317 |
| 15962 | 2965 | 6866 | 2247 | 2576 | 18624 | 13803 | 8821 | 12537 | 18487 | 2386 | 15498 |
| 10683 | 8191 | 15227 | 10351 | 14762 | 14780 | 14761 | 1568 | 1570 | 1823 | 11984 | 13721 |
| 7268 | 10834 | 14940 | 2764 | 6670 | 1577 | 8050 | 4950 | 17318 | 17533 | 743 | 1273 |
| 13143 | 2621 | 6532 | 6553 | 10775 | 11080 | 10240 | 6827 | 13829 | 14551 | 14376 | 8122 |
| 11203 | 11186 | 9158 | 4943 | 11793 | 4606 | 4451 | 4941 | 4925 | 4421 | 4923 | 4447 |
| 4439 | 13094 | 16831 | 5632 | 16842 | 7972 | 8319 | 4463 | 18291 | 1153 | 5593 | 14256 |
| 15648 | 10411 | 15778 | 3836 | 8646 | 11057 | 10802 | 2547 | 10675 | 10705 | 12481 | 17144 |
| 11933 | 9742 | 760 | 816 | 2393 | 15797 | 15801 | 4227 | 10125 | 15598 | 14171 | 4499 |
| 16812 | 14907 | 3633 | 2579 | 6096 | 11005 | | | | | | |
| 214:17205 | 12417 | 5182 | 11592 | 1218 | 18584 | 9104 | 3562 | 17644 | 15108 | 2456 | 12621 |
| 2781 | 16359 | 2519 | 11488 | 17691 | 5523 | 3592 | 4365 | 17685 | 18564 | 9255 | 3981 |
| 17471 | 14932 | 6896 | 17261 | 14309 | 4464 | | | | | | |
| 215:11565 | 17980 | 9870 | 6731 | 7560 | 8022 | 11032 | 6899 | 11020 | 17043 | 17521 | 13209 |
| 6078 | 3756 | 6314 | 4852 | 7764 | 17958 | 8539 | 3600 | 2654 | 15211 | 8089 | 10817 |
| 882 | 2355 | 12320 | 10576 | 857 | | | | | | | |
| 216: 4548 | 1814 | 1811 | 18872 | 5005 | 8334 | 4062 | 18447 | 18595 | | | |
| 217:10206 | 9379 | 12609 | 7556 | 16810 | 18633 | 7572 | 6772 | 9785 | 18400 | 4394 | 8373 |
| 3586 | 13142 | 13290 | 11170 | 2118 | 4015 | 4419 | 3154 | 4988 | 15178 | 2746 | 16499 |
| 7153 | 17079 | 12443 | 9989 | 7666 | 3147 | 16089 | 8157 | 15468 | 13125 | 13112 | 1739 |
| 16612 | 16627 | 16628 | 7970 | | | | | | | | |
| 218:14011 | 6366 | 12194 | 19166 | 14872 | 8536 | 9025 | 15878 | 5246 | 1466 | 10734 | 18375 |
| 19051 | 3284 | 11851 | 1926 | 16644 | 15376 | 15389 | 19007 | 13011 | 16761 | 14930 | 17632 |
| 17097 | 11908 | 10938 | 10056 | 5151 | 1836 | 696 | 16029 | 14064 | 3132 | 16092 | 16678 |
| 14296 | 5107 | 9978 | 12711 | 18145 | 4922 | | | | | | |
| 219: 1266 | 12114 | 9582 | 1006 | 14040 | 2995 | 2574 | 14277 | 1487 | 12000 | 15871 | 545 |
| 17801 | 6330 | 11820 | 4719 | 4718 | 7544 | 2089 | 6943 | 5642 | 3726 | 15195 | 15394 |
| 15397 | 4250 | 16556 | 6724 | 9453 | 12301 | 8101 | 15949 | 7115 | 16130 | 3091 | 15504 |
| 5479 | 9235 | 18681 | 12554 | 6544 | 9548 | 12244 | 14153 | 18006 | 13786 | 4349 | 944 |
| 15755 | 5484 | 11621 | 9159 | 4302 | 731 | 19190 | 13815 | 4148 | 1580 | 2614 | |
| 220:17643 | 10449 | 11152 | 786 | 6249 | 12066 | 11428 | 11925 | 8370 | 4891 | 10927 | 3165 |
| 2073 | 1089 | 2824 | 12422 | 12197 | 9475 | 18563 | 14844 | 5667 | 5051 | 7973 | 17063 |
| 16714 | 12884 | 8011 | 16641 | 16666 | 6510 | 19040 | 4239 | 14002 | 16784 | 2028 | 10196 |
| 16806 | 16609 | 10112 | 4683 | 7040 | 10524 | 11614 | 15345 | 5811 | 2298 | 3507 | 15078 |
| 14109 | 12786 | 4216 | 13136 | 18305 | 3837 | 5287 | 12384 | 17153 | 5425 | 7719 | 17446 |
| 5024 | 14077 | 1542 | 14745 | 3608 | 6018 | 7127 | 18148 | 18190 | 1256 | 9632 | 13003 |
| 8636 | 4022 | 12857 | 10814 | 3807 | 15717 | 2030 | 10386 | 15202 | | | |
| 221: 9897 | | | | | | | | | | | |
| 222:16503 | 6946 | 12512 | 8166 | 7603 | 13367 | 17779 | 17108 | 5677 | 12483 | 4102 | 3789 |
| 8969 | 6176 | 4890 | 8723 | 5819 | 17099 | 6806 | 13035 | 12703 | 18886 | 4929 | 7208 |
| 12867 | 8233 | 11471 | 2758 | 17866 | 8378 | 10583 | 7058 | 5503 | 7152 | 12439 | 15584 |
| 8164 | 7820 | 9948 | 5042 | 10327 | 12796 | 9868 | 16562 | 10986 | 16295 | 19149 | 3946 |
| 8716 | 15320 | 18991 | 18402 | 1483 | 13460 | 17671 | 10764 | 2962 | 17886 | 17864 | 10600 |
| 10129 | 2817 | 2842 | 1695 | 16096 | 16759 | 15370 | 16173 | 13739 | 9388 | 8737 | 9863 |
| 10045 | 2379 | 17056 | 18355 | 1404 | 10235 | 18253 | 14229 | 15388 | 10130 | 2085 | 12476 |

(continued)

| SEQ ID NO: | homolog SEQ ID NOs | | | | | | | | | | |
|---|---|---|---|---|---|---|---|---|---|---|---|
| 9115 | 4120 | 3415 | 7852 | 14972 | 14948 | 10772 | 14572 | 8634 | 6691 | 8768 | 12084 |
| 1558 | 16813 | 13074 | 5000 | 14208 | 12827 | 3545 | 7743 | 17653 | 16255 | 8780 | 6849 |
| 13159 | 2512 | 17454 | 5691 | 2761 | 6441 | 8258 | 13861 | 2288 | 18638 | 679 | 927 |
| 17856 | 10134 | 15581 | 6881 | 10442 | 11642 | | | | | | |
| 223: 5413 | 6647 | 13564 | 11787 | 2900 | 17371 | 5719 | 18538 | 15525 | 16814 | | |
| 224: 5509 | 13877 | 15894 | 17059 | 12524 | 14960 | 15820 | 11498 | 4900 | 3665 | 14166 | 6804 |
| 1956 | 10375 | 3288 | 15483 | 14981 | 7447 | 12253 | 3522 | 5292 | 8172 | | |
| 225:19221 | 12606 | 17232 | 9256 | 19165 | 15129 | 4321 | 4677 | 9193 | 9192 | 9191 | 9195 |
| 17128 | 4398 | 15386 | 16721 | 7270 | 15262 | 9606 | 9608 | 6404 | 3730 | 4073 | 8257 |
| 5783 | 7717 | 17495 | 17574 | 18125 | 10532 | 2806 | 8453 | | | | |
| 226: 5355 | 10381 | 12506 | 9031 | | | | | | | | |
| 227:19210 | 13824 | 18756 | 2469 | 12148 | 19017 | 8260 | 8261 | 12127 | 18655 | 8112 | 7521 |
| 7032 | 14012 | 3050 | 11148 | 4438 | 1566 | 16589 | 8148 | 15936 | 11007 | 7676 | 16727 |
| 17306 | 14797 | 11542 | 14204 | 3088 | 11432 | 2312 | 18876 | 16653 | 8788 | 7477 | 1897 |
| 11235 | 7590 | 5594 | 17421 | 1171 | 2099 | 18271 | 4351 | 19063 | 15226 | 14546 | 18240 |
| 16886 | 18163 | 18160 | 14050 | 17165 | 8432 | 8098 | 7178 | 12519 | 15335 | 15336 | 15559 |
| 18118 | 13139 | 11776 | 1732 | 18200 | 2705 | 15454 | 14111 | 7831 | 1225 | 17488 | 14609 |
| 13273 | 12285 | 12720 | 771 | 1304 | 17239 | 18465 | 10463 | 6196 | 5321 | 10439 | 1315 |
| 12739 | 2300 | 13232 | 3894 | 3310 | 11754 | 17956 | 16710 | 1595 | 9767 | 12365 | 12980 |
| 312 | 878 | 19048 | 14611 | 1373 | 10205 | 3247 | 15298 | 7562 | 10090 | 3516 | 17816 |
| 14670 | 5400 | 7355 | 9322 | 5109 | 12003 | 11372 | 9135 | 17202 | 8964 | 12126 | 11501 |
| 15692 | 16891 | 14540 | 2285 | 1520 | 8928 | 3021 | 13685 | 15478 | 12561 | 13562 | 11325 |
| 3904 | 3248 | 7591 | 8153 | 8174 | 11539 | 18693 | 12236 | 10326 | 16285 | 14984 | 7073 |
| 7485 | 12635 | 18797 | 15967 | 18439 | 13075 | 4234 | 4864 | 18936 | 3640 | 11030 | 2385 |
| 6954 | 1768 | 2406 | 1951 | 16202 | 10053 | 11969 | 6069 | 10741 | 4310 | 4346 | 7301 |
| 15020 | 7030 | 13902 | 3676 | 15245 | 7967 | 2114 | 9755 | 9758 | 11041 | 1399 | 607 |
| 3844 | 5187 | 1248 | 15419 | 5059 | 16475 | 12458 | 11038 | 14975 | 2305 | 10096 | 10098 |
| 12979 | 16663 | 2458 | 3448 | 14447 | 9214 | 9217 | 3996 | 7238 | 12789 | 2038 | 6685 |
| 19174 | 5335 | 4231 | 17456 | 11781 | 4994 | 14877 | 14271 | 742 | 13905 | 16267 | 11286 |
| 4963 | 13433 | 13634 | 11742 | 10897 | 15826 | 1081 | 15615 | 12163 | 7022 | 11209 | 8104 |
| 13217 | 1514 | 1513 | 11401 | 13849 | 9872 | 3392 | 7714 | 17437 | 13797 | 3209 | 5004 |
| 16821 | 12740 | 13800 | 12560 | 5279 | 8730 | 9292 | 9265 | 9298 | 6765 | 9810 | 2380 |
| 18328 | 16833 | 1946 | 9995 | 6746 | 13050 | 9269 | 12948 | 6484 | 17397 | 14869 | 14870 |
| 6414 | 17620 | 14459 | 2373 | 434 | 3656 | 423 | 5401 | 16694 | 18030 | 5537 | 6578 |
| 12369 | 12850 | 900 | 10894 | 10868 | 8960 | 16150 | 14909 | 2286 | 14529 | 12399 | 15856 |
| 8705 | 16311 | 9271 | 8715 | 9294 | 2423 | 2855 | 8463 | 9779 | 12614 | 17865 | 12958 |
| 228: 3536 | 1432 | 1435 | 9457 | 3524 | 2486 | 15369 | 15566 | 8392 | 15107 | 8262 | 4848 |
| 11182 | 1390 | 12883 | 12866 | 16691 | 12187 | 8176 | | | | | |
| 229:11285 | 6998 | 4222 | 1194 | 3135 | 690 | 7978 | 12021 | 17770 | 4522 | 10591 | 3470 |
| 5744 | 10394 | 9316 | 16338 | 10498 | 6985 | 6465 | 10932 | 16071 | 6165 | 14004 | 8007 |
| 1454 | 18502 | 4382 | 3345 | 8449 | 13229 | 482 | 13158 | 471 | 13890 | 11377 | 13258 |
| 11770 | 18675 | 2967 | 3041 | 9750 | 10249 | 9108 | 17334 | 18037 | 18728 | 7830 | 1674 |
| 1812 | 3749 | 10193 | 14618 | 2134 | 13432 | 4763 | 4579 | 7829 | 2269 | 2651 | 7694 |
| 12856 | 14831 | 16875 | 5171 | 3878 | 13759 | 8459 | 14779 | 18185 | 9776 | 11388 | 12319 |
| 8800 | 15396 | 11006 | 11865 | 781 | 2655 | 9557 | 1652 | 18641 | 13834 | 883 | 1441 |
| 17087 | 19062 | 1555 | 10780 | 5359 | 15260 | 15387 | 1477 | 18785 | 18732 | 8532 | 4811 |
| 6656 | 14777 | 10943 | 17228 | 6869 | 6289 | 693 | 18549 | 12475 | 17800 | 10372 | 14986 |
| 2345 | 15774 | 18087 | 18112 | 10403 | 10425 | 10356 | 10444 | 5049 | 17881 | 7747 | 7751 |
| 2408 | 15831 | 15137 | 2702 | 11149 | 10004 | 6144 | 4336 | 14513 | 13952 | 14531 | 6916 |
| 15501 | 12581 | 5620 | 6080 | 19010 | 12938 | 9860 | 18618 | 16724 | 4517 | 4518 | 8087 |

(continued)

| SEQ ID NO: | homolog SEQ ID NOs | | | | | | | | | | |
|---|---|---|---|---|---|---|---|---|---|---|---|
| 15741 | 4127 | 1790 | 13173 | 7904 | 13171 | 1511 | 15876 | 11013 | 18888 | 11027 | 19169 |
| 7975 | 3702 | 2402 | 7358 | 18232 | 9847 | 1293 | 3935 | 18026 | 5050 | 12496 | 11265 |
| 445 | 8793 | 17777 | 18956 | 3689 | 15600 | 4183 | 12296 | 9947 | 6217 | 2318 | 9905 |
| 10822 | 18897 | 10020 | 8608 | 4703 | 12426 | 2450 | 11693 | 13869 | 4378 | 5451 | 12152 |
| 13395 | 3602 | 11015 | 6386 | 14969 | 14971 | 12677 | 12679 | 10108 | 11200 | 2362 | 13275 |
| 13272 | 10488 | 1952 | 9959 | 13141 | 1597 | 9919 | 13656 | 18699 | 16615 | 9593 | 528 |
| 11155 | 2311 | 12062 | 6200 | 7555 | 16257 | 13871 | 11381 | 8072 | 17475 | 17975 | 9464 |
| 15555 | 14752 | 11676 | 9806 | 9636 | 18597 | 16145 | 10274 | 6007 | 803 | 11807 | 2508 |
| 4932 | 18774 | 12096 | 4136 | 1786 | 13203 | 10939 | 670 | 18646 | 16184 | 18599 | 2581 |
| 16610 | 716 | 14042 | 8562 | 14736 | 14186 | 13709 | 2192 | 8832 | 11194 | 4318 | 7133 |
| 13421 | 7722 | 12759 | 8612 | 1003 | 16212 | 6268 | 7759 | 12304 | 18917 | 10512 | 14485 |
| 1335 | 426 | 10569 | 15216 | 904 | 8558 | 5540 | 5521 | 18933 | 8714 | 16607 | 1525 |
| 4021 | 8226 | 16343 | 17502 | 9713 | 14937 | 6678 | 15165 | 4655 | 5966 | 1262 | 18385 |
| 15085 | 5600 | 19146 | 15327 | 3657 | 567 | 16109 | 7392 | 5220 | 6531 | 6901 | |
| 230: 2130 | 5614 | 18926 | 11907 | 12420 | 4735 | 2310 | 5895 | 15061 | 9587 | 2282 | 11177 |
| 7927 | 8439 | 14508 | 9449 | 4903 | 6372 | 13595 | 3196 | 15544 | 444 | 2920 | 6319 |
| 5298 | | | | | | | | | | | |
| 231: 1909 | 14373 | 14375 | 12706 | 8598 | 11874 | 6536 | 7394 | 9724 | 13690 | 867 | 9458 |
| 4523 | 1703 | 13763 | 6958 | 14051 | 15346 | 19124 | 5833 | 13480 | 17342 | 13749 | 660 |
| 13092 | 1043 | 3787 | 3666 | 4743 | 1789 | 7649 | 5971 | 2220 | 13980 | 15157 | 9574 |
| 5328 | 11666 | 10119 | 5694 | 17374 | 18016 | 12563 | 2301 | 5762 | 11980 | 19089 | 1594 |
| 16383 | 17962 | 574 | 10694 | 10755 | 11071 | 8789 | | | | | |
| 232: 8297 | 5825 | 6027 | 9280 | 10798 | 11842 | 13809 | 8602 | 3051 | 16110 | 15280 | 5826 |
| 4654 | 4160 | 13647 | 16427 | 8769 | 17058 | 13265 | 16636 | 17796 | 5282 | 11608 | 18218 |
| 5544 | 4639 | 3751 | 1980 | 1599 | 1367 | 5678 | 16090 | 8359 | 982 | 980 | 15158 |
| 233:13431 | 7705 | 4748 | 2942 | | | | | | | | |
| 234: 6174 | 6173 | 5382 | 5585 | 13230 | 416 | 11225 | 654 | 17280 | 15733 | 10352 | 2087 |
| 2106 | 7696 | 7472 | 13351 | 5940 | 16730 | 15960 | 14680 | 7112 | 17267 | 15783 | 12008 |
| 15950 | 18731 | 18038 | 6273 | 19246 | 440 | 16299 | 18152 | 4005 | 5505 | 5331 | 15201 |
| 15155 | 17171 | | | | | | | | | | |
| 235:13748 | 17177 | 17005 | 17507 | 2003 | 2731 | 14085 | 19141 | 3528 | 7773 | 12053 | |
| 236:18719 | 6450 | 2954 | 2513 | 11280 | 3537 | 12705 | 8142 | 1722 | 15652 | 6054 | 2368 |
| 8633 | 16862 | 2353 | 8396 | 6821 | 901 | 1338 | 8023 | 2885 | 5209 | 1400 | 10805 |
| 14006 | 19175 | 920 | 11551 | 5722 | 15177 | | | | | | |
| 237: 9531 | 15827 | 11924 | 13062 | 15631 | 18390 | 15024 | 17683 | 9770 | 8469 | 447 | 16320 |
| 10886 | 4868 | 5684 | 12984 | 10616 | 11070 | 6145 | 17628 | 9655 | 18501 | 12917 | 16620 |
| 597 | 18585 | 13613 | 12010 | 9368 | 16625 | 7805 | 18988 | 17040 | 2689 | 18413 | 15186 |
| 4542 | 7399 | 3101 | 7259 | 8108 | 17966 | 5070 | 4026 | 3314 | 8938 | 18997 | 16673 |
| 3681 | 10328 | 5476 | 7286 | 2797 | 2795 | 6805 | 4951 | 10810 | 6689 | 3620 | 2650 |
| 14993 | 439 | 5143 | 11726 | 8225 | 12969 | 13519 | 18776 | 3574 | 3419 | 10778 | 17155 |
| 15100 | 9912 | 592 | 462 | 13348 | 3653 | 17555 | 18894 | 19069 | 3737 | 5311 | 15989 |
| 6483 | 2095 | 11816 | 5552 | 11157 | 18998 | 5013 | 1198 | 11659 | 14169 | 1837 | 6978 |
| 16566 | 19112 | 754 | 5846 | 9635 | 3808 | 13332 | 9836 | 10998 | 10558 | 14850 | 3711 |
| 11087 | 7095 | 6789 | 14582 | 2405 | 17733 | 2395 | 2191 | 14821 | 17898 | 18539 | 11894 |
| 19049 | 13683 | 4869 | 7423 | 810 | 16624 | 14264 | 11173 | 7205 | 2304 | 12791 | 18911 |
| 540 | 7390 | 15746 | 4661 | 14474 | 12750 | 12431 | 1930 | 14703 | 16154 | 5100 | 14265 |
| 7779 | 4485 | 11790 | 10166 | 19098 | 17981 | 18351 | 18316 | 13483 | 3429 | 5764 | 18602 |
| 17718 | 5081 | 16590 | 2682 | 9244 | 15241 | 1591 | 6844 | 19002 | 1306 | 17939 | 12928 |
| 13095 | 5145 | 11495 | 5383 | 9618 | 7331 | 6645 | 7908 | 8026 | 15413 | 16201 | 16348 |
| 6937 | 18476 | 14667 | 4293 | 4973 | 13840 | 9324 | 17032 | 12167 | 10184 | 4904 | 3931 |

(continued)

| SEQ ID NO: | homolog SEQ ID NOs | | | | | | | | | | |
|---|---|---|---|---|---|---|---|---|---|---|---|
| 4933 | 3240 | 1440 | 13853 | 10065 | 14734 | 11467 | 11799 | 16412 | 15244 | 3709 | 3663 |
| 6202 | 3325 | 18397 | 919 | 1255 | 17985 | 17487 | 8231 | 9424 | 16669 | 6940 | 15923 |
| 18306 | 7486 | 1129 | 7915 | 17212 | 18228 | 4913 | 9838 | 18846 | 17927 | 15550 | 4884 |
| 5386 | 7487 | 11184 | 7359 | 6942 | 737 | 1844 | 16997 | 1729 | 13810 | 3821 | 12333 |
| 11543 | 16481 | 7489 | 14980 | 10354 | | | | | | | |
| 238: 5969 | 19041 | 17447 | 17839 | 13044 | 6002 | 5996 | 6451 | 6051 | 5841 | 5420 | 5975 |
| 3313 | 5976 | 6030 | 4007 | 6044 | 6032 | 6035 | 7314 | 5390 | 5402 | 5406 | 5404 |
| 5442 | 5483 | 5460 | 5447 | 5462 | 5466 | 5448 | 5403 | 5409 | 8111 | 6279 | 5010 |
| 5457 | 13908 | 18485 | 10958 | 15764 | 15541 | 7610 | 6194 | 1287 | 2415 | 722 | 9613 |
| 18552 | 15880 | 6449 | 6448 | 18427 | 1941 | 18981 | 813 | 17756 | 16493 | 16643 | 8243 |
| 16018 | 8285 | 5367 | 681 | 17196 | 1880 | 13382 | 13353 | 6922 | 7721 | 6921 | 13781 |
| 602 | 16375 | 16374 | 1074 | 17578 | 12505 | 15049 | 16420 | 10531 | 6595 | 14804 | 3356 |
| 436 | 5388 | 6589 | 13334 | 1533 | 1682 | 11580 | 12397 | 16098 | 13659 | 6440 | 2483 |
| 14084 | 12530 | 3910 | 19087 | 19100 | 14923 | 18518 | 12688 | 13533 | 14541 | 14664 | 14880 |
| 6006 | 6025 | 6048 | 6947 | 6462 | 6467 | 6950 | 6471 | 6466 | 6470 | 5429 | 5982 |
| 13335 | 5873 | 10054 | 2377 | 2376 | 2960 | 9973 | 5950 | 5427 | 5999 | 6005 | 17954 |
| 5077 | 2687 | 812 | 698 | 11828 | 7347 | 3916 | 16904 | 8032 | 5443 | 5444 | 6845 |
| 6861 | 774 | 6097 | 7864 | 6681 | 14657 | 14656 | 14322 | 2315 | 12334 | 11946 | 9215 |
| 16352 | 16388 | 6184 | 19241 | 4820 | 10263 | 5192 | 4384 | 18473 | 18304 | 2125 | 12402 |
| 3477 | 17630 | 8856 | 12634 | 8795 | 6223 | 7518 | 9472 | 2061 | 7998 | 14430 | 17592 |
| 13210 | 4691 | 11026 | 13772 | 2757 | 14910 | 10539 | 1080 | 12022 | 17482 | 17713 | 12113 |
| 7912 | 18577 | 13796 | 13788 | 6701 | 5397 | 7440 | 13096 | 15002 | 13893 | 6826 | 5886 |
| 2947 | 5423 | 5973 | 5468 | | | | | | | | |
| 239: 7931 | 13758 | 8114 | 3373 | 11499 | 11529 | 1416 | 3738 | 13929 | 15590 | 1947 | 10552 |
| 663 | 16209 | 16266 | 6195 | 18471 | 8560 | 14830 | 12408 | 10330 | 3753 | 5574 | 10312 |
| 5387 | 10060 | 2091 | 2632 | 16023 | 6345 | 13028 | 16568 | 16816 | 1275 | 13202 | 4405 |
| 1977 | 5058 | 9376 | 14258 | 9597 | 18150 | 15433 | 1990 | 3791 | 7832 | 12466 | 10094 |
| 2631 | 13168 | 12715 | 4562 | 16993 | 2448 | 10742 | 17882 | 16751 | 11056 | 3456 | 1427 |
| 1894 | 13370 | 1955 | 1637 | 15678 | | | | | | | |
| 240:18268 | 843 | 14440 | 12547 | 863 | 849 | 1380 | 877 | 899 | 5838 | 17044 | 18986 |
| 6087 | 1561 | 7134 | 12815 | 18752 | 9764 | 17742 | 13930 | 17791 | 4063 | 5217 | 1087 |
| 2750 | 13402 | 6192 | 18436 | 8296 | 13072 | 16555 | 3399 | 18332 | 15403 | 1358 | 13201 |
| 4618 | 12737 | 12313 | 2681 | 6906 | 12257 | 5347 | 9811 | 2778 | 18084 | 13271 | 3849 |
| 15380 | 9079 | 5286 | 17837 | 12566 | 12567 | 12570 | 15930 | 18679 | 3212 | 16901 | 3880 |
| 4979 | 7632 | 12905 | 11429 | 14344 | 636 | 16963 | 15406 | 12534 | 14968 | 17309 | 8138 |
| 18401 | 18515 | 17014 | 17565 | 3768 | 9667 | 1496 | 15404 | 4486 | 16528 | 18777 | 14536 |
| 241: 7389 | 4712 | 18984 | 2834 | 7240 | 7425 | 7411 | 3882 | 2159 | 7263 | 7262 | 4838 |
| 15917 | 7350 | 7269 | 7288 | 7239 | 1037 | 8013 | 6310 | 7326 | 7209 | 7237 | 7235 |
| 7221 | 7299 | 7296 | 3899 | 15830 | 16083 | 4115 | 2148 | 8292 | 18656 | 7538 | 5827 |
| 14078 | 13597 | 7517 | 7511 | 7494 | 7198 | 11579 | 7426 | 7444 | 3270 | 15500 | 8339 |
| 627 | 1316 | 19032 | 11289 | 6299 | 1170 | 3567 | 3150 | 3151 | 8702 | 7648 | 7468 |
| 13733 | 10019 | 10017 | 11534 | 10089 | 4520 | 16146 | 11919 | 11926 | 11887 | 15101 | 11886 |
| 15102 | 3432 | 18952 | 18380 | 15422 | 8333 | 8284 | 8313 | 2075 | 6604 | 10245 | 3095 |
| 7407 | 10445 | 2441 | 10489 | 10658 | 7473 | 15243 | 4952 | 7044 | 3124 | 16107 | 2221 |
| 2241 | 7332 | 7339 | 7336 | 1359 | 1360 | 8314 | 17763 | 8310 | 12788 | 8318 | 7964 |
| 8340 | 8343 | 8346 | 1962 | 8847 | 2058 | 2413 | 2416 | 16080 | 14542 | 7363 | 3127 |
| 8003 | 7373 | 7372 | 15767 | 12500 | 16065 | 6387 | 14998 | 11845 | 11762 | 11109 | 15506 |
| 7190 | 5075 | 5076 | 7367 | 7365 | 13195 | 7449 | 7467 | 6802 | 7491 | 7448 | 7535 |
| 7520 | 7513 | 7533 | 7515 | 7290 | 7294 | 1975 | 13363 | 688 | 12002 | 692 | 12927 |
| 12921 | 8317 | | | | | | | | | | |

(continued)

SEQ ID NO:    homolog SEQ ID NOs

```
242: 8884  11880   8404  14936  10220  13873  14731  19162   9931    728   7746   2765
      9313  11758  15528  15679   3801   6959
243: 6493   7546  16565   6399   3230  15571  10046  16614
244: 6067  18383  14924   7767    500  11341   8391  18054   9488  14419  15709   2123
      6463   8207  11093  16740   4794
245: 6356  10815   5961   3168  13304   7307   3538   9862  18320  10893  17933   9310
      2801  14818   6864
246: 1702  18677  18678  16723   3824
247: 9993   6350   2350   3660  13279   6773    833  13639  14922   1925  15263  13746
     10150  11999  10994   4619  12040   8110  12045  18224   3989  14287    443  18853
     16144  14510   8332  17924   4224   4989   9778  14496  18885
248:17218   8464  14878   5889   5871   5869   5920   5919   5144  16260   4470  11700
      2569   4480   4481   1067  19234  17408   9777  15352  13852  15549  13123  10984
      7295  13833   5464   7742   7738   7740  16169  11981   1726   5866   5864   5914
      5835   5839   5834   6468  11794   5893  10285   5917   5915   5861   4458   3378
      8006  12101  12540   6524  14197   1213  10988   7939  13889  17151  16020  16044
     13018   6266   9286  14709  12147  15902  18955   7996   3302   1807   3643  15994
       668  15993  11303  11315   5891   5890  11226  14904  10319  10318  10320  10301
     10300  10297  10322  18548  10284   9861  11008   4328   3779
249: 6780   5494   6854  15194   6088   2074  16016  10722  17010   9739   9706   9707
      5452   9678  17017   9722    493   3339   6621    496  10507  18235   8249  10055
     13499   2237  18808   6046   1677  10950  14628   6208  13735  16093
250: 8596  10295   6751  17589  14070   7098   7038   8951   4311  10311  10232
251: 8165  16137  11727  14848  10797   6870  15697  10219   2543  16471  18468    994
      4654  17908  17268   3959   9832   2808  15373   1237   3647  10686  15612  11675
      9784  16308  10978  15081  16090    982    980  15158
252: 5700  10459  14135  16707  10635  10022    652  10461   9410  10216   9602  16193
      6261  16715
253:15564  16365   3189   7229   6136  12380   9773   7880   7495   3648  11586  10617
     15395   5490   7523   7999   7532  16605   6957   8187  15574  18540  10138  10140
      1018   6766   2447   2433   3052   1186   5859   4179   1019   5303   1189  10139
     10143   4305   2399   3428   4306   6822   9455   3149   7484   2398   5380   4304
      9413   1187   6127   1241  18838    450   4874  11239   4893   6917   7500   9358
      9435   1085   4896   8624   6837   8081   4145   1822  10767   3535  15569  10732
     14952  18828  10079  18778   4121  16057  11153  17223   4347  17531  10638  13982
     16515  17774  10902   2175  17000   6764   6335   4406   6128   7811   1665  15653
       504  18289  11587   9507   6050  13490  13492   5006  15225   1047   4835  17918
     11808  17365  17363   6878  19092  18823   2719   9344  10875   5018  14749   5736
     13845    579  12944   2821   3032   5475   5456  16834  16986  14473   1845  18340
     15071    457   2837  15068   3413  14266  17618   3416   2841  16807  18622  18623
     17736   5553   5511  16913   1154  13787    551  16936  15134  16914    599   1103
     15032  14996  14489  14495    424    562    596  19235  16200  18098  18767  13087
     13081  13089   9698   4339  11800  11802  11652   5068   5052   5230   6547   7219
     12394   6405   7222  11028   7663  13663  13083   3825   6548   7001  13106   9236
      8416  10317   5712   5727  12386   9896   5016   7107   3856   2669   7129   3712
      2686  12346   9873   3816   5017   7608   6570  10983   7140   9789   7660  10935
      6850   4358   7421
254: 5008  12646  17688   8974   7014  13777  16381  18947   6884   5695   8070   6494
     18567   5248  10972   8883   2749  17493   5368   4695   2619  17562    565    866
      7934  18910   2163   8893   7103   8331   2771  17230  10464  12038   6156  11251
```

(continued)

| SEQ ID NO: | homolog SEQ ID NOs | | | | | | | | | | |
|---|---|---|---|---|---|---|---|---|---|---|---|
| 6447 | 7626 | 12981 | 17857 | 18307 | 13138 | 7233 | 10185 | 14032 | 2442 | 10198 | 7579 |
| 10288 | 5236 | 17638 | 5130 | 14500 | 5679 | 6723 | 17712 | 14696 | 18814 | 7293 | 3229 |
| 10783 | 5784 | 1563 | 12227 | 12937 | 3202 | 13523 | 18544 | 2290 | 2092 | 18131 | 18458 |
| 6187 | 2328 | 9027 | 9029 | 7272 | 4260 | 16038 | 14698 | | | | |
| 255:13844 | 13458 | 16344 | 13531 | 8991 | 10799 | 11067 | 14090 | 4077 | 19037 | 2887 | 8049 |
| 11386 | 11116 | 14226 | 7690 | 12362 | 8745 | 8824 | 8279 | 7431 | 2378 | 6457 | 691 |
| 689 | 15215 | 10186 | 18272 | 19197 | 7202 | 2174 | 1280 | 10910 | 15451 | 2634 | 5194 |
| 3064 | 16764 | 9386 | 16140 | 9008 | 5381 | 11014 | 2599 | 19137 | 3083 | 6341 | 2620 |
| 7439 | | | | | | | | | | | |
| 256:18791 | 7383 | 5747 | 11955 | 5810 | 13684 | 7469 | 8124 | 3372 | 8114 | 3373 | 8123 |
| 4217 | 17629 | 4204 | 591 | 6282 | 4519 | 14590 | 14131 | 9918 | 18109 | 18122 | 18107 |
| 18123 | 10592 | 7481 | 7463 | 2612 | 6371 | 6375 | 10073 | 14990 | 11339 | 14608 | 11060 |
| 7879 | 630 | 4828 | 3591 | 6406 | 15590 | 11021 | 17060 | 790 | 5787 | 2670 | 6720 |
| 15939 | 2455 | 8857 | 13434 | 11738 | 3008 | 9102 | 17680 | 1196 | 10417 | 13536 | 14243 |
| 17599 | 2902 | 9828 | 18056 | 5234 | 17355 | 18761 | 9371 | 11729 | 4637 | 10566 | 16772 |
| 12408 | 14108 | 9500 | 10312 | 12735 | 5387 | 17976 | 5569 | 1191 | 15171 | 10790 | 17721 |
| 17700 | 17675 | 17672 | 17720 | 17696 | 17677 | 17654 | 17719 | 17668 | 17704 | 17642 | 17702 |
| 17698 | 17652 | 6324 | 6380 | 17768 | 15277 | 15321 | 15302 | 15275 | 15326 | 15324 | 15305 |
| 15301 | 15283 | 15308 | 15236 | 15306 | 15329 | 15255 | 15271 | 7606 | 1292 | 14494 | 1603 |
| 4524 | 11636 | 15988 | 18060 | 15311 | 2602 | 8083 | 5114 | 4690 | 3719 | 4672 | 17429 |
| 17430 | 4233 | 8801 | 6584 | 7117 | 12873 | 15833 | 907 | 1575 | 8208 | 7089 | 6782 |
| 2275 | 10256 | 15480 | 12342 | 11054 | 17184 | 9375 | 9393 | 6297 | 8043 | 18829 | 3281 |
| 11962 | 9376 | 16594 | 6181 | 6879 | 6185 | 10213 | 7891 | 5849 | 12308 | 15458 | 2082 |
| 13755 | 11977 | 5283 | 15433 | 15434 | 7662 | 18141 | 18135 | 18166 | 18146 | 18143 | 18313 |
| 5892 | 1829 | 2536 | 10168 | 15018 | 3116 | 3117 | 4803 | 11056 | 11602 | 18167 | 18179 |
| 18178 | 18198 | 18180 | 18177 | 18199 | 2128 | 4574 | 6875 | 10412 | 6321 | 3049 | 560 |
| 257:10236 | 1778 | 2762 | 8305 | 15457 | 1517 | 18065 | 3918 | 4604 | 14043 | 1834 | 15769 |
| 10542 | 13088 | 16947 | 9842 | 2117 | 1780 | 1783 | 6887 | 10786 | 3092 | 3074 | 2425 |
| 518 | 6885 | 18899 | 4918 | 8216 | 4901 | 16336 | 4899 | 16103 | 7720 | 3649 | 4873 |
| 4866 | 4875 | 628 | 1283 | 15291 | 13148 | 3611 | 1217 | 9366 | 15616 | | |
| 258:17425 | 15853 | 8859 | 9683 | 7091 | 5216 | 2201 | 2205 | 11262 | 3346 | 13482 | 13109 |
| 13107 | 13108 | 9794 | 8281 | 14641 | 13444 | 2715 | 1066 | 6137 | 12970 | 6142 | 6140 |
| 17183 | 6163 | 6141 | 17181 | 17192 | 9831 | 5159 | 13051 | 14297 | 10948 | 7031 | 811 |
| 16445 | 7171 | 15854 | 13126 | 710 | 17296 | 17319 | 17285 | 17286 | 873 | 9312 | 16119 |
| 16120 | 10141 | 6168 | 17224 | 4466 | 14934 | 13246 | 14008 | 11772 | 10397 | 13068 | 13455 |
| 17650 | 10953 | 14295 | 1735 | 10585 | 13288 | 8496 | 6041 | 7961 | 19038 | 6612 | 1629 |
| 6017 | 6013 | 14627 | 11526 | 15297 | 4441 | 6040 | 6148 | 9420 | 7099 | 8711 | 19132 |
| 11098 | 3943 | 10624 | 6800 | | | | | | | | |
| 259:12322 | 4714 | 7090 | 7343 | 3817 | 9397 | 4548 | 3010 | 2769 | 9109 | 3414 | 14347 |
| 13708 | 13372 | 5943 | 11868 | 11465 | 10063 | 4752 | 17817 | 13361 | 13360 | 13357 | 5105 |
| 5573 | 9024 | 6424 | 13872 | 13868 | 7092 | 4717 | 4716 | 7087 | 6575 | 17611 | 18568 |
| 4570 | 15716 | 10227 | 8510 | 7634 | 10157 | | | | | | |
| 260: 6150 | 16463 | 4354 | 8132 | 5126 | 13216 | 2066 | 7809 | 7718 | 2988 | 12642 | 13615 |
| 10029 | 17953 | 14902 | 16838 | 18868 | 8290 | 5596 | | | | | |
| 261: 2464 | 6735 | 15726 | 18074 | 4533 | 7642 | 7488 | 10955 | 10687 | 7802 | 3503 | 13093 |
| 8940 | 2616 | 12104 | 8987 | 18900 | 3879 | 8561 | 4110 | 16434 | 2625 | 16297 | 10309 |
| 4244 | 1026 | 8472 | 8897 | 17846 | 740 | 3692 | 12517 | 16930 | 18079 | 655 | 5583 |
| 14539 | 3696 | 8399 | 11724 | 9362 | 5698 | 10595 | 14766 | 5215 | 4170 | 9716 | 19244 |
| 7813 | 5862 | 585 | 4135 | 648 | 19138 | 11445 | 2739 | 10042 | 18353 | 8100 | 2019 |
| 2690 | 19052 | 12373 | 13945 | 16595 | 1140 | 12664 | 1560 | 16857 | 13624 | 12453 | 13808 |

(continued)

| SEQ ID NO: | homolog SEQ ID NOs | | | | | | | | | | |
|---|---|---|---|---|---|---|---|---|---|---|---|
| 5173 | 13766 | 6628 | 9260 | 12260 | 8687 | 18121 | 17463 | 1120 | 7797 | 13969 | 17724 |
| 5314 | 16826 | 2063 | 1088 | 14372 | 9553 | 17994 | 2673 | 16245 | 6149 | 8458 | 19093 |
| 7324 | 10811 | 7710 | 17194 | 17284 | 12321 | 12033 | 10898 | 10152 | 9002 | 13471 | 5605 |
| 8885 | 4972 | 13637 | 9833 | 12092 | 1700 | 19012 | 2081 | 13874 | 11373 | 2852 | 11365 |
| 18154 | 11821 | 9406 | 17339 | 4707 | 1102 | 1166 | 1787 | 11844 | 5166 | 10551 | 12580 |
| 19135 | 3752 | 15624 | 1063 | 14987 | 1535 | 17889 | 8739 | 13192 | 2052 | 610 | 1714 |
| 10124 | 18536 | 8139 | 16889 | 5258 | 2981 | 8679 | 4369 | 16639 | 16037 | 7893 | |
| 262: 2033 | 2767 | 3670 | 4459 | 4432 | 16170 | 10905 | 16867 | 8606 | 18942 | 7697 | 3565 |
| 14407 | 8288 | | | | | | | | | | |
| 263: 1035 | 9303 | 17522 | 3596 | 16423 | 10242 | 10744 | 14550 | 18944 | 13113 | 10874 | 8143 |
| 8831 | 7585 | 8109 | 1310 | 5138 | 850 | 11127 | 7958 | 8986 | 2453 | 2560 | 4205 |
| 4004 | 4675 | 4673 | 10345 | 17998 | 1374 | 821 | 8649 | 8481 | 9682 | 7380 | 6431 |
| 3360 | 3374 | 5754 | 11483 | 13329 | 1203 | 1340 | 1339 | 4399 | 3283 | 6668 | 6381 |
| 6241 | 7284 | 7512 | 6230 | 11328 | 15128 | 12214 | 10951 | 11193 | 6497 | 2195 | 16892 |
| 16809 | 4053 | 15715 | 15901 | 16798 | 11263 | 1719 | 3634 | 2158 | 5478 | 15076 | 1257 |
| 13172 | 15036 | 14456 | 14458 | 3438 | 18769 | 2703 | 12701 | 18877 | 14893 | 6253 | 18425 |
| 3403 | 3914 | 5746 | 4245 | 5745 | 17084 | 15381 | 17772 | 15379 | 9309 | 10296 | 12410 |
| 5703 | 15992 | 3780 | 18405 | 2278 | 4687 | 7254 | 4645 | 15181 | 9150 | 10286 | 18596 |
| 6188 | 9199 | 10890 | 10879 | 15662 | 18680 | 17535 | 16335 | 16334 | 564 | 15325 | 9790 |
| 15285 | 2090 | 15282 | 6549 | 4833 | 1118 | 7329 | 18041 | 15620 | 16076 | 8589 | 10725 |
| 17516 | 14852 | 6421 | 5690 | 5688 | 5119 | 12105 | 18454 | 13900 | 6564 | 14418 | 14619 |
| 4297 | 10339 | 10360 | 10639 | 12762 | 12763 | 12741 | 14511 | 13719 | 10909 | 10773 | 16790 |
| 9869 | 1727 | 6880 | 10960 | 11304 | 7110 | 5769 | 13197 | 7056 | 9945 | 5261 | 17830 |
| 14774 | 14773 | 12199 | 3137 | 5595 | 14014 | 2941 | 2957 | 17451 | 9205 | 2466 | 12448 |
| 15051 | 3366 | 18339 | 9670 | 13214 | 1239 | 3486 | 18561 | 7055 | 9712 | 10923 | 13525 |
| 8438 | 13359 | 2418 | 9400 | 11234 | 2070 | 18866 | 15848 | 7243 | 7241 | 2726 | 10370 |
| 7457 | 14298 | 1838 | 10174 | 11329 | 12913 | 10405 | 11972 | 3408 | 12813 | 15659 | 8843 |
| 2880 | 13850 | 4880 | 4385 | 17717 | 17265 | 10460 | 11220 | 15829 | 9685 | 15344 | 2178 |
| 11324 | 12998 | 18296 | 15580 | 12407 | 18636 | 16217 | 1071 | 4776 | 2384 | 16294 | 517 |
| 1409 | 13377 | 442 | 855 | 3498 | 5899 | 7009 | 3369 | 9524 | 7955 | 17042 | 2463 |
| 6860 | 797 | 13680 | 1818 | 8229 | 6189 | 1892 | 2482 | 5086 | 11387 | 12139 | 19047 |
| 19046 | 2561 | 11674 | 16039 | 19016 | 6993 | 4118 | 16629 | 14564 | 16309 | 2735 | 832 |
| 4286 | 11133 | 1920 | 16941 | 7793 | 7847 | 3846 | 12352 | 3027 | 2187 | 7317 | 11751 |
| 16395 | 14634 | | | | | | | | | | |
| 264: 7571 | 12286 | 11656 | 6312 | 7806 | 13041 | 4308 | | | | | |
| 265:13716 | 16282 | 3622 | 16128 | 2432 | 5091 | 7256 | 5094 | 7466 | 7464 | 5092 | 1341 |
| 1344 | 16034 | 16318 | 1345 | 5493 | 10131 | 13605 | 14704 | 4059 | 10144 | 19195 | 9113 |
| 266:16179 | 6376 | | | | | | | | | | |
| 267:10695 | 10080 | 15222 | 3336 | 16070 | 17906 | 15461 | 16778 | 11541 | 8321 | 14840 | 16441 |
| 14461 | 3078 | 16553 | 14929 | 14615 | 18225 | 1799 | 3781 | 9074 | 1362 | 8894 | 9856 |
| 13182 | 12587 | 11210 | 16207 | 19053 | 9477 | 3450 | 8475 | 11378 | 10077 | 18659 | 5908 |
| 11662 | 10239 | 3546 | 9196 | 11788 | 16729 | 12687 | 17436 | 12472 | 14335 | 14313 | 3350 |
| 5657 | 13623 | 11806 | 15691 | 18889 | 5828 | 12962 | 7549 | 17152 | 14791 | 7328 | 7327 |
| 15844 | 10068 | 11042 | 19158 | 16711 | 15570 | 16355 | 8068 | 10996 | | | |
| 268: 2886 | 9957 | 17982 | 10633 | 7244 | 6714 | 1461 | 2113 | 6871 | 836 | 13502 | 6798 |
| 15168 | 18396 | 3234 | 2200 | 9380 | 5880 | 9621 | 8447 | 9787 | 12043 | 15448 | 9694 |
| 13218 | 4705 | 13753 | 14029 | 2998 | | | | | | | |
| 269:10688 | 4111 | 17553 | 10437 | 15096 | 5485 | 9680 | 10385 | 10653 | 11747 | 16874 | 14594 |
| 16211 | 14686 | 17690 | 8241 | 4055 | 7427 | 7803 | 1987 | 4186 | 4214 | 15651 | 1363 |
| 8864 | 11780 | 13023 | 7633 | 12224 | 6490 | 13751 | 8037 | 3589 | 11702 | 5195 | 5909 |

(continued)

| SEQ ID NO: | homolog SEQ ID NOs | | | | | | | | | | |
|---|---|---|---|---|---|---|---|---|---|---|---|
| 2222 | 9907 | 14842 | 17686 | 6624 | 7566 | 3583 | 12297 | 12724 | 14468 | 10207 | 18644 |
| 10608 | 1622 | 4841 | 16104 | 1605 | 16551 | 18689 | 12343 | 9207 | 2436 | 3822 | 16818 |
| 10642 | 18674 | 2352 | 4626 | 1675 | 17661 | 3134 | 617 | 2708 | 961 | 3106 | 10629 |
| 14827 | 11171 | 6053 | 12518 | 7784 | 5323 | 2580 | 9759 | 1709 | 2179 | 18964 | 14835 |
| 6852 | 13452 | 11718 | 10486 | 13090 | 18057 | 3263 | 8170 | 8744 | 16373 | 1654 | 15378 |
| 14170 | 9209 | 2584 | 3407 | 889 | 1439 | 10830 | 1585 | 4816 | 16025 | 12545 | 13281 |
| 13346 | 2590 | 1758 | 9650 | 17819 | 18091 | 16148 | 3389 | 17615 | 14976 | 10238 | 13875 |
| 2611 | 14065 | 5696 | 12279 | 6074 | 6729 | 2745 | 19185 | 5681 | 16920 | 13388 | 14163 |
| 8813 | 3618 | 14834 | 15087 | 3721 | 1485 | 9949 | 10283 | 7215 | 3309 | 13415 | 5439 |
| 4469 | 5933 | 17289 | 11789 | 6384 | 5731 | 10264 | 7605 | 8842 | 1099 | 14475 | 3909 |
| 13435 | 15200 | 7527 | 11012 | 18453 | 15198 | 4161 | 4104 | 18753 | 12971 | 17172 | 9631 |
| 12235 | 8497 | 13245 | 18019 | 16873 | 3661 | 4577 | 6121 | 6122 | 17843 | 14241 | 6559 |
| 15677 | 16369 | 18284 | 6292 | 1387 | 19123 | 4430 | 18460 | 6119 | 14240 | 4809 | 5037 |
| 3176 | 14246 | 822 | 19212 | 3604 | 8652 | 11848 | 9111 | 17377 | 14244 | 10482 | 5532 |
| 14726 | | | | | | | | | | | |
| 270: 8430 | 11246 | 888 | 19061 | 13527 | 4203 | 9211 | 18507 | 12612 | 6037 | 9813 | 12222 |
| 4042 | 17351 | 7357 | 2554 | 15576 | 2530 | 17209 | 1254 | 553 | 6291 | 4213 | 8660 |
| 8211 | 13160 | 12189 | 4353 | 2593 | 12910 | 18282 | 14806 | 17244 | 10015 | 2741 | 7736 |
| 6107 | 16064 | 536 | 683 | | | | | | | | |
| 271: 4877 | 12594 | 12592 | 12593 | 1168 | 17709 | 14622 | 9708 | 3469 | 12557 | 7679 | 13757 |
| 6458 | 11104 | 10895 | 7774 | 6856 | 6232 | 12922 | 3157 | 8983 | 11307 | 15719 | 12415 |
| 10416 | 6486 | 7002 | 1957 | 16783 | 5624 | 16422 | 11181 | 7291 | 1626 | 1648 | 5699 |
| 2204 | 719 | 6203 | 5597 | 6211 | 12836 | 12810 | 17491 | 17492 | 18863 | 8852 | 18169 |
| 17979 | 9998 | 10497 | 12247 | 18972 | 16293 | 11825 | 9133 | 5644 | 7508 | 4196 | 18115 |
| 16165 | 15640 | 12480 | 1900 | 2109 | 1803 | 1697 | 5219 | 12900 | 13417 | 18843 | 17971 |
| 2072 | 9088 | 12825 | 17178 | 18422 | 11750 | 10836 | 11653 | 13491 | 11689 | 11447 | 7592 |
| 19119 | 3201 | 3872 | 19058 | 6475 | 860 | 5497 | 10226 | 9099 | 13710 | 10051 | 6443 |
| 8235 | 3185 | 11456 | 806 | 429 | 8818 | 10483 | 17327 | 13496 | 11075 | 4592 | 412 |
| 11434 | 10429 | 11433 | 7422 | 7409 | 7408 | 15322 | 7907 | 13817 | 17105 | 7191 | 17711 |
| 10243 | 15851 | 13418 | 13147 | 2556 | 3866 | 2791 | 8273 | 3086 | 3070 | 3072 | 5538 |
| 11777 | 6320 | 16611 | 6100 | 14771 | 16623 | 3786 | 8242 | 18082 | 19027 | 13724 | 15983 |
| 1891 | 2867 | 7775 | 11619 | 13324 | 6616 | 6618 | 1125 | 18094 | 17722 | 15364 | 6683 |
| 2157 | 12555 | 13024 | 3783 | 14194 | 14316 | 5306 | 6778 | 15785 | 1282 | 18433 | 3128 |
| 14481 | 1991 | 8973 | 5580 | 5591 | 11725 | 2566 | 10376 | 13740 | 6263 | 1971 | 808 |
| 3748 | 17701 | 17699 | 6514 | 18566 | 6515 | 2929 | 3160 | 3162 | 8065 | 13297 | 13642 |
| 14227 | 10467 | 9564 | 4460 | 17984 | 13310 | 14903 | 13295 | 458 | 5622 | 3047 | 4732 |
| 12237 | 4733 | 18448 | 14026 | 12245 | 17550 | 13325 | 16872 | 6622 | 9208 | 11356 | 10084 |
| 4865 | 11454 | 4461 | 5057 | 1856 | 7815 | 6736 | 15122 | 17560 | 15649 | 4241 | 14979 |
| 8033 | 2588 | 2812 | 2571 | 4071 | 964 | 2367 | 963 | 12429 | 10641 | 3853 | 6277 |
| 489 | 5174 | 11089 | 13091 | 10627 | 15133 | 18593 | 3984 | 9394 | 10678 | 10661 | 9365 |
| 14707 | 10023 | 10677 | 9319 | 15093 | 6963 | 13933 | 15000 | 2932 | 18188 | 14891 | 18189 |
| 15816 | 13816 | 5516 | 671 | 5424 | 15377 | 10753 | 661 | 15431 | 12258 | 3839 | 10438 |
| 5562 | 6313 | 12903 | 3897 | 7885 | 12208 | 9401 | 9143 | 6513 | 14927 | 16596 | 13242 |
| 5450 | 11198 | 18338 | 18696 | 17858 | 17860 | 18786 | 5393 | 7594 | 12681 | 3631 | 2024 |
| 2005 | 4693 | 1526 | 2397 | 8766 | 11511 | 1179 | 1723 | 15556 | 5205 | 10095 | 4713 |
| 272: 2476 | 4605 | 3590 | 17269 | 12552 | 12550 | 7501 | 14751 | 15392 | 5592 | 11796 | 16372 |
| 4149 | 18640 | 6540 | 15725 | 18637 | 18044 | 9775 | 14074 | 17762 | 9431 | 16817 | 2976 |
| 2041 | 10920 | 10918 | 10919 | 6246 | 6245 | 3529 | 4129 | 11740 | 9275 | 11166 | 2997 |
| 7120 | 16741 | 14429 | 4680 | 14300 | 930 | 18404 | 10446 | 13276 | 14192 | 17400 | |
| 273: 7563 | 7558 | 12847 | 18712 | 16770 | 17497 | 544 | 10669 | 10670 | 17546 | 5362 | 18031 |

(continued)

| SEQ ID NO: | homolog SEQ ID NOs | | | | | | | | | |
|---|---|---|---|---|---|---|---|---|---|---|
| 10396 | 6596 | 11136 | 3476 | 3497 | 622 | | | | | |
| 274:13296 | 2414 | 7482 | 715 | 5029 | 17139 | 8523 | 12280 | 5587 | 9163 | 13837 | 7094 |
| 3044 | 4280 | 2153 | 1112 | 4738 | 4010 | 5324 | 10529 | 10800 | 18319 | 7203 | 6138 |
| 5128 | 10413 | 17368 | 9596 | 3799 | 5190 | 8551 | 2711 | 8694 | 12467 | 5167 | 4009 |
| 3862 | 9114 | 6874 | 16580 | 10234 | 6060 | 13741 | 8220 | 7478 | 11407 | 13856 | 11295 |
| 15070 | 13298 | 4415 | 1606 | 18172 | 6499 | 13961 | 14716 | 3655 | 10066 | 2800 | 7266 |
| 18464 | 16192 | 12428 | 17016 | 1810 | 7183 | 9659 | 15622 | 4254 | 18781 | 824 | 3874 |
| 5645 | 16717 | 15897 | 2604 | 11084 | 9641 | 9704 | 10541 | 3208 | 13670 | 1672 | 7074 |
| 5735 | 2600 | 5765 | 6912 | 5346 | 13728 | 13703 | 12240 | 12238 | 9145 | 15565 | 11169 |
| 1405 | 9756 | 15771 | 5179 | 5706 | 15772 | 12901 | 6709 | 14021 | 4168 | 18206 | 11626 |
| 14139 | 2779 | 886 | 11568 | 1437 | 9403 | 16000 | 16756 | 12529 | 13059 | 15915 | 12623 |
| 3252 | 1720 | 12209 | 16495 | 19227 | 9883 | 18208 | 5924 | 11326 | 13150 | 3307 | 8365 |
| 6995 | 12798 | 4949 | 14471 | 13560 | 8423 | 12166 | 9082 | 5041 | 6058 | 19128 | 7756 |
| 17440 | 14799 | 2510 | 13503 | 10992 | 12818 | 6613 | 11175 | 12853 | 793 | 2299 | 8291 |
| 12461 | 7179 | 18106 | 14045 | 2984 | 11966 | 741 | 2565 | 2532 | 11685 | 3928 | 10924 |
| 8919 | 1372 | 7471 | 6106 | 13429 | 13236 | 18258 | 6786 | 6823 | 2446 | 3040 | 3042 |
| 14676 | 10735 | 724 | 727 | 9601 | 15533 | 1649 | 10137 | 4857 | 4842 | 5365 | 644 |
| 6812 | 10912 | 3603 | 12030 | 14912 | 8616 | 9170 | 2322 | 3245 | 3250 | 3260 | 12840 |
| 5264 | 16328 | 8655 | 6283 | 13350 | 10698 | 8590 | 10697 | 11885 | 11195 | 9476 | 14668 |
| 15473 | 11567 | 12864 | 9709 | 14945 | 12215 | 4742 | 14584 | 16884 | 16447 | 1996 | 10199 |
| 2734 | 6807 | 13557 | 8666 | 15469 | 17395 | 14955 | 14176 | 5518 | 18711 | 2827 | 13154 |
| 2898 | 5322 | 5319 | 4739 | 8890 | 17300 | 4495 | 5063 | 10549 | 13137 | 4195 | 1854 |
| 2527 | 19139 | 9946 | 15252 | 12776 | 5280 | 10043 | 935 | 17588 | 4373 | 9663 | 17287 |
| 8521 | 6169 | 11290 | 10749 | 17136 | 17121 | 7881 | 14438 | 4888 | 5759 | 16329 | 2927 |
| 15532 | 1013 | 18288 | 17804 | 13542 | 8044 | 16866 | 1327 | 12341 | 4863 | 16757 | 9526 |
| 15323 | 6503 | 10634 | 17375 | 10795 | 1116 | 14645 | 6760 | 893 | 10543 | 5316 | 17304 |
| 5733 | 17142 | 14098 | 8524 | 12290 | 10516 | 7883 | 909 | 7984 | 778 | 14722 | 16684 |
| 12239 | 3006 | 15438 | 17394 | 17665 | 11558 | 8342 | 18264 | 14928 | 14478 | 17344 | 13381 |
| 7085 | 13720 | 18912 | 17156 | 2264 | 12779 | 1586 | 5275 | 10989 | 5818 | 11896 | 11897 |
| 5619 | 10840 | 5543 | 1650 | 10582 | 11709 | 16690 | 14269 | 14810 | 4431 | 4788 | 4790 |
| 7944 | 16870 | 7387 | 6769 | 7960 | 12491 | 18360 | 5026 | 6658 | 16937 | 12538 | 4612 |
| 10580 | 18193 | 3979 | 8422 | 3818 | 8505 | 8507 | 8504 | 17778 | 1759 | 8816 | 14646 |
| 11797 | 4240 | 6897 | 15192 | 6066 | 11963 | 13198 | 18204 | 18963 | 2573 | 14426 | 7752 |
| 15906 | 16059 | 1220 | 10047 | | | | | | | |
| 275: 6567 | 7596 | 11364 | 11453 | 12046 | 5398 | 13331 | 16182 | 8916 | 1973 | 5797 | 2518 |
| 891 | 18583 | 13323 | 733 | 12250 | 8328 | 5812 | 9967 | 3368 | 10248 | 3335 | 3895 |
| 11052 | 890 | 13631 | 12434 | 2940 | 12421 | 5281 | 9331 | 17926 | 19188 | 6236 | 17460 |
| 16804 | 5671 | 5831 | 16672 | 15497 | 17552 | 11625 | 14132 | 11383 | 9972 | 12803 | 11064 |
| 4584 | 6770 | 13599 | 15368 | 17312 | 1216 | 10021 | 16522 | 2292 | 1932 | 4386 | 8493 |
| 8247 | 6504 | 15981 | 15979 | 5650 | 5649 | 5636 | 17849 | 6811 | 18134 | 763 | 6640 |
| 16829 | 15593 | 7070 | 4330 | 3192 | 14604 | 8972 | 14445 | 17869 | 2903 | 6742 | 8271 |
| 8817 | 6090 | 5296 | 15391 | 13676 | 16526 | 2983 | 6105 | 3619 | 16242 | 6828 | 2683 |
| 19079 | 14386 | 8407 | 13407 | 14384 | 14377 | 14357 | 3028 | 15348 | 4817 | 14803 | 10025 |
| 13175 | 13161 | 14681 | 6147 | 13177 | 17575 | 17576 | 14449 | 17873 | 4915 | 3262 | 16036 |
| 17573 | 10706 | 4912 | 13077 | 18586 | 9900 | 18940 | 711 | 3290 | 16031 | 6781 | 18658 |
| 18668 | 18670 | 18671 | 18666 | 9878 | 3171 | 3207 | 880 | 3843 | 7249 | 3754 | 4372 |
| 5498 | 10341 | 13017 | 6682 | 8822 | 8212 | 6976 | 1984 | 7674 | 1906 | 14209 | 10975 |
| 9849 | 16316 | 6726 | 3249 | 11988 | 11991 | 5817 | 4766 | 10578 | 10662 | 430 | 14671 |
| 17412 | 15883 | 5437 | 11773 | | | | | | | |
| 276: 6415 | 11627 | 16734 | 2790 | 12959 | 6986 | 10082 | 18111 | 1354 | 12820 | 3964 | |

(continued)

| SEQ ID NO: | homolog SEQ ID NOs | | | | | | | | | |
|---|---|---|---|---|---|---|---|---|---|---|
| 277:18650 | 5609 | 10555 | 10768 | 10537 | 17527 | 6952 | 15558 | 10041 | 18763 | 7499 |
| 278:15266 | 15482 | 12044 | 12553 | 4614 | 11192 | 2672 | 11183 | 9823 | 14522 | 16221 | 3544 |
| 6281 | 5112 | 1760 | 18159 | 8238 | 8228 | 5482 | 8190 | 1965 | | |
| 279: 6567 | 11364 | 749 | 11453 | 12046 | 10052 | 17706 | 18365 | 8385 | 5398 | 1544 | 16182 |
| 12144 | 16111 | 3341 | 9565 | 9725 | 15117 | 15657 | 533 | 1137 | 2518 | 1887 | 1885 |
| 10842 | 13590 | 3994 | 2630 | 16766 | 15463 | 13036 | 9551 | 936 | 11557 | 18445 | 17217 |
| 9331 | 4033 | 4622 | 9786 | 11206 | 10471 | 4352 | 4386 | 1932 | 8493 | 8247 | 6504 |
| 15979 | 15981 | 9941 | 8839 | 15593 | 2313 | 916 | 918 | 2006 | 7070 | 4330 | 18529 |
| 10148 | 12565 | 10731 | 4682 | 10251 | 16953 | 15509 | 1699 | 7405 | 16425 | 9783 | 10979 |
| 5799 | 15687 | 18301 | 1950 | 7096 | 910 | 1135 | 16009 | 3792 | 3289 | 14462 | 14467 |
| 14464 | 17453 | 13343 | 13384 | 6719 | 13341 | 13729 | 2729 | 3534 | 2831 | 16392 | 12337 |
| 12350 | 6742 | 7072 | 7075 | 8817 | 6944 | 7570 | 3680 | 13593 | 12535 | 18281 | 19020 |
| 11696 | 4598 | 8914 | 14214 | 4159 | 4105 | 15044 | 16776 | 5558 | 16175 | 4191 | 15013 |
| 12168 | 16055 | 10803 | 2983 | 16242 | 6105 | 8407 | 9666 | 12514 | 4363 | 6781 | 8770 |
| 9878 | 3171 | 5652 | 17141 | 6726 | 3249 | 11991 | 11988 | 5817 | 4766 | 10662 | 17219 |
| 430 | 14671 | 4886 | 16660 | 5437 | 11773 | 5761 | 5410 | 18705 | 13903 | | |
| 280: 6173 | 6174 | 2365 | 17259 | 4411 | 4410 | 5588 | 11225 | 15721 | 14985 | 7696 | 11506 |
| 13351 | 5940 | 10368 | 6787 | 1959 | 12498 | 2454 | 16341 | 15783 | 18676 | 11293 | 4855 |
| 14524 | 17078 | 10180 | 5505 | 15201 | 15155 | 17171 | | | | | |
| 281:17893 | 7011 | 6207 | 11190 | 17567 | 12492 | 19233 | 19215 | 4300 | 7003 | 18070 | 16733 |
| 1554 | 9581 | 726 | 13534 | 2346 | 14727 | 18724 | 10857 | 13387 | 9212 | 9168 | 8573 |
| 7609 | 18442 | 6632 | | | | | | | | | |
| 282:18444 | 2704 | 17326 | 11744 | 5133 | 10947 | 2939 | 7795 | 9151 | 17727 | 19011 | 18210 |
| 7640 | 17186 | 13914 | 16210 | 7796 | 9182 | 16289 | 11073 | 10329 | 18021 | 486 | 3056 |
| 19189 | 2823 | 2718 | 19238 | 18251 | 17572 | 9916 | 7688 | 13457 | 19033 | 5682 | 5161 |
| 4926 | 8080 | 7018 | 6841 | 11443 | 11095 | 9499 | 17065 | 7800 | 7601 | 19208 | 15218 |
| 5336 | 11076 | 4603 | 5978 | 5434 | 4035 | 12125 | 16835 | 3501 | 5152 | 1055 | 16745 |
| 4798 | 4689 | 6204 | 11348 | 14326 | 16719 | 1911 | 13223 | 11698 | 14123 | 2728 | 17489 |
| 15170 | 7007 | 6500 | 18519 | 18845 | 2992 | 11872 | 15371 | 12170 | 5290 | 1076 | 11637 |
| 15888 | 1378 | 18303 | 6977 | 8150 | 12792 | 5180 | 4001 | 10701 | 11105 | 11240 | 6832 |
| 13140 | 8860 | 9478 | 1883 | 13213 | 8435 | 17266 | 17026 | 13464 | 9672 | 18431 | 3659 |
| 8631 | 16129 | 14941 | 14947 | 14446 | 11292 | 1493 | 13419 | 17608 | 7564 | 6391 | 18516 |
| 10217 | 13189 | 2412 | 15180 | 5510 | 18242 | 18310 | 13799 | 18244 | 2324 | 14518 | 605 |
| 8344 | 5123 | 8185 | 1105 | 10401 | 467 | 762 | 13128 | 6945 | 8993 | 9326 | 16082 |
| 11795 | 5213 | 5623 | 10420 | 2991 | 2993 | 2096 | 960 | 12805 | 15251 | 15269 | 12807 |
| 12808 | 15270 | 7006 | 12219 | 3037 | 4391 | 12138 | 16426 | 16377 | 9381 | 17166 | |
| 283: 1002 | 570 | 11876 | 2649 | 17648 | 4380 | 18089 | 12995 | 10728 | 17836 | 473 | 498 |
| 497 | 494 | 479 | 478 | 546 | 532 | 531 | 456 | 1015 | 5353 | 5378 | 15904 |
| 12957 | 2173 | 17947 | 10011 | 2928 | 8192 | 4334 | 8436 | 10105 | 16670 | 8736 | 5369 |
| 14463 | 773 | 19068 | 10713 | 10613 | 14516 | 10631 | 12652 | 10059 | 18613 | 15333 | 6327 |
| 1206 | 4265 | 5431 | 4488 | 13563 | 11252 | 14434 | 2444 | 4287 | 11202 | 4967 | 15204 |
| 6609 | 6389 | 17214 | 9049 | 11603 | 12082 | 4333 | 5394 | 4878 | 14480 | 15253 | 13831 |
| 15728 | 16916 | 16176 | 5417 | 13947 | 492 | 14257 | 12736 | 5030 | 809 | 15363 | 18256 |
| 6671 | 14781 | 15290 | 4190 | 9653 | 3334 | 3142 | 11578 | 7479 | 14674 | 7636 | 7429 |
| 9011 | 6705 | 16995 | 13053 | 8444 | 18496 | 3690 | 3684 | 12996 | 8128 | 16753 | 11423 |
| 14784 | 17995 | 8105 | 2055 | 1197 | 18721 | 13278 | 14156 | 17631 | 16351 | 11812 | 921 |
| 6316 | 6511 | 9688 | 8236 | 2236 | 1666 | 8183 | 14222 | 10270 | 10871 | 13354 | 10281 |
| 14643 | 9877 | 8303 | 10404 | 3508 | 14918 | 11058 | 10754 | 12023 | 19160 | 14926 | 12436 |
| 7311 | 1532 | 16945 | 16640 | 2936 | 12181 | 940 | 15494 | 1817 | 14016 | 6809 | 14081 |
| 5214 | 2516 | 10962 | 12360 | 14278 | 18013 | 9804 | 13658 | 9805 | 16113 | 1205 | 8511 |

(continued)

| SEQ ID NO: | homolog SEQ ID NOs | | | | | | | | | | |
|---|---|---|---|---|---|---|---|---|---|---|---|
| 3525 | 3575 | 7878 | 7199 | 7600 | 1982 | 16131 | 16501 | 2291 | 11620 | 3695 | 16984 |
| 14292 | 18773 | 7186 | 6323 | 10513 | 8845 | 5757 | 18101 | 8446 | 16588 | 5206 | 18358 |
| 17179 | 16606 | 15686 | 12142 | 10606 | 12316 | 7617 | 1093 | 8519 | 8670 | 17382 | 3800 |
| 2626 | 13178 | 17695 | 1423 | 1940 | 18278 | 6601 | 6598 | 6593 | 6300 | 9545 | 9495 |
| 9511 | 16384 | 2953 | 18423 | 13134 | 1744 | 13300 | 7493 | 10787 | 7818 | 18632 | 14702 |
| 4730 | 543 | 542 | 15813 | 15825 | 15773 | 15794 | 7790 | 7792 | 449 | 474 | 476 |
| 1020 | 12896 | 1634 | 10792 | 12939 | 15517 | 17601 | 14591 | 2504 | 2344 | 5239 | 13365 |
| 10738 | 5272 | 3907 | 12787 | 1422 | 1424 | 1426 | 1425 | 19071 | 14066 | 16496 | 11686 |
| 5098 | 8250 | 5868 | 9903 | 15732 | 4474 | 5641 | 13540 | 13553 | 4126 | 13784 | 2325 |
| 7406 | 2261 | 8915 | 18702 | 3635 | 9611 | 8644 | 7577 | 12242 | 12269 | 12246 | 12271 |
| 11172 | 5545 | 15259 | 17564 | 4679 | 8798 | 17419 | 4927 | 18557 | 12287 | 12288 | 5930 |
| 5928 | 5923 | 5935 | 4130 | 13063 | 2794 | 1707 | 4094 | 1716 | 3417 | 2027 | 11025 |
| 7823 | 7826 | 17538 | 6576 | 6577 | 6574 | 5948 | 7932 | 7947 | 2012 | 12569 | 6675 |
| 828 | 12106 | 11826 | 3708 | 2515 | 5501 | 5922 | 5901 | 5897 | 9808 | 3022 | 11269 |
| 11272 | 11268 | 13793 | 17409 | 14651 | 1584 | 7567 | 17116 | 9026 | 7438 | 5942 | 11535 |
| 435 | 507 | 506 | 508 | 510 | 554 | 8989 | 8959 | 8956 | 3505 | 18140 | 9977 |
| 8791 | 16310 | 17659 | 5330 | 7514 | 6280 | 14200 | 16736 | 11690 | 454 | 452 | 549 |
| 513 | 525 | 529 | 526 | 1017 | 1016 | 14261 | 17867 | 18881 | | | |
| 284: 9361 | 16893 | 9841 | 9508 | 9489 | 9510 | 8548 | 2151 | 9737 | 8904 | 9699 | 9733 |
| 8878 | 9765 | 8911 | 9771 | 8935 | 4737 | 11143 | 11141 | 9599 | 17965 | 17946 | 17964 |
| 17930 | 17871 | 17945 | 17942 | 17925 | 2429 | 2430 | 17944 | 17921 | 2431 | 17872 | 11759 |
| 11211 | 11749 | 11746 | 11767 | 11233 | 11249 | 11099 | 12527 | 7839 | 13474 | 6634 | 12615 |
| 19181 | 11446 | 8245 | 13314 | 7012 | 9594 | 13153 | 10676 | 2100 | 18285 | 18354 | 1169 |
| 18730 | 7863 | 8452 | 9676 | 8923 | 14136 | 13408 | 4155 | 15234 | 6994 | 16883 | 12775 |
| 11442 | 11441 | 11444 | 11418 | 11402 | 11403 | 11421 | 11420 | 11424 | 11425 | 18440 | 14044 |
| 10044 | 1907 | 16100 | 2578 | 17862 | 18275 | 14037 | 7727 | 902 | 14299 | 11996 | 11521 |
| 11993 | 11519 | 11525 | 11522 | 11500 | 11520 | 11502 | 11990 | 11487 | 11503 | 11497 | 11496 |
| 11462 | 11466 | 11459 | 11468 | 11463 | 1731 | 6352 | 9028 | 10971 | 14876 | 17905 | 1007 |
| 3912 | 18214 | 2424 | 5743 | 14161 | 3251 | 9701 | 4116 | 13262 | 16196 | 16194 | 14886 |
| 15430 | 3744 | 15496 | 538 | 12595 | 12454 | 572 | 14919 | 10603 | 6393 | 2474 | 18937 |
| 1350 | 12992 | 4791 | 14059 | 10593 | 6231 | 8906 | 1357 | 17622 | 13397 | 12455 | 6425 |
| 13336 | 4445 | 601 | 2279 | 9629 | 4564 | 4514 | 4515 | 4530 | 9668 | 9673 | 3298 |
| 18324 | 14202 | 14217 | 7836 | 7995 | 14054 | 14052 | 10447 | 13260 | 9743 | 9740 | 3296 |
| 6847 | 7835 | 18362 | 18346 | 18368 | 18348 | 18361 | 18344 | 10389 | 5830 | 9745 | 9744 |
| 9734 | 9738 | 5898 | 5896 | 5900 | 5959 | 5956 | 5951 | 5954 | 5902 | 5904 | 5931 |
| 5934 | 5906 | 5905 | 14576 | 11634 | 3067 | 17147 | 17162 | 12627 | 18342 | 18367 | 10707 |
| 6343 | 6340 | 11120 | 7435 | 7434 | 3295 | 2856 | 18352 | 14219 | 14223 | 18326 | 14205 |
| 14206 | 16199 | 16161 | 16312 | 16181 | 16174 | 16205 | 16159 | 15704 | 15701 | 16135 | 6905 |
| 6403 | 6367 | 6979 | 6939 | 6931 | 6908 | 7028 | 7027 | 7051 | 6935 | 6930 | 6982 |
| 7088 | 7050 | 6996 | 6910 | 7084 | 7081 | 3301 | 18321 | 14207 | 10407 | 14201 | 16085 |
| 11852 | 11841 | 11830 | 18849 | 4290 | 8356 | 15621 | 13592 | 9691 | 10911 | 8256 | 17163 |
| 17159 | 17160 | 14938 | 4909 | 19243 | 12355 | 11757 | 1229 | 1231 | 9627 | 8814 | 17187 |
| 9822 | 9486 | 19151 | 12596 | 19152 | 13551 | 13607 | 19142 | 19154 | 12590 | 19153 | 12628 |
| 13627 | 13610 | 13570 | 13567 | 13569 | 13632 | 13644 | 13645 | 13629 | 13651 | 13649 | 12617 |
| 19196 | 11827 | 11786 | 11801 | 11798 | 11782 | 11769 | 13633 | 11124 | 4276 | 15799 | 15730 |
| 15776 | 15759 | 9072 | 8275 | 14415 | 3276 | 15817 | 9692 | 8865 | 8908 | 9741 | 4544 |
| 9671 | 8863 | 4594 | 4597 | 4541 | 9647 | 4560 | 8820 | 9077 | 9633 | 13601 | 18798 |
| 18794 | 10819 | 11333 | 11311 | 11342 | 11337 | 11336 | 11334 | 13814 | 15375 | 6867 | 6502 |
| 12768 | 13425 | 8239 | 1903 | 13934 | 13960 | 13956 | 13936 | 17174 | 15112 | 3232 | 3233 |
| 2359 | 5824 | 10492 | 5266 | 14180 | 3722 | 5850 | 10430 | 10432 | 10427 | 10408 | 10410 |

(continued)

SEQ ID NO:    homolog SEQ ID NOs

| | | | | | | | | | | | |
|---|---|---|---|---|---|---|---|---|---|---|---|
| 10414 | 5844 | 10426 | 10468 | 10452 | 10453 | 14379 | 10525 | 10547 | 10553 | 10570 | 10577 |
| 6810 | 6794 | 6790 | 6796 | 6813 | 6833 | 6815 | 6831 | 17566 | 6190 | 10237 | 10088 |
| 17358 | 9958 | 18903 | 18833 | 18520 | 4587 | 4590 | 4536 | 13045 | 15942 | 2775 | 15428 |
| 7650 | 17148 | 17157 | 9287 | 4268 | 4253 | 18883 | 13477 | 12390 | 12613 | 19171 | 14402 |
| 19173 | 19177 | 12629 | 12631 | 19156 | 1669 | 9637 | 4258 | 12862 | 9649 | 8837 | 13571 |
| 9843 | 9513 | 13603 | 13584 | 13583 | 13588 | 13587 | 9818 | 9516 | 9480 | 9481 | 9795 |
| 9793 | 9484 | 4065 | 4038 | 3526 | 3520 | 3492 | 3518 | 3504 | 3472 | 3523 | 3459 |
| 4043 | 4036 | 4066 | 3558 | 3557 | 4020 | 3556 | 4019 | 3540 | 3542 | 2968 | 4034 |
| 4060 | 4032 | 4947 | 18852 | 18851 | 11998 | 12013 | 12035 | 11997 | 12057 | 12055 | 12014 |
| 12039 | 12019 | 12034 | 12017 | 12011 | 12061 | 12037 | 12060 | 12041 | 18856 | 4270 | 10450 |
| 6432 | 6429 | 6422 | 16725 | 16742 | 16701 | 16705 | 16743 | 16722 | 16706 | 12395 | 12378 |
| 12376 | 12326 | 12381 | 12345 | 12382 | 12361 | 16700 | 16681 | 16685 | 16682 | 16680 | 16136 |
| 16698 | 16133 | 16561 | 16598 | 16586 | 16564 | 16584 | 16578 | 16540 | 16538 | 12357 | 9768 |
| 8934 | 9702 | 9730 | 8881 | 4272 | 4257 | 18859 | 18873 | 18871 | 4259 | 4273 | 18858 |
| 11353 | 11352 | 11350 | 18834 | 18836 | 18875 | 18902 | 18901 | 9735 | 8903 | 8876 | 8902 |
| 8877 | 3588 | 1869 | 7078 | 16399 | 11481 | 11480 | 11485 | 11484 | 13012 | | |
| 285:11893 | 9361 | 16893 | 9508 | 9510 | 9841 | 9489 | 8548 | 2151 | 9737 | 8904 | 9699 |
| 9733 | 8878 | 9765 | 8911 | 9771 | 8935 | 17925 | 17965 | 17964 | 2430 | 17945 | 17942 |
| 17871 | 17930 | 2431 | 17946 | 2429 | 17872 | 17944 | 17921 | 11759 | 11211 | 11767 | 11749 |
| 11746 | 11233 | 11249 | 11099 | 12527 | 7839 | 13474 | 6634 | 19181 | 12615 | 11446 | 16547 |
| 8245 | 13314 | 7012 | 9594 | 13153 | 10676 | 1169 | 18285 | 18354 | 18730 | 7863 | 8452 |
| 9676 | 8923 | 14136 | 13408 | 13475 | 4155 | 15234 | 6994 | 16883 | 12775 | 11442 | 11441 |
| 11444 | 11421 | 11420 | 11402 | 11418 | 11403 | 11424 | 11425 | 1305 | 18440 | 14044 | 10044 |
| 1907 | 16100 | 2578 | 17862 | 18275 | 14037 | 7727 | 902 | 14299 | 11525 | 11522 | 11496 |
| 11993 | 11500 | 11503 | 11521 | 11519 | 11502 | 11996 | 11487 | 11520 | 11990 | 11497 | 11462 |
| 11468 | 11459 | 11466 | 11463 | 1731 | 6352 | 9028 | 10971 | 14876 | 17905 | 1007 | 3912 |
| 18214 | 2424 | 5743 | 1204 | 3251 | 14161 | 9701 | 4116 | 13262 | 4502 | 16194 | 16196 |
| 14886 | 15430 | 3744 | 15496 | 538 | 12595 | 12454 | 572 | 14919 | 10603 | 6393 | 15683 |
| 2474 | 14250 | 18937 | 1350 | 12992 | 4791 | 14059 | 10593 | 6231 | 8906 | 1357 | 17622 |
| 13397 | 12455 | 6425 | 13336 | 4445 | 601 | 2279 | 9629 | 4564 | 4514 | 4515 | 4530 |
| 9673 | 9668 | 18324 | 3298 | 14202 | 7836 | 14217 | 7995 | 14054 | 14052 | 10447 | 13260 |
| 9743 | 9740 | 3296 | 6847 | 7835 | 18362 | 18346 | 18348 | 18368 | 18361 | 18344 | 10389 |
| 5830 | 9745 | 9744 | 9734 | 9738 | 5898 | 5896 | 5900 | 5956 | 5959 | 5951 | 5954 |
| 5902 | 5904 | 5934 | 5931 | 5906 | 5905 | 14576 | 3067 | 17147 | 12627 | 18342 | 18367 |
| 10707 | 6343 | 6340 | 18321 | 3301 | 14207 | 10407 | 14201 | 16085 | 11852 | 11830 | 11841 |
| 8356 | 13592 | 9691 | 18195 | 10069 | 9922 | 11349 | 11618 | 15487 | 10182 | 3804 | 4909 |
| 19243 | 12355 | 11757 | 10954 | 9627 | 8814 | 17187 | 9822 | 9486 | 19151 | 19152 | 12596 |
| 13607 | 13551 | 19142 | 19154 | 19153 | 12628 | 12590 | 13627 | 13610 | 13632 | 13570 | 13567 |
| 13629 | 13569 | 13644 | 13645 | 13651 | 12617 | 19196 | 4986 | 11389 | 4987 | 11398 | 11827 |
| 11786 | 11782 | 11801 | 11798 | 11769 | 13633 | 11124 | 4276 | 15799 | 15759 | 15776 | 15730 |
| 9072 | 8275 | 14673 | 15817 | 9692 | 8865 | 9741 | 8908 | 9671 | 4597 | 4544 | 4594 |
| 8863 | 4541 | 9647 | 4560 | 8820 | 9077 | 9633 | 13601 | 18798 | 18794 | 13814 | 18690 |
| 6439 | 14133 | 12768 | 13425 | 9224 | 4509 | 2132 | 10382 | 1903 | 13934 | 13956 | 13960 |
| 13936 | 17174 | 15112 | 4944 | 1133 | 2359 | 5824 | 10492 | 5266 | 14180 | 3722 | 5850 |
| 10430 | 10432 | 10427 | 10408 | 10414 | 10410 | 5844 | 10426 | 10468 | 10453 | 10452 | 14379 |
| 10525 | 10547 | 10570 | 10553 | 10577 | 6810 | 6794 | 6790 | 6796 | 6813 | 6833 | 6815 |
| 6831 | 6748 | 6749 | 17566 | 6190 | 10237 | 10088 | 17358 | 9958 | 18520 | 4590 | 4587 |
| 4536 | 13045 | 15942 | 2775 | 15428 | 7650 | 17148 | 17157 | 9287 | 4268 | 4253 | 18883 |
| 13477 | 12390 | 19171 | 12613 | 19177 | 19173 | 14402 | 12629 | 12631 | 19156 | 1669 | 9637 |
| 4258 | 12862 | 9649 | 8837 | 13587 | 9513 | 13603 | 13584 | 9843 | 13588 | 13571 | 13583 |

(continued)

| SEQ ID NO: | homolog SEQ ID NOs | | | | | | | | | | |
|---|---|---|---|---|---|---|---|---|---|---|---|
| 9516 | 9818 | 9480 | 9793 | 9481 | 9795 | 9484 | 4065 | 4038 | 3526 | 3520 | 3492 |
| 3472 | 3518 | 3504 | 3523 | 3459 | 4043 | 4036 | 4066 | 4019 | 4020 | 3557 | 3556 |
| 3540 | 3542 | 3558 | 4034 | 4060 | 4032 | 4947 | 12061 | 11997 | 12035 | 12013 | 11998 |
| 12055 | 12057 | 12019 | 12039 | 12034 | 12017 | 12014 | 12060 | 12011 | 12041 | 12037 | 18856 |
| 4270 | 10450 | 6432 | 6429 | 6422 | 16725 | 12326 | 12395 | 12376 | 12378 | 16743 | 16742 |
| 16722 | 16706 | 16705 | 16701 | 12381 | 12345 | 12382 | 12361 | 16681 | 16685 | 16682 | 16700 |
| 16136 | 16698 | 16680 | 16133 | 16598 | 16586 | 16564 | 16561 | 16578 | 16584 | 16540 | 16538 |
| 12357 | 9768 | 8934 | 9702 | 9730 | 8881 | 4272 | 4257 | 4273 | 11353 | 11352 | 11350 |
| 18875 | 9735 | 8903 | 8876 | 8902 | 8877 | 1869 | 3588 | 16399 | 11480 | 11481 | 11485 |
| 11484 | 13012 | | | | | | | | | | |
| 286: 2777 | 5039 | 16750 | 1471 | 3285 | 2150 | 10618 | 8803 | 11961 | 2889 | 6846 | 3739 |
| 1995 | 3694 | 17476 | 12331 | 12602 | 7992 | 10443 | 10584 | 2551 | 12607 | 18523 | 16013 |
| 7530 | 4332 | 17943 | 9036 | 8140 | 14652 | 9799 | 6082 | 2538 | 5800 | 6597 | 12897 |
| 15296 | | | | | | | | | | | |
| 287: 9566 | 5643 | 6213 | 14305 | 9062 | 12764 | 1510 | 7131 | 14437 | 14832 | 5009 | 2243 |
| 12243 | 6637 | 4770 | 3936 | 13672 | 11255 | 11221 | 5921 | 7391 | 1432 | 1435 | 9457 |
| 15566 | 8392 | 5977 | 14724 | 12745 | 2394 | 16446 | 15400 | 12866 | 12883 | 3287 | 7541 |
| 6534 | 7866 | 15513 | 14892 | | | | | | | | |
| 288:15365 | 18498 | 13541 | 7184 | 7109 | 1034 | 7848 | 1309 | 1974 | 5407 | 4483 | 3120 |
| 15279 | 6672 | 19131 | 14159 | 8869 | 2364 | 10622 | 4552 | 13626 | 10133 | 10574 | 461 |
| 17086 | 6452 | 4595 | 3599 | 3294 | 11158 | 18667 | | | | | |
| 289:16368 | 12503 | 3630 | 9835 | 13104 | 15863 | 12124 | 11301 | 13962 | 8408 | 17039 | 12299 |
| 8002 | 7757 | 7854 | 2421 | 17941 | 9614 | 9944 | 947 | 7876 | 3401 | 2851 | 18366 |
| 5872 | 12894 | 5554 | 6523 | 3967 | 10214 | 17229 | 11034 | 6907 | 17354 | 15997 | 17279 |
| 15293 | 11343 | 12782 | 3434 | 8664 | 10980 | 11284 | 7151 | 5262 | 5760 | 15952 | 13577 |
| 8200 | 5778 | 14238 | 2958 | 3987 | 6408 | 4512 | 4532 | 4535 | 4513 | 13926 | 8393 |
| 2633 | 2640 | 913 | 15415 | 11965 | 16677 | 6715 | 13715 | 4773 | 19199 | 8085 | 1519 |
| 13439 | 15432 | 14319 | 13712 | 8738 | 11125 | 16975 | 6895 | 8732 | 12851 | 477 | 14392 |
| 15611 | 4147 | 9651 | 6491 | 6488 | 3806 | 13714 | 17754 | 17750 | 460 | 12140 | 3732 |
| 1981 | 2760 | 18374 | 632 | 8844 | 2235 | 13441 | 8846 | 6661 | 6659 | 865 | 14395 |
| 14433 | 14958 | 8575 | 7966 | | | | | | | | |
| 290:11915 | 12531 | 10674 | 5108 | 10673 | 10671 | 5110 | 10656 | 15342 | 7321 | 11367 | 11854 |
| 5888 | 14613 | 5002 | 7871 | 18610 | 18248 | 5952 | 11256 | 11273 | 1008 | 9456 | 9482 |
| 1098 | 17829 | 10769 | 8759 | 15842 | 9296 | 11737 | 16394 | 15796 | 11222 | 12888 | 17948 |
| 15606 | 15608 | 13316 | 8168 | 13652 | 16659 | 15073 | 13630 | 10782 | 17520 | 17523 | 7375 |
| 12579 | 4555 | 16775 | 18273 | 8782 | 8833 | 8828 | 5913 | | | | |
| 291: 9661 | 14871 | 18815 | 14431 | 12470 | 9259 | 5881 | 17640 | 2549 | 5199 | 3645 | 16654 |
| 14864 | 17074 | 1797 | 10867 | 4767 | 18221 | 9718 | 751 | 13915 | 18164 | 9703 | 6358 |
| 739 | 12122 | 15132 | 14974 | 8785 | 2372 | 6430 | 14127 | 1550 | 18042 | 7607 | 1574 |
| 8968 | 14165 | 14000 | 12131 | 19081 | 16106 | 2645 | 17386 | 6251 | 15629 | 11509 | 17303 |
| 11118 | 16393 | 9726 | 11174 | 4106 | 10619 | 5469 | 2895 | 7622 | 19078 | 15694 | 5576 |
| 11721 | 12733 | 17199 | 15873 | | | | | | | | |
| 292:15315 | 12929 | 13897 | 13194 | 6285 | 13409 | 13879 | 5211 | 428 | 4840 | 623 | 9603 |
| 9165 | 2034 | 13804 | | | | | | | | | |
| 293: 1857 | 13794 | 6221 | 18932 | 10387 | 11856 | 4252 | 4256 | 4255 | 3425 | 12892 | 11901 |
| 8638 | 8630 | 978 | 5301 | 5141 | 15472 | 1119 | 11213 | 13891 | 2603 | 8637 | 12207 |
| 16699 | 14669 | 12027 | 15111 | 13878 | 2754 | 11266 | 9938 | 10663 | 7917 | 17855 | 2691 |
| 8215 | 717 | 483 | 2996 | 8158 | 13042 | 3905 | 19024 | 3005 | 9180 | 3573 | 10211 |
| 16275 | 10831 | 13060 | 6460 | 704 | 1245 | 17402 | 1160 | 1753 | 4132 | 11888 | 18985 |
| 16323 | 17693 | 7097 | 11035 | 15312 | 1997 | 18441 | 5708 | 7627 | 16437 | 8154 | 1791 |

(continued)

| SEQ ID NO: | homolog SEQ ID NOs | | | | | | | | | | |
|---|---|---|---|---|---|---|---|---|---|---|---|
| 19031 | 8274 | 3741 | 8747 | 6445 | 12846 | 11110 | 18117 | 7167 | 10833 | 15412 | 17468 |
| 10824 | 16088 | 12354 | 15985 | 6684 | 17751 | 4202 | 14270 | 885 | 19125 | 8213 | 14055 |
| 1676 | 14412 | 4092 | 4079 | 2784 | 4093 | 2239 | 12447 | 18014 | 14252 | 18350 | 2529 |
| 3662 | 1065 | 15060 | 10364 | 16853 | 15185 | 2643 | 18058 | 3747 | 1498 | 1443 | 1881 |
| 8360 | 12988 | 11224 | 12576 | 631 | 3784 | 7653 | 10644 | 8103 | 2707 | 7143 | 952 |
| 11597 | 6171 | 18896 | 19187 | 2242 | 9521 | 9760 | 15027 | 6679 | 15641 | 11298 | 6919 |
| 1036 | 2296 | 18614 | 2154 | 11537 | 13573 | 3099 | 10710 | 5656 | 11644 | 15999 | 973 |
| 13920 | 19091 | 18890 | 2891 | 18318 | 3629 | 1252 | 879 | 5997 | 1961 | 7982 | 7936 |
| 294: 7105 | 3084 | 1994 | 18783 | 2497 | 7816 | 18184 | 3440 | 14124 | 18751 | 1967 | 15867 |
| 14672 | 7886 | 17222 | 1877 | 17983 | 17378 | 9387 | 3723 | 9501 | 16163 | 14128 | 6267 |
| 8384 | 5802 | 8872 | 3745 | 18948 | 14939 | 2793 | 16550 | 7047 | 2103 | 612 | 1522 |
| 15922 | 13547 | 13079 | 15943 | 5970 | 5968 | 4359 | 17714 | 10659 | 16490 | | |
| 295:11915 | 12531 | 17198 | 7368 | 2744 | 18649 | 3181 | 14613 | 8489 | 5002 | 7871 | 13313 |
| 18248 | 12632 | 11291 | 6927 | 1098 | 17829 | 10769 | 11161 | 488 | 14181 | 16980 | 14805 |
| 3254 | 2964 | 9225 | 9296 | 14276 | 11739 | 15796 | 7315 | 11804 | 15606 | 15608 | 13316 |
| 5763 | 12456 | 13652 | 16659 | 10739 | 12059 | 10782 | 17523 | 7375 | 10346 | 8720 | 16775 |
| 2653 | 18273 | 8782 | 8833 | 5913 | | | | | | | |
| 296: 862 | 5137 | 16001 | 12941 | 770 | 15554 | 18605 | 8082 | 3087 | 8425 | 2773 | 6680 |
| 6816 | 5218 | 18416 | 5855 | 1839 | 14492 | 16795 | 3595 | 1910 | 13985 | 10118 | 12109 |
| 7502 | 14989 | 5062 | 3113 | 10472 | 7870 | 1136 | 3790 | 8761 | 8515 | 14301 | 13855 |
| 5798 | 7675 | 15995 | 13529 | 1460 | 10887 | 18807 | 12633 | 7553 | | | |
| 297: 438 | 13973 | 13308 | 11956 | 16007 | 7173 | 7175 | 12391 | 2026 | 8512 | 16573 | 16216 |
| 11162 | 7158 | 2209 | 9879 | 16890 | 10061 | 9391 | 8377 | 11208 | 13866 | 15446 | 10737 |
| 16951 | 18861 | 3386 | 14949 | 16049 | 2268 | 422 | 2498 | 12158 | 11196 | 999 | 11422 |
| 6909 | 971 | 17103 | 12945 | 16236 | 14279 | 1815 | 5256 | 15390 | 801 | 4660 | 4185 |
| 5533 | 3055 | 15173 | 13105 | 8055 | 2461 | 5801 | 2810 | 15573 | 17102 | 17242 | 11720 |
| 15557 | 6135 | 14995 | 2480 | 13243 | 5433 | | | | | | |
| 298:11915 | 12531 | 15342 | 7321 | 11367 | 5888 | 14613 | 8489 | 5002 | 7871 | 18610 | 18248 |
| 5952 | 11253 | 1008 | 15996 | 1098 | 17829 | 10769 | 17874 | 4262 | 6648 | 9296 | 16112 |
| 2214 | 16394 | 17950 | 5329 | 18025 | 16063 | 17293 | 15606 | 15608 | 6546 | 4664 | 4292 |
| 10730 | 18652 | 12515 | 11405 | 18158 | 3549 | 13330 | 3902 | 6434 | 11902 | 9414 | 17888 |
| 2240 | 17635 | 14946 | 12828 | 8168 | 13652 | 16659 | 15073 | 13630 | 10782 | 17520 | 17523 |
| 7375 | 12579 | 4555 | 16775 | 8782 | 8833 | 8828 | 5913 | | | | |
| 299: 414 | 1917 | 1934 | 1936 | 1364 | 14900 | 8480 | 12103 | 17664 | 4215 | 5090 | 10064 |
| 10748 | 4581 | 3710 | 7147 | 15265 | 14787 | 5241 | 3993 | 17960 | 12064 | 10702 | 17494 |
| 13847 | 14233 | 7728 | 11629 | 9459 | 11576 | 12484 | 6071 | 3597 | 18443 | 1876 | 10502 |
| 6159 | 6154 | 6160 | 17140 | 7783 | 10647 | 17310 | 17311 | 17393 | 17332 | 17314 | 17372 |
| 17366 | 17388 | 17349 | 17364 | 17345 | 17317 | 17369 | 16206 | 17390 | 16927 | 16906 | 16839 |
| 16226 | 17418 | 16925 | 16825 | 16190 | 16228 | 16948 | 13935 | 13163 | 2803 | 17357 | 18002 |
| 410 | 14420 | 10711 | 853 | 839 | 6111 | 2440 | 15718 | 4166 | 12588 | 1480 | 8764 |
| 11330 | 3359 | 17935 | 6269 | 13179 | 14129 | 3075 | 2639 | 2657 | 15001 | 6623 | 6619 |
| 6649 | 6642 | 5607 | 7804 | 6646 | 9579 | 15233 | 12379 | 18050 | 17729 | 14558 | 12543 |
| 12546 | 14954 | 409 | 5200 | 10554 | 7507 | 3337 | 7801 | 2712 | 11045 | 6157 | 11716 |
| 12255 | 12259 | 11719 | 1915 | 13946 | 1212 | 1210 | 1236 | 1240 | 1242 | 1771 | 1769 |
| 1800 | 1777 | 1796 | 1794 | 1802 | 1269 | 1805 | 1244 | 1773 | 1272 | 1247 | 1775 |
| 1267 | 1264 | 1261 | 1214 | 7754 | 12783 | 1502 | 14776 | 17545 | 17503 | 17499 | 6363 |
| 14199 | 17682 | 16929 | 17526 | 14196 | 17540 | 17684 | 12050 | 2170 | 2176 | 2149 | 2147 |
| 17258 | 2145 | 12029 | 17252 | 12048 | 1642 | 17294 | 17292 | 17282 | 2873 | 17336 | 12024 |
| 13998 | 11552 | 3881 | 3451 | 17233 | 14483 | 14506 | 3978 | 14486 | 14482 | 13999 | 3939 |
| 3887 | 14507 | 14487 | 3940 | 11528 | 14013 | 13996 | 13997 | 18688 | 18722 | 6334 | 5664 |

(continued)

| SEQ ID NO: | homolog SEQ ID NOs | | | | | | | | | | |
|---|---|---|---|---|---|---|---|---|---|---|---|
| 8553 | 2931 | 4402 | 4401 | 15288 | 6179 | 17089 | 7845 | 17088 | 17055 | 17067 | 6182 |
| 7247 | 17064 | 17465 | 17481 | 17469 | 17467 | 13921 | 13899 | 12068 | 12069 | 12063 | 12065 |
| 9522 | 13942 | 13924 | 14829 | 5375 | 12544 | 19216 | 5610 | 7450 | 4163 | 9575 | 7246 |
| 9628 | 9059 | 4165 | 6857 | 6838 | 8944 | 7667 | 9634 | 7668 | 9652 | 7101 | 12424 |
| 15341 | 5621 | 5608 | 11722 | 12248 | 11687 | 11714 | 12217 | 11695 | 11691 | 11697 | 12220 |
| 11694 | 12249 | 12212 | 9731 | 12216 | 11723 | 12210 | 5724 | 15847 | | | |
| 300:17992 | 14813 | 6116 | 9580 | 2888 | 7234 | 8357 | 9926 | 14330 | 15460 | 6476 | 11921 |
| 8487 | 19217 | 12340 | 14973 | 18168 | 6988 | 14092 | 13614 | 9589 | 1042 | 12559 | 12558 |
| 10145 | 13807 | 3131 | 12159 | 7765 | 17049 | 491 | 5373 | 16006 | 13937 | 16869 | 16844 |
| 13943 | 13939 | 13917 | 18662 | 1028 | 18302 | 8760 | 8210 | 7918 | 968 | 6155 | 18132 |
| 4210 | 4212 | 4208 | 465 | 12388 | 1139 | 2520 | 7618 | 19214 | 6643 | 11734 | 11547 |
| 11050 | 7539 | 10093 | 8107 | 10903 | 12707 | 10224 | 4971 | 14365 | 16659 | 18553 | 9554 |
| 5668 | 1601 | 16122 | 1462 | 10321 | 11228 | 9914 | 18521 | 17523 | 16603 | 16599 | 16600 |
| 14253 | 10841 | 12283 | 13811 | 12709 | 5758 | 3941 | 3385 | 9514 | 9509 | 8569 | 18996 |
| 9826 | 15445 | 15986 | 7285 | 18975 | 3467 | 9261 | 16796 | 16794 | 15896 | 15895 | |
| 301:10900 | 8457 | 13754 | 4644 | 5428 | 3481 | 1939 | 10756 | 4267 | 18389 | 14849 | 6797 |
| 2161 | 10604 | 16911 | 13860 | 9866 | 9857 | 14350 | 3615 | 1201 | 1403 | 1653 | 19242 |
| 15740 | 4248 | 18572 | 5142 | 13938 | 7102 | 11968 | 3015 | 10490 | 2894 | 18449 | 14314 |
| 18543 | 17977 | 17076 | 425 | 10771 | 5506 | | | | | | |
| 302: 6516 | 3244 | | | | | | | | | | |
| 303: 9157 | 6322 | 11978 | 11130 | 7319 | 6594 | 12072 | 12604 | 1630 | 15835 | 15625 | 2667 |
| 11873 | 10353 | 7250 | 12387 | 18220 | 5040 | 16397 | 744 | 682 | 13620 | 15592 | 3376 |
| 7637 | 5327 | 15172 | 9136 | 3454 | 8680 | 3406 | 2563 | 8386 | 15628 | 583 | 17951 |
| 16220 | 16241 | 15828 | 2354 | 1711 | 18844 | 14933 | 14606 | 8084 | 2951 | 2023 | 12230 |
| 1131 | 1022 | 7453 | 6888 | 3757 | 3490 | 3410 | 965 | 14448 | 10758 | 3736 | 13099 |
| 5418 | 3869 | 16033 | 5419 | 9116 | 5463 | 9118 | 14280 | | | | |
| 304:13016 | 4501 | 12800 | 15199 | 17376 | 16048 | 7771 | 6495 | 2349 | 13559 | 15944 | 16686 |
| 11650 | 4749 | 7034 | 12604 | 1630 | 12213 | 1041 | 8874 | 12774 | 13164 | 15543 | 16462 |
| 2893 | 9060 | 8653 | 3035 | 6124 | 2097 | 11863 | 3682 | 3447 | 14809 | 7584 | 15021 |
| 3973 | 13598 | 10693 | 4630 | | | | | | | | |
| 305: 3217 | 3571 | | | | | | | | | | |
| 306: 9517 | 16283 | 17490 | 7415 | 537 | 6886 | 2849 | 12335 | 14505 | 13819 | 6814 | 6115 |
| 17752 | 9736 | 18191 | 8424 | 2155 | 19176 | 17694 | 8682 | 15351 | 5379 | 11881 | 11938 |
| 16662 | 17579 | 11271 | 16360 | 7659 | 16032 | 9586 | 7670 | 675 | 6708 | 13311 | 11396 |
| 4758 | 17299 | 5191 | 7346 | 8937 | 16585 | 5606 | 502 | 8374 | 3761 | 16767 | 3107 |
| 12145 | 13731 | 8754 | 11287 | 1587 | 17321 | 939 | 11556 | 13038 | 2216 | 5067 | 17391 |
| 5842 | 5661 | 9584 | 18461 | 17532 | 15765 | 12358 | 17023 | 16827 | 854 | 15019 | 3746 |
| 18406 | 3158 | 4638 | 16647 | 9956 | 5285 | 10517 | 6010 | 8267 | 3102 | 8875 | 17859 |
| 3533 | 5163 | 14049 | 12819 | 8830 | 10422 | 1545 | 11903 | 18692 | 1408 | 5782 | 10419 |
| 8743 | 2507 | 14022 | 15821 | 4156 | 18202 | 14875 | 3437 | 2493 | 5683 | 14057 | 2774 |
| 2307 | 11546 | 2862 | 18609 | 15789 | 16720 | 12528 | 4710 | 7020 | 14477 | 8362 | 15861 |
| 12266 | 3664 | 5927 | 10107 | 911 | 10257 | 9367 | 12658 | 15086 | 2329 | 12591 | 10770 |
| 17651 | 14336 | 501 | 3598 | 8283 | 9930 | 7604 | 10652 | 19129 | 5069 | 14901 | 15868 |
| 14717 | 1446 | 11406 | 12649 | 3069 | 5305 | 17167 | 2725 | 1528 | 841 | 7652 | 12267 |
| 19206 | 6331 | 417 | 12854 | 18813 | 2969 | 4834 | 15665 | 14583 | 9988 | 3933 | 12960 |
| 8189 | 8063 | 18363 | 9630 | 3955 | 8707 | 13020 | 13437 | 2029 | 7702 | 16563 | 9892 |
| 9894 | 9909 | 5345 | 4993 | 17674 | 14807 | 17748 | 923 | 13039 | 12302 | 12305 | 4593 |
| 6333 | 4582 | 4585 | 4504 | 4505 | 4507 | 4525 | 4528 | 4580 | 8892 | 16476 | 3071 |
| 15617 | 13247 | 16828 | 13307 | 12568 | 16763 | 13025 | 4662 | 9544 | 9547 | 2848 | 8455 |
| 1875 | 17554 | 14725 | 6339 | 12637 | 3861 | 16635 | 9893 | 16631 | 9377 | 4729 | 18714 |

(continued)

| SEQ ID NO: | homolog SEQ ID NOs | | | | | | | | | | |
|---|---|---|---|---|---|---|---|---|---|---|---|
| 5265 | 7136 | 17773 | 19222 | 15795 | 6317 | 17731 | 13215 | 15661 | 6496 | 15845 | 3683 |
| 18173 | 8985 | 3238 | 16239 | 8182 | 847 | 8939 | 13251 | 17330 | 15347 | 1090 | 6654 |
| 16830 | 14533 | 1434 | 10162 | 1307 | 1608 | 12047 | 9254 | 4623 | 6733 | 9845 | 3976 |
| 11791 | 6023 | 10860 | 8387 | 8388 | 3436 | 1289 | 4200 | 11245 | 14746 | 18697 | 10596 |
| 9834 | 4867 | 18801 | 4048 | 4500 | 4051 | 4125 | 4041 | 6284 | 16362 | 6481 | 2203 |
| 3253 | 17931 | 15047 | 13987 | 1184 | 17227 | 13858 | 6911 | 1625 | 7441 | 15207 | 4112 |
| 1349 | 17655 | 3809 | 17405 | 15646 | 11605 | 16230 | 15136 | 12284 | 11241 | 11022 | 9598 |
| 16189 | 16789 | 8351 | 11101 | 3405 | 17442 | 6972 | 13910 | 898 | 13694 | 11524 | 12489 |
| 6077 | 12564 | 18758 | 16224 | 10614 | 12449 | 9505 | 4294 | 14642 | 15832 | 1908 | 11003 |
| 9272 | 12730 | 18514 | 6417 | 17206 | 1004 | 475 | 12462 | 19223 | 18691 | 3383 | 6603 |
| 6725 | 3889 | 2521 | 17328 | 842 | 17029 | 3484 | 17880 | 2854 | 17158 | 11122 | 15188 |
| 19211 | 2820 | 7370 | 5183 | 5883 | 12698 | 4454 | 7017 | 10197 | 5646 | 9715 | 1157 |
| 12651 | 7968 | 7684 | 1763 | 7207 | 2196 | 13537 | 12099 | 2202 | 2253 | 3365 | 8144 |
| 13004 | 16497 | 15811 | 6265 | 2124 | 8582 | 7976 | 7231 | 18294 | 6438 | 17932 | 6485 |
| 13284 | 18660 | 16797 | 5384 | 6617 | 15439 | 18738 | 18562 | 13641 | 3118 | 11948 | 3760 |
| 5015 | 11023 | 3770 | 18364 | 1660 | 19130 | 1072 | 4534 | 12826 | 18768 | 2417 | 17234 |
| 2882 | 8417 | 9871 | 5837 | 9911 | 9132 | 11165 | 3873 | 13863 | 17480 | 9515 | 4131 |
| 6588 | 18170 | 16222 | 4230 | 5085 | 9852 | 8230 | 8227 | 18403 | 9997 | 2267 | 16180 |
| 18524 | 17253 | 7957 | 4709 | 14254 | 6788 | 4558 | 15464 | 10901 | 12863 | 16622 | 6999 |
| 4559 | 19065 | 4408 | 16389 | 18716 | 942 | 17799 | 2335 | 4291 | 14534 | 15459 | 7048 |
| 9917 | 8849 | 9747 | 6004 | 9915 | 4583 | 16306 | 3700 | 14454 | 3960 | 12902 | 9166 |
| 9769 | 2495 | 9891 | 9890 | 3163 | 4044 | 9913 | 8502 | | | | |
| 307:16966 | 1919 | 12583 | 14001 | 11131 | 6894 | 6752 | 19213 | 6877 | 6738 | 5028 | 11478 |
| 12673 | 16234 | 15971 | 15875 | 12694 | 12533 | 13333 | 11870 | 12183 | 12169 | 12146 | 12093 |
| 6307 | 12143 | 12171 | 12089 | 12074 | 12123 | 12073 | 12137 | 12118 | 12117 | 12051 | 12665 |
| 3465 | 18929 | 8042 | 12488 | 12878 | 13761 | 17647 | 6876 | 6840 | 6272 | 6835 | 11243 |
| 10315 | 19201 | 9766 | 4261 | 15639 | 16402 | 19042 | 7713 | 7791 | 16291 | 6973 | 721 |
| 1355 | 7033 | 11065 | 9853 | 14782 | 18575 | 14554 | 3292 | 6118 | 8034 | 13958 | 13312 |
| 1115 | 12766 | 8654 | 12755 | 10949 | 16650 | 16061 | 13056 | 2685 | 18534 | 4047 | 11361 |
| 8443 | 17255 | 5349 | 1847 | 9017 | 446 | 3511 | 12923 | 4315 | 3637 | 10479 | 18867 |
| 2225 | 9308 | 11987 | 12463 | 3000 | 5517 | 17776 | 14688 | 13231 | 15599 | 7869 | 11941 |
| 17438 | 16655 | 15176 | 6229 | 11914 | 18483 | 8880 | 14025 | 14630 | 18182 | 17877 | 15768 |
| 4778 | 13925 | 8338 | 5136 | 14825 | 4905 | 9898 | 7630 | 1639 | 11601 | 17037 | 2945 |
| 10431 | 17797 | 9885 | 7574 | 13621 | 10863 | 9055 | 11151 | 11755 | 7946 | 9333 | 6329 |
| 4591 | 4091 | 13981 | 5065 | 16803 | 14822 | 6964 | 3463 | 10454 | 2177 | 9360 | 11197 |
| 13234 | 15420 | 10808 | 13424 | 10292 | 8329 | 2865 | 13338 | 13339 | 16185 | 10202 | 8955 |
| 15242 | 8322 | 8223 | 16632 | 15869 | 4551 | 10418 | 2231 | 3358 | 17915 | 9080 | 15089 |
| 6338 | 2733 | 11560 | 12364 | 18802 | 12721 | 4025 | 10092 | 15814 | 3303 | 6611 | 7498 |
| 13931 | 1868 | 8572 | 9825 | 14345 | 10581 | 10007 | 18356 | 8308 | 6981 | 10752 | 8173 |
| 2904 | 2938 | 12670 | 15524 | 5073 | 14565 | 14327 | 4609 | 17256 | 18506 | 11581 | 9098 |
| 6914 | 3347 | 13049 | 18254 | 8152 | 8151 | 1224 | 10181 | 2869 | 14649 | 12087 | 9901 |
| 7952 | 1465 | 11039 | 2613 | 4283 | 3706 | 5396 | 10832 | 3362 | 5953 | 8848 | 17193 |
| 7462 | 8895 | 14087 | 12914 | 18645 | 2396 | 9188 | 10926 | 11609 | 6271 | 7379 | 3493 |
| 14614 | 3502 | 19167 | 13184 | 11475 | 419 | 18267 | 8970 | 9234 | 6938 | 16418 | 13285 |
| 18292 | 18269 | 18212 | 10727 | 18238 | 12508 | 12510 | 709 | 315 | 17725 | 8434 | 13254 |
| 861 | 17182 | 12639 | 13959 | 6309 | 2860 | 8674 | 12332 | 8623 | 8648 | 8678 | 12309 |
| 8647 | 8629 | 8697 | 18930 | 18017 | 15456 | 9239 | 3499 | 1491 | 3279 | 7455 | 13389 |
| 18685 | 13086 | 1576 | 8896 | 4057 | 3140 | 1215 | 8021 | 8040 | 11229 | 16223 | 4381 |
| 3805 | 7364 | 313 | 11613 | 14318 | 7069 | 18729 | 4708 | 12085 | | | |
| 308:17723 | 13349 | 5426 | 10342 | 646 | 8286 | 11681 | 15655 | 7162 | 2974 | 11778 | 13876 |

(continued)

| SEQ ID NO: | homolog SEQ ID NOs | | | | | | | | | | |
|---|---|---|---|---|---|---|---|---|---|---|---|
| 18386 | 10114 | 3206 | 2802 | 8806 | 8486 | 14598 | 6551 | 3387 | 18415 | 4506 | 19086 |
| 720 | 13013 | 8851 | 6410 | 18004 | 1501 | 6469 | 11711 | 8962 | 16976 | 6226 | 1146 |
| 8776 | 5079 | 13857 | 14566 | 18277 | 2698 | 3433 | 15273 | 9154 | 8961 | 1782 | 12468 |
| 2069 | 5555 | 4356 | 15859 | 12450 | 17580 | 17519 | 12015 | 4117 | 12311 | 12833 | 903 |
| 17841 | 3317 | 8289 | 13436 | 3400 | 4303 | 5816 | 15343 | 14218 | 3390 | 2829 | 15319 |
| 5048 | 4640 | 11046 | 9300 | 18120 | 5536 | 5686 | 10941 | 8685 | 12753 | 15582 | 12001 |
| 15643 | 5455 | 16989 | 2119 | 14187 | 11932 | 18938 | 1334 | 15886 | 938 | 4813 | 3811 |
| 10654 | 15534 | 18497 | 14811 | 1552 | 2661 | 5080 | 15338 | 6315 | 5225 | 13561 | 15213 |
| 1490 | 14525 | 15884 | 12359 | 6669 | 955 | 18171 | 5358 | 14624 | 2813 | 2542 | 12020 |
| 1749 | 1631 | 12016 | 6303 | 6949 | 7842 | 16125 | 18957 | 16094 | 7410 | 2120 | 16642 |
| 12078 | 11363 | 10559 | 12794 | 19168 | 13662 | 15228 | 13544 | 2606 | 18630 | 18727 | 5929 |
| 1497 | 976 | 11871 | 3924 | 5803 | 1809 | 7834 | 2156 | 15294 | 9809 | 5613 | 12582 |
| 819 | 10715 | 15196 | 13261 | 16533 | 11473 | 12028 | 18392 | 2674 | 4882 | 18532 | 3509 |
| 14873 | 10440 | 4142 | 8651 | 6824 | 745 | 9875 | 16149 | 17563 | 17373 | 18734 | 18230 |
| 8224 | 13813 | 8272 | 3398 | 4907 | 13315 | 4016 | 1330 | 18116 | 6568 | 17201 | 11312 |
| 15066 | 14302 | 4800 | 18992 | 4086 | 4549 | 309 | 6162 | 5244 | 5565 | 1899 | 11667 |
| 14224 | 16251 | 7386 | 6416 | 14759 | 14889 | 3100 | 10072 | 9146 | | | |
| 309:17723 | 13349 | 5426 | 646 | 8286 | 11681 | 15655 | 7162 | 2974 | 11778 | 13876 | 18386 |
| 10114 | 1104 | 3206 | 2802 | 8806 | 8486 | 14598 | 6551 | 3387 | 18415 | 4506 | 19086 |
| 720 | 13013 | 8851 | 6410 | 18004 | 1501 | 6469 | 11711 | 8962 | 16976 | 6226 | 1146 |
| 9010 | 8776 | 5079 | 13857 | 14566 | 18277 | 2698 | 3433 | 15273 | 9154 | 8961 | 1782 |
| 12468 | 2069 | 5555 | 4356 | 15859 | 12450 | 17580 | 17519 | 12015 | 4117 | 12311 | 12833 |
| 903 | 17841 | 3317 | 8289 | 13436 | 3400 | 4303 | 5816 | 15343 | 14218 | 3390 | 2829 |
| 15319 | 5048 | 4640 | 11046 | 9300 | 18120 | 5536 | 5686 | 10941 | 12753 | 15582 | 12001 |
| 15643 | 5455 | 2119 | 14187 | 11932 | 18938 | 15886 | 1334 | 938 | 4813 | 3811 | 15534 |
| 10654 | 18497 | 14811 | 1552 | 2661 | 5080 | 15338 | 5225 | 13561 | 1490 | 15213 | 14525 |
| 15884 | 12359 | 6669 | 955 | 18171 | 5358 | 14624 | 2813 | 2542 | 12020 | 1749 | 1631 |
| 12016 | 6303 | 7842 | 18957 | 16125 | 16094 | 7410 | 2120 | 16642 | 12078 | 11363 | 10559 |
| 12794 | 19168 | 13662 | 15228 | 13544 | 2606 | 18630 | 18727 | 5929 | 1497 | 976 | 11871 |
| 3924 | 5803 | 1809 | 7834 | 2156 | 15294 | 9809 | 5613 | 12582 | 819 | 10715 | 15196 |
| 13261 | 16533 | 12028 | 18392 | 2674 | 4882 | 18532 | 3509 | 14873 | 4142 | 10440 | 8651 |
| 6824 | 9875 | 16149 | 17563 | 17373 | 18734 | 8224 | 18230 | 13813 | 8272 | 3398 | 4907 |
| 4016 | 1330 | 13315 | 18116 | 6568 | 15447 | 11312 | 15066 | 14302 | 4800 | 18992 | 4086 |
| 5244 | 5565 | 1899 | 11667 | 14224 | 16251 | 308 | 7386 | 6416 | 14759 | 14889 | 3100 |
| 10072 | 9146 | | | | | | | | | | |
| 310:10103 | 18209 | 3920 | 6083 | 11927 | 11920 | 17472 | 4786 | 17648 | 10273 | 6306 | 9064 |
| 11347 | 7551 | 6834 | 6260 | 7334 | 11100 | 5786 | 17161 | 16591 | 12957 | 15792 | 17989 |
| 13513 | 14343 | 10276 | 5369 | 3379 | 1500 | 8808 | 10294 | 1398 | 10594 | 823 | 5710 |
| 18613 | 7856 | 14705 | 13453 | 6480 | 18733 | 15333 | 3235 | 4488 | 6348 | 1223 | 5579 |
| 469 | 6158 | 2323 | 15204 | 12704 | 7135 | 2864 | 5709 | 13549 | 16473 | 12094 | 11847 |
| 15189 | 7404 | 18021 | 16376 | 15728 | 17758 | 17759 | 10289 | 5408 | 2799 | 11658 | 9568 |
| 17803 | 1830 | 1347 | 15363 | 15824 | 3963 | 16520 | 17045 | 2659 | 4190 | 7035 | 7889 |
| 1332 | 11043 | 18965 | 13554 | 4100 | 4076 | 1488 | 17195 | 7119 | 7429 | 14837 | 14242 |
| 8145 | 8222 | 8282 | 6690 | 6705 | 10469 | 14943 | 3690 | 3684 | 6191 | 16970 | 14567 |
| 1302 | 14760 | 10891 | 10888 | 14125 | 13193 | 13805 | 9451 | 12868 | 17957 | 11774 | 2050 |
| 15443 | 2709 | 1596 | 17631 | 11812 | 4600 | 3958 | 17798 | 2617 | 17937 | 6512 | 3483 |
| 753 | 10957 | 6419 | 16465 | 9968 | 9604 | 17150 | 19182 | 9662 | 7557 | 3962 | 5101 |
| 13495 | 9576 | 17734 | 4975 | 3891 | 6104 | 8565 | 17439 | 12438 | 852 | 18005 | 14679 |
| 2768 | 3555 | 7354 | 6809 | 2693 | 1475 | 19143 | 2516 | 5214 | 12911 | 11562 | 3868 |
| 5630 | 3865 | 3819 | 5007 | 6948 | 14549 | 2971 | 7275 | 459 | 19164 | 5974 | 12430 |

(continued)

| SEQ ID NO: | homolog SEQ ID NOs | | | | | | | | | | |
|---|---|---|---|---|---|---|---|---|---|---|---|
| 5655 | 967 | 14881 | 17890 | 17331 | 14820 | 7963 | 10759 | 18574 | 16972 | 18773 | 837 |
| 2068 | 2133 | 8845 | 19013 | 19054 | 12054 | 659 | 17185 | 640 | 3444 | 14093 | 6479 |
| 14245 | 10973 | 7988 | 6296 | 3530 | 8812 | 464 | 16581 | 3767 | 15891 | 15332 | 1402 |
| 13224 | 796 | 5729 | 2002 | 14571 | 6300 | 9442 | 18423 | 12656 | 9032 | 10645 | 15161 |
| 11805 | 15637 | 12696 | 5239 | 8059 | 7922 | 17929 | 6756 | 13692 | 3094 | 1298 | 11589 |
| 8861 | 3514 | 3093 | 18298 | 1301 | 4779 | 11321 | 3090 | 13713 | 11607 | 19071 | 1621 |
| 18085 | 5907 | 15732 | 13127 | 11970 | 1178 | 14188 | 9969 | 12336 | 10649 | 10172 | 6305 |
| 4760 | 2694 | 3243 | 15374 | 8000 | 18588 | 13076 | 17379 | 6898 | 10609 | 9061 | 3321 |
| 5901 | 9808 | 3022 | 19029 | 2112 | 2759 | 9640 | 16516 | 2738 | 15881 | 6161 | 3225 |
| 17681 | 11663 | 14225 | 11836 | 18207 | 6210 | 17844 | 11690 | 11113 | 7824 | 10588 | |
| 311:16284 | 3606 | 17484 | 3724 | 12088 | 11103 | 5785 | 8336 | 4133 | 5435 | 12206 | 18097 |
| 7672 | 3473 | 10039 | 15998 | 16601 | 11983 | 18950 | 16664 | 16546 | 6636 | 7838 | 10509 |
| 16277 | 12119 | 13264 | 14523 | 7959 | 1624 | 6101 | 6558 | 6991 | 17341 | 3713 | 18780 |
| 6732 | 14843 | 10839 | 11368 | 11376 | 7124 | 2198 | 11419 | 10982 | 5660 | 10500 | 4144 |
| 13648 | 18842 | 16054 | 4831 | 5477 | 19155 | 1618 | 5333 | 2295 | 10956 | 16508 | 1271 |
| 635 | 5739 | 7739 | 16017 | 14157 | 6758 | 9889 | 14914 | 12966 | 7210 | 3982 | 17122 |
| 10837 | 12989 | 6556 | 2697 | 15231 | 12324 | 7578 | 11435 | 3445 | 18211 | 15350 | 15035 |
| 2143 | 11631 | 16505 | 5245 | 1415 | 1572 | 6829 | 15515 | 15116 | 827 | 1386 | 4407 |
| 7692 | 12842 | 18133 | 4853 | 9605 | 5531 | 3036 | 4948 | 16400 | 12572 | 17847 | 3054 |
| 13870 | 12907 | 7292 | 10290 | 17401 | 2526 | 15417 | 16488 | 10985 | 3934 | 18892 | 19082 |
| 7937 | 16671 | 8368 | 18511 | 818 | 13398 | 14650 | 10501 | 6270 | 14970 | 12668 | 13951 |
| 7076 | 9820 | 11096 | 14699 | 17676 | 16290 | 17907 | 15362 | 5385 | 15054 | 3855 | 5338 |
| 10212 | 17090 | 9087 | 2224 | 12811 | 12933 | 4815 | 8067 | 9429 | 13643 | 15267 | 687 |
| 6819 | 12318 | 2260 | 485 | 6093 | 9503 | 3108 | 7687 | 10087 | 17708 | 3704 | 11310 |
| 17316 | 18100 | 2782 | 3166 | 4676 | 7310 | 10111 | 10921 | 2460 | 16998 | 13700 | 1896 |
| 3890 | 3893 | 11460 | 1365 | 4697 | 5147 | 8506 | 17646 | 12460 | 10187 | 13390 | 2371 |
| 13266 | 12499 | 15209 | 1819 | 8062 | 1842 | 16386 | 16407 | 12516 | 17315 | 4818 | 7619 |
| 12585 | 6346 | 16345 | 9939 | 15062 | 17660 | 18237 | 18639 | 16965 | 18174 | 11756 | 7709 |
| 12790 | 15507 | 5527 | 5421 | 5857 | 13673 | 19101 | 922 | 13675 | 2047 | 15119 | 17607 |
| 15523 | 5604 | 2583 | 2462 | 11728 | 3180 | 3264 | 15426 | 15516 | 9555 | 10457 | 2383 |
| 10519 | 14033 | | | | | | | | | | |
| 312:19210 | 19017 | 2469 | 12148 | 8868 | 9122 | 8261 | 8260 | 12127 | 18655 | 10968 | 4278 |
| 7032 | 14012 | 3050 | 4438 | 1566 | 16589 | 8148 | 15936 | 18723 | 14802 | 11007 | 7676 |
| 11542 | 14797 | 3088 | 18432 | 15023 | 5711 | 4424 | 12601 | 17203 | 15601 | 15229 | 15790 |
| 10278 | 11432 | 7413 | 18876 | 16653 | 11235 | 6737 | 1684 | 6956 | 9399 | 1897 | 9520 |
| 8790 | 7451 | 8788 | 7477 | 17421 | 4351 | 19063 | 15226 | 16886 | 18163 | 18160 | 7887 |
| 17356 | 9131 | 9840 | 10435 | 14050 | 17165 | 8098 | 15335 | 15336 | 18118 | 19218 | 2079 |
| 1732 | 2705 | 7831 | 14111 | 14609 | 17488 | 13273 | 1225 | 14741 | 12285 | 12720 | 17703 |
| 3155 | 1304 | 17239 | 771 | 11531 | 6196 | 6407 | 6374 | 3679 | 4189 | 11047 | 18086 |
| 4317 | 16026 | 1757 | 18381 | 13635 | 10439 | 11504 | 15742 | 17243 | 17247 | 5416 | 8996 |
| 6566 | 6509 | 14737 | 1315 | 5853 | 7602 | 1598 | 13609 | 13378 | 6003 | 6112 | 13287 |
| 12739 | 3813 | 12645 | 6983 | 15316 | 2300 | 3894 | 3310 | 6507 | 18804 | 5203 | 18754 |
| 11533 | 4836 | 17956 | 11754 | 9830 | 16710 | 12891 | 13671 | 9767 | 12365 | 14611 | 19048 |
| 1373 | 17645 | 10205 | 7562 | 3247 | 15298 | 10090 | 3516 | 14670 | 14578 | 5400 | 7355 |
| 7287 | 6657 | 12597 | 3186 | 12801 | 9322 | 2963 | 12003 | 11372 | 9135 | 8891 | 18576 |
| 8964 | 12126 | 15692 | 16891 | 1520 | 15478 | 3021 | 13685 | 12561 | 11325 | 13562 | 3904 |
| 3248 | 7591 | 8174 | 11539 | 16285 | 14984 | 17248 | 15849 | 1431 | 17593 | 14701 | 7485 |
| 6569 | 18797 | 13075 | 11030 | 4234 | 1951 | 2406 | 6694 | 8640 | 11969 | 4529 | 17591 |
| 1573 | 2001 | 14631 | 16366 | 15065 | 3460 | 15627 | 3676 | 7030 | 15020 | 1278 | 7967 |
| 2114 | 9755 | 9758 | 11041 | 1399 | 607 | 3844 | 5187 | 1248 | 15419 | 16475 | 5059 |

(continued)

| SEQ ID NO: | homolog SEQ ID NOs | | | | | | | | | | |
|---|---|---|---|---|---|---|---|---|---|---|---|
| 12458 | 11038 | 14975 | 10096 | 12979 | 16663 | 2458 | 3448 | 14447 | 17143 | 8069 | 8074 |
| 9214 | 9217 | 3996 | 12789 | 7238 | 19174 | 227 | 12457 | 16704 | 5335 | 6029 | 17456 |
| 4994 | 11781 | 14877 | 4963 | 13433 | 13634 | 11742 | 1081 | 15826 | 15615 | 12163 | 7022 |
| 11209 | 13217 | 8104 | 13738 | 13750 | 13742 | 1514 | 7714 | 3392 | 9872 | 11401 | 13849 |
| 13797 | 16821 | 3209 | 5004 | 9810 | 2380 | 18328 | 10246 | 13309 | 16833 | 16143 | 1946 |
| 6746 | 13050 | 17027 | 12948 | 6484 | 17397 | 14869 | 14870 | 13608 | 7545 | 14441 | 6414 |
| 11135 | 15223 | 15074 | 15007 | 17620 | 14459 | 434 | 423 | 5401 | 2373 | 18030 | 16694 |
| 5537 | 6578 | 12369 | 12850 | 900 | 8960 | 10894 | 10868 | 14909 | 2286 | 14529 | 16150 |
| 12399 | 15856 | 18280 | 17002 | 516 | 15539 | 8705 | 4792 | 16311 | 11664 | 8715 | 2423 |
| 2855 | 8463 | 17865 | 9779 | 12958 | | | | | | | |
| 313:16966 | 643 | 4364 | 1919 | 12583 | 14001 | 11131 | 11866 | 6894 | 6752 | 19213 | 6877 |
| 6738 | 5028 | 11478 | 5031 | 12673 | 6883 | 16234 | 15875 | 15971 | 12694 | 13333 | 11870 |
| 12533 | 12089 | 12074 | 12093 | 12073 | 12051 | 12146 | 12143 | 12183 | 12123 | 12665 | 12117 |
| 6307 | 12137 | 12118 | 12171 | 12169 | 15777 | 2586 | 9343 | 7157 | 16204 | 4467 | 5936 |
| 5848 | 2944 | 3465 | 18929 | 8042 | 12488 | 10936 | 12878 | 5308 | 13761 | 10847 | 17647 |
| 6876 | 6835 | 6272 | 6840 | 11243 | 19201 | 10315 | 9766 | 15639 | 16402 | 1251 | 19042 |
| 8345 | 7713 | 17138 | 7791 | 14235 | 9974 | 16291 | 6973 | 721 | 14514 | 1355 | 7033 |
| 11065 | 8783 | 18451 | 18470 | 9853 | 14782 | 8294 | 14554 | 3292 | 6118 | 8034 | 8326 |
| 7882 | 13312 | 1115 | 12766 | 8654 | 11936 | 15710 | 14341 | 12674 | 12755 | 14585 | 15809 |
| 10949 | 16787 | 10621 | 16244 | 15680 | 15675 | 16650 | 13056 | 16061 | 2685 | 925 | 18534 |
| 4047 | 16841 | 11361 | 15666 | 8443 | 17255 | 9128 | 5349 | 1847 | 3511 | 12923 | 4315 |
| 10473 | 13515 | 9149 | 9152 | 10479 | 18867 | 2225 | 9308 | 5129 | 11987 | 3000 | 5517 |
| 17117 | 17091 | 17739 | 13231 | 15599 | 15256 | 7869 | 7260 | 1005 | 16655 | 15176 | 11941 |
| 17438 | 16917 | 6229 | 11914 | 18483 | 8880 | 14025 | 14630 | 1176 | 14629 | 18182 | 5047 |
| 17877 | 19064 | 2724 | 15768 | 3835 | 4778 | 13925 | 15822 | 8338 | 5136 | 14825 | 4905 |
| 9898 | 7630 | 1639 | 11601 | 17037 | 11036 | 15239 | 2945 | 10431 | 7289 | 17797 | 9885 |
| 7574 | 4849 | 13621 | 10863 | 9055 | 11628 | 11151 | 12401 | 11755 | 16894 | 8403 | 7946 |
| 9333 | 6329 | 4591 | 4589 | 4091 | 13981 | 15919 | 11572 | 11657 | 18700 | 4138 | 5065 |
| 470 | 17414 | 16358 | 16803 | 11665 | 6964 | 14822 | 10703 | 307 | 844 | 19122 | 3975 |
| 5260 | 3463 | 18429 | 10454 | 2177 | 9360 | 11197 | 14283 | 13234 | 15420 | 8184 | 9953 |
| 17197 | 10808 | 13188 | 13424 | 7005 | 10292 | 8329 | 2865 | 2866 | 2403 | 13339 | 13338 |
| 1290 | 16185 | 11883 | 10202 | 15242 | 8955 | 8322 | 11191 | 11212 | 18103 | 17940 | 17254 |
| 8223 | 6379 | 16632 | 17934 | 15869 | 2503 | 3636 | 4551 | 10418 | 2231 | 2872 | 3358 |
| 17915 | 15089 | 6338 | 2733 | 11560 | 12364 | 5805 | 18802 | 9140 | 8445 | 2502 | 3312 |
| 7828 | 12721 | 4696 | 8008 | 872 | 4025 | 10092 | 15882 | 15814 | 12371 | 3303 | 6611 |
| 13977 | 7498 | 13931 | 1868 | 8572 | 14345 | 9825 | 10581 | 10007 | 12149 | 18356 | 8308 |
| 7036 | 6981 | 10752 | 14493 | 8173 | 8136 | 2938 | 12670 | 2904 | 6932 | 5073 | 14565 |
| 2037 | 14327 | 4609 | 17256 | 2721 | 18506 | 16138 | 6028 | 16225 | 16203 | 16156 | 16198 |
| 9121 | 18322 | 16246 | 16178 | 16172 | 9129 | 16219 | 9117 | 16243 | 16158 | 11581 | 3347 |
| 6914 | 13049 | 18254 | 8151 | 8152 | 1224 | 10181 | 2869 | 9901 | 1465 | 11039 | 6971 |
| 3706 | 2613 | 4283 | 5396 | 10832 | 3362 | 5953 | 8848 | 17193 | 7462 | 18645 | 2396 |
| 9188 | 10926 | 6271 | 7379 | 19167 | 14614 | 3493 | 3502 | 13184 | 11475 | 419 | 2246 |
| 1541 | 420 | 10820 | 1865 | 1864 | 19193 | 5121 | 11566 | 3364 | 15356 | 16339 | 18267 |
| 8970 | 9234 | 2382 | 6938 | 16418 | 13285 | 18212 | 18292 | 18269 | 10727 | 18238 | 18293 |
| 6362 | 18215 | 18239 | 12508 | 12510 | 709 | 1152 | 315 | 17725 | 17697 | 8434 | 13254 |
| 861 | 13959 | 17182 | 12639 | 16406 | 12542 | 12541 | 6309 | 2860 | 3570 | 8648 | 12309 |
| 8647 | 12332 | 8623 | 8674 | 8629 | 8678 | 18930 | 18017 | 15456 | 3499 | 1491 | 3279 |
| 7455 | 13389 | 13086 | 18685 | 1576 | 8896 | 4057 | 3140 | 1215 | 8021 | 11229 | 16223 |
| 4381 | 3805 | 7364 | 3228 | 15465 | 11613 | 9609 | 14318 | 7069 | 18729 | 4708 | 12085 |
| 314:17506 | 3672 | 14034 | 18715 | 14894 | 10880 | 1849 | 16469 | 512 | 10254 | 10470 | 5726 |

(continued)

| SEQ ID NO: | homolog SEQ ID NOs | | | | | | | | | | |
|---|---|---|---|---|---|---|---|---|---|---|---|
| 14472 | 10171 | 14406 | 3718 | 16079 | 9837 | 9339 | 5104 | 17771 | 5508 | 5020 | 834 |
| 17380 | 15334 | 5096 | 4999 | 9288 | 8295 | 5472 | 13752 | 1964 | 16799 | 2401 | 13069 |
| 13691 | 13422 | 7593 | 2540 | 2717 | 5882 | 11638 | 13697 | 17811 | 5581 | 4456 | 1381 |
| 15933 | 2472 | 7620 | 16823 | 4482 | 2452 | 7400 | 16262 | 17807 | 18192 | 13790 | 18757 |
| 18673 | 10586 | 4772 | 12650 | 18934 | 3174 | 5720 | 2083 | 2874 | 4669 | 9314 | 9839 |
| 9762 | 10310 | 13888 | 5627 | 9147 | 8405 | 3728 | 11400 | 6409 | 15786 | 2828 | 15900 |
| 3340 | 3474 | 1459 | 892 | 13842 | 16047 | 11176 | 15514 | 13638 | 969 | 12485 | 7833 |
| 7242 | 10503 | 11553 | 4417 | 11411 | 1986 | 1221 | 1227 | 3527 | 18941 | 12683 | 12977 |
| 12129 | 15069 | 5885 | 1534 | 16021 | 15292 | 17543 | 4538 | 2792 | 5539 | 5519 | 17904 |
| 876 | 3164 | 18558 | 1300 | 10402 | 13522 | 14027 | 15033 | 18919 | 18259 | 4068 | 2850 |
| 17343 | 19026 | 4479 | 10493 | 5549 | 1874 | 18434 | 645 | 9644 | 14808 | 5548 | 6344 |
| 15331 | 13114 | 3446 | 2644 | 17210 | 13825 | 5662 | 15153 | 16313 | 7693 | 12780 | 16574 |
| 4686 | 11440 | 14816 | 4338 | 16346 | 1636 | 19236 | 17410 | 10101 | 7456 | 991 | 12872 |
| 3699 | 4028 | 6992 | 9962 | 4496 | 3842 | 16910 | 12042 | 2255 | 7046 | 17485 | 15064 |
| 817 | 8460 | 4635 | 12802 | 15113 | 4751 | 14557 | 1668 | 6768 | 8195 | 14547 | 2541 |
| 6164 | 13760 | 1788 | 12403 | 15927 | 17057 | 1507 | 4337 | 7148 | 3237 | 19060 | 16254 |
| 13277 | 9223 | 15237 | 9177 | 15837 | 2341 | 10611 | 11649 | 2838 | 18156 | 12666 | 16249 |
| 3138 | 6863 | 17584 | 2129 | 6563 | 15963 | 14009 | 3652 | 9433 | 2262 | 2534 | 13801 |
| 3231 | 14588 | 7872 | 11469 | 13974 | 17240 | 11323 | 14501 | 16931 | 12006 | 441 | 18417 |
| 2918 | 12097 | 12837 | 10016 | 18066 | 15815 | 3716 | 13070 | 14839 | 10827 | 10253 | 14272 |
| 13944 | 1870 | 15148 | 9697 | 10347 | 12080 | 19157 | 9054 | 18345 | 4503 | 12292 | 4395 |
| 16938 | 463 | 7974 | 14120 | 16183 | 3471 | 4782 | 14865 | 15284 | 15286 | 6396 | 8031 |
| 8017 | 18816 | 9981 | 9980 | 11274 | 17812 | 14966 | 10272 | 3420 | 3624 | 16040 | 3020 |
| 1155 | 1159 | 13046 | 8442 | | | | | | | | |
| 315:16966 | 643 | 4364 | 1375 | 1919 | 11131 | 11866 | 11486 | 6894 | 3621 | 6752 | 5028 |
| 11478 | 12673 | 6883 | 16234 | 15971 | 15875 | 12694 | 13333 | 12533 | 11870 | 12169 | 12183 |
| 12171 | 6307 | 12665 | 12143 | 12137 | 12123 | 12118 | 12117 | 12146 | 12074 | 12089 | 12051 |
| 12093 | 12073 | 15777 | 2586 | 9343 | 18929 | 8042 | 12488 | 10936 | 12878 | 5308 | 13761 |
| 17647 | 6876 | 6835 | 6272 | 6840 | 11243 | 19201 | 10315 | 15639 | 16402 | 19042 | 7713 |
| 7791 | 9974 | 16291 | 6973 | 15084 | 3115 | 721 | 14514 | 7033 | 1355 | 11065 | 1512 |
| 18451 | 18470 | 9853 | 14782 | 8294 | 18575 | 3292 | 8034 | 6118 | 8654 | 11936 | 15710 |
| 14341 | 12755 | 14585 | 10949 | 16787 | 15680 | 15675 | 16244 | 10621 | 16340 | 16061 | 925 |
| 18534 | 4047 | 11361 | 17255 | 12923 | 4315 | 10473 | 9152 | 13515 | 9149 | 9357 | 10479 |
| 18867 | 9308 | 2225 | 11987 | 12463 | 5517 | 17117 | 17091 | 17739 | 15599 | 13231 | 7869 |
| 7260 | 1005 | 11941 | 17438 | 16655 | 15176 | 16917 | 19150 | 8880 | 14025 | 14630 | 14629 |
| 18182 | 17877 | 2724 | 15768 | 3835 | 4778 | 13925 | 8338 | 10373 | 14825 | 9898 | 7630 |
| 1639 | 11601 | 17037 | 10431 | 7574 | 10863 | 9055 | 11151 | 11755 | 8403 | 9333 | 6329 |
| 4591 | 13981 | 15919 | 11657 | 11572 | 17414 | 470 | 16803 | 6964 | 14822 | 10703 | 3975 |
| 19122 | 307 | 844 | 5260 | 3463 | 2177 | 9360 | 11197 | 14283 | 13234 | 9953 | 8184 |
| 10808 | 13424 | 10292 | 8329 | 2865 | 2866 | 13338 | 13339 | 16185 | 10202 | 15242 | 8955 |
| 6473 | 11191 | 11212 | 18103 | 2638 | 8223 | 6379 | 16632 | 15869 | 10418 | 2231 | 9080 |
| 6295 | 15089 | 6338 | 2733 | 11560 | 12364 | 5805 | 18802 | 8445 | 2502 | 3312 | 7828 |
| 1747 | 12721 | 4696 | 8008 | 872 | 4025 | 10092 | 15814 | 3303 | 6611 | 13977 | 1096 |
| 13931 | 1868 | 14345 | 8572 | 9825 | 5794 | 10581 | 10007 | 18356 | 8308 | 7036 | 6981 |
| 10752 | 14493 | 8173 | 8136 | 2938 | 12670 | 2904 | 6932 | 5073 | 14565 | 14327 | 4609 |
| 17256 | 18506 | 16219 | 16178 | 6028 | 16243 | 16138 | 18322 | 16158 | 16225 | 16172 | 16203 |
| 16156 | 9121 | 16198 | 16246 | 9129 | 11581 | 6914 | 13049 | 8151 | 8152 | 1224 | 10181 |
| 2869 | 9901 | 1465 | 11039 | 4283 | 2613 | 3706 | 5396 | 10832 | 3362 | 7462 | 18645 |
| 9188 | 10926 | 6271 | 7379 | 19167 | 14614 | 3493 | 13184 | 3502 | 11475 | 419 | 17288 |
| 16086 | 4069 | 10820 | 1865 | 1864 | 420 | 3364 | 19193 | 18267 | 8970 | 9234 | 16418 |

(continued)

| SEQ ID NO: | homolog SEQ ID NOs | | | | | | | | | | |
|---|---|---|---|---|---|---|---|---|---|---|---|
| 18292 | 18212 | 10727 | 18269 | 18238 | 18293 | 18239 | 6362 | 18215 | 12508 | 709 | 915 |
| 1152 | 17725 | 13254 | 861 | 13959 | 12639 | 17182 | 12541 | 12542 | 2860 | 6309 | 8648 |
| 8697 | 8623 | 12332 | 12309 | 15456 | 3499 | 1491 | 3279 | 7455 | 8896 | 4057 | 16223 |
| 4381 | 3805 | 3228 | 15465 | 313 | 11613 | 14318 | 7069 | 18729 | 4708 | 12085 | |
| 316:18488 | 18007 | 3371 | 18022 | 6240 | 5577 | 1141 | 4858 | 15264 | 13008 | 8036 | 7857 |
| 16648 | 14599 | 16141 | 17352 | 11264 | 2922 | 7855 | 12100 | 16569 | 4670 | 11835 | 7156 |
| 2615 | 16602 | 15671 | 16213 | 1068 | 10487 | 18989 | 7745 | 13625 | 8276 | 11591 | 15968 |
| 18537 | 13558 | 1106 | 2391 | 17916 | 11931 | 7844 | 18020 | 10668 | 5894 | 2814 | 16160 |
| 4671 | 4553 | 13144 | 7837 | 8429 | 9643 | 9229 | 9330 | 12172 | 9648 | 15361 | 12367 |
| 18376 | 8325 | 3269 | 16474 | 6652 | 2059 | 10267 | 2067 | 10188 | 3795 | 3794 | 974 |
| 5364 | 7376 | 8980 | 15893 | 16484 | 2807 | 2826 | 15414 | 9081 | 10784 | 3221 | 11000 |
| 16877 | 358 | 18878 | 11643 | 14101 | 17431 | 14104 | 11922 | 14106 | 14118 | 14103 | 11917 |
| 14117 | 11877 | 9156 | 2076 | 7599 | 18286 | 4992 | 7700 | 9492 | 17313 | 12079 | 14348 |
| 4084 | 9494 | 7706 | 1297 | 17046 | 3961 | 5911 | 12363 | 8462 | 8465 | 1521 | 13528 |
| 11661 | 18323 | 10495 | 18481 | 1858 | 9034 | 9035 | 18850 | 7483 | 6754 | 17200 | 7821 |
| 11939 | 13965 | 4088 | 11316 | 6817 | 17715 | 11701 | 788 | 7905 | 18810 | 5360 | 14586 |
| 1033 | 14950 | 16858 | 8889 | 15056 | 15910 | 11640 | 9983 | 15575 | 11391 | 17626 | 17605 |
| 17606 | 17623 | 16231 | 16151 | 16155 | 16095 | 13014 | 16069 | 12870 | 3257 | 6401 | 17505 |
| 17504 | 17525 | 17524 | 1679 | 2836 | 11399 | 7377 | 432 | 16819 | 10332 | 2411 | 10331 |
| 2410 | 12098 | 12865 | 3223 | 1027 | 13770 | 13767 | 16781 | 16777 | 16779 | 16801 | 17741 |
| 16774 | 4860 | 15510 | 12777 | 12778 | 14071 | 14067 | 14053 | 14069 | 14091 | 14072 | 14075 |
| 8376 | 11001 | 9987 | 7853 | 5184 | 7393 | 7395 | 3219 | 3220 | 17517 | 3203 | 17996 |
| 3205 | 17513 | 10899 | 3204 | 18474 | 18023 | 9302 | 1882 | 17692 | 3594 | 3098 | 1993 |
| 6587 | 16786 | 17678 | 7822 | 18795 | 14425 | 2040 | 14424 | 14895 | 13347 | 18509 | 13344 |
| 18011 | 3992 | 4426 | 15577 | 11803 | 18607 | 12719 | 3019 | 2751 | 1413 | 1611 | 15567 |
| 5584 | 4242 | 18484 | 18027 | 18467 | 18028 | 17973 | 17972 | 17970 | 17969 | 7432 | 4113 |
| 4887 | 4360 | 12444 | 1657 | 8005 | 17457 | 10146 | 563 | 18067 | 7398 | 4018 | 13922 |
| 5489 | 530 | 1353 | 5529 | 6153 | 8586 | 6257 | 7997 | 19219 | 8567 | 12135 | 5520 |
| 18325 | 18513 | 18472 | 11430 | 15382 | 18181 | 7353 | 7349 | 7351 | 7348 | 7218 | 7214 |
| 7201 | 7224 | 7220 | 7200 | 17333 | 2490 | 10136 | 15058 | 19237 | 15045 | 15907 | 16396 |
| 16398 | 15912 | 15057 | 15636 | 2471 | 16403 | 15079 | 15075 | 18489 | 363 | 8942 | |
| 317:10700 | 2789 | 1693 | 18488 | 18007 | 10307 | 6070 | 15804 | 12667 | 12669 | 9807 | 11134 |
| 16489 | 16485 | 15579 | 4935 | 7730 | 2883 | 18491 | 357 | 12474 | 11486 | 15568 | 10324 |
| 1141 | 4114 | 4023 | 1324 | 1516 | 8779 | 8066 | 8071 | 8383 | 8036 | 7857 | 16769 |
| 18034 | 5299 | 8547 | 7169 | 7318 | 7344 | 8910 | 8912 | 8092 | 19202 | 8015 | 8690 |
| 14341 | 12620 | 15737 | 16340 | 15484 | 15467 | 14520 | 14452 | 14455 | 16969 | 14400 | 1724 |
| 12630 | 9906 | 2004 | 524 | 14484 | 4721 | 3025 | 7855 | 12963 | 18219 | 16511 | 16512 |
| 3275 | 16587 | 4756 | 17235 | 1571 | 2857 | 15727 | 10976 | 10974 | 12274 | 12273 | 13779 |
| 13783 | 10612 | 18384 | 9855 | 5254 | 10252 | 13600 | 18187 | 16897 | 7678 | 11829 | 2404 |
| 9908 | 5704 | 14398 | 15012 | 5140 | 2309 | 6667 | 10169 | 6704 | 16922 | 15850 | 6152 |
| 11114 | 5014 | 8102 | 9465 | 6114 | 2638 | 18541 | 4403 | 15642 | 11464 | 2905 | 11622 |
| 8995 | 1743 | 1762 | 1746 | 19009 | 1346 | 7223 | 2208 | 2193 | 17096 | 7104 | 12577 |
| 5887 | 6803 | 11121 | 18419 | 14992 | 7575 | 10188 | 3795 | 3794 | 974 | 7376 | 8980 |
| 2807 | 2826 | 7369 | 3221 | 16877 | 11643 | 14101 | 17431 | 14104 | 11922 | 14106 | 14118 |
| 14103 | 11917 | 14117 | 16324 | 16321 | 8905 | 10383 | 15152 | 2076 | 7599 | 12174 | 12185 |
| 12182 | 18286 | 13955 | 17910 | 14653 | 7417 | 12493 | 18627 | 16619 | 10333 | 1521 | 17614 |
| 17616 | 17619 | 11661 | 18323 | 10495 | 18481 | 1001 | 18455 | 7303 | 7306 | 7304 | 16744 |
| 3625 | 7860 | 4775 | 17411 | 18535 | 1033 | 15056 | 15910 | 7850 | 11458 | 17423 | 17422 |
| 10530 | 10527 | 10535 | 15563 | 3257 | 1704 | 1706 | 14317 | 6401 | 17505 | 17504 | 17525 |
| 17524 | 15208 | 11409 | 11410 | 2505 | 6022 | 14351 | 16028 | 7381 | 7377 | 432 | 17322 |

(continued)

| SEQ ID NO: | homolog SEQ ID NOs | | | | | | | | | | |
|---|---|---|---|---|---|---|---|---|---|---|---|
| 15540 | 6527 | 5564 | 5567 | 15538 | 12098 | 12865 | 3223 | 4128 | 11048 | 1117 | 6455 |
| 13770 | 13767 | 16099 | 845 | 18437 | 15972 | 15973 | 16097 | 3580 | 14537 | 10850 | 10852 |
| 13827 | 15803 | 12523 | 8855 | 16421 | 10106 | 11083 | 11745 | 4860 | 12777 | 12778 | 14071 |
| 14067 | 14053 | 14069 | 14091 | 14072 | 14075 | 8376 | 11001 | 9987 | 7853 | 5184 | 7393 |
| 7395 | 3219 | 3220 | 17517 | 3203 | 17996 | 3205 | 17513 | 10899 | 3204 | 18474 | 18023 |
| 8058 | 16661 | 9537 | 7732 | 11831 | 15167 | 7258 | 5125 | 10598 | 17302 | 18438 | 7525 |
| 16517 | 11964 | 13291 | 11188 | 16233 | 7480 | 12520 | 6579 | 13347 | 18509 | 13344 | 18011 |
| 7526 | 11573 | 6489 | 13802 | 11785 | 11585 | 3344 | 9693 | 17863 | 1377 | 9412 | 1040 |
| 18484 | 18027 | 18467 | 18028 | 17973 | 17972 | 17970 | 17969 | 6464 | 3216 | 10159 | 11232 |
| 10456 | 998 | 11571 | 4985 | 4969 | 4968 | 4962 | 4942 | 14048 | 4370 | 3286 | 503 |
| 12392 | 17021 | 5556 | 3215 | 6951 | 10681 | 7213 | 3129 | 5208 | 6043 | 9884 | 620 |
| 6698 | 7398 | 13922 | 6759 | 6761 | 6763 | 4298 | 7130 | 7141 | 1286 | 18325 | 1553 |
| 1564 | 8604 | 15411 | 10151 | 17595 | 15945 | 327 | 17353 | 1384 | 7353 | 7349 | 7351 |
| 7348 | 7218 | 7214 | 7201 | 7224 | 7220 | 7200 | 2490 | 12445 | 12412 | 12411 | 8097 |
| 7923 | 4277 | 11898 | 11906 | 11911 | 11909 | 11934 | 6086 | 17673 | 4895 | 15058 | 19237 |
| 15045 | 15907 | 16396 | 16398 | 15912 | 15057 | 15636 | 3112 | 16403 | 15079 | 15075 | 10699 |
| 13848 | 18489 | 6779 | 418 | 17149 | | | | | | | |
| 318:12599 | 499 | 18756 | 7177 | 8260 | 11959 | 18655 | 10968 | 8112 | 4278 | 7032 | 3823 |
| 14255 | 3548 | 1566 | 16589 | 16232 | 8773 | 8775 | 2276 | 2312 | 7316 | 8907 | 11235 |
| 12972 | 10692 | 17421 | 14546 | 18163 | 18160 | 7004 | 8135 | 7178 | 15335 | 15336 | 15470 |
| 4765 | 14111 | 1225 | 5814 | 4193 | 13232 | 8948 | 1200 | 11059 | 17361 | 12365 | 13450 |
| 2535 | 17627 | 10090 | 3516 | 6657 | 7287 | 11489 | 9322 | 5109 | 3778 | 9014 | 12003 |
| 3354 | 15913 | 17202 | 14866 | 9711 | 2285 | 8928 | 14738 | 10494 | 14577 | 11588 | 18862 |
| 19117 | 954 | 8153 | 13468 | 9213 | 1431 | 7485 | 4537 | 4429 | 11904 | 12478 | 12758 |
| 12095 | 14845 | 1768 | 12437 | 16579 | 15770 | 2675 | 13701 | 7914 | 9070 | 10222 | 14563 |
| 2114 | 9758 | 9755 | 11041 | 5187 | 1248 | 2305 | 10098 | 10096 | 11267 | 19200 | 14447 |
| 9214 | 9217 | 3996 | 12789 | 7238 | 2038 | 6685 | 19174 | 5335 | 4231 | 18968 | 10872 |
| 8252 | 15615 | 12163 | 11209 | 5713 | 2380 | 6538 | 13050 | 3892 | 6414 | 11753 | 17620 |
| 5399 | 16694 | 18030 | 5537 | 16265 | 17092 | 6578 | 12369 | 12850 | 900 | 10894 | 8960 |
| 10868 | 11664 | 8715 | 2423 | 8463 | 9779 | 12614 | | | | | |
| 319: 1589 | 13174 | 17604 | 12728 | 8019 | 1121 | 5422 | 814 | 2032 | 3576 | 403 | 12973 |
| 5779 | 586 | 7625 | 13532 | 14465 | 8035 | 5342 | 14260 | 12366 | 13545 | 11833 | 15130 |
| 16303 | 3048 | 15435 | 2835 | 12465 | 8722 | 4550 | 15303 | 15548 | 15934 | 3788 | 10866 |
| 10597 | 2843 | 6036 | 13084 | 15810 | 8431 | 10743 | 5277 | 5274 | 9812 | 9270 | 2756 |
| 1410 | 18832 | 3278 | 11379 | 13579 | 11278 | 4074 | 7651 | 8619 | 13040 | 8311 | 3715 |
| 16215 | 15077 | 14079 | 15855 | 12165 | 4158 | 14545 | 5156 | 12317 | 14284 | 1515 | 7176 |
| 4843 | 14556 | 12712 | 12300 | 9895 | 9617 | 18976 | 7164 | 12344 | 3521 | 1713 | 9817 |
| 8246 | 14610 | 4404 | 18916 | 18913 | 3122 | 11641 | 5193 | 5344 | 15313 | 4566 | 4856 |
| 5071 | 5093 | 4448 | 8287 | 13657 | 6526 | 4058 | 4427 | 6492 | 3925 | 10279 | 13235 |
| 2054 | 8492 | 5680 | 7588 | 11513 | 15092 | 977 | 6674 | 12978 | 14138 | 14142 | 19136 |
| 8941 | 12026 | 6528 | 11069 | 13846 | 18499 | 15946 | 14691 | 5796 | 15067 | 10116 | 4991 |
| 9572 | 9076 | 3395 | 18068 | 9445 | 17137 | 8421 | 13493 | 10070 | 12598 | 12451 | 5291 |
| 12469 | 9327 | 12751 | 12747 | 1725 | 12393 | 11867 | 6201 | 2489 | 7875 | 15355 | 19025 |
| 10704 | 2049 | 10788 | 15175 | 2284 | 16846 | 8929 | 5947 | 13843 | 15877 | 12575 | 2501 |
| 3898 | 8155 | 13467 | 7071 | 7873 | 13993 | 15899 | 13380 | 6767 | 18512 | 11884 | 11979 |
| 3330 | 15689 | 16649 | 4862 | 8196 | 6687 | 2400 | 11639 | 2206 | 6934 | 11974 | 3561 |
| 17225 | 17146 | 16272 | 5307 | 8299 | 6795 | 5491 | 4199 | 12086 | 13575 | 6650 | 3908 |
| 12452 | 9496 | 12843 | 7943 | 4725 | 12680 | 12281 | 8051 | 358 | 12760 | 4561 | 2302 |
| 8823 | 6716 | 7236 | 10428 | 18993 | 18407 | 1484 | 18587 | 1395 | 9297 | 4090 | 12363 |
| 8462 | 8465 | 1521 | 11345 | 10914 | 8048 | 3177 | 2098 | 1828 | 5023 | 13427 | 5602 |

(continued)

| SEQ ID NO: | homolog SEQ ID NOs | | | | | | | | | | |
|---|---|---|---|---|---|---|---|---|---|---|---|
| 5276 | 15578 | 11178 | 2819 | 615 | 9528 | 14414 | 9527 | 11254 | 3937 | 5441 | 1651 |
| 8198 | 12353 | 10853 | 8300 | 10913 | 10928 | 6110 | 5189 | 4050 | 12823 | 14443 | 11949 |
| 16408 | 11950 | 13618 | 13617 | 8525 | 8047 | 14589 | 17449 | 9626 | 10655 | 7897 | 16072 |
| 10344 | 1825 | 16933 | 5370 | 1337 | 16438 | 5326 | 2492 | 15690 | 4379 | 2818 | 13301 |
| 17586 | 9487 | 949 | 3316 | 11112 | 8259 | 10981 | 15481 | 11590 | 1960 | 10987 | 10959 |
| 327 | 6605 | 17324 | 1442 | 14285 | 16347 | 3109 | 8163 | 9798 | 2999 | 6258 | 19224 |
| 11771 | 10012 | 5150 | 13606 | 363 | 331 | 14915 | 19232 | 2868 | 18598 | 8400 | 8016 |
| 9887 | 12616 | | | | | | | | | | |
| 320:15632 | 2952 | 18051 | 1872 | 3610 | 1143 | 8771 | 9483 | 12004 | 11992 | 10480 | 8336 |
| 4133 | 12206 | 1141 | 7895 | 10039 | 16601 | 11983 | 10259 | 6521 | 16664 | 6287 | 8036 |
| 14786 | 12091 | 12228 | 18962 | 14523 | 7959 | 1698 | 6558 | 1392 | 17879 | 6216 | 6732 |
| 4889 | 17426 | 4101 | 11419 | 8871 | 10630 | 5660 | 18687 | 5945 | 6925 | 5477 | 13616 |
| 15059 | 18276 | 14625 | 7855 | 10956 | 9543 | 14068 | 14195 | 3384 | 17085 | 14647 | 10436 |
| 19111 | 2582 | 9763 | 1531 | 5352 | 11010 | 3327 | 14602 | 18949 | 5178 | 14833 | 3564 |
| 10837 | 7935 | 6556 | 2697 | 15231 | 10250 | 1721 | 19094 | 16923 | 14173 | 4694 | 16114 |
| 14956 | 11138 | 10083 | 15037 | 18032 | 1014 | 9937 | 3923 | 19096 | 7692 | 5154 | 12842 |
| 14677 | 3036 | 12572 | 17847 | 5502 | 13294 | 7292 | 10290 | 10985 | 1326 | 5578 | 5496 |
| 16671 | 15401 | 16633 | 14650 | 13687 | 7867 | 4516 | 3489 | 2481 | 9181 | 1640 | 17370 |
| 1859 | 9696 | 16524 | 9721 | 11096 | 15425 | 14800 | 15362 | 15449 | 2265 | 2374 | 2181 |
| 12009 | 14700 | 10716 | 9087 | 2723 | 12811 | 3421 | 18898 | 8500 | 11713 | 17270 | 18029 |
| 11943 | 9460 | 17570 | 15160 | 6926 | 4444 | 10794 | 6710 | 6707 | 4243 | 14028 | 6791 |
| 4228 | 19004 | 3795 | 10188 | 3794 | 974 | 7376 | 8980 | 12318 | 2807 | 2826 | 15644 |
| 2680 | 6093 | 358 | 11643 | 4153 | 838 | 14101 | 17431 | 11922 | 14104 | 14106 | 14118 |
| 14103 | 11917 | 14117 | 11877 | 9156 | 17708 | 2076 | 7599 | 18286 | 16788 | 18725 | 702 |
| 6728 | 1024 | 3627 | 12363 | 8462 | 8465 | 1521 | 11661 | 18323 | 10495 | 3704 | 15618 |
| 1858 | 17316 | 18100 | 2782 | 4676 | 3166 | 7310 | 10111 | 1033 | 7850 | 13576 | 15542 |
| 16151 | 16155 | 16095 | 3257 | 6401 | 17505 | 17504 | 17525 | 17524 | 1896 | 1207 | 7377 |
| 432 | 16819 | 10332 | 2411 | 10331 | 2410 | 12865 | 3223 | 4128 | 11048 | 1117 | 13770 |
| 13767 | 16781 | 16777 | 16779 | 16801 | 17741 | 16774 | 17646 | 4860 | 12777 | 12778 | 14071 |
| 14067 | 14053 | 14069 | 14091 | 14072 | 14075 | 8376 | 11001 | 9987 | 7853 | 5184 | 7393 |
| 7395 | 3219 | 3220 | 17517 | 3203 | 17996 | 3205 | 17513 | 10899 | 3204 | 12460 | 1407 |
| 10187 | 13347 | 6722 | 10917 | 13636 | 16218 | 5693 | 11859 | 5188 | 8765 | 11074 | 7398 |
| 13922 | 12516 | 17315 | 6346 | 16345 | 9939 | 15062 | 18325 | 18698 | 17660 | 16965 | 327 |
| 7298 | 5459 | 18530 | 17826 | 12176 | 15633 | 7353 | 7349 | 7351 | 7348 | 7218 | 7214 |
| 7201 | 7224 | 7220 | 7200 | 2490 | 1737 | 3491 | 13673 | 13675 | 15119 | 13602 | 15058 |
| 19237 | 3264 | 15516 | 363 | 10316 | 16637 | 2383 | 10379 | 10952 | 10563 | | |
| 321: 4787 | 1070 | 1143 | 14654 | 12004 | 9483 | 11994 | 11992 | 10480 | 8077 | 7492 | 18097 |
| 1141 | 7895 | 10039 | 16601 | 11983 | 18391 | 9162 | 16546 | 6287 | 8036 | 16050 | 12091 |
| 15109 | 5055 | 14523 | 7959 | 1698 | 6558 | 4665 | 9086 | 14143 | 3713 | 1389 | 17879 |
| 6216 | 6732 | 4288 | 10839 | 16448 | 11376 | 17426 | 7384 | 15099 | 11717 | 18687 | 3375 |
| 6925 | 2064 | 13616 | 18615 | 18276 | 1079 | 7855 | 10956 | 16326 | 14023 | 14195 | 18092 |
| 2595 | 1419 | 5878 | 14647 | 8095 | 9681 | 9889 | 1418 | 7628 | 1691 | 11010 | 3327 |
| 1291 | 14602 | 5178 | 18949 | 8748 | 10837 | 9976 | 15008 | 12989 | 6556 | 6602 | 4099 |
| 15231 | 12324 | 11078 | 12716 | 18545 | 15123 | 19094 | 1721 | 11505 | 16923 | 16087 | 11631 |
| 4694 | 4728 | 14956 | 14961 | 17360 | 12636 | 15037 | 18032 | 1014 | 19096 | 9937 | 3923 |
| 5154 | 12842 | 10269 | 14580 | 17215 | 4853 | 9605 | 4948 | 15110 | 12572 | 17847 | 5502 |
| 13294 | 16560 | 7292 | 14293 | 10985 | 1326 | 5496 | 5578 | 13565 | 16671 | 15401 | 9097 |
| 16633 | 17335 | 818 | 13398 | 13687 | 4516 | 7867 | 9181 | 17370 | 1640 | 2481 | 16524 |
| 9820 | 11096 | 18560 | 16290 | 4367 | 2374 | 2265 | 2181 | 3855 | 5338 | 16697 | 9087 |
| 3421 | 4284 | 13612 | 9460 | 15160 | 17570 | 6926 | 4444 | 13949 | 6326 | 10794 | 10325 |

(continued)

| SEQ ID NO: | homolog SEQ ID NOs | | | | | | | | | |
|---|---|---|---|---|---|---|---|---|---|---|
| 4243 | 6710 | 6707 | 14028 | 6791 | 4228 | 19004 | 10188 | 3795 | 3794 | 974 | 7376 |
| 8980 | 2807 | 7729 | 15644 | 2680 | 1890 | 6093 | 358 | 11643 | 10156 | 14101 | 17431 |
| 14104 | 11922 | 14106 | 14118 | 14103 | 11917 | 14117 | 11877 | 9156 | 17708 | 2076 | 7599 |
| 18286 | 17450 | 10636 | 10883 | 19245 | 2706 | 6728 | 16273 | 1024 | 3627 | 4931 | 10230 |
| 13967 | 10244 | 5157 | 3863 | 17448 | 17445 | 13484 | 12298 | 523 | 16683 | 14982 | 14290 |
| 11257 | 16905 | 18550 | 17602 | 17600 | 16815 | 656 | 17707 | 2933 | 4668 | 7938 | 12926 |
| 11953 | 18469 | 12363 | 8462 | 8465 | 8395 | 1521 | 11661 | 18323 | 3704 | 15618 | 1858 |
| 17316 | 17901 | 1025 | 4676 | 7310 | 13111 | 3166 | 9748 | 15261 | 7953 | 14931 | 10111 |
| 17383 | 6243 | 14337 | 11009 | 802 | 1489 | 9751 | 5535 | 19225 | 4741 | 8379 | 9986 |
| 5615 | 9570 | 7631 | 18249 | 1579 | 18874 | 13594 | 14957 | 2833 | 13097 | 11855 | 17444 |
| 15990 | 800 | 2048 | 5863 | 1033 | 7850 | 13576 | 15542 | 16151 | 16155 | 16095 | 3257 |
| 6401 | 17505 | 17504 | 17525 | 17524 | 17093 | 15754 | 18311 | 6989 | 11460 | 14526 | 7397 |
| 11457 | 1896 | 12536 | 12551 | 1207 | 1365 | 18241 | 7377 | 432 | 16819 | 10332 | 2411 |
| 10331 | 2410 | 12865 | 3223 | 4128 | 11048 | 1117 | 13770 | 13767 | 16781 | 16777 | 16779 |
| 16801 | 17741 | 16774 | 17646 | 4860 | 12777 | 12778 | 14071 | 14067 | 14053 | 14069 | 14091 |
| 14072 | 14075 | 8376 | 11001 | 9987 | 7853 | 5184 | 7393 | 7395 | 3219 | 3220 | 17517 |
| 3203 | 17996 | 3205 | 17513 | 10899 | 3204 | 4087 | 5453 | 600 | 10187 | 12890 | 1263 |
| 2658 | 12511 | 2978 | 16319 | 11645 | 9321 | 14259 | 12460 | 12499 | 5106 | 3023 | 13950 |
| 1407 | 1842 | 3668 | 11242 | 10666 | 8675 | 8209 | 5693 | 9304 | 5717 | 1495 | 8302 |
| 13979 | 6722 | 12398 | 8064 | 10221 | 16218 | 9573 | 7398 | 13922 | 3342 | 12516 | 11763 |
| 2356 | 17878 | 17315 | 16011 | 9939 | 15062 | 18325 | 18698 | 17660 | 14814 | 16965 | 2892 |
| 17826 | 12176 | 18530 | 15633 | 7353 | 7349 | 7351 | 7348 | 7218 | 7214 | 7201 | 7224 |
| 7220 | 7200 | 2490 | 5527 | 1737 | 3491 | 13673 | 13675 | 15523 | 3264 | 15516 | 16637 |
| 10316 | 8850 | 2383 | 9979 | 10952 | | | | | | | |
| 322:13867 | 13356 | 18488 | 18007 | 3183 | 12200 | 18931 | 2950 | 6222 | 3371 | 18022 | 360 |
| 5198 | 14399 | 1141 | 15264 | 17705 | 8488 | 14597 | 11044 | 8036 | 7857 | 4692 | 3130 |
| 12522 | 17069 | 10657 | 3462 | 13989 | 1149 | 6120 | 11450 | 8772 | 10736 | 17216 | 9050 |
| 6700 | 9882 | 18213 | 18369 | 14503 | 9964 | 15053 | 16115 | 7855 | 18183 | 9714 | 15235 |
| 18772 | 14715 | 3139 | 7921 | 9020 | 14812 | 701 | 2770 | 3381 | 7111 | 7814 | 9167 |
| 13764 | 3884 | 17541 | 3901 | 1277 | 12193 | 12982 | 4219 | 6713 | 2609 | 3190 | 11470 |
| 8428 | 19080 | 2937 | 2587 | 15925 | 15924 | 2921 | 2742 | 590 | 8886 | 9243 | 17583 |
| 11582 | 6545 | 15518 | 6109 | 10855 | 5127 | 14268 | 9185 | 2748 | 10188 | 3795 | 3794 |
| 974 | 7376 | 8980 | 10081 | 14401 | 16645 | 2807 | 2826 | 15414 | 3221 | 11000 | 16877 |
| 358 | 11643 | 13747 | 14101 | 17431 | 14104 | 11922 | 14106 | 14118 | 14103 | 11917 | 14117 |
| 11877 | 9156 | 2076 | 7599 | 18286 | 17450 | 10636 | 10883 | 19245 | 2706 | 17448 | 17445 |
| 13484 | 12363 | 8462 | 8465 | 1521 | 11661 | 18323 | 10495 | 18481 | 5492 | 1858 | 3922 |
| 17901 | 1025 | 15261 | 1033 | 15056 | 15910 | 16151 | 16155 | 16095 | 13014 | 16069 | 3257 |
| 6401 | 17505 | 17504 | 17525 | 17524 | 1530 | 2022 | 7377 | 432 | 16819 | 10332 | 2411 |
| 10331 | 2410 | 12098 | 12865 | 3223 | 4128 | 11048 | 1117 | 13770 | 13767 | 16781 | 16777 |
| 16779 | 16801 | 17741 | 16774 | 4860 | 15510 | 12777 | 12778 | 14071 | 14067 | 14053 | 14069 |
| 14091 | 14072 | 14075 | 8376 | 11001 | 9987 | 7853 | 5184 | 7393 | 7395 | 3219 | 3220 |
| 17517 | 3203 | 17996 | 3205 | 17513 | 10899 | 3204 | 18474 | 18023 | 600 | 13347 | 18509 |
| 13344 | 18011 | 8209 | 8302 | 18008 | 10221 | 15948 | 17612 | 8147 | 11081 | 18398 | 16052 |
| 2853 | 15154 | 15669 | 4108 | 413 | 16888 | 8441 | 3479 | 1048 | 17549 | 9749 | 17291 |
| 14470 | 2008 | 13574 | 5988 | 3547 | 13320 | 12107 | 4540 | 6031 | 16301 | 6076 | 18372 |
| 10579 | 4109 | 11366 | 19231 | 11108 | 8482 | 5412 | 12150 | 14436 | 18176 | 1610 | 674 |
| 6891 | 13730 | 8642 | 6095 | 3638 | 993 | 17479 | 11156 | 6014 | 4301 | 1954 | 10818 |
| 7039 | 15055 | 12418 | 10003 | 1924 | 13798 | 11179 | 15926 | 13552 | 9569 | 18113 | 9432 |
| 15846 | 14535 | 3145 | 12007 | 16132 | 12934 | 1538 | 16847 | 15583 | 6741 | 5534 | 14502 |
| 4641 | 7188 | 15672 | 4220 | 11219 | 12375 | 7172 | 3983 | 2230 | 6072 | 5854 | 17508 |

(continued)

| SEQ ID NO: | homolog SEQ ID NOs | | | | | | | | | | |
|---|---|---|---|---|---|---|---|---|---|---|---|
| | 17766 | 18490 | 15885 | 4617 | 4368 | 16850 | 13940 | 3991 | 1557 | 11615 | 2624 | 18508 |
| | 18457 | 10864 | 18750 | 6697 | 1128 | 12487 | 18977 | 5445 | 11199 | 10746 | 16932 | 5601 |
| | 16270 | 559 | 5072 | 16012 | 1609 | 13543 | 18010 | 5876 | 18551 | 11710 | 16973 | 8611 |
| | 17125 | 17786 | 896 | 15150 | 6585 | 6520 | 13129 | 4965 | 3423 | 18175 | 2832 | 10945 |
| | 18855 | 16525 | 7611 | 1748 | 5964 | 14612 | 11300 | 17123 | 1873 | 14105 | 1285 | 15156 |
| | 11382 | 8729 | 12956 | 17420 | 6607 | 6377 | 5035 | 9405 | 13286 | 9963 | 8621 | 9241 |
| | 13064 | 2473 | 14203 | 19067 | 17080 | 10484 | 16852 | 9591 | 6583 | 5237 | 3885 | 18484 |
| | 18027 | 18467 | 18028 | 17973 | 17972 | 17970 | 17969 | 5302 | 16042 | 6762 | 8202 | 17109 |
| | 9781 | 3057 | 12195 | 18035 | 14637 | 2924 | 5223 | 595 | 5651 | 5083 | 15409 | 17949 |
| | 13812 | 7398 | 13922 | 18325 | 17657 | 18513 | 18472 | 17113 | 17362 | 7353 | 7349 | 7351 |
| | 7348 | 7218 | 7214 | 7201 | 7224 | 7220 | 7200 | 2490 | 6747 | 4859 | 15058 | 19237 |
| | 15045 | 15907 | 16396 | 16398 | 15912 | 15057 | 15636 | 16403 | 15079 | 15075 | 18489 | 13426 |
| | 5879 | 12748 | | | | | | | | | | |
| 323: | 8509 | 1134 | 18488 | 18007 | 1916 | 3371 | 18022 | 8888 | 12349 | 10147 | 14312 | 15530 |
| | 13340 | 18024 | 1141 | 12261 | 15264 | 9171 | 10433 | 11548 | 8036 | 7857 | 15149 | 17899 |
| | 2020 | 9112 | 4452 | 4484 | 2223 | 11002 | 7352 | 6808 | 7161 | 17556 | 6400 | 7983 |
| | 2949 | 1905 | 8251 | 4178 | 4037 | 2160 | 7855 | 3729 | 10163 | 14692 | 2870 | 4621 |
| | 15928 | 5084 | 11180 | 7057 | 11633 | 9253 | 16002 | 13530 | 3418 | 11611 | 17115 | 17307 |
| | 17434 | 13885 | 9657 | 11119 | 15654 | 12427 | 3412 | 4802 | 5132 | 6456 | 1469 | 3913 |
| | 3614 | 13992 | 17416 | 16667 | 11029 | 13494 | 5616 | 2334 | 2102 | 11128 | 18331 | 15535 |
| | 16171 | 9966 | 6234 | 18709 | 6008 | 11068 | 15752 | 16073 | 16342 | 8681 | 8695 | 6474 |
| | 6357 | 9359 | 3239 | 11051 | 16468 | 10672 | 11216 | 17792 | 3226 | 13446 | 4327 | 14062 |
| | 7614 | 2101 | 12204 | 18837 | 1898 | 5001 | 15038 | 7000 | 4563 | 3830 | 12987 | 10357 |
| | 8622 | 16416 | 18809 | 13442 | 10188 | 3795 | 3794 | 974 | 7376 | 8980 | 2807 | 2826 |
| | 15414 | 11189 | 3221 | 11000 | 16877 | 358 | 11643 | 14101 | 17431 | 14104 | 11922 | 14106 |
| | 14118 | 14103 | 11917 | 14117 | 9717 | 11877 | 9156 | 2076 | 7599 | 18286 | 2975 | 17450 |
| | 10636 | 10883 | 19245 | 2706 | 17448 | 17445 | 13484 | 13267 | 12765 | 11397 | 7419 | 12363 |
| | 8462 | 8465 | 1521 | 11661 | 18323 | 10495 | 18481 | 1858 | 17901 | 1025 | 15261 | 14337 |
| | 11009 | 802 | 1489 | 9751 | 5535 | 19225 | 4741 | 8379 | 9986 | 5615 | 9570 | 18249 |
| | 1579 | 18874 | 14957 | 2833 | 13097 | 11855 | 17444 | 2048 | 5863 | 16811 | 12374 | 13302 |
| | 5440 | 14857 | 13516 | 10812 | 9419 | 6771 | 11768 | 12141 | 18295 | 11218 | 3543 | 6242 |
| | 11404 | 18052 | 2875 | 1963 | 2636 | 7150 | 5500 | 15645 | 3024 | 19039 | 805 | 11538 |
| | 17169 | 2943 | 12915 | 4471 | 17129 | 2420 | 9530 | 8029 | 15921 | 16041 | 11094 | 10747 |
| | 13795 | 2144 | 17511 | 12731 | 6924 | 15147 | 18864 | 7598 | 2259 | 7950 | 18104 | 7123 |
| | 13183 | 9498 | 651 | 5099 | 8353 | 13883 | 4389 | 2662 | 11940 | 19159 | 16364 | 4653 |
| | 14754 | 12986 | 3439 | 9888 | 4998 | 8161 | 19192 | 17068 | 10691 | 934 | 606 | 769 |
| | 8468 | 18297 | 1397 | 3167 | 10721 | 18909 | 18127 | 820 | 8767 | 11231 | 10209 | 18039 |
| | 4769 | 4271 | 914 | 5454 | 17761 | 1436 | 16749 | 12983 | 7990 | 3461 | 11482 | 2488 |
| | 19116 | 2605 | 15929 | 1132 | 11154 | 5499 | 16543 | 12081 | 4575 | 10781 | 15011 | 17427 |
| | 14182 | 608 | 1033 | 15056 | 15910 | 7850 | 13576 | 15542 | 16151 | 16155 | 15597 | 16095 |
| | 13014 | 16069 | 3257 | 14796 | 6401 | 17505 | 17504 | 17525 | 17524 | 1010 | 2022 | 7377 |
| | 432 | 16819 | 10332 | 2411 | 10331 | 2410 | 12098 | 12865 | 3223 | 4128 | 11048 | 1117 |
| | 13770 | 13767 | 16781 | 16777 | 16779 | 16801 | 17741 | 16774 | 5158 | 4860 | 15510 | 12777 |
| | 12778 | 14071 | 14067 | 14053 | 14069 | 14091 | 14072 | 14075 | 8376 | 11001 | 9987 | 7853 |
| | 5184 | 7393 | 7395 | 3219 | 3220 | 17517 | 3203 | 17996 | 3205 | 17513 | 10899 | 3204 |
| | 18474 | 18023 | 600 | 7911 | 16191 | 13238 | 15975 | 4924 | 9729 | 14251 | 6091 | 4326 |
| | 6820 | 13828 | 4812 | 9933 | 17784 | 16424 | 4768 | 15749 | 7309 | 10904 | 2071 | 4194 |
| | 15043 | 2025 | 10355 | 13347 | 18509 | 13344 | 18011 | 8209 | 8302 | 10221 | 17990 | 6825 |
| | 13135 | 15948 | 8147 | 11081 | 18398 | 17612 | 16052 | 2853 | 15154 | 15669 | 4108 | 413 |
| | 16888 | 8441 | 3479 | 1048 | 17549 | 9749 | 17291 | 14470 | 2008 | 13574 | 5988 | 3547 |

(continued)

| SEQ ID NO: | homolog SEQ ID NOs | | | | | | | | | |
|---|---|---|---|---|---|---|---|---|---|---|
| 13320 | 12107 | 4540 | 6031 | 16301 | 6076 | 18372 | 10579 | 4109 | 11366 | 19231 | 11108 |
| 8482 | 5412 | 12150 | 14436 | 18176 | 1610 | 674 | 6891 | 13730 | 8642 | 6095 | 3638 |
| 993 | 17479 | 11156 | 6014 | 4301 | 1954 | 10818 | 7039 | 15055 | 12418 | 10003 | 1924 |
| 13798 | 11179 | 15926 | 13552 | 9569 | 18113 | 9432 | 15846 | 14535 | 3145 | 12007 | 16132 |
| 12934 | 1538 | 16847 | 15583 | 6741 | 5534 | 14502 | 4641 | 7188 | 15672 | 4220 | 11219 |
| 12375 | 7172 | 3983 | 2230 | 6072 | 5854 | 17508 | 17766 | 18490 | 15885 | 4617 | 4368 |
| 16850 | 13940 | 3991 | 1557 | 11615 | 2624 | 18508 | 18457 | 10864 | 18750 | 6697 | 1128 |
| 12487 | 18977 | 5445 | 11199 | 10746 | 16932 | 5601 | 16270 | 559 | 5072 | 16012 | 1609 |
| 13543 | 18010 | 5876 | 18551 | 11710 | 16973 | 8611 | 17786 | 896 | 15150 | 6585 | 6520 |
| 13129 | 4965 | 18175 | 2832 | 17125 | 10945 | 3423 | 18855 | 16525 | 1748 | 5964 | 7611 |
| 14612 | 11300 | 17123 | 14105 | 1285 | 15156 | 11382 | 12956 | 17420 | 6607 | 6377 | 1873 |
| 5035 | 9405 | 13286 | 8729 | 9963 | 8621 | 9241 | 13064 | 2473 | 14203 | 19067 | 4331 |
| 17080 | 10484 | 16852 | 4017 | 9591 | 3885 | 6583 | 5237 | 18484 | 18027 | 18467 | 18028 |
| 17973 | 17972 | 17970 | 17969 | 5302 | 16042 | 6762 | 8202 | 17109 | 9781 | 3057 | 12195 |
| 18035 | 14637 | 2924 | 5223 | 595 | 5651 | 5083 | 15409 | 17949 | 19109 | 11395 | 17509 |
| 10365 | 16956 | 9754 | 9720 | 13131 | 5389 | 6525 | 18579 | 18245 | 18559 | 15802 | 6580 |
| 13199 | 10789 | 12935 | 13695 | 14236 | 14994 | 5044 | 6962 | 15953 | 11413 | 2116 | 18736 |
| 3803 | 13906 | 7398 | 13922 | 15230 | 9222 | 2858 | 19186 | 18325 | 18513 | 18472 | 17119 |
| 327 | 12058 | 14220 | 15762 | 7353 | 7349 | 7351 | 7348 | 7218 | 7214 | 7201 | 7224 |
| 7220 | 7200 | 18951 | 550 | 569 | 548 | 555 | 18970 | 552 | 571 | 576 | 18973 |
| 566 | 556 | 568 | 18967 | 18971 | 18927 | 18954 | 18953 | 17025 | 17030 | 16532 | 17028 |
| 17031 | 17024 | 6349 | 2490 | 13768 | 8873 | 3506 | 4724 | 6862 | 3554 | 4984 | 10907 |
| 15058 | 19237 | 15045 | 15907 | 16396 | 16398 | 15912 | 15057 | 15636 | 16403 | 15079 | 15075 |
| 13211 | 18489 | 363 | 12855 | 14320 | 9450 | 6933 | | | | | |
| 324: 9727 | 17604 | 8019 | 17534 | 5342 | 14260 | 12366 | 11833 | 15130 | 15303 | 15548 | 578 |
| 2843 | 10597 | 6036 | 3271 | 10743 | 13040 | 8311 | 16215 | 12165 | 5156 | 14545 | 12317 |
| 1515 | 9617 | 18976 | 12344 | 13678 | 9850 | 9817 | 10925 | 16912 | 3080 | 15313 | 4566 |
| 6526 | 5635 | 10279 | 9228 | 815 | 2054 | 8508 | 11583 | 3845 | 5204 | 7588 | 15092 |
| 12713 | 3464 | 977 | 15385 | 9792 | 12978 | 14138 | 12026 | 3293 | 1369 | 12116 | 9076 |
| 18068 | 12469 | 9327 | 12751 | 6201 | 19025 | 7875 | 12432 | 2049 | 10788 | 16846 | 18665 |
| 7873 | 11979 | 3330 | 15689 | 17238 | 4586 | 8514 | 2400 | 17974 | 2546 | 17225 | 15437 |
| 8299 | 4199 | 6795 | 5491 | 12086 | 12452 | 12843 | 7943 | 12052 | 6716 | 7236 | 10428 |
| 12363 | 8462 | 11345 | 8048 | 2098 | 5602 | 13427 | 1651 | 7682 | 8300 | 10853 | 12353 |
| 8198 | 10913 | 10928 | 5189 | 11954 | 11949 | 11950 | 16408 | 4232 | 4289 | 14167 | 2492 |
| 15690 | 10153 | 15481 | 11590 | 3698 | 13133 | 10987 | 10959 | 328 | 327 | 14285 | 8163 |
| 9798 | 6258 | 594 | 11771 | 17226 | 331 | 6717 | 19232 | 2907 | 18598 | 2868 | 8016 |
| 15159 | | | | | | | | | | | |
| 325:14605 | 6517 | 16865 | 17621 | 6498 | 10877 | 4478 | 535 | 7655 | 7490 | 11610 | 12325 |
| 10826 | 12155 | 329 | 359 | 362 | 360 | 357 | 9904 | 12647 | 12682 | 11322 | 13239 |
| 8952 | 4187 | 1141 | 4906 | 6775 | 3519 | 6177 | 17529 | 16576 | 10915 | 19075 | 8253 |
| 9925 | 8036 | 7079 | 14890 | 10540 | 2772 | 1228 | 9307 | 5251 | 8710 | 2343 | 9153 |
| 7253 | 6428 | 3776 | 8966 | 16276 | 8950 | 5611 | 8203 | 14908 | 4343 | 6064 | 5226 |
| 6370 | 1046 | 3282 | 3553 | 10371 | 7861 | 13057 | 10076 | 5103 | 18747 | 15674 | 3566 |
| 16824 | 4745 | 7855 | 4568 | 10122 | 3173 | 14748 | 7024 | 1998 | 18270 | 18414 | 2000 |
| 9723 | 16513 | 7623 | 3841 | 11201 | 10882 | 14758 | 7077 | 3123 | 15800 | 2884 | 8411 |
| 9932 | 14183 | 7748 | 11559 | 13821 | 13207 | 5113 | 4397 | 5376 | 8746 | 17170 | 18657 |
| 9642 | 15684 | 14665 | 326 | 15221 | 11049 | 941 | 18982 | 8593 | 6099 | 15254 | 1202 |
| 16046 | 12502 | 17716 | 9851 | 15240 | 14039 | 1414 | 18848 | 2451 | 4881 | 9525 | 5374 |
| 14767 | 14281 | 6641 | 17952 | 12133 | 15614 | 7506 | 15039 | 17536 | 2009 | 13100 | 8427 |
| 6859 | 9928 | 19163 | 4498 | 2340 | 1509 | 433 | 13478 | 14041 | 14568 | 4457 | 18018 |

(continued)

| SEQ ID NO: | homolog SEQ ID NOs | | | | | | | | | | |
|---|---|---|---|---|---|---|---|---|---|---|---|
| 17111 | 1472 | 9268 | 9056 | 10303 | 5984 | 19184 | 18805 | 15184 | 6590 | 747 | 858 |
| 1988 | 17838 | 16755 | 13510 | 14046 | 9788 | 7458 | 8799 | 8811 | 4319 | 14371 | 3222 |
| 14453 | 13005 | 1232 | 13882 | 5228 | 9624 | 2044 | 6117 | 10188 | 3795 | 3794 | 974 |
| 15693 | 18573 | 7376 | 8980 | 2807 | 2826 | 11958 | 18157 | 10192 | 3850 | 358 | 8304 |
| 11643 | 16949 | 2912 | 14101 | 17431 | 14104 | 11922 | 14106 | 14118 | 14103 | 11917 | 14117 |
| 11877 | 9156 | 1742 | 2076 | 7599 | 18286 | 2961 | 2816 | 8027 | 11540 | 897 | 17297 |
| 14617 | 12032 | 14635 | 2786 | 12115 | 1581 | 19172 | 8841 | 2722 | 3304 | 3031 | 18527 |
| 3971 | 3488 | 14435 | 3300 | 653 | 17450 | 10636 | 10883 | 19245 | 12363 | 8462 | 8465 |
| 1521 | 11661 | 18323 | 10495 | 1858 | 5752 | 18642 | 14905 | 11072 | 16126 | 15626 | 1970 |
| 11741 | 1549 | 2732 | 16716 | 11426 | 1770 | 6081 | 9927 | 11814 | 614 | 9502 | 8796 |
| 1798 | 7054 | 1033 | 7850 | 13576 | 15542 | 16151 | 16155 | 16095 | 3257 | 6401 | 17505 |
| 17504 | 17525 | 17524 | 18409 | 15499 | 8232 | 16518 | 7377 | 432 | 13504 | 13500 | 16819 |
| 10332 | 2411 | 10331 | 2410 | 12865 | 5789 | 3223 | 4128 | 11048 | 1117 | 13770 | 13767 |
| 16781 | 16777 | 16779 | 16801 | 17741 | 16774 | 4860 | 12777 | 12778 | 14071 | 14067 | 14053 |
| 14069 | 14091 | 14072 | 14075 | 8376 | 11001 | 9987 | 7853 | 5184 | 7393 | 7395 | 3219 |
| 3220 | 17517 | 3203 | 17996 | 3205 | 17513 | 10899 | 3204 | 11670 | 14581 | 14168 | 11682 |
| 12108 | 4806 | 15638 | 6252 | 19127 | 1701 | 5131 | 8012 | 3793 | 15088 | 12482 | 12339 |
| 600 | 6047 | 14198 | 11699 | 7787 | 19095 | 10717 | 12562 | 3733 | 3009 | 12416 | 9506 |
| 8209 | 6561 | 3146 | 8302 | 859 | 8778 | 10221 | 3688 | 17462 | 14789 | 17884 | 15014 |
| 6858 | 7396 | 16527 | 17887 | 4569 | 4361 | 9985 | 2465 | 3152 | 9337 | 18012 | 12479 |
| 1428 | 3121 | 12526 | 7010 | 6034 | 6248 | 5302 | 11518 | 3532 | 7398 | 13922 | 16467 |
| 9227 | 13725 | 3045 | 14411 | 11840 | 6227 | 2107 | 5626 | 3017 | 649 | 5687 | 19170 |
| 18325 | 17413 | 13970 | 18612 | 8010 | 7401 | 19015 | 328 | 7461 | 14921 | 4492 | 15663 |
| 7353 | 7349 | 7351 | 7348 | 7218 | 7214 | 7201 | 7224 | 7220 | 7200 | 2490 | 10210 |
| 1914 | 11630 | 12067 | 10526 | 7360 | 17483 | 990 | 2676 | 9138 | 1772 | 10340 | 333 |
| 4344 | 2478 | 7841 | 363 | 1767 | 4651 | 11660 | 15488 | 7901 | 7724 | | |
| 326:14605 | 6517 | 16865 | 16885 | 17621 | 6498 | 10877 | 4478 | 535 | 7655 | 7490 | 11610 |
| 12325 | 10826 | 12155 | 362 | 359 | 357 | 360 | 329 | 9904 | 12647 | 12682 | 11322 |
| 13239 | 8952 | 4187 | 2763 | 1274 | 1141 | 4906 | 6775 | 3519 | 6177 | 2747 | 18261 |
| 11684 | 17529 | 16576 | 10915 | 19075 | 8253 | 9925 | 8036 | 7079 | 4625 | 7770 | 14890 |
| 10540 | 2772 | 18979 | 1228 | 9307 | 5251 | 8710 | 2343 | 9153 | 7253 | 6428 | 3776 |
| 8966 | 16276 | 18393 | 17006 | 14683 | 8950 | 5611 | 8203 | 14908 | 4343 | 6064 | 5226 |
| 6370 | 1046 | 3282 | 3553 | 10371 | 7861 | 13057 | 10076 | 5103 | 18747 | 15674 | 3566 |
| 16824 | 18741 | 17597 | 2126 | 4745 | 7855 | 4568 | 10122 | 3173 | 14748 | 7024 | 1998 |
| 18270 | 15272 | 18414 | 2000 | 9723 | 16513 | 7623 | 3841 | 11201 | 10882 | 14758 | 7077 |
| 3123 | 325 | 15800 | 2884 | 8411 | 9932 | 14183 | 7748 | 11559 | 13821 | 13207 | 5113 |
| 4397 | 5376 | 8746 | 17170 | 18657 | 1764 | 875 | 11550 | 9642 | 15684 | 14665 | 11049 |
| 941 | 17189 | 18982 | 16732 | 8593 | 6099 | 15254 | 1202 | 16046 | 12502 | 17716 | 3827 |
| 5116 | 4146 | 9851 | 15240 | 14039 | 1414 | 17928 | 17894 | 18848 | 2451 | 522 | 4881 |
| 9525 | 5374 | 14767 | 14281 | 6641 | 17952 | 12133 | 15614 | 7506 | 15039 | 17536 | 2009 |
| 13100 | 8427 | 6859 | 9928 | 19163 | 4498 | 2340 | 1509 | 2780 | 1486 | 433 | 13478 |
| 14041 | 14568 | 4457 | 18018 | 17111 | 1472 | 9268 | 9056 | 10303 | 5984 | 19184 | 18805 |
| 15184 | 17669 | 6590 | 5317 | 5461 | 747 | 858 | 1988 | 17838 | 16755 | 13510 | 14046 |
| 9788 | 7458 | 8799 | 8811 | 4319 | 14371 | 3222 | 14453 | 13005 | 1232 | 13882 | 5228 |
| 9624 | 6117 | 2044 | 10188 | 3795 | 3794 | 974 | 15693 | 18573 | 7376 | 8980 | 2807 |
| 2826 | 11958 | 11654 | 15238 | 3291 | 12252 | 18157 | 10192 | 3850 | 9625 | 8304 | 8603 |
| 11643 | 16949 | 2912 | 14101 | 17431 | 14104 | 11922 | 14106 | 14118 | 14103 | 11917 | 14117 |
| 11877 | 9156 | 1742 | 2076 | 7599 | 18286 | 2961 | 8027 | 2816 | 12032 | 11540 | 897 |
| 17297 | 14617 | 14635 | 2786 | 12115 | 1581 | 19172 | 8841 | 2722 | 3304 | 18527 | 3031 |
| 17821 | 17385 | 10997 | 14435 | 3300 | 653 | 17450 | 10636 | 10883 | 19245 | 3971 | 3488 |

(continued)

| SEQ ID NO: | homolog SEQ ID NOs | | | | | | | | | | |
|---|---|---|---|---|---|---|---|---|---|---|---|
| 2706 | 17448 | 17445 | 13484 | 10247 | 12363 | 8462 | 8465 | 1521 | 11661 | 18323 | 1858 |
| 5752 | 18642 | 14905 | 11072 | 16126 | 15626 | 1970 | 11741 | 16716 | 1770 | 11426 | 1549 |
| 9927 | 11814 | 9502 | 614 | 6081 | 2732 | 1798 | 8796 | 7054 | 13263 | 9753 | 13032 |
| 17901 | 1025 | 15261 | 14337 | 11009 | 802 | 1489 | 9751 | 5535 | 19225 | 4741 | 8379 |
| 9986 | 5615 | 9570 | 18249 | 1579 | 18874 | 14957 | 2833 | 13097 | 11855 | 17444 | 2048 |
| 5863 | 16811 | 12374 | 1033 | 7850 | 13576 | 15542 | 16151 | 16155 | 16095 | 3257 | 6401 |
| 17505 | 17504 | 17525 | 17524 | 18409 | 15499 | 8232 | 16518 | 5559 | 9033 | 6961 | 3195 |
| 15502 | 7377 | 432 | 13504 | 13500 | 16819 | 10332 | 2411 | 10331 | 2410 | 12865 | 5789 |
| 3223 | 4128 | 11048 | 1117 | 13770 | 13767 | 16781 | 16777 | 16779 | 16801 | 17741 | 16774 |
| 4860 | 12777 | 12778 | 14071 | 14067 | 14053 | 14069 | 14091 | 14072 | 14075 | 8376 | 11001 |
| 9987 | 7853 | 5184 | 7393 | 7395 | 3219 | 3220 | 17517 | 3203 | 17996 | 3205 | 17513 |
| 10899 | 3204 | 11670 | 14581 | 14168 | 12108 | 11682 | 4806 | 6252 | 15638 | 19127 | 5131 |
| 1701 | 3793 | 8012 | 15088 | 12482 | 12339 | 600 | 1430 | 18717 | 10571 | 13132 | 2647 |
| 8686 | 9426 | 6047 | 11699 | 14198 | 7787 | 10717 | 19095 | 12562 | 3733 | 3009 | 12416 |
| 9506 | 6561 | 8209 | 3146 | 8302 | 859 | 8778 | 10221 | 3688 | 17462 | 14789 | 17884 |
| 15014 | 6858 | 4361 | 7396 | 16527 | 4569 | 17887 | 2465 | 9985 | 18012 | 9337 | 12479 |
| 3121 | 1428 | 12526 | 7010 | 7398 | 13922 | 16467 | 9227 | 13725 | 3045 | 14411 | 11840 |
| 6227 | 2107 | 5626 | 3017 | 649 | 5687 | 19170 | 18325 | 17413 | 13970 | 18612 | 8010 |
| 7401 | 19015 | 328 | 7461 | 14921 | 4492 | 15663 | 7353 | 7349 | 7351 | 7348 | 7218 |
| 7214 | 7201 | 7224 | 7220 | 7200 | 2490 | 10210 | 1914 | 11630 | 10526 | 12067 | 7360 |
| 17483 | 990 | 2676 | 9138 | 1772 | 18635 | 10340 | 333 | 4344 | 2478 | 1751 | 7841 |
| 1767 | 4651 | 11660 | 15488 | 7901 | | | | | | | |
| 327:15072 | 18488 | 18007 | 9610 | 3775 | 9607 | 3371 | 18022 | 9772 | 3587 | 3646 | 3626 |
| 3623 | 3605 | 360 | 5669 | 1833 | 18114 | 14999 | 9687 | 1141 | 4861 | 8045 | 15264 |
| 11935 | 19083 | 8036 | 7857 | 16570 | 5268 | 5235 | 7145 | 5870 | 6620 | 10640 | 18589 |
| 10476 | 17211 | 3947 | 5034 | 18317 | 4157 | 18510 | 7855 | 3915 | 11082 | 10349 | 17126 |
| 1913 | 14073 | 10520 | 6696 | 14675 | 12497 | 10231 | 5640 | 3617 | 8159 | 1342 | 12396 |
| 14967 | 15398 | 3877 | 18205 | 1444 | 8485 | 12841 | 4970 | 1296 | 14659 | 6073 | 317 |
| 332 | 12025 | 887 | 15872 | 14897 | 13021 | 3998 | 17919 | 17868 | 16067 | 1616 | 1878 |
| 6009 | 11314 | 547 | 2796 | 13566 | 13927 | 5860 | 2980 | 15914 | 9417 | 4366 | 17477 |
| | | | | | | | | | | | |
| 5293 | 4666 | 6056 | 767 | 5618 | 16102 | 12310 | 15083 | 2136 | 11779 | 13414 | 3319 |
| 9174 | 9169 | 7113 | 18279 | 9434 | 869 | 12657 | 14439 | 12954 | 14521 | 780 | 8186 |
| 9746 | 7582 | 7597 | 16451 | 10188 | 3795 | 3794 | 974 | 7376 | 8980 | 13650 | 2807 |
| 2826 | 15414 | 12722 | 3221 | 11000 | 16877 | 11643 | 5414 | 15634 | 14101 | 17431 | 14104 |
| 11922 | 14106 | 14118 | 14103 | 11917 | 14117 | 11877 | 9156 | 2076 | 15756 | 7599 | 18286 |
| 4123 | 10977 | 5267 | 4264 | 4246 | 4226 | 4247 | 4221 | 4211 | 4209 | 3742 | 17450 |
| 10636 | 10883 | 19245 | 2706 | 17448 | 17445 | 13484 | 12363 | 8462 | 8465 | 1521 | 11661 |
| 18323 | 10495 | 18481 | 1858 | 19106 | 17961 | 3487 | 5697 | 16728 | 1033 | 15056 | 15910 |
| 7850 | 13576 | 15542 | 16151 | 16155 | 16095 | 13014 | 16069 | 3257 | 6401 | 17505 | 17504 |
| 17525 | 17524 | 1966 | 2022 | 7377 | 432 | 8618 | 16819 | 10332 | 2411 | 10331 | 2410 |
| 12098 | 12865 | 3223 | 4128 | 11048 | 1117 | 13770 | 13767 | 16781 | 16777 | 16779 | 16801 |
| 17741 | 16774 | 4860 | 15510 | 12777 | 12778 | 14071 | 14067 | 14053 | 14069 | 14091 | 14072 |
| 14075 | 8376 | 11001 | 9987 | 7853 | 5184 | 7393 | 7395 | 3219 | 3220 | 17517 | 3203 |
| 17996 | 3205 | 17513 | 10899 | 3204 | 18474 | 18023 | 5912 | 12513 | 600 | 7430 | 7911 |
| 16191 | 13238 | 15975 | 4924 | 9729 | 6091 | 4326 | 6820 | 13828 | 4812 | 9933 | 17784 |
| 16424 | 4768 | 15749 | 7309 | 10904 | 2071 | 4194 | 15043 | 2025 | 10355 | 5253 | 13347 |
| 18509 | 13344 | 18011 | 3930 | 15274 | 2484 | 6139 | 18484 | 18027 | 18467 | 18028 | 17973 |
| 17972 | 17970 | 17969 | 13244 | 9306 | 12654 | 7398 | 9689 | 13922 | 6420 | 15605 | 14007 |
| 13995 | 2438 | 2437 | 19194 | 13145 | 3988 | 15004 | 11945 | 4150 | 10313 | 10359 | 18325 |

(continued)

| SEQ ID NO: | homolog SEQ ID NOs | | | | | | | | | | |
|---|---|---|---|---|---|---|---|---|---|---|---|
| 18513 | 16544 | 14378 | 18243 | 18234 | 18231 | 18233 | 18229 | 18472 | 4879 | 15706 | 2293 |
| 15916 | 7353 | 7349 | 7351 | 7348 | 7218 | 7214 | 7201 | 7224 | 7220 | 7200 | 2490 |
| 6757 | 4810 | 15090 | 319 | 316 | 322 | 320 | 323 | 15058 | 19237 | 15045 | 15907 |
| 16396 | 16398 | 15912 | 15057 | 15636 | 2327 | 16403 | 15079 | 15075 | 10665 | 18489 | 363 |
| 331 | 2956 | 15490 | 14175 | | | | | | | | |
| 328:14605 | 6517 | 16865 | 16885 | 9665 | 3001 | 17621 | 6498 | 10877 | 1351 | 708 | 4478 |
| 535 | 7655 | 7490 | 11610 | 12325 | 12155 | 359 | 329 | 9904 | 12647 | 12682 | 11322 |
| 13239 | 8952 | 7452 | 4187 | 6693 | 2763 | 1274 | 1141 | 629 | 17824 | 7900 | 4906 |
| 6775 | 6177 | 3519 | 2747 | 18261 | 11684 | 17529 | 16576 | 10915 | 2317 | 19075 | 2548 |
| 8253 | 4198 | 9925 | 8036 | 7079 | 4625 | 7770 | 14890 | 14154 | 10540 | 2772 | 18979 |
| 8825 | 1228 | 9307 | 8673 | 5251 | 8710 | 2343 | 9153 | 6428 | 1481 | 17991 | 3776 |
| 8966 | 12834 | 16276 | 18393 | 17006 | 14683 | 8950 | 11563 | 4180 | 5611 | 519 | 8203 |
| 14908 | 4343 | 6872 | 6064 | 5226 | 6370 | 1046 | 3282 | 3553 | 16693 | 848 | 10371 |
| 15841 | 7612 | 7861 | 13057 | 5103 | 18747 | 17001 | 15674 | 3566 | 16824 | 18741 | 17597 |
| 2126 | 2014 | 4745 | 7855 | 4568 | 10122 | 10969 | 3173 | 2544 | 14748 | 7024 | 1998 |
| 18270 | 15272 | 14644 | 18414 | 2000 | 9723 | 5269 | 16513 | 7623 | 3841 | 11201 | 10505 |
| 10882 | 14758 | 2552 | 5991 | 7077 | 8626 | 3123 | 5046 | 325 | 8727 | 777 | 13918 |
| 15800 | 2884 | 3466 | 8448 | 17617 | 8411 | 8918 | 4089 | 12752 | 9932 | 14183 | 7748 |
| 11559 | 13821 | 13207 | 7858 | 15656 | 13994 | 5113 | 6395 | 825 | 4397 | 12175 | 5376 |
| 8746 | 17170 | 18657 | 875 | 1764 | 11550 | 9642 | 15684 | 14665 | 326 | 15221 | 2042 |
| 11049 | 13548 | 6784 | 941 | 18982 | 16732 | 17189 | 332 | 8593 | 6099 | 1740 | 15546 |
| 18142 | 15254 | 1202 | 1281 | 15791 | 16046 | 15474 | 13180 | 11077 | 12502 | 17716 | 3827 |
| 5116 | 4146 | 9851 | 15240 | 987 | 14039 | 12727 | 1414 | 17894 | 17928 | 12662 | 18848 |
| 522 | 2451 | 4881 | 13061 | 9525 | 10424 | 5374 | 427 | 14767 | 10179 | 14281 | 6641 |
| 17952 | 12133 | 15614 | 17071 | 7506 | 3584 | 15039 | 17536 | 2009 | 13100 | 8427 | 16760 |
| 6859 | 9928 | 19163 | 7166 | 4498 | 14733 | 2340 | 3068 | 1816 | 1509 | 2780 | 1486 |
| 433 | 16785 | 13478 | 18959 | 17854 | 14041 | 16004 | 14568 | 15205 | 4457 | 2876 | 18018 |
| 14884 | 1600 | 17111 | 10757 | 1472 | 766 | 9268 | 9056 | 10303 | 5984 | 19184 | 18805 |
| 18151 | 15184 | 17669 | 18943 | 6590 | 5461 | 5317 | 6134 | 747 | 858 | 1988 | 17838 |
| 10028 | 16755 | 13423 | 5944 | 13510 | 9955 | 14046 | 9788 | 7458 | 8799 | 8811 | 4319 |
| 14371 | 6600 | 1850 | 17710 | 3222 | 4455 | 5432 | 14453 | 13005 | 1232 | 18987 | 5228 |
| 13882 | 9624 | 2044 | 6117 | 10338 | 10188 | 3795 | 3794 | 974 | 15693 | 18573 | 7376 |
| 8980 | 2807 | 2826 | 11958 | 11654 | 15238 | 3291 | 12252 | 18157 | 10192 | 3850 | 9625 |
| 358 | 8304 | 8603 | 11643 | 16949 | 2912 | 1565 | 15603 | 13762 | 2805 | 2809 | 8467 |
| 2804 | 10050 | 17132 | 14101 | 17431 | 14104 | 11922 | 14106 | 14118 | 14103 | 11917 | 14117 |
| 11877 | 9156 | 1742 | 2076 | 7599 | 18286 | 2961 | 2360 | 8027 | 2816 | 11540 | 897 |
| 17297 | 14617 | 12032 | 3304 | 3031 | 18527 | 14635 | 2786 | 12115 | 1581 | 19172 | 8841 |
| 2722 | 17450 | 10636 | 10883 | 19245 | 2706 | 3971 | 3488 | 17821 | 17385 | 17448 | 17445 |
| 3300 | 13484 | 10997 | 14435 | 653 | 10247 | 12363 | 8462 | 8465 | 1521 | 11661 | 18323 |
| 10495 | 1858 | 18642 | 5752 | 14905 | 11072 | 16126 | 15626 | 1970 | 614 | 9927 | 9502 |
| 6081 | 8796 | 1798 | 1549 | 11741 | 11426 | 16716 | 1770 | 11814 | 2732 | 13032 | 7054 |
| 17901 | 1025 | 13263 | 9753 | 15261 | 14337 | 11009 | 802 | 1489 | 9751 | 5535 | 19225 |
| 4741 | 8379 | 9986 | 5615 | 9570 | 18249 | 1579 | 18874 | 14957 | 2833 | 13097 | 11855 |
| 17444 | 13520 | 2048 | 5863 | 16811 | 12374 | 13302 | 5440 | 14857 | 13516 | 10812 | 9419 |
| 6771 | 11768 | 12141 | 3543 | 6242 | 11404 | 10175 | 18052 | 1963 | 2636 | 17662 | 7150 |
| 2875 | 5500 | 15645 | 3024 | 19039 | 805 | 11538 | 2943 | 12915 | 17169 | 4471 | 17129 |
| 2420 | 9530 | 8029 | 15921 | 16041 | 11094 | 18222 | 10747 | 13795 | 2144 | 17511 | 12731 |
| 6924 | 15147 | 18864 | 7123 | 7598 | 2259 | 7950 | 18104 | 13183 | 9498 | 651 | 5099 |
| 8353 | 13883 | 1033 | 7850 | 13576 | 15542 | 16151 | 16155 | 16095 | 3257 | 6401 | 17505 |
| 17504 | 17525 | 17524 | 18409 | 15499 | 8232 | 6961 | 16518 | 15502 | 5559 | 9033 | 3195 |

(continued)

| SEQ ID NO: | homolog SEQ ID NOs | | | | | | | | | | |
|---|---|---|---|---|---|---|---|---|---|---|---|
| 7377 | 432 | 13504 | 13500 | 2411 | 16819 | 10332 | 10331 | 2410 | 12865 | 5789 | 3223 |
| 4128 | 11048 | 1117 | 13770 | 13767 | 16781 | 16777 | 16779 | 16801 | 17741 | 16774 | 4860 |
| 12777 | 12778 | 14071 | 14067 | 14053 | 14069 | 14091 | 14072 | 14075 | 8376 | 11001 | 9987 |
| 7853 | 5184 | 7393 | 7395 | 3219 | 3220 | 17517 | 3203 | 17996 | 3205 | 17513 | 10899 |
| 3204 | 11670 | 14581 | 14168 | 11682 | 12108 | 6252 | 15638 | 15088 | 1701 | 4806 | 19127 |
| 3793 | 5131 | 1430 | 8012 | 600 | 10571 | 2647 | 12339 | 12482 | 13132 | 18717 | 5066 |
| 9426 | 8686 | 7911 | 13347 | 6047 | 14198 | 11699 | 7787 | 19095 | 3733 | 10717 | 3009 |
| 9506 | 12562 | 12416 | 8209 | 6561 | 8778 | 8302 | 3146 | 10221 | 859 | 3688 | 15948 |
| 8147 | 14789 | 17462 | 17884 | 15014 | 6858 | 18012 | 4361 | 4569 | 16527 | 7396 | 17887 |
| 2465 | 9985 | 3152 | 13120 | 5302 | 3121 | 7010 | 1428 | 6034 | 6248 | 16042 | 6762 |
| 11518 | 8202 | 17109 | 9781 | 3532 | 3057 | 12195 | 18035 | 14637 | 2924 | 12479 | 5223 |
| 2218 | 9337 | 595 | 12526 | 17290 | 5651 | 2890 | 5083 | 15409 | 581 | 2226 | 17949 |
| 6501 | 15384 | 19109 | 7049 | 11159 | 11395 | 7613 | 14638 | 12405 | 1009 | 15823 | 12586 |
| 5832 | 468 | 13131 | 5389 | 6525 | 7398 | 13922 | 16467 | 13725 | 3045 | 14411 | 11840 |
| 6227 | 2107 | 5626 | 3017 | 5027 | 649 | 5687 | 5560 | 19170 | 18325 | 17413 | 13970 |
| 18612 | 8010 | 7401 | 19015 | 7461 | 6703 | 453 | 14921 | 4492 | 15663 | 7353 | 7349 |
| 7351 | 7348 | 7218 | 7214 | 7201 | 7224 | 7220 | 7200 | 2490 | 10210 | 7497 | 5053 |
| 1914 | 11630 | 10526 | 7360 | 12067 | 17483 | 990 | 2676 | 9138 | 1772 | 18635 | 17757 |
| 10340 | 5732 | 333 | 15080 | 321 | 324 | 15058 | 19237 | 4344 | 2478 | 1751 | 7841 |
| 1767 | 4651 | 11660 | 15488 | 7901 | 7724 | | | | | | |
| 329:17587 | 18488 | 18007 | 3371 | 18022 | 12974 | 8171 | 13047 | 1141 | 15264 | 8036 | 7857 |
| 17407 | 5542 | 7855 | 325 | 326 | 332 | 3829 | 10188 | 3795 | 3794 | 974 | 7376 |
| 8980 | 2807 | 2826 | 15414 | 3221 | 11000 | 16877 | 11643 | 14101 | 17431 | 14104 | 11922 |
| 14106 | 14118 | 14103 | 11917 | 14117 | 11877 | 9156 | 15521 | 15519 | 15512 | 2076 | 7599 |
| 18286 | 17450 | 10636 | 10883 | 19245 | 2706 | 17448 | 17445 | 13484 | 12363 | 8462 | 8465 |
| 1521 | 11661 | 18323 | 10495 | 18481 | 1858 | 17901 | 1025 | 15261 | 14337 | 11009 | 802 |
| 1489 | 9751 | 5535 | 19225 | 4741 | 8379 | 9986 | 5615 | 9570 | 18249 | 1579 | 18874 |
| 14957 | 2833 | 13097 | 11855 | 17444 | 2048 | 5863 | 16811 | 12374 | 13302 | 5440 | 14857 |
| 13516 | 10812 | 9419 | 6771 | 11768 | 12141 | 3543 | 6242 | 11404 | 18052 | 1963 | 2636 |
| 7150 | 2875 | 5500 | 15645 | 3024 | 19039 | 805 | 11538 | 2943 | 12915 | 17169 | 4471 |
| 17129 | 2420 | 9530 | 8029 | 15921 | 16041 | 11094 | 10747 | 12731 | 13795 | 2144 | 17511 |
| 6924 | 15147 | 18864 | 7598 | 2259 | 7950 | 18104 | 7123 | 13183 | 9498 | 651 | 5099 |
| 8353 | 13883 | 4389 | 2662 | 11940 | 19159 | 16364 | 4653 | 14754 | 12986 | 3439 | 9888 |
| 4998 | 8161 | 19192 | 17068 | 10691 | 934 | 606 | 769 | 8468 | 18297 | 1397 | 3167 |
| 10721 | 18909 | 18127 | 820 | 8767 | 11231 | 10209 | 18039 | 4769 | 4271 | 914 | 5454 |
| 17761 | 1436 | 16749 | 12983 | 7990 | 3461 | 11482 | 2488 | 2605 | 15929 | 19116 | 1132 |
| 11154 | 5499 | 16543 | 12081 | 4575 | 10781 | 15011 | 17427 | 14182 | 11494 | 4324 | 686 |
| 8119 | 16545 | 10123 | 1033 | 15056 | 15910 | 7850 | 13576 | 15542 | 6776 | 16151 | 16155 |
| 16095 | 13014 | 16069 | 3257 | 6401 | 17505 | 17504 | 17525 | 17524 | 2022 | 7377 | 432 |
| 16819 | 10332 | 2411 | 10331 | 2410 | 12098 | 12865 | 3223 | 4128 | 11048 | 1117 | 13770 |
| 13767 | 16781 | 16777 | 16779 | 16801 | 17741 | 16774 | 4860 | 15510 | 15508 | 12777 | 12778 |
| 14071 | 14067 | 14053 | 14069 | 14091 | 14072 | 14075 | 8376 | 11001 | 9987 | 7853 | 5184 |
| 7393 | 7395 | 3219 | 3220 | 17517 | 3203 | 17996 | 3205 | 17513 | 10899 | 3204 | 18474 |
| 18023 | 600 | 7911 | 16191 | 13238 | 15975 | 4924 | 9729 | 6091 | 4326 | 6820 | 13828 |
| 4812 | 9933 | 17784 | 16424 | 4768 | 15749 | 7309 | 10904 | 2071 | 4194 | 15043 | 2025 |
| 10355 | 5253 | 18478 | 2062 | 13347 | 18509 | 13344 | 18011 | 8209 | 8302 | 10221 | 15948 |
| 8147 | 11081 | 18398 | 17612 | 16052 | 2853 | 15154 | 15669 | 4108 | 413 | 16888 | 8441 |
| 3479 | 1048 | 17549 | 9749 | 17291 | 14470 | 2008 | 13574 | 5988 | 3547 | 13320 | 12107 |
| 4540 | 6031 | 16301 | 6076 | 18372 | 10579 | 4109 | 11366 | 19231 | 11108 | 8482 | 5412 |
| 12150 | 14436 | 18176 | 1610 | 674 | 6891 | 13730 | 8642 | 6095 | 3638 | 993 | 17479 |

(continued)

| SEQ ID NO: | homolog SEQ ID NOs | | | | | | | | | | |
|---|---|---|---|---|---|---|---|---|---|---|---|
| 11156 | 6014 | 4301 | 1954 | 10818 | 7039 | 15055 | 12418 | 10003 | 1924 | 13798 | 11179 |
| 15926 | 13552 | 9569 | 18113 | 9432 | 15846 | 14535 | 3145 | 12007 | 1128 | 16132 | 12934 |
| 1538 | 16847 | 15583 | 6741 | 5534 | 14502 | 4641 | 7188 | 15672 | 4220 | 11219 | 12375 |
| 7172 | 3983 | 2230 | 6072 | 5854 | 17508 | 17766 | 18490 | 16932 | 15885 | 4617 | 4368 |
| 16850 | 13940 | 3991 | 1557 | 11615 | 2624 | 18508 | 18457 | 10864 | 18750 | 6697 | 12487 |
| 18977 | 5445 | 11199 | 10746 | 5601 | 16270 | 559 | 5072 | 16012 | 1609 | 13543 | 18010 |
| 5876 | 18551 | 11710 | 16973 | 8611 | 17786 | 896 | 15150 | 6585 | 6520 | 13129 | 4965 |
| 18175 | 2832 | 10945 | 3423 | 17125 | 18855 | 16525 | 1748 | 5964 | 11300 | 7611 | 14612 |
| 17123 | 14105 | 1285 | 15156 | 11382 | 12956 | 17420 | 6607 | 6377 | 1873 | 5035 | 9405 |
| 13286 | 8729 | 9963 | 8621 | 9241 | 13064 | 2473 | 14203 | 19067 | 17080 | 10484 | 16852 |
| 9591 | 6583 | 5237 | 3885 | 11942 | 14024 | 5941 | 2321 | 17997 | 12578 | 4450 | 13443 |
| 18484 | 18027 | 18467 | 18028 | 17973 | 17972 | 17970 | 17969 | 5302 | 16042 | 6762 | 8202 |
| 17109 | 9781 | 3057 | 12195 | 18035 | 14637 | 2924 | 5223 | 595 | 5651 | 5083 | 15409 |
| 17949 | 19109 | 11395 | 13131 | 5389 | 6525 | 18579 | 18245 | 18559 | 15802 | 6580 | 13199 |
| 10789 | 12935 | 13695 | 14236 | 5044 | 6962 | 15953 | 11413 | 2116 | 18736 | 3803 | 13906 |
| 7398 | 13922 | 18325 | 18513 | 18472 | 6413 | 7353 | 7349 | 7351 | 7348 | 7218 | 7214 |
| 7201 | 7224 | 7220 | 7200 | 2490 | 15058 | 19237 | 15045 | 15907 | 16396 | 16398 | 15912 |
| 15057 | 15636 | 16403 | 15079 | 15075 | 10664 | 18489 | 363 | | | | |
| 330:14603 | 515 | 18387 | 18343 | 2331 | 4920 | 18684 | 3585 | 1086 | 2105 | 10280 | 4477 |
| 534 | 16554 | 580 | 5939 | 12908 | 11544 | 9876 | 8805 | 3452 | 7516 | 362 | 357 |
| 360 | 5160 | 7225 | 7197 | 13693 | 11971 | 8978 | 7475 | 8946 | 4188 | 6695 | 4175 |
| 17891 | 18486 | 5737 | 5750 | 15281 | 8580 | 1376 | 1379 | 17822 | 7898 | 10455 | 14342 |
| 7382 | 7888 | 7445 | 738 | 5438 | 17922 | 9221 | 17902 | 7986 | 4172 | 12385 | 14898 |
| 15214 | 12314 | 3725 | 15232 | 8116 | 18161 | 2487 | 2559 | 19074 | 4201 | 8255 | 9921 |
| 14137 | 7777 | 5356 | 14888 | 14152 | 6278 | 2470 | 2755 | 18980 | 718 | 16531 | 1230 |
| 3927 | 8672 | 9283 | 3995 | 6079 | 2339 | 851 | 11164 | 7252 | 9267 | 3883 | 1479 |
| 17993 | 1590 | 2294 | 3774 | 5271 | 6390 | 8965 | 11604 | 10999 | 10176 | 17396 | 18394 |
| 17008 | 14682 | 13895 | 15309 | 484 | 17399 | 8933 | 5612 | 9198 | 6892 | 6990 | 9137 |
| 8204 | 8167 | 19059 | 18580 | 17301 | 6873 | 4340 | 11451 | 6065 | 9085 | 11944 | 11248 |
| 18110 | 10733 | 7794 | 5227 | 7083 | 8278 | 15410 | 16695 | 3552 | 7053 | 2668 | 15839 |
| 10369 | 14061 | 7862 | 13065 | 12871 | 3847 | 16010 | 12618 | 5102 | 5723 | 5348 | 9583 |
| 9532 | 15763 | 13830 | 17548 | 15673 | 5777 | 18062 | 18742 | 15685 | 17435 | 421 | 835 |
| 1904 | 3265 | 9700 | 2180 | 2753 | 4596 | 12734 | 10970 | 18093 | 18831 | 2545 | 10816 |
| 7026 | 12691 | 6318 | 2499 | 1999 | 2567 | 19001 | 1394 | 6365 | 9719 | 12315 | 8324 |
| 11811 | 5666 | 16514 | 18475 | 15956 | 10933 | 3273 | 5918 | 3838 | 706 | 10504 | 2553 |
| 10861 | 5990 | 9018 | 8809 | 8627 | 8584 | 13596 | 2608 | 10275 | 12278 | 8725 | 779 |
| 17013 | 4078 | 3280 | 18893 | 18315 | 14793 | 18945 | 4573 | 3468 | 2115 | 18906 | 8412 |
| 2897 | 8921 | 7583 | 9677 | 14390 | 13256 | 14184 | 7750 | 11561 | 7723 | 7768 | 13823 |
| 8577 | 13165 | 7859 | 18546 | 7116 | 5043 | 4449 | 2137 | 6398 | 7799 | 7196 | 12151 |
| 10862 | 18748 | 5377 | 3616 | 17168 | 11492 | 15246 | 18824 | 12600 | 895 | 1785 | 14661 |
| 15224 | 16802 | 985 | 326 | 14632 | 13546 | 16571 | 9996 | 16735 | 332 | 17191 | 18450 |
| 18771 | 1741 | 18129 | 6098 | 15547 | 15586 | 15257 | 15805 | 1279 | 16479 | 15476 | 11079 |
| 13181 | 16731 | 12486 | 18648 | 16167 | 4143 | 15009 | 15010 | 17134 | 12729 | 1417 | 10964 |
| 14712 | 5504 | 5721 | 3199 | 2622 | 13058 | 2979 | 10423 | 8307 | 3641 | 4854 | 3541 |
| 10183 | 735 | 16996 | 732 | 17406 | 9929 | 4647 | 12132 | 17070 | 14450 | 8676 | 17537 |
| 14527 | 14925 | 11247 | 5570 | 16918 | 18300 | 6997 | 13078 | 7933 | 12177 | 10965 | 7654 |
| 19120 | 14122 | 8609 | 10040 | 1506 | 2233 | 3802 | 17276 | 16782 | 19107 | 18939 | 3104 |
| 10995 | 1912 | 16003 | 8348 | 8420 | 13916 | 14569 | 15206 | 15711 | 8756 | 7204 | 18526 |
| 6423 | 11090 | 14885 | 1583 | 14210 | 10760 | 17110 | 10117 | 768 | 1474 | 2743 | 3191 |
| 3911 | 8963 | 9058 | 10304 | 19183 | 5960 | 15372 | 18803 | 8355 | 18130 | 750 | 16208 |

(continued)

| SEQ ID NO: | homolog SEQ ID NOs | | | | | | | | | | |
|---|---|---|---|---|---|---|---|---|---|---|---|
| 2485 | 1433 | 1895 | 2007 | 18492 | 17590 | 5481 | 5304 | 6130 | 3953 | 5967 | 748 |
| 856 | 9398 | 1989 | 18983 | 10032 | 1308 | 16754 | 13420 | 17236 | 1270 | 10884 | 13511 |
| 9952 | 4531 | 14047 | 9791 | 584 | 6920 | 6129 | 6133 | 14369 | 17710 | 6599 | 16186 |
| 14499 | 14532 | 18914 | 3224 | 5430 | 4453 | 6290 | 4141 | 4547 | 14451 | 13001 | 5357 |
| 5676 | 10109 | 18990 | 1226 | 13884 | 6373 | 11384 | 16391 | 15289 | 9623 | 10336 | 2046 |
| 10225 | 5637 | 18760 | 2679 | 15287 | 15299 | 15005 | 10538 | 2274 | 15858 | 2366 | 4715 |
| 18226 | 7226 | 10000 | 14899 | 12251 | 11655 | 2326 | 12860 | 12845 | 12858 | 15650 | 4472 |
| 18162 | 7699 | 13374 | 1938 | 6890 | 2517 | 358 | 13718 | 9245 | 3740 | 8301 | 10036 |
| 10037 | 11598 | 13156 | 6062 | 11813 | 4731 | 9348 | 6264 | 18880 | 2358 | 3478 | 2537 |
| 16692 | 8028 | 12689 | 15658 | 12574 | 1734 | 17135 | 12440 | 3578 | 17624 | 18905 | 3691 |
| 11371 | 14828 | 6677 | 14887 | 14573 | 6627 | 16604 | 3255 | 4171 | 10829 | 5926 | 18882 |
| 5653 | 2514 | 7948 | 13401 | 11986 | 7884 | 18359 | 3148 | 17208 | 7280 | 18155 | 13200 |
| 5115 | 19203 | 15440 | 13521 | 13501 | 12684 | 5790 | 561 | 14678 | 4152 | 7008 | 7589 |
| 17500 | 5692 | 3103 | 16843 | 8691 | 14121 | 4961 | 15699 | 18887 | 9148 | 13225 | 11743 |
| 4031 | 10545 | 13773 | 9282 | 6505 | 2815 | 11385 | 4383 | 5212 | 11549 | 3097 | 9160 |
| 603 | 3512 | 11976 | 13010 | 1548 | 10287 | 8901 | 2266 | 12643 | 8381 | 14274 | 11452 |
| 16500 | 16464 | 16442 | 16552 | 16470 | 13726 | 7065 | 7063 | 14056 | 10680 | 7067 | 7043 |
| 17663 | 14404 | 11839 | 11995 | 2011 | 3141 | 12589 | 18452 | 3852 | 6660 | 2217 | 4465 |
| 4565 | 3018 | 1851 | 13717 | 14662 | 18960 | 14978 | 15034 | 15793 | 17415 | 10448 | 11989 |
| 6298 | 11606 | 12404 | 7683 | 2094 | 10265 | 13451 | 9523 | 9057 | 6506 | 14626 | 14596 |
| 17585 | 4757 | 2467 | 2131 | 1958 | 16227 | 12425 | 8409 | 455 | 7459 | 3609 | 8867 |
| 4350 | 2093 | 19030 | 2060 | 13896 | 8024 | 15664 | 1766 | 15442 | 6244 | 7496 | 4892 |
| 10377 | 11975 | 3002 | 3671 | 4437 | 9335 | 19108 | 17895 | 16401 | 10085 | 16164 | 1056 |
| 4981 | 1848 | 11707 | 15836 | 5124 | 1138 | 18857 | 3644 | 10102 | 10006 | 9263 | 10291 |
| 12852 | 3475 | 6442 | 16263 | 9780 | 5177 | 11318 | 1765 | 19178 | 18695 | 18669 | 9242 |
| 4650 | 8073 | 7725 | 10485 | 17246 | | | | | | | |
| 331:14603 | 515 | 18387 | 18343 | 10567 | 2331 | 4920 | 18684 | 3585 | 2105 | 10280 | 534 |
| 4477 | 16554 | 580 | 5939 | 12908 | 11544 | 9876 | 8805 | 3452 | 7516 | 360 | 357 |
| 362 | 359 | 329 | 5160 | 7225 | 7197 | 13693 | 11971 | 8978 | 7475 | 8946 | 4188 |
| 6695 | 4175 | 17891 | 18486 | 15281 | 8580 | 1376 | 1379 | 17822 | 7898 | 14342 | 10455 |
| 7888 | 7382 | 7445 | 738 | 5438 | 17922 | 9221 | 17902 | 7986 | 4172 | 12385 | 14898 |
| 11160 | 15214 | 12314 | 3725 | 15232 | 8116 | 18161 | 2487 | 19074 | 2559 | 8255 | 4201 |
| 9552 | 9921 | 14137 | 7777 | 5356 | 14888 | 14152 | 6278 | 2470 | 2755 | 18980 | 718 |
| 16531 | 1230 | 3927 | 9283 | 8672 | 3995 | 6079 | 2339 | 851 | 11164 | 7252 | 9267 |
| 3883 | 1479 | 17993 | 2294 | 3774 | 5271 | 6390 | 8965 | 10999 | 11604 | 10176 | 17008 |
| 14682 | 17396 | 18394 | 13895 | 15309 | 17399 | 484 | 5612 | 8933 | 9198 | 6892 | 6990 |
| 9137 | 8204 | 8167 | 19059 | 18580 | 17301 | 4340 | 6873 | 11451 | 6065 | 9085 | 11944 |
| 11248 | 18110 | 10733 | 5227 | 7794 | 7083 | 8278 | 15410 | 3552 | 16695 | 7053 | 2668 |
| 15839 | 10369 | 14061 | 13065 | 7862 | 12871 | 3847 | 16010 | 12618 | 5102 | 5723 | 5348 |
| 9583 | 9532 | 15763 | 17548 | 13830 | 15673 | 5777 | 18062 | 18742 | 15685 | 17435 | 835 |
| 421 | 9700 | 1904 | 3265 | 2180 | 2753 | 4596 | 12734 | 10970 | 18093 | 18831 | 2545 |
| 7026 | 10816 | 2499 | 12691 | 6318 | 1999 | 2567 | 1394 | 6365 | 19001 | 9719 | 8324 |
| 11811 | 12315 | 5666 | 16514 | 18475 | 10933 | 15956 | 3273 | 3838 | 706 | 10504 | 5990 |
| 10861 | 2553 | 9018 | 8809 | 8627 | 8584 | 13596 | 2608 | 10275 | 12278 | 8725 | 779 |
| 17013 | 4078 | 3280 | 8926 | 18315 | 18893 | 14793 | 18945 | 4573 | 3468 | 2115 | 18906 |
| 2897 | 8412 | 8921 | 7583 | 9677 | 14390 | 13256 | 14184 | 11561 | 7750 | 7723 | 13823 |
| 8577 | 13165 | 7859 | 18546 | 7116 | 5043 | 4449 | 2137 | 6398 | 7799 | 7196 | 12151 |
| 18748 | 10862 | 5377 | 3616 | 7581 | 17168 | 11492 | 895 | 15695 | 15224 | 16802 | 985 |
| 14632 | 326 | 13546 | 9996 | 16571 | 18999 | 18450 | 18771 | 18129 | 6098 | 1741 | 15547 |
| 15586 | 15805 | 15257 | 16479 | 1279 | 15476 | 11079 | 13181 | 16731 | 12486 | 18648 | 16167 |

(continued)

| SEQ ID NO: | homolog SEQ ID NOs | | | | | | | | | | |
|---|---|---|---|---|---|---|---|---|---|---|---|
| 5118 | 15009 | 15010 | 17134 | 12729 | 10964 | 1417 | 14712 | 5721 | 5504 | 3199 | 2622 |
| 13058 | 2979 | 10423 | 8307 | 4854 | 3641 | 3541 | 10183 | 16996 | 735 | 732 | 17406 |
| 9929 | 4647 | 12132 | 17070 | 14450 | 8676 | 17537 | 14527 | 14925 | 11247 | 5570 | 16918 |
| 18300 | 6997 | 13078 | 7933 | 12177 | 10965 | 7654 | 19120 | 14122 | 8609 | 10040 | 1506 |
| 2233 | 3802 | 17276 | 16782 | 19107 | 18939 | 3104 | 10995 | 1912 | 16003 | 8420 | 8348 |
| 13916 | 14569 | 15206 | 16935 | 15711 | 8756 | 7204 | 18526 | 6423 | 11090 | 1583 | 14885 |
| 14210 | 17110 | 10760 | 10117 | 1474 | 2743 | 768 | 3191 | 3911 | 8963 | 9058 | 10304 |
| 19183 | 5960 | 15372 | 18803 | 8355 | 750 | 18130 | 16208 | 2485 | 1433 | 2007 | 18492 |
| 1895 | 17590 | 5481 | 5304 | 6130 | 3953 | 748 | 856 | 9398 | 1989 | 18983 | 10032 |
| 1308 | 16754 | 13420 | 17236 | 1270 | 10884 | 12757 | 13511 | 9952 | 4531 | 14047 | 9791 |
| 584 | 6920 | 6129 | 6133 | 14369 | 17710 | 6599 | 16186 | 14532 | 14499 | 18914 | 2449 |
| 3224 | 5430 | 4453 | 4547 | 4141 | 6290 | 13001 | 14451 | 5357 | 5676 | 10109 | 13884 |
| 1226 | 18990 | 6373 | 15289 | 16391 | 11384 | 2046 | 9623 | 10336 | 10225 | 5637 | 2679 |
| 18760 | 15287 | 15299 | 15005 | 10538 | 2274 | 2366 | 15858 | 4715 | 18226 | 7226 | 10000 |
| 14899 | 12251 | 11655 | 2326 | 12860 | 15650 | 12845 | 4472 | 12858 | 18162 | 7699 | 13374 |
| 1938 | 6890 | 2517 | 358 | 13718 | 9245 | 3740 | 8301 | 10036 | 10037 | 11598 | 13156 |
| 6062 | 11813 | 4731 | 9348 | 6264 | 18880 | 2358 | 16692 | 8028 | 3478 | 2537 | 12689 |
| 15658 | 12440 | 17135 | 12574 | 1734 | 3578 | 17624 | 18905 | 3691 | 11371 | 14828 | 6677 |
| 14887 | 14573 | 6627 | 16604 | 3255 | 4171 | 5653 | 18882 | 5926 | 10829 | 13669 | 2514 |
| 7948 | 13401 | 11986 | 7884 | 18359 | 3148 | 17208 | 7280 | 18155 | 13200 | 5115 | 19203 |
| 15440 | 13521 | 13501 | 12684 | 5790 | 561 | 4152 | 7008 | 7589 | 17500 | 3103 | 5692 |
| 16843 | 8691 | 4961 | 14121 | 9148 | 4031 | 15699 | 11743 | 13225 | 18887 | 10545 | 13773 |
| 9282 | 6505 | 11549 | 11385 | 4383 | 5212 | 2815 | 730 | 3097 | 9160 | 603 | 11976 |
| 13010 | 1548 | 3512 | 8381 | 4054 | 10287 | 8901 | 2266 | 11452 | 16442 | 16464 | 16470 |
| 16500 | 16552 | 13726 | 7063 | 7067 | 7043 | 10680 | 7065 | 14056 | 17663 | 14404 | 11839 |
| 11995 | 2011 | 3141 | 12589 | 18452 | 4465 | 3852 | 6660 | 2217 | 3018 | 1851 | 13717 |
| 14662 | 18960 | 14978 | 15034 | 15793 | 17415 | 10448 | 12404 | 11606 | 7683 | 2094 | 13451 |
| 9523 | 10265 | 9057 | 14626 | 6506 | 14596 | 17585 | 4757 | 2467 | 2131 | 1958 | 16227 |
| 12425 | 8409 | 327 | 328 | 455 | 7459 | 3609 | 8867 | 19030 | 4350 | 2060 | 2093 |
| 13896 | 8024 | 15664 | 1766 | 15442 | 6244 | 7496 | 4892 | 10377 | 11975 | 3002 | 3671 |
| 4437 | 9335 | 19108 | 17895 | 16401 | 10085 | 16164 | 1056 | 4981 | 1848 | 11707 | 324 |
| 322 | 316 | 319 | 15836 | 333 | 323 | 321 | 320 | 5124 | 8234 | 18857 | 1138 |
| 3644 | 10102 | 10006 | 9263 | 619 | 12852 | 10291 | 3475 | 5177 | 6442 | 16263 | 9780 |
| 11318 | 1765 | 19178 | 18669 | 9242 | 4650 | 8073 | 7725 | 17246 | 10485 | | |
| 332:14603 | 515 | 18343 | 18387 | 10567 | 2331 | 4920 | 18684 | 3585 | 2105 | 10280 | 4477 |
| 580 | 18136 | 8805 | 3452 | 7516 | 359 | 360 | 5160 | 7225 | 7197 | 13693 | 11971 |
| 7475 | 8978 | 8946 | 6695 | 4188 | 4175 | 17891 | 18486 | 5737 | 5750 | 15281 | 8580 |
| 1376 | 1379 | 17822 | 7898 | 14342 | 7445 | 738 | 5438 | 17922 | 9221 | 17902 | 7986 |
| 4172 | 12385 | 15214 | 12314 | 15232 | 8116 | 18161 | 2487 | 2559 | 19074 | 4201 | 8255 |
| 9921 | 7777 | 5356 | 14152 | 14888 | 6278 | 18980 | 2755 | 2470 | 718 | 16531 | 1230 |
| 3927 | 8672 | 9283 | 3995 | 6079 | 851 | 11164 | 7252 | 9267 | 3883 | 17993 | 3774 |
| 5271 | 6390 | 8965 | 10999 | 11604 | 10176 | 18394 | 17396 | 13895 | 8933 | 9137 | 8204 |
| 8167 | 18580 | 19059 | 17301 | 6873 | 4340 | 11451 | 6065 | 9085 | 11944 | 11248 | 18110 |
| 10733 | 5227 | 8278 | 15410 | 16695 | 3552 | 15839 | 10369 | 14061 | 13065 | 3847 | 16010 |
| 5102 | 5723 | 9532 | 15763 | 13830 | 15673 | 5777 | 18742 | 17435 | 421 | 835 | 9700 |
| 1904 | 3265 | 2180 | 2753 | 4596 | 10970 | 18093 | 2545 | 10816 | 7026 | 12691 | 2499 |
| 19001 | 6365 | 1394 | 9719 | 8324 | 11811 | 16514 | 18475 | 10933 | 15956 | 3273 | 3838 |
| 706 | 10504 | 2553 | 10861 | 8809 | 8627 | 8584 | 13596 | 2608 | 779 | 8725 | 12278 |
| 17013 | 4078 | 3280 | 18893 | 18315 | 14793 | 18945 | 3468 | 8921 | 8412 | 14390 | 13256 |
| 14184 | 7750 | 11561 | 7723 | 13823 | 8577 | 13165 | 7859 | 18546 | 7116 | 5043 | 2137 |

(continued)

| SEQ ID NO: | homolog SEQ ID NOs | | | | | | | | | | |
|---|---|---|---|---|---|---|---|---|---|---|---|
| 6398 | 7196 | 12151 | 18748 | 10862 | 5377 | 3616 | 17168 | 15246 | 18824 | 11492 | 12600 |
| 1785 | 15224 | 985 | 16802 | 13546 | 9996 | 18450 | 18771 | 1741 | 15547 | 6098 | 15257 |
| 15476 | 13181 | 18648 | 12486 | 16167 | 15009 | 15010 | 17134 | 12729 | 1417 | 10964 | 14712 |
| 5721 | 3199 | 2622 | 13058 | 2979 | 10423 | 8307 | 3641 | 4854 | 3541 | 10183 | 16996 |
| 17406 | 4647 | 12132 | 17070 | 14450 | 8676 | 17537 | 14527 | 14925 | 11247 | 5570 | 6997 |
| 16918 | 18300 | 13078 | 7933 | 10965 | 12177 | 7654 | 14122 | 8609 | 10040 | 1506 | 16782 |
| 19107 | 18939 | 10995 | 3104 | 1912 | 16003 | 8420 | 15206 | 14569 | 16935 | 15711 | 8756 |
| 7204 | 18526 | 6423 | 11090 | 14885 | 1583 | 14210 | 10760 | 17110 | 10117 | 768 | 2743 |
| 1474 | 3191 | 3911 | 8963 | 9058 | 10304 | 5960 | 18803 | 750 | 2485 | 1433 | 1895 |
| 2007 | 18492 | 17590 | 5304 | 856 | 748 | 9398 | 1989 | 18983 | 10032 | 1308 | 13420 |
| 16754 | 17236 | 1270 | 12757 | 9952 | 13511 | 14047 | 9791 | 584 | 6599 | 17710 | 14369 |
| 16186 | 14532 | 14499 | 18914 | 2449 | 3224 | 4453 | 14451 | 5357 | 18990 | 1226 | 9623 |
| 10336 | 15287 | 15299 | 15005 | 10538 | 2274 | 15858 | 4715 | 18226 | 7226 | 11655 | 2326 |
| 14899 | 10000 | 12251 | 15650 | 12860 | 12858 | 12845 | 18162 | 13374 | 7699 | 1938 | 6890 |
| 2517 | 13718 | 358 | 9245 | 8301 | 10036 | 10037 | 11598 | 13156 | 6062 | 11813 | 6264 |
| 18880 | 2358 | 3478 | 2537 | 16692 | 8028 | 15658 | 12689 | 12574 | 1734 | 17135 | 12440 |
| 17450 | 3578 | 17624 | 18905 | 3691 | 11371 | 14828 | 14573 | 14887 | 6677 | 6627 | 3255 |
| 16604 | 4171 | 16840 | 5926 | 10829 | 18882 | 5653 | 13669 | 2514 | 7948 | 13401 | 11986 |
| 7884 | 18359 | 3148 | 17208 | 7280 | 18155 | 13200 | 5115 | 19203 | 15440 | 13521 | 12684 |
| 5790 | 561 | 14678 | 3956 | 4152 | 7589 | 7008 | 17500 | 5692 | 3103 | 16843 | 4961 |
| 14121 | 8691 | 9148 | 4031 | 15699 | 13225 | 18887 | 11743 | 10545 | 13773 | 9282 | 6505 |
| 11549 | 4383 | 2815 | 11385 | 5212 | 730 | 3097 | 9160 | 603 | 13010 | 1548 | 11976 |
| 3512 | 10287 | 8901 | 2266 | 8381 | 4054 | 6522 | 4061 | 14274 | 11452 | 16464 | 16442 |
| 16470 | 16500 | 16552 | 13726 | 7065 | 7063 | 10680 | 7043 | 7067 | 14056 | 17663 | 14404 |
| 11839 | 11995 | 12589 | 18452 | 3852 | 6660 | 2217 | 4565 | 1851 | 3018 | 13717 | 14662 |
| 18960 | 14978 | 15034 | 15793 | 10448 | 17415 | 11989 | 6298 | 11606 | 12404 | 7683 | 2094 |
| 13451 | 9523 | 10265 | 6506 | 2467 | 2131 | 1958 | 16227 | 12425 | 8409 | 7459 | 8867 |
| 4350 | 2093 | 2060 | 13896 | 19030 | 15664 | 1766 | 15442 | 7496 | 4892 | 10377 | 11975 |
| 3002 | 3671 | 4437 | 9335 | 17895 | 16401 | 10085 | 16164 | 1056 | 4981 | 1848 | 11707 |
| 7139 | 5124 | 15836 | 322 | 18857 | 1138 | 3644 | 10102 | 10006 | 9263 | 619 | 10291 |
| 12852 | 3475 | 16263 | 6442 | 9780 | 5177 | 11318 | 1765 | 19178 | 18669 | 18695 | 4650 |
| 7725 | 17246 | 10485 | | | | | | | | | |
| 333:17621 | 6498 | 10877 | 4478 | 535 | 7655 | 7490 | 11610 | 12155 | 359 | 329 | 357 |
| 9904 | 12647 | 12682 | 11322 | 13239 | 8952 | 4187 | 2763 | 1274 | 1141 | 4906 | 6775 |
| 6177 | 3519 | 17529 | 16576 | 10915 | 19075 | 8253 | 9925 | 8036 | 7079 | 4625 | 7770 |
| 14890 | 10540 | 2772 | 9307 | 5251 | 8710 | 2343 | 9153 | 6428 | 1481 | 3776 | 8966 |
| 16276 | 18393 | 17006 | 14683 | 8950 | 14908 | 4343 | 6064 | 5226 | 6370 | 3282 | 3553 |
| 10371 | 7861 | 13057 | 18747 | 15674 | 3566 | 16824 | 18741 | 17597 | 4745 | 7855 | 4568 |
| 10122 | 3173 | 14748 | 7024 | 1998 | 18270 | 15272 | 18414 | 2000 | 9723 | 7623 | 3841 |
| 11201 | 10882 | 14758 | 7077 | 3123 | 325 | 8727 | 777 | 15800 | 2884 | 8411 | 14183 |
| 7748 | 11559 | 13821 | 13207 | 5113 | 4397 | 5376 | 8746 | 18657 | 9642 | 15684 | 14665 |
| 326 | 15221 | 11049 | 941 | 18982 | 8593 | 6099 | 15254 | 1202 | 16046 | 11077 | 12502 |
| 17716 | 9851 | 15240 | 14039 | 18848 | 522 | 2451 | 4881 | 9525 | 5374 | 14767 | 14281 |
| 6641 | 17952 | 12133 | 15614 | 7506 | 15039 | 17536 | 2009 | 13100 | 8427 | 6859 | 9928 |
| 19163 | 4498 | 2340 | 1509 | 433 | 13478 | 14041 | 14568 | 4457 | 18018 | 17111 | 1472 |
| 9268 | 9056 | 10303 | 5984 | 19184 | 18805 | 15184 | 6590 | 5461 | 747 | 858 | 1988 |
| 17838 | 16755 | 13510 | 14046 | 7458 | 8799 | 8811 | 4319 | 14371 | 3222 | 14453 | 13005 |
| 1232 | 5228 | 9624 | 2044 | 6117 | 10188 | 3795 | 3794 | 974 | 15693 | 18573 | 7376 |
| 8980 | 2807 | 2826 | 11958 | 18157 | 10192 | 3850 | 9625 | 8603 | 11643 | 16949 | 2912 |
| 14101 | 17431 | 14104 | 11922 | 14106 | 14118 | 14103 | 11917 | 14117 | 11877 | 9156 | 1742 |

(continued)

| SEQ ID NO: | homolog SEQ ID NOs | | | | | | | | | | |
|---|---|---|---|---|---|---|---|---|---|---|---|
| 2076 | 7599 | 18286 | 2961 | 2816 | 8027 | 11540 | 897 | 17297 | 12032 | 14617 | 14635 |
| 2786 | 12115 | 1581 | 19172 | 8841 | 3304 | 2722 | 18527 | 3031 | 17450 | 10636 | 10883 |
| 19245 | 3971 | 3488 | 2706 | 12363 | 8462 | 8465 | 1521 | 11661 | 18323 | 10495 | 1858 |
| 18642 | 5752 | 14905 | 11072 | 16126 | 15626 | 1970 | 11741 | 6081 | 16716 | 11426 | 614 |
| 8796 | 9927 | 9502 | 1770 | 1798 | 11814 | 1549 | 2732 | 1033 | 7850 | 13576 | 15542 |
| 16151 | 16155 | 16095 | 3257 | 6401 | 17505 | 17504 | 17525 | 17524 | 18409 | 8232 | 15499 |
| 6961 | 7377 | 432 | 13504 | 13500 | 16819 | 10332 | 2411 | 10331 | 2410 | 12865 | 5789 |
| 3223 | 4128 | 11048 | 1117 | 13770 | 13767 | 16781 | 16777 | 16779 | 16801 | 17741 | 16774 |
| 4860 | 12777 | 12778 | 14071 | 14067 | 14053 | 14069 | 14091 | 14072 | 14075 | 8376 | 11001 |
| 9987 | 7853 | 5184 | 7393 | 7395 | 3219 | 3220 | 17517 | 3203 | 17996 | 3205 | 17513 |
| 10899 | 3204 | 11670 | 14581 | 14168 | 12108 | 11682 | 15638 | 6252 | 19127 | 4806 | 1701 |
| 3793 | 5131 | 15088 | 13347 | 6047 | 11699 | 14198 | 7787 | 19095 | 3733 | 3009 | 10717 |
| 12562 | 9506 | 8209 | 12416 | 6561 | 8778 | 8302 | 3146 | 10221 | 859 | 3688 | 15948 |
| 8147 | 17462 | 14789 | 17884 | 15014 | 6858 | 17887 | 2465 | 7396 | 9985 | 4361 | 4569 |
| 16527 | 18012 | 3152 | 5302 | 13120 | 6034 | 16042 | 6762 | 12479 | 3121 | 8202 | 17109 |
| 9781 | 1428 | 6248 | 3057 | 12195 | 11518 | 7010 | 9337 | 18035 | 14637 | 2924 | 7398 |
| 13922 | 16467 | 13725 | 3045 | 14411 | 11840 | 6227 | 2107 | 5626 | 3017 | 649 | 5687 |
| 19170 | 18325 | 17413 | 13970 | 18612 | 8010 | 3601 | 7401 | 19015 | 328 | 7461 | 14921 |
| 4492 | 15663 | 7353 | 7349 | 7351 | 7348 | 7218 | 7214 | 7201 | 7224 | 7220 | 7200 |
| 2490 | 10210 | 1914 | 11630 | 7360 | 10526 | 12067 | 17483 | 990 | 2676 | 9138 | 1772 |
| 18635 | 10340 | 15058 | 19237 | 4344 | 2478 | 1751 | 7841 | 1767 | 4651 | 11660 | 15488 |
| 7901 | 7724 | | | | | | | | | | |
| 334:13396 | 17207 | 7300 | 12940 | 10409 | 6167 | 6302 | 16961 | 3782 | 14913 | 12949 | 6274 |
| 1445 | 8025 | 4162 | 5021 | 10714 | 4634 | 11369 | 6304 | 16188 | 14951 | 7942 | 7940 |
| 14879 | 4636 | 9184 | 9183 | 840 | 3277 | 17325 | 3714 | 15114 | 17528 | 17582 | 5185 |
| 4423 | 19198 | 14370 | 11230 | 15609 | 10851 | 2333 | 11889 | 4722 | 8931 | 15588 | 1827 |
| 680 | 11357 | 16987 | 3034 | 7121 | 14512 | 16943 | 2908 | 16250 | 18410 | 495 | 12608 |
| 3954 | 2539 | 4400 | 14817 | 8967 | 8298 | | | | | | |
| 335:17955 | 13227 | 14444 | 9858 | 3875 | 7843 | 5082 | 5361 | 8900 | 16139 | 16124 | 4656 |
| 16646 | 13371 | 6550 | 5840 | 6552 | 8352 | 13524 | 11651 | 9350 | 7366 | 15095 | 9106 |
| 2189 | 17114 | 15179 | 13572 | 17818 | 12991 | 884 | 8197 | 17100 | 7580 | 17298 | 7561 |
| 18531 | 14944 | 12090 | 12849 | 8713 | 2165 | 481 | 10266 | 5405 | 7903 | 10155 | 10154 |
| 5547 | 6554 | 5176 | 11960 | 18654 | 8367 | 18775 | 3308 | 3306 | 15052 | 6482 | |
| 336:12812 | 6537 | 6555 | 17107 | 18839 | 15750 | 6539 | 8474 | 19239 | 12797 | 15782 | 4902 |
| 15115 | 9000 | 9001 | 9065 | 9067 | 9105 | 9103 | 17825 | 6565 | 6562 | 6557 | 9004 |
| 9003 | 9012 | 16968 | 2568 | 9474 | 9470 | 1122 | 1147 | 1755 | 8315 | 8306 | 8312 |
| 14711 | 14713 | 14695 | 7673 | 7677 | 14772 | 14775 | 2435 | 11752 | 13152 | 6435 | 4085 |
| 4083 | 6533 | 6535 | 8614 | 8615 | 8617 | 8620 | 8518 | 8537 | 8542 | 8543 | 8570 |
| 12746 | 8576 | 8592 | 8594 | 8601 | 8610 | 1645 | 1673 | 8635 | 8639 | 8641 | 8643 |
| 8661 | 8663 | 8665 | 9257 | 9258 | 9264 | 9266 | 9262 | 9299 | 9295 | 9317 | 9318 |
| 9320 | 9323 | 9338 | 9342 | 9341 | 9345 | 9364 | 9346 | 9373 | 9374 | 9389 | 9392 |
| 9390 | 9396 | 9404 | 9408 | 9407 | 9411 | 9409 | 9423 | 9427 | 9425 | 9441 | 9428 |
| 9443 | 9444 | 9447 | 9467 | 9448 | 10005 | 10001 | 10008 | 10014 | 10024 | 10013 | 10027 |
| 10031 | 10034 | 8599 | 8597 | 8477 | 8478 | 8490 | 8479 | 8513 | 1692 | 1754 | 1710 |
| 1752 | 1045 | 17015 | 15357 | 15358 | 9042 | 9041 | 12442 | 15367 | 9100 | 9101 | 9120 |
| 9119 | 9123 | 9124 | 16510 | 1050 | 984 | 2213 | 2212 | 1659 | 1656 | 8981 | 8979 |
| 9005 | 9007 | 7760 | 7737 | 7734 | 9044 | 9043 | 9069 | 9071 | 12814 | 2254 | 2258 |
| 2272 | 2273 | 8160 | 4433 | 6702 | 6721 | 3343 | 6193 | 16536 | 19006 | 12307 | 3854 |
| 3851 | 12771 | 1662 | 1661 | 2139 | 2138 | 2171 | 8566 | 8568 | 8667 | 8669 | 10026 |
| 2162 | 2146 | 2210 | 2190 | 2142 | 2141 | 4959 | 1144 | 1148 | 17053 | 13859 | 1049 |

(continued)

| SEQ ID NO: | homolog SEQ ID NOs | | | | | | | | | | |
|---|---|---|---|---|---|---|---|---|---|---|---|
| 1053 | 1052 | 1124 | 17011 | 16959 | 1059 | 1058 | 1054 | 13862 | 1142 | 1127 | 1061 |
| 1062 | 1075 | 1077 | 1064 | 16992 | 16915 | 16964 | 16878 | 16876 | 16859 | 17012 | 16899 |
| 16921 | 16946 | 1182 | 1095 | 1094 | 16856 | 1097 | 16854 | 16851 | 1126 | 13864 | 16939 |
| 16942 | 1100 | 16940 | 13841 | 16960 | 14325 | 16944 | 16994 | 17052 | 16879 | 1108 | 1145 |
| 16991 | 16880 | 17018 | 16967 | 13839 | 17048 | 16926 | 17022 | 1177 | 1114 | 1113 | 16895 |
| 1111 | 1175 | 17047 | 1110 | 4898 | 5589 | 5599 | 4983 | 16542 | 13892 | 16864 | 16988 |
| 17051 | 14321 | 14323 | 19043 | 2338 | 1536 | 2164 | 9068 | 1664 | 16509 | 1044 | 983 |
| 6146 | 4976 | 3764 | 4974 | 3763 | 15421 | 9190 | 11574 | 8757 | 10135 | 14728 | 11967 |
| 3187 | 4958 | 8755 | 11575 | 1172 | 1276 | 17066 | 16924 | 16962 | 1083 | 1092 | 1078 |
| 1082 | 1181 | 16990 | 16903 | 16896 | 4982 | 4894 | 1694 | 1728 | 1750 | 1784 | 1635 |
| 12761 | 8471 | 5674 | 4720 | 4702 | 16162 | 16593 | 16429 | 16592 | 13465 | 16651 | 12975 |
| 14562 | 14228 | 13445 | 7037 | 12725 | 9992 | 13383 | 9994 | 13865 | 14324 | 16882 | 1539 |
| 1540 | 1165 | 1164 | 1537 | 1185 | 1193 | 1188 | 1209 | 1190 | 1211 | 1192 | 16687 |
| 16688 | 16689 | 3125 | 9504 | 1328 | 18686 | 1945 | 14841 | 14262 | 13283 | 9073 | 9075 |
| 13355 | 17912 | 17911 | 12471 | 4663 | 13744 | 6639 | 2970 | 5233 | 6727 | 8683 | 18083 |
| 16805 | 16999 | 1109 | 16617 | 10334 | 1733 | 4064 | 16482 | 12338 | 2688 | 8323 | 7698 |
| 11227 | 1776 | 1602 | 1619 | 1620 | 1690 | 1689 | 1736 | 1781 | 4784 | 1615 | 1667 |
| 1774 | 1779 | 1756 | 1738 | 1623 | 1641 | 1671 | 1717 | 12773 | 13030 | 15977 | 15991 |
| 12233 | 5806 | 912 | 18647 | 5201 | 1678 | 15537 | 15536 | 16043 | 11137 | 17959 | 4029 |
| 10751 | 9422 | 7965 | 3773 | 17175 | 15696 | 9656 | 14239 | 1456 | 8237 | 3965 | 2720 |
| 4396 | 15798 | 4098 | 13654 | 16332 | 1457 | 8240 | 5097 | 7868 | 8335 | 6224 | 17118 |
| 7782 | 6255 | 6237 | 7776 | 6233 | 14548 | 15040 | 15041 | 8516 | 8517 | 12031 | 12049 |
| 12781 | 12784 | 6582 | 6560 | 14328 | 4917 | 14355 | 14359 | 14367 | 14396 | 19230 | 10194 |
| 10191 | 10451 | 9437 | 1647 | 9436 | 9439 | 9440 | 9846 | 7906 | 2642 | 9463 | 13611 |
| 8571 | 10086 | 662 | 19008 | 6288 | 4736 | 14530 | 846 | 9216 | 12880 | 8162 | 14794 |
| 14790 | 14788 | 15359 | 13027 | 14387 | 11668 | 8337 | 8341 | 12881 | 12882 | 13026 | 13391 |
| 12955 | 12785 | 12804 | 12806 | 13191 | 13220 | 10610 | 10605 | 10607 | 10623 | 10626 | 10625 |
| 10533 | 18925 | 14732 | 14714 | 14729 | 14730 | 17061 | 14750 | 12898 | 7639 | 7641 | 7624 |
| 13186 | 13185 | 4801 | 13976 | 8330 | 7761 | 16558 | 9139 | 9089 | 9090 | 12899 | 9046 |
| 9045 | 13385 | 12932 | 9040 | 9038 | 9013 | 9015 | 9096 | 8218 | 13006 | 13009 | 11928 |
| 11916 | 11918 | 11923 | 11335 | 13190 | 13187 | 7691 | 13406 | 13394 | 13392 | 12879 | 12877 |
| 3727 | 18357 | 13517 | 3085 | 12328 | 12329 | 6664 | 6665 | 6666 | 12660 | 4761 | 11671 |
| 14600 | 8169 | 6360 | 3351 | 10190 | 5867 | 3349 | 16478 | 16472 | 16477 | 7680 | 16504 |
| 13155 | 2297 | 14382 | 14391 | 4797 | 15682 | 2785 | 12327 | 13048 | 18495 | 14115 | 17082 |
| 12990 | 917 | 15503 | 5572 | 9144 | 5334 | 4799 | 5675 | 14308 | 14394 | 14366 | 14338 |
| 14859 | 14310 | 14385 | 14860 | 14858 | 14332 | 14356 | 1688 | 1687 | 9560 | 11126 | 8014 |
| 2169 | 2166 | 1696 | 8495 | 8491 | 12793 | 2227 | 2215 | 9469 | 8538 | 8540 | 9289 |
| 9290 | 9291 | 15908 | 10650 | 13029 | 13031 | 13033 | 16432 | 6354 | 6353 | 5957 | 9874 |
| 17852 | 11476 | 2846 | 5526 | 5528 | 19207 | 16548 | 12951 | 2135 | 12953 | 5530 | 12952 |
| 14720 | 3496 | 5582 | 2342 | 15247 | 15911 | 17347 | 15712 | 13002 | 1680 | 2185 | 2184 |
| 7322 | 4953 | 4759 | 4762 | 1130 | 3826 | 3812 | 3815 | 12767 | 8645 | 8473 | 8461 |
| 12795 | 9126 | 9127 | 9094 | 9009 | 1683 | 1685 | 12861 | 13043 | 9093 | 9091 | 9078 |
| 12904 | 12906 | 1448 | 11362 | 1449 | 1450 | 9325 | 12931 | 14358 | 2915 | 8053 | 14498 |
| 11392 | 4282 | 7781 | 16258 | 9550 | 9590 | 4521 | 4977 | 18683 | 15969 | 16454 | 1011 |
| 1246 | 996 | 11260 | 946 | 943 | 932 | 18672 | 14854 | 4980 | 15980 | 15976 | 15978 |
| 15974 | 15874 | 15982 | 16521 | 12799 | 8419 | 11340 | 10885 | 16256 | 10835 | 10856 | 10806 |
| 13518 | 3443 | 14861 | 16748 | 16758 | 8949 | 15743 | 13836 | 7261 | 17637 | 2188 | 2186 |
| 14388 | 14397 | 15744 | 6427 | 6412 | 6411 | 14333 | 12961 | 16261 | 13674 | 14819 | 9048 |
| 9047 | 12816 | 14747 | 12835 | 12809 | 12832 | 12817 | 1806 | 18749 | 4362 | 1325 | 9864 |
| 1801 | 10091 | 8794 | 8786 | 8117 | 8113 | 8115 | 8060 | 8057 | 8093 | 5874 | 8838 |

(continued)

| SEQ ID NO: | homolog SEQ ID NOs | | | | | | | | | | |
|---|---|---|---|---|---|---|---|---|---|---|---|
| | 8829 | 8836 | 8858 | 8826 | 8810 | 8827 | 8815 | 9471 | 8054 | 5768 | 8056 | 5843 |
| | 8075 | 5829 | 5788 | 8149 | 5808 | 5807 | 8076 | 8078 | 8120 | 8118 | 8125 | 8130 |
| | 8127 | 8977 | 8976 | 9529 | 9538 | 9536 | 9559 | 9539 | 9540 | 18257 | 16900 | 7763 |
| | 11331 | 11313 | 7658 | 7661 | 14795 | 11338 | 14815 | 14307 | 13912 | 1804 | 15957 | 3848 |
| | 3831 | 3834 | 12744 | 4181 | 4740 | 14753 | 14230 | 4182 | 4711 | 8668 | 8758 | 8693 |
| | 8692 | 8726 | 8724 | 8728 | 8701 | 8706 | 8704 | 8689 | 8721 | 8709 | 8708 | 8774 |
| | 8781 | 8777 | 8784 | 8752 | 8731 | 14363 | 10892 | 4387 | 12605 | 17432 | 6543 | 10268 |
| | 8178 | 7656 | 7644 | 14769 | 14755 | 14693 | 14689 | 1638 | 1670 | 1712 | 1715 | 1730 |
| | 2238 | 2234 | 9063 | 14862 | 1681 | 1663 | 2251 | 2250 | 14311 | 14331 | 4911 | 4914 |
| | 4930 | 4934 | 14334 | 4936 | 14353 | 14354 | 4939 | 14361 | 14389 | 4978 | 1646 | 1617 |
| | 1604 | 1708 | 3870 | 3871 | 12743 | 8574 | 16919 | 9369 | 9370 | 2211 | 2252 | 8498 |
| | 8499 | 4897 | 4908 | 4919 | 4937 | 4955 | 4957 | 4960 | 16507 | 16486 | 16480 | 16491 |
| | 1032 | 1030 | 981 | 979 | 962 | 959 | 956 | 2232 | 2229 | 14329 | 14155 | 14306 |
| | 14340 | 14339 | 14393 | 14851 | 14853 | 14855 | 4821 | 7851 | 11477 | | | |
| 337: | 4095 | 15046 | 10521 | 11731 | 1628 | 9595 | 7433 | 633 | 7621 | 18399 | 1383 | 7646 |
| | 5781 | 12525 | 14082 | 6175 | 5446 | 17124 | 2637 | 14579 | 18542 | 8588 | 804 | 8650 |
| | 5284 | 18904 | 5658 | 8106 | 6336 | 4263 | 11332 | 12874 | 6478 | 13913 | 9066 | 13379 |
| | 17596 | 18592 | 13115 | 2010 | 2419 | 4845 | 14997 | 18740 | 618 | 3431 | 3970 | 10518 |
| | 14706 | 16668 | 9305 | 10374 | 18493 | 5038 | 10849 | 14836 | 2271 | 992 | 9179 | 16837 |
| | 8717 | 10496 | 18477 | 7920 | 10916 | 3559 | 10271 | 17999 | 4030 | 18694 | 6969 | 4377 |
| | 9285 | 2666 | 10774 | 17320 | 13447 | 10398 | 3876 | 2057 | 18631 | 17938 | 558 | 17050 |
| | 15751 | 10685 | 10348 | 7899 | 13923 | 8201 | 604 | 6712 | 2475 | 19003 | 2910 | 16091 |
| | 2140 | 10720 | 3323 | 9194 | 11223 | 19145 | 10602 | 657 | 17840 | 11593 | 11595 | 18216 |
| | 16978 | 13665 | 12968 | 16708 | 15761 | 4008 | 9556 | 11066 | 3402 | 5250 | 17466 | 6487 |
| | 16702 | 11097 | 11261 | 15834 | 16616 | 4700 | 4701 | 9490 | 6730 | 3210 | 1853 | 14663 |
| | 14660 | 10844 | 13655 | 4649 | 6868 | 16252 | 4885 | 1523 | 15210 | 12821 | 1183 | 15471 |
| | 2051 | 5972 | 4643 | 3628 | 1352 | 5987 | 6581 | 18812 | 14639 | 16535 | 9679 | 11416 |
| | 14991 | 7257 | 4274 | 9585 | 6918 | 2388 | 17101 | 4046 | 16235 | 2330 | 12769 | 4218 |
| | 881 | 5507 | 11957 | 3735 | 14383 | 16187 | 2043 | 5111 | 10878 | 17577 | 19147 | 343 |
| | 17755 | 12154 | 7436 | 13269 | 13270 | 4312 | 7297 | 2111 | 3014 | 9674 | 4608 | 10651 |
| | 11599 | 13358 | | | | | | | | | | |
| 338: | 10161 | 1021 | 6688 | 9452 | 16287 | 16288 | 15688 | 15702 | 15143 | 16317 | 15707 | 15713 |
| | 15714 | 15729 | 6744 | 14587 | 10601 | 14607 | 3011 | 12699 | 13448 | 9210 | 7228 | 7062 |
| | 7064 | 7066 | 2700 | 8792 | 17658 | 19226 | 18581 | 7704 | 14010 | 16417 | 1222 | 7789 |
| | 611 | 11394 | 8456 | 13854 | 13461 | 4750 | 2550 | 14364 | 19133 | 16371 | 11853 | 908 |
| | 3758 | 1219 | 7812 | 7093 | 7068 | 14739 | 666 | 6001 | 17547 | 7416 | 14427 | 12134 |
| | 15094 | 16314 | 2594 | 15063 | 12622 | 1314 | 15738 | 15705 | 15731 | 15734 | 15736 | 14785 |
| | 634 | 15145 | 2811 | 4375 | 16005 | 15760 | 6740 | 16286 | 9982 | 1607 | 5288 | 3077 |
| | 3076 | 3079 | 3060 | 17569 | 5586 | 1163 | 1294 | 1299 | 7042 | 7060 | 1318 | 1320 |
| | 1323 | 7531 | 10165 | | | | | | | | | |
| 339: | 13280 | 7465 | 13170 | 13488 | 8094 | 7023 | 15591 | 15585 | 15589 | 13440 | 10740 | 14846 |
| | 15781 | 14380 | 7542 | 4997 | 6793 | 6530 | 15127 | 14107 | 14099 | 12692 | 12638 | 6743 |
| | 4176 | 8397 | 8398 | 12234 | 12202 | 12231 | 12184 | 8401 | 12178 | 12201 | 12188 | 12226 |
| | 8402 | 1852 | 18620 | 17417 | 1931 | 18095 | 18096 | 18081 | 18076 | 17392 | 17346 | 1795 |
| | 1793 | 17515 | 17496 | 18138 | 17518 | 18108 | 8418 | 3043 | 18459 | 8099 | 9311 | 16771 |
| | 2036 | 14555 | 8712 | 1468 | 4416 | 4414 | 4413 | 4436 | 4435 | 4371 | 4357 | 4355 |
| | 4376 | 3798 | 3797 | 3315 | 11764 | 5938 | 3236 | 15423 | 18556 | 1084 | 9329 | 9332 |
| | 9334 | 9352 | 9349 | 9353 | 9355 | 9354 | 1556 | 12179 | 7144 | 4314 | 7509 | 15131 |
| | 13118 | 16078 | 945 | 13430 | 18194 | 4956 | 6929 | 1412 | 4783 | 12306 | 9385 | 12690 |
| | 12672 | 4342 | 13055 | 13054 | 7021 | 4081 | 7703 | 3033 | 17154 | 9984 | 15399 | 7217 |

(continued)

| SEQ ID NO: | homolog SEQ ID NOs | | | | | | | | | | |
|---|---|---|---|---|---|---|---|---|---|---|---|
| 9277 | 13222 | 13034 | 341 | 11004 | 15248 | 1463 | 2015 | 17433 | 12644 | 15941 | 10807 |
| 3266 | 8742 | 1972 | 3510 | 9558 | 4266 | 626 | 7388 | 14560 | 7227 | 13555 | 17967 |
| 4546 | 8700 | 17272 | 10335 | 18466 | 1060 | 3720 | 12985 | 9961 | 12732 | 18869 | 8954 |
| 2752 | 13820 | 10361 | 15865 | 2500 | 10465 | 18069 | 18784 | 16053 | 775 | 2727 | 16534 |
| 13326 | 15757 | 12838 | 11061 | 3693 | 13785 | 10142 | 11449 | 11354 | 9684 | 5074 | 9660 |
| 12700 | 15166 | 15169 | 13456 | 13485 | 13469 | 13473 | 13487 | 13505 | 13506 | 12663 | 12661 |
| 5471 | 17875 | 16908 | 13205 | 17054 | 9363 | 989 | 988 | 4140 | 6615 | 6572 | 6635 |
| 6591 | 6608 | 6592 | 11985 | 6631 | 6662 | 6633 | 6573 | 6638 | 3200 | 10745 | 1012 |
| 15212 | 14003 | 8819 | 17558 | 3396 | 13373 | 19044 | 7278 | 8887 | 11236 | 5875 | 11288 |
| 16665 | 5242 | 4870 | 10506 | 725 | 15807 | 18290 | 11584 | 11815 | 10709 | 5665 | 8913 |
| 4443 | 4446 | 4940 | 18124 | 18126 | 6102 | 10718 | 13110 | 5792 | 18314 | 9497 | 3370 |
| 3367 | 11346 | 7951 | 13887 | 1123 | 7016 | 8221 | 13167 | 1644 | 9351 | 6673 | 16460 |
| 18895 | 16626 | 1321 | 13321 | 3707 | 6276 | 7282 | 17173 | 5804 | 19036 | 12603 | 15028 |
| 11016 | 18046 | 8269 | 8121 | 2935 | 14015 | 17073 | 16077 | 9816 | 3949 | 6418 | 11142 |
| 17987 | 1761 | 15163 | 15613 | 15594 | 15124 | 15595 | 15162 | 15146 | 15144 | 15126 | 15138 |
| 15121 | 15140 | 15164 | 15139 | 3828 | 3810 | 3832 | 3814 | 3796 | 16726 | 10777 | 13774 |
| 13776 | 10726 | 11111 | 10708 | 10724 | 10729 | 11982 | 13771 | 11437 | 8696 | 18408 | 9083 |
| 11185 | 5772 | 5773 | 5793 | 5795 | 7325 | 5718 | 5716 | 5775 | 7308 | 7313 | 11294 |
| 13971 | 13968 | 13919 | 13986 | 13966 | 13898 | 13983 | 13941 | 13948 | 5742 | 5738 | 7755 |
| 7753 | 5740 | 5809 | 5753 | 5776 | 5755 | 17404 | 5756 | 5771 | 5263 | 13375 | 13376 |
| 8038 | 14491 | 18265 | 7335 | 17735 | 1470 | 6663 | 1478 | 1476 | 1455 | 1451 | 1935 |
| 1473 | 8041 | 1937 | 1458 | 1933 | 1452 | 5249 | 1447 | 11296 | 11297 | 11282 | 11259 |
| 11277 | 11299 | 11279 | 11417 | 11344 | 11415 | 11258 | 2990 | 14315 | 10158 | 2987 | 18033 |
| 18144 | 18603 | 18600 | 18147 | 1821 | 1893 | 2985 | 2986 | 2989 | 6848 | 17794 | 8531 |
| 8557 | 8554 | 8535 | 8527 | 8550 | 8751 | 9638 | 9616 | 9639 | 9615 | 9620 | 9052 |
| 9612 | 8994 | 8992 | 8920 | 8924 | 3380 | 10785 | 6038 | 10491 | 4075 | 16618 | 7274 |
| 6061 | 5955 | 7685 | 17176 | 11024 | 17188 | 5903 | 8096 | 8061 | 16575 | 16559 | 16506 |
| 16597 | 10546 | 17145 | 7669 | 5958 | 8947 | 8927 | 8930 | 8925 | 9016 | 9019 | 8957 |
| 8984 | 8988 | 8990 | 9023 | 6337 | 5820 | 5822 | 9051 | 7330 | 5064 | 9053 | 18015 |
| 11669 | 5821 | 14528 | 14509 | 5813 | 15700 | 2445 | 11351 | 6436 | 14764 | 8797 | 13073 |
| 17133 | | | | | | | | | | | |
| 340:13196 | 16439 | 6745 | 4610 | 11899 | 10523 | 19022 | 5232 | 11306 | 2056 | 2570 | 18196 |
| 609 | 5025 | 5019 | 7865 | 15314 | 2678 | 12655 | 9546 | 9454 | 13253 | 18958 | 1923 |
| 4335 | 9039 | 11412 | 8309 | 7587 | 15840 | 8854 | 5981 | 9219 | 16268 | 15747 | 4154 |
| 6311 | 13498 | 4599 | 5366 | 3114 | 9197 | 17805 | 3156 | 14735 | 1918 | 13226 | 12950 |
| 7323 | 7733 | 8437 | 9356 | 12112 | 17458 | 16494 | 13907 | 17075 | 9200 | 9250 | 9276 |
| 9252 | 9278 | 9279 | 9281 | 6045 | 5162 | 7615 | 7595 | 8879 | 7374 | 9867 | 6042 |
| 7749 | 8020 | 3743 | 8718 | 16238 | 16530 | 15604 | 18077 | 14517 | 14553 | 1871 | 1069 |
| 2878 | 2845 | 12697 | 9970 | 12964 | 12965 | 3572 | 17241 | 4279 | 18703 | 616 | 17038 |
| 17036 | 17007 | 17009 | 17034 | 17035 | 17003 | 17004 | 2716 | 1388 | 14685 | 12624 | 3453 |
| 2426 | 4819 | 12501 | 3673 | 10048 | 1499 | 2916 | 4850 | 5117 | 7647 | 14030 | 9934 |
| 15766 | 13237 | 539 | 11479 | 18921 | 7137 | 17775 | 5670 | 16492 | 18336 | 18337 | 1862 |
| 6718 | 11040 | 13556 | 3974 | 6965 | 5054 | 3081 | 6968 | 7212 | 13037 | 16679 | 6364 |
| 2629 | 2077 | 3766 | 6851 | 9951 | 7731 | 9942 | 5707 | 15735 | 10648 | 11283 | 2381 |
| 2363 | 18601 | 10074 | 3268 | 2684 | 4642 | 9218 | 664 | 2494 | 2601 | 8219 | 17656 |
| 10990 | 7785 | 3331 | 5146 | 4493 | 4620 | 2917 | 16768 | 15720 | 8671 | 15843 | 9645 |
| 5541 | 6103 | 11053 | 14038 | 15349 | 2088 | 795 | 3297 | 1000 | 1559 | 12161 | 15353 |
| 13769 | 2270 | 10599 | 11748 | 18825 | 18373 | 3182 | 10477 | 13228 | 5351 | 5273 | 17329 |
| 10825 | 12254 | 8870 | 1199 | 4883 | 10343 | 12967 | 4238 | 1562 | 17883 | 11472 | 17019 |
| 16370 | 10801 | 18263 | 14621 | 12659 | 15703 | 593 | 11508 | 12110 | 12723 | 12111 | 4392 |

(continued)

| SEQ ID NO: | homolog SEQ ID NOs | | | | | | | | | | |
|---|---|---|---|---|---|---|---|---|---|---|---|
| 11861 | 4169 | 14911 | 16024 | 18137 | 17510 | 10528 | 18935 | 14916 | 17827 | 18611 | 15191 |
| 12909 | 18197 | 1658 | 2086 | 16157 | 11091 | 12303 | 13953 | 12295 | 13403 | 14690 | 527 |
| 3357 | 2859 | 11106 | 17963 | 1655 | 16934 | 15042 | 16307 | 2152 | 3172 | 3999 | 2695 |
| 4223 | 1968 | 14466 | 15787 | 5255 | 12229 | 11570 | 11600 | 15937 | 7082 | 5673 | 6369 |
| 8380 | 5325 | 3322 | 3328 | 5672 | 6368 | 13585 | 11692 | 17220 | 3531 | 3073 | 1889 |
| 8613 | 17603 | 13282 | 6059 | 14374 | 6092 | 15505 | 3382 | 10113 | 9943 | 9960 | 13705 |
| 10510 | 12419 | 8371 | 1356 | 697 | 2104 | 868 | 15905 | 6254 | 10906 | 12136 | 9705 |
| 6214 | 589 | 2558 | 7015 | 7333 | 7345 | 7340 | 15278 | 15258 | 15276 | 7342 | 4805 |
| 10215 | 18817 | 18643 | 12946 | 1331 | 1333 | 11882 | 13568 | 2641 | 9802 | 10173 | 17561 |
| 11973 | 6328 | 16066 | 13399 | 15310 | 10568 | 6842 | 4296 | 9829 | 12919 | 13707 | 6529 |
| 6625 | 1039 | 5395 | 4067 | 4526 | 9438 | 10350 | 15249 | 15857 | 13015 | 9800 | 17845 |
| 3551 | 16820 | 7979 | 1633 | 9827 | 7991 | 9824 | 11672 | 9865 | 9204 | 9284 | 9220 |
| 9249 | 8579 | 9175 | 9246 | 8549 | 9173 | 9248 | 8563 | 9232 | 9203 | 8578 | 8559 |
| 9172 | 9178 | 8585 | 8581 | 8552 | 8556 | 9176 | 8587 | 13212 | 10149 | 13765 | 9201 |
| 9226 | 14964 | 1718 | 7993 | 12641 | 18059 | 9231 | 3494 | 14770 | 9563 | 7665 | 10399 |
| 4688 | 6342 | 7025 | 16979 | 16279 | 16302 | 16981 | 16413 | 16433 | 16414 | 16330 | 16333 |
| 16331 | 16971 | 16296 | 16350 | 16954 | 16356 | 16974 | 16353 | 16300 | 16431 | 16304 | 16292 |
| 16357 | 16955 | 16952 | 16325 | 16385 | 16435 | 16411 | 16410 | 16280 | 16430 | 16957 | 16354 |
| 16387 | 16985 | 16415 | 16327 | 16455 | 16457 | 16390 | 16380 | 16950 | 16436 | 16382 | 16274 |
| 16452 | 16456 | 16278 | 16453 | 5858 | 2370 | 14410 | 18969 | 3274 | 1393 | 8975 | 573 |
| 7715 | 15030 | 1249 | 14601 | 17639 | 7403 | 1504 | 8265 | 10515 | 8316 | 15104 | 1901 |
| 14063 | 11766 | 12976 | 11736 | 12162 | 1902 | 7470 | 15551 | 8361 | 10367 | 14060 | 8052 |
| 9801 | 9803 | 7474 | 1051 | 2959 | 4990 | 7193 | 12916 | 17670 | 12887 | 10565 | 16240 |
| 8866 | 11564 | 12294 | 12293 | 17789 | 14742 | 13578 | 3256 | | | | |
| 341:13280 | 7465 | 13170 | 13488 | 8094 | 7023 | 15591 | 15585 | 15589 | 13440 | 10740 | 14846 |
| 15781 | 7542 | 4997 | 6793 | 6530 | 15127 | 14107 | 14099 | 12692 | 12638 | 6743 | 8397 |
| 8398 | 12234 | 12202 | 12231 | 12184 | 8401 | 12178 | 1852 | 18620 | 17417 | 1931 | 18095 |
| 18096 | 18081 | 18076 | 17392 | 17346 | 1795 | 1793 | 17515 | 17496 | 18138 | 17518 | 18108 |
| 8418 | 3043 | 18459 | 8099 | 9311 | 16771 | 2036 | 14555 | 8712 | 1468 | 4413 | 4414 |
| 4416 | 4436 | 4435 | 4357 | 4355 | 4371 | 4376 | 3798 | 3797 | 3315 | 11764 | 5938 |
| 3236 | 15423 | 18556 | 1084 | 9329 | 9332 | 9334 | 9352 | 9349 | 9353 | 9355 | 9354 |
| 1556 | 12179 | 7144 | 4314 | 7509 | 15131 | 13118 | 16078 | 945 | 13430 | 18194 | 4956 |
| 6929 | 4783 | 12306 | 9385 | 12690 | 12672 | 4342 | 13055 | 13054 | 7021 | 4081 | 7703 |
| 3033 | 17154 | 9984 | 15399 | 7217 | 9277 | 13222 | 13034 | 15248 | 1463 | 2015 | 17433 |
| 12644 | 15941 | 10807 | 3266 | 8742 | 1972 | 3510 | 9558 | 4266 | 626 | 7388 | 14560 |
| 7227 | 13555 | 17967 | 4546 | 11878 | 8700 | 17272 | 18466 | 1060 | 3720 | 12985 | 9961 |
| 18869 | 12732 | 8954 | 2752 | 13820 | 10361 | 15865 | 2500 | 10465 | 18069 | 18784 | 16053 |
| 775 | 2727 | 16534 | 13326 | 15757 | 12838 | 3693 | 13785 | 10142 | 11449 | 11354 | 9684 |
| 5074 | 9660 | 12700 | 15166 | 15169 | 13456 | 13485 | 13469 | 13473 | 13487 | 13506 | 13505 |
| 12661 | 12663 | 5471 | 17875 | 13205 | 16908 | 17054 | 9363 | 989 | 988 | 4140 | 6633 |
| 6608 | 6592 | 6591 | 6631 | 6573 | 6635 | 11985 | 6615 | 6572 | 6662 | 6638 | 3200 |
| 1012 | 10745 | 15212 | 14003 | 8819 | 3396 | 13373 | 17558 | 19044 | 7278 | 8887 | 16665 |
| 5875 | 11288 | 11236 | 5242 | 4870 | 725 | 10506 | 11584 | 15807 | 18290 | 10709 | 5665 |
| 8913 | 4443 | 4446 | 18124 | 18126 | 6102 | 10718 | 13110 | 5792 | 18314 | 9497 | 3370 |
| 3367 | 15852 | 11346 | 7951 | 13887 | 1123 | 7016 | 8221 | 1644 | 13167 | 9351 | 16460 |
| 6673 | 18895 | 5487 | 3178 | 16626 | 1321 | 3707 | 6276 | 7282 | 13321 | 5804 | 17173 |
| 12603 | 18046 | 8269 | 2935 | 14015 | 8121 | 17073 | 16077 | 9816 | 3949 | 6418 | 11142 |
| 6209 | 1761 | 15146 | 15163 | 15613 | 15594 | 15162 | 15595 | 15124 | 15121 | 15144 | 15140 |
| 15164 | 15138 | 15126 | 15139 | 3828 | 3832 | 3814 | 3810 | 3796 | 16726 | 10777 | 13774 |
| 13776 | 10726 | 11111 | 10708 | 10724 | 10729 | 11982 | 13771 | 11437 | 8696 | 18408 | 9083 |

(continued)

| SEQ ID NO: | homolog SEQ ID NOs | | | | | | | | | |
|---|---|---|---|---|---|---|---|---|---|---|
| 11185 | 5773 | 5772 | 5793 | 5795 | 7325 | 5718 | 5716 | 5775 | 7308 | 7313 | 11294 |
| 13971 | 13968 | 13986 | 13898 | 13919 | 13966 | 13983 | 13948 | 13941 | 7755 | 7753 | 5738 |
| 5742 | 5740 | 5809 | 5753 | 5776 | 5755 | 17404 | 5756 | 5771 | 5263 | 13376 | 13375 |
| 8038 | 14491 | 18265 | 7335 | 17735 | 1451 | 1470 | 8041 | 1933 | 1478 | 1455 | 1935 |
| 1473 | 1458 | 6663 | 1476 | 1937 | 1452 | 5249 | 1447 | 11296 | 11297 | 11299 | 11259 |
| 11282 | 11277 | 11279 | 11417 | 11344 | 11415 | 11258 | 2990 | 14315 | 10158 | 2987 | 18033 |
| 18603 | 18144 | 18147 | 18600 | 1821 | 1893 | 2985 | 2986 | 2989 | 6848 | 17794 | 8531 |
| 8557 | 8554 | 8535 | 8527 | 8550 | 8751 | 9616 | 9638 | 9615 | 9620 | 9639 | 9052 |
| 9612 | 8994 | 8992 | 8924 | 8920 | 3380 | 7669 | 10785 | 17145 | 5958 | 16618 | 16506 |
| 11024 | 10491 | 4075 | 7685 | 16559 | 8096 | 6038 | 5955 | 17188 | 7274 | 6061 | 17176 |
| 16597 | 8061 | 5903 | 16575 | 10546 | 8947 | 8927 | 8925 | 8930 | 9019 | 9016 | 8984 |
| 8957 | 8988 | 8990 | 9023 | 6337 | 5820 | 5822 | 9051 | 7330 | 5064 | 9053 | 18015 |
| 11669 | 5821 | 14509 | 14528 | 5813 | 15700 | 339 | 2445 | 11351 | 6436 | 14764 | 8797 |
| 13073 | 17133 | | | | | | | | | | |
| 342: 4275 | 14267 | 13342 | 6967 | 16887 | 3675 | 9675 | 6057 | 3840 | 17900 | 16902 | 18661 |
| 10421 | 17260 | 3986 | 15808 | 7849 | 8997 | 16487 | 15022 | 4823 | 12754 | 10395 | 8534 |
| 13581 | 2017 | 5522 | 7701 | 2701 | 9728 | 17790 | 12191 | 4072 | 6250 | 7712 | 7019 |
| 12120 | 7086 | 4916 | 18255 | 8520 | 18744 | 13466 | 18395 | 2589 | 9658 | 17251 | 12180 |
| 4996 | 5774 | 10305 | 4826 | 3919 | 11530 | 6902 | 2861 | 4554 | 7041 | 2825 | 18879 |
| 7681 | 3326 | 2822 | 8206 | 1820 | 3218 | 15526 | 18435 | 14428 | 14561 | 13249 | 10813 |
| 11879 | 4832 | 15268 | 2787 | 6653 | 12610 | 13972 | 3977 | 7443 | 4056 | 17876 | 12742 |
| 17551 | 9095 | 830 | 12330 | 18456 | 5153 | 17559 | 14538 | 3441 | 4281 | 6388 | 929 |
| 12203 | 12005 | 16713 | 14490 | 3329 | 17610 | 15818 | 10223 | 1832 | 17892 | 16739 | 19247 |
| 19209 | 8382 | 6262 | 3193 | 5986 | 8544 | 10873 | 2844 | 11163 | 3318 | 5155 | 18764 |
| 11864 | 6332 | 19115 | 19114 | 11647 | 8254 | 9757 | 17455 | 18569 | 13668 | 16305 | 19055 |
| 18604 | 14213 | 19057 | 18621 | 18578 | 14211 | 18907 | 13293 | 13257 | 13319 | 13318 | 13255 |
| 13233 | 13274 | 13240 | 1288 | 14112 | 705 | 1322 | 1313 | 14151 | 14149 | 14096 | 669 |
| 1265 | 703 | 700 | 639 | 13682 | 14145 | 14134 | 647 | 1259 | 14146 | 1243 | 14113 |
| 1268 | 14130 | 13704 | 1317 | 707 | 14178 | 14148 | 14116 | 736 | 757 | 756 | 677 |
| 14119 | 13679 | 14172 | 13688 | 14097 | 667 | 782 | 14088 | 783 | 678 | 14174 | 734 |
| 14089 | 729 | 642 | 791 | 758 | 759 | 1233 | 761 | 787 | 784 | 1238 | 13686 |
| 5633 | 3144 | 14179 | 6378 | 18102 | 19076 | 19103 | 19099 | 19102 | 19090 | 19088 | 18743 |
| 9920 | 13711 | 13723 | 1195 | 19220 | 1467 | 17833 | 10277 | 5278 | 7877 | 6039 | 5512 |
| 6020 | 6019 | 6016 | 6015 | 5994 | 5995 | 6012 | 5993 | 5515 | 5032 | 11146 | 19134 |
| 2994 | 17095 | 8583 | 3311 | 11705 | 15026 | 10508 | 1167 | 12675 | 10097 | 1101 | 1091 |
| 5963 | 8953 | 10466 | | | | | | | | | |
| 343: 4095 | 15046 | 10521 | 1628 | 9595 | 7433 | 7621 | 18399 | 1383 | 5781 | 12525 | 14082 |
| 6175 | 5446 | 17124 | 2637 | 14579 | 8588 | 804 | 8650 | 5284 | 18904 | 8106 | 6336 |
| 4263 | 11332 | 12874 | 6478 | 13913 | 9066 | 13379 | 17596 | 18592 | 13115 | 2010 | 2419 |
| 4845 | 14997 | 18740 | 618 | 3431 | 3970 | 10518 | 14706 | 16668 | 9305 | 10374 | 18493 |
| 5038 | 10849 | 14836 | 2271 | 992 | 9179 | 16837 | 8717 | 10496 | 18477 | 7920 | 10916 |
| 3559 | 10271 | 17999 | 4030 | 18694 | 6969 | 4377 | 9285 | 2666 | 11862 | 10774 | 13447 |
| 10398 | 3876 | 2057 | 18631 | 17938 | 17050 | 10348 | 604 | 6712 | 2475 | 19003 | 2910 |
| 16091 | 2140 | 10720 | 3323 | 9194 | 14883 | 11223 | 19145 | 10602 | 657 | 17840 | 11593 |
| 11595 | 18216 | 16978 | 13665 | 12968 | 15761 | 3402 | 4008 | 16708 | 17466 | 9556 | 11066 |
| 6487 | 5250 | 16702 | 11261 | 15834 | 16616 | 4700 | 4701 | 9490 | 17897 | 6730 | 3210 |
| 1853 | 14666 | 10844 | 14663 | 13655 | 14660 | 4649 | 6868 | 4648 | 6855 | 16252 | 4885 |
| 1523 | 15210 | 12821 | 1183 | 15471 | 2051 | 5972 | 4643 | 3628 | 1352 | 5987 | 14639 |
| 16535 | 9679 | 14991 | 11416 | 7257 | 2330 | 4274 | 9585 | 6918 | 2388 | 17101 | 4046 |
| 16235 | 12769 | 4685 | 881 | 5507 | 11957 | 3735 | 14383 | 16187 | 2043 | 5111 | 10878 |

(continued)

SEQ ID NO:   homolog SEQ ID NOs

```
        17577  19147  17755  12154   7436  13269  13270   4312   7297   2111   3014   9674
         4608  10651  11599    337   2316  13358
344: 5300   3500   4027   7686   9421   4052   8763  14058   5561   2575  18884  15708
         4543   1686  13252  13166   4602  14826  18707   5525   8193   7080  17793    986
         9233  14403
345: 5980   9971   4070   2303   9479   8998   7155  11775    933
346:18418  12504  14095   1860   5339   1942  18388  11380    752  11688  14723   7312
        18500   6219  12070   1382  15676  13413  19034  10573  16153  13734  18099  17389
         1808   8009  14640  13476  14408   8018   7100   3096   2598  13386    625  11250
        11018   3111  10260  18653   2016    637  10620  13082   2021  11275   8483  13851
        15961  10302  12726  18421   6212   3159   2443   3658
347:11270  17077   4946   4945  15527  18554  15862  15870  15860  13080  12083  14086
        18704  15951  14593   5514   8137  18330  17072   5748   4003   7232   9301   8840
        14744   3550   6166  17459  15531   4830  12695  14036   7418   7827  14592   3352
        16349   7118  15429   5566  10536   2972  15722   3016   1928   1508   3985  14595
        13481   9084  14959  13221  10228  16121  15492  12993   8591  15602   2592  11832
         1949   9134   9588   2207  16909   5169   5148  11680  17594  10679   5728   6123
         4235  11214  15511  12368  11088  18835  11905  10115  15436  15660   7540   3170
        11393  10848  15970  12714   2257  16752   3677   3857   6325   5243  14768  15918
        18582  14405  10218  11890   9037  16519   6235  12549  18827   2287  12400  15098
         7528
348:13176   4345   6836  18766   3513  16800  19180   2524  12509   5751   7573   4388
349:11912   5568   6508   3945  11062  10942   8564   5617  16983   5480  19110   2468
         3581  10481   7817  10475   5989   7810   3957   4139   6900   9519   8199   1162
        17815  16982  13169  11144  14543    490   5095   4658   5436   2277  16315   7320
         8369   7909   7971  14917   4572   5603   5011
351: 1944   3388  11320  11302  11319  11317  11305   7165   1174  11523  18349  13775
         7657   7283   3563  13470   3951  18978   3197   3932   3046   3062   3065   3063
         5791   3938  11517  12121  11055   4011  16549   3495   2533   3650  15029  17486
          451  17381   4511  14953   9732  15182   2627  10876  18227    511  19073   8217
        12521  15724   4425  15491   7189   8088  17498  17501  18186
352:13975   6206  17120  13306  11947   1705  16008   3435  19023   7536   6974   2031
        13640   1494  18480  11512   3888   7159  18706   6893  11448    714   4753  10937
         6126   6218   8698  13988  10384  16502  11673  19019  10167   7265   4613   9695
        14636  17295  14469  15141   2783
353: 8835  11783   9669  12756   2337  13479   9821   8090  13818
354:18918  14457
355:17083  10967   5149  19161  12075  11238  18924   4468  11390  10560  17452  17271
        17530   2078
356:16848  17598  13404   2320   4652   6293  12539   3765   8280  13130    520   3952
         9848  15670
357: 3703  18488  18007   3371  18022  12829   4322  10690   1141  15264   8036   7857
          924    975   7855   7926    325    326   4938  11635  10188   3795   3794    974
         7376   8980   2807   2826  15414   3221  11000  16877    358  11643  14101  17431
        14104  11922  14106  14118  14103  11917  14117  11877   9156  15521  15519  15512
         2076   7599  18286  17450  10636  10883  19245   2706  17448  17445  13484  12363
         8462   8465   1521  11661  18323  10495  18481   1858  17901   1025  15261  14337
        11009    802   1489   9751   5535  19225   4741   8379   9986   5615   9570  18249
         1579  18874  14957   2833  13097  11855  17444   2048   5863  16811  12374  13302
         5440  14857  13516  10812   9419   6771  11768  12141   3543   6242  11404  18052
```

| SEQ ID NO: | homolog SEQ ID NOs | | | | | | | | | | |
|---|---|---|---|---|---|---|---|---|---|---|---|
| 1963 | 2636 | 7150 | 2875 | 5500 | 15645 | 3024 | 19039 | 805 | 11538 | 2943 | 12915 |
| 17169 | 4471 | 15921 | 17129 | 2420 | 9530 | 8029 | 16041 | 11094 | 10747 | 13795 | 2144 |
| 17511 | 12731 | 6924 | 15147 | 18864 | 7598 | 2259 | 7950 | 18104 | 7123 | 13183 | 9498 |
| 651 | 5099 | 8353 | 13883 | 4389 | 2662 | 11940 | 19159 | 16364 | 4653 | 14754 | 12986 |
| 3439 | 9888 | 4998 | 8161 | 19192 | 17068 | 10691 | 934 | 606 | 769 | 8468 | 18297 |
| 1397 | 3167 | 10721 | 18909 | 18127 | 820 | 8767 | 11231 | 10209 | 18039 | 4769 | 4271 |
| 914 | 5454 | 17761 | 1436 | 16749 | 12983 | 7990 | 3461 | 11482 | 2488 | 2605 | 15929 |
| 19116 | 1132 | 11154 | 5499 | 16543 | 12081 | 4575 | 10781 | 15011 | 17427 | 14182 | 11494 |
| 4324 | 686 | 11427 | 8119 | 16545 | 1033 | 15056 | 15910 | 7850 | 13576 | 15542 | 16151 |
| 16155 | 16095 | 13014 | 16069 | 3257 | 6401 | 17505 | 17504 | 17525 | 17524 | 2022 | 7377 |
| 432 | 16819 | 10332 | 2411 | 10331 | 2410 | 12098 | 12865 | 3223 | 4128 | 11048 | 1117 |
| 13770 | 13767 | 16781 | 16777 | 16779 | 16801 | 17741 | 16774 | 4860 | 15510 | 12777 | 12778 |
| 14071 | 14067 | 14053 | 14069 | 14091 | 14072 | 14075 | 8376 | 11001 | 9987 | 7853 | 5184 |
| 7393 | 7395 | 3219 | 3220 | 17517 | 3203 | 17996 | 3205 | 17513 | 10899 | 3204 | 18474 |
| 18023 | 600 | 7911 | 16191 | 13238 | 15975 | 4924 | 9729 | 6091 | 4326 | 6820 | 13828 |
| 4812 | 9933 | 17784 | 16424 | 4768 | 15749 | 7309 | 10904 | 2071 | 4194 | 15043 | 2025 |
| 10355 | 5253 | 18478 | 13347 | 18509 | 13344 | 18011 | 8209 | 8302 | 10221 | 15948 | 8147 |
| 11081 | 18398 | 17612 | 16052 | 2853 | 15154 | 15669 | 4108 | 413 | 16888 | 8441 | 3479 |
| 1048 | 17549 | 9749 | 17291 | 14470 | 2008 | 13574 | 5988 | 3547 | 13320 | 12107 | 4540 |
| 6031 | 16301 | 6076 | 18372 | 10579 | 4109 | 11366 | 19231 | 11108 | 8482 | 5412 | 12150 |
| 14436 | 18176 | 1610 | 674 | 6891 | 13730 | 8642 | 6095 | 3638 | 993 | 17479 | 11156 |
| 6014 | 4301 | 1954 | 10818 | 7039 | 15055 | 12418 | 10003 | 1924 | 13798 | 11179 | 15926 |
| 13552 | 9569 | 18113 | 9432 | 15846 | 14535 | 3145 | 12007 | 16132 | 12934 | 1538 | 16847 |
| 15583 | 6741 | 5534 | 14502 | 4641 | 7188 | 15672 | 4220 | 11219 | 12375 | 7172 | 3983 |
| 2230 | 6072 | 5854 | 17508 | 17766 | 18490 | 15885 | 4617 | 4368 | 16850 | 13940 | 3991 |
| 1557 | 11615 | 2624 | 18508 | 18457 | 10864 | 18750 | 6697 | 1128 | 12487 | 18977 | 5445 |
| 11199 | 10746 | 16932 | 5601 | 16270 | 559 | 5072 | 16012 | 1609 | 13543 | 18010 | 5876 |
| 18551 | 11710 | 16973 | 8611 | 17786 | 896 | 15150 | 6585 | 6520 | 13129 | 4965 | 18175 |
| 2832 | 10945 | 3423 | 17125 | 18855 | 16525 | 1748 | 5964 | 7611 | 14612 | 11300 | 17123 |
| 14105 | 1285 | 15156 | 11382 | 12956 | 17420 | 6607 | 6377 | 1873 | 5035 | 9405 | 13286 |
| 8729 | 9963 | 8621 | 9241 | 13064 | 2473 | 14203 | 19067 | 17080 | 10484 | 16852 | 9591 |
| 6583 | 5237 | 3885 | 11942 | 14024 | 5941 | 6783 | 2321 | 18484 | 18027 | 18467 | 18028 |
| 17973 | 17972 | 17970 | 17969 | 5302 | 16042 | 6762 | 8202 | 17109 | 9781 | 3057 | 12195 |
| 18035 | 14637 | 2924 | 5223 | 595 | 5651 | 5083 | 15409 | 17949 | 19109 | 11395 | 13131 |
| 5389 | 6525 | 18579 | 18245 | 18559 | 15802 | 6580 | 13199 | 10789 | 12935 | 13695 | 14236 |
| 5044 | 6962 | 15953 | 11413 | 2116 | 18736 | 3803 | 13906 | 7398 | 13922 | 18325 | 18513 |
| 18472 | 328 | 16450 | 7353 | 7349 | 7351 | 7348 | 7218 | 7214 | 7201 | 7224 | 7220 |
| 7200 | 2490 | 5078 | 333 | 15058 | 19237 | 15045 | 15907 | 16396 | 16398 | 15912 | 15057 |
| 15636 | 16403 | 15079 | 15075 | 11327 | 18489 | | | | | | |
| 358: 7510 | 2877 | 514 | 3654 | 3669 | 15610 | 18488 | 18007 | 3371 | 18022 | 8268 | 8266 |
| 14102 | 360 | 359 | 329 | 362 | 357 | 11217 | 7969 | 9372 | 8541 | 7890 | 18428 |
| 11507 | 18426 | 1141 | 15264 | 8036 | 7857 | 19000 | 613 | 8263 | 13146 | 19018 | 19126 |
| 18606 | 6915 | 6347 | 13472 | 10628 | 8494 | 13268 | 2909 | 7855 | 11569 | 13550 | 6426 |
| 2973 | 325 | 7807 | 14215 | 3026 | 12947 | 15493 | 326 | 15366 | 11129 | 6026 | 3133 |
| 5061 | 2597 | 18920 | 2219 | 18525 | 4049 | 7576 | 18045 | 2110 | 5513 | 16459 | 19228 |
| 14697 | 16792 | 16718 | 3560 | 18726 | 4631 | 9142 | 4307 | 4348 | 6586 | 4615 | 4629 |
| 4995 | 4616 | 699 | 3687 | 5856 | 10188 | 3795 | 3794 | 974 | 7376 | 8980 | 9347 |
| 9315 | 2807 | 2826 | 13328 | 18424 | 10646 | 5320 | 13791 | 17988 | 15414 | 3221 | 11000 |
| 16877 | 11643 | 14101 | 17431 | 14104 | 11922 | 14106 | 14118 | 14103 | 11917 | 14117 | 11877 |
| 9156 | 2076 | 7599 | 18286 | 17450 | 10636 | 10883 | 19245 | 2706 | 17448 | 17445 | 13484 |

(continued)

| SEQ ID NO: | homolog SEQ ID NOs | | | | | | | | | | |
|---|---|---|---|---|---|---|---|---|---|---|---|
| | 18745 | 15681 | 5992 | 2522 | 2523 | 15520 | 19014 | 6220 | 12363 | 8462 | 8465 | 1521 |
| | 11661 | 18323 | 10495 | 18481 | 1858 | 17901 | 1025 | 15261 | 14337 | 11009 | 802 | 1489 |
| | 9751 | 5535 | 19225 | 4741 | 8379 | 9986 | 5615 | 9570 | 18249 | 1579 | 18874 | 14957 |
| | 2833 | 13097 | 11855 | 17444 | 2048 | 5863 | 12374 | 16811 | 13302 | 5440 | 14857 | 13516 |
| | 10812 | 9419 | 6771 | 11768 | 5500 | 12141 | 3543 | 6242 | 11404 | 18052 | 1963 | 2636 |
| | 7150 | 2875 | 15645 | 3024 | 19039 | 12915 | 805 | 11538 | 2943 | 17169 | 4471 | 9797 |
| | 17129 | 2420 | 8029 | 9530 | 15921 | 2555 | 7989 | 1311 | 5122 | 16041 | 11094 | 10747 |
| | 13795 | 2144 | 17511 | 12731 | 6924 | 14303 | 15147 | 18864 | 7598 | 2259 | 7950 | 18104 |
| | 7123 | 13183 | 9498 | 651 | 5099 | 8353 | 13883 | 4389 | 2662 | 11940 | 19159 | 16364 |
| | 14754 | 18608 | 4653 | 12986 | 17068 | 3439 | 9888 | 4998 | 8161 | 19192 | 10691 | 934 |
| | 606 | 769 | 8468 | 18297 | 1397 | 3167 | 10721 | 18909 | 18127 | 820 | 11516 | 4271 |
| | 8767 | 11231 | 10209 | 18039 | 4769 | 914 | 2605 | 5454 | 17761 | 1436 | 12263 | 16749 |
| | 12435 | 12983 | 19116 | 7990 | 3461 | 11482 | 2488 | 14294 | 2357 | 15929 | 1132 | 11154 |
| | 5499 | 16543 | 12081 | 4575 | 10781 | 15011 | 17427 | 14182 | 11494 | 4324 | 686 | 1033 |
| | 15056 | 15910 | 7850 | 13576 | 15542 | 16151 | 16155 | 16095 | 11455 | 13014 | 16069 | 3257 |
| | 4390 | 6401 | 17505 | 17504 | 17525 | 17524 | 6799 | 3353 | 6792 | 2022 | 3424 | 3404 |
| | 3409 | 792 | 7377 | 432 | 16819 | 10332 | 2411 | 10331 | 2410 | 18533 | 12098 | 12865 |
| | 3223 | 4128 | 11048 | 1117 | 13770 | 13767 | 16781 | 16777 | 16779 | 16801 | 17741 | 16774 |
| | 4860 | 15510 | 12777 | 12778 | 14071 | 14067 | 14053 | 14069 | 14091 | 14072 | 14075 | 8376 |
| | 11001 | 9987 | 7853 | 5184 | 7393 | 7395 | 3219 | 3220 | 17517 | 3203 | 17996 | 3205 |
| | 17513 | 10899 | 3204 | 18474 | 18023 | 600 | 17986 | 18923 | 575 | 4812 | 7911 | 16191 |
| | 13238 | 15975 | 4924 | 9729 | 6091 | 4326 | 6820 | 13828 | 9933 | 17784 | 16424 | 4768 |
| | 14684 | 9518 | 15749 | 7309 | 13646 | 10904 | 2071 | 6198 | 4194 | 15043 | 2025 | 10355 |
| | 5253 | 13347 | 18509 | 13344 | 18011 | 8209 | 8302 | 10221 | 1303 | 14421 | 17237 | 10579 |
| | 15948 | 16301 | 1954 | 8147 | 11081 | 2008 | 13320 | 18398 | 17612 | 7188 | 16052 | 3547 |
| | 12007 | 3983 | 2853 | 15154 | 15669 | 4108 | 413 | 16888 | 8441 | 3479 | 1048 | 17549 |
| | 9749 | 17291 | 14470 | 13574 | 5988 | 12107 | 4540 | 6031 | 6076 | 18372 | 4109 | 11366 |
| | 19231 | 11108 | 8482 | 5412 | 12150 | 14436 | 18176 | 1610 | 674 | 6891 | 13730 | 8642 |
| | 6095 | 3638 | 993 | 17479 | 11156 | 6014 | 4301 | 10818 | 7039 | 15055 | 12418 | 10003 |
| | 1924 | 13798 | 11179 | 15926 | 13552 | 9569 | 18113 | 9432 | 15846 | 14535 | 3145 | 16132 |
| | 12934 | 1538 | 16847 | 15583 | 6741 | 5534 | 14502 | 15885 | 4641 | 15672 | 4220 | 11219 |
| | 12375 | 7172 | 2230 | 6072 | 5854 | 17508 | 17766 | 18490 | 4617 | 559 | 4368 | 16850 |
| | 13940 | 3991 | 1557 | 11615 | 2624 | 18508 | 18457 | 10864 | 18750 | 6697 | 1128 | 12487 |
| | 18977 | 5445 | 11199 | 10746 | 16932 | 5601 | 16270 | 5072 | 16012 | 1609 | 13543 | 18010 |
| | 6520 | 5876 | 18551 | 11710 | 16973 | 8611 | 17786 | 896 | 15150 | 6585 | 13129 | 4965 |
| | 18175 | 2832 | 10945 | 3423 | 17125 | 18855 | 16525 | 1748 | 5964 | 7611 | 6607 | 14612 |
| | 11300 | 17123 | 14105 | 1285 | 15156 | 11382 | 12956 | 17420 | 6377 | 1873 | 5035 | 9405 |
| | 13286 | 8729 | 9963 | 8621 | 9241 | 13064 | 2473 | 14203 | 19067 | 17080 | 10484 | 16852 |
| | 9591 | 6583 | 5237 | 3885 | 18484 | 18027 | 18467 | 18028 | 17973 | 17972 | 17970 | 17969 |
| | 5302 | 16042 | 6762 | 8202 | 17109 | 9781 | 3057 | 12195 | 18035 | 14637 | 2924 | 5223 |
| | 5651 | 595 | 5083 | 15409 | 17949 | 19109 | 11395 | 8126 | 3348 | 12648 | 1969 | 13131 |
| | 5389 | 6525 | 18579 | 18245 | 18559 | 15802 | 6580 | 13199 | 10789 | 12935 | 13695 | 14236 |
| | 5044 | 6962 | 15953 | 11413 | 2116 | 18736 | 11857 | 1258 | 1235 | 3803 | 1582 | 755 |
| | 13906 | 7398 | 13922 | 1826 | 18325 | 18513 | 18472 | 328 | 327 | 18793 | 6125 | 18821 |
| | 1158 | 6132 | 1546 | 12173 | 4539 | 12936 | 18946 | 18922 | 7353 | 7349 | 7351 | 7348 |
| | 7218 | 7214 | 7201 | 7224 | 7220 | 7200 | 2490 | 320 | 322 | 333 | 324 | 323 |
| | 321 | 319 | 316 | 15058 | 19237 | 15045 | 15907 | 16396 | 16398 | 15912 | 15057 | 15636 |
| | 16403 | 15079 | 15075 | 8656 | 18489 | 331 | 363 | 330 | 4045 | | | |
| 359: | 18246 | 18488 | 18007 | 3371 | 18022 | 16861 | 3411 | 13792 | 5847 | 5845 | 1141 | 15264 |
| | 8036 | 7857 | 5865 | 9815 | 5372 | 16808 | 5252 | 7273 | 7855 | 13022 | 13463 | 13462 |

(continued)

| SEQ ID NO: | homolog SEQ ID NOs | | | | | | | | | | |
|---|---|---|---|---|---|---|---|---|---|---|---|
| 326 | 332 | 3697 | 4490 | 4040 | 4024 | 10188 | 3795 | 3794 | 974 | 7376 | 8980 |
| 2807 | 2826 | 6239 | 15414 | 1879 | 3221 | 11000 | 16877 | 11643 | 14101 | 17431 | 14104 |
| 11922 | 14106 | 14118 | 14103 | 11917 | 14117 | 11877 | 9156 | 15521 | 15519 | 15512 | 2076 |
| 7599 | 18286 | 17450 | 10636 | 10883 | 19245 | 2706 | 17448 | 17445 | 13484 | 12363 | 8462 |
| 8465 | 1521 | 11661 | 18323 | 10495 | 18481 | 1858 | 17901 | 1025 | 15261 | 14337 | 11009 |
| 802 | 1489 | 9751 | 5535 | 19225 | 4741 | 8379 | 9986 | 5615 | 9570 | 18249 | 1579 |
| 18874 | 14957 | 2833 | 13097 | 11855 | 17444 | 2048 | 5863 | 16811 | 12374 | 13302 | 5440 |
| 14857 | 13516 | 10812 | 9419 | 6771 | 11768 | 12141 | 3543 | 6242 | 11404 | 18052 | 1963 |
| 2636 | 7150 | 2875 | 5500 | 15645 | 3024 | 19039 | 805 | 11538 | 2943 | 12915 | 17169 |
| 4471 | 17129 | 2420 | 9530 | 8029 | 15921 | 16041 | 11094 | 10747 | 13795 | 2144 | 17511 |
| 12731 | 6924 | 15147 | 18864 | 7598 | 2259 | 7950 | 18104 | 7123 | 13183 | 9498 | 651 |
| 5099 | 8353 | 13883 | 4389 | 2662 | 11940 | 19159 | 16364 | 4653 | 14754 | 12986 | 3439 |
| 9888 | 4998 | 8161 | 19192 | 17068 | 10691 | 934 | 606 | 769 | 8468 | 18297 | 1397 |
| 3167 | 10721 | 18909 | 18127 | 820 | 8767 | 11231 | 10209 | 18039 | 4769 | 4271 | 914 |
| 5454 | 17761 | 1436 | 16749 | 12983 | 7990 | 3461 | 11482 | 2488 | 2605 | 15929 | 19116 |
| 1132 | 11154 | 5499 | 16543 | 12081 | 4575 | 10781 | 15011 | 17427 | 14182 | 11494 | 4324 |
| 686 | 8119 | 16545 | 1033 | 15056 | 15910 | 7850 | 13576 | 15542 | 16151 | 16155 | 16095 |
| 13014 | 16069 | 3257 | 6401 | 17505 | 17504 | 17525 | 17524 | 2022 | 7377 | 432 | 16819 |
| 10332 | 2411 | 10331 | 2410 | 12098 | 12865 | 3223 | 4128 | 11048 | 1117 | 13770 | 13767 |
| 16781 | 16777 | 16779 | 16801 | 17741 | 16774 | 4860 | 15510 | 12777 | 12778 | 14071 | 14067 |
| 14053 | 14069 | 14091 | 14072 | 14075 | 8376 | 11001 | 9987 | 7853 | 5184 | 7393 | 7395 |
| 3219 | 3220 | 17517 | 3203 | 17996 | 3205 | 17513 | 10899 | 3204 | 18474 | 18023 | 600 |
| 7911 | 16191 | 13238 | 15975 | 4924 | 9729 | 6091 | 4326 | 6820 | 13828 | 4812 | 9933 |
| 17784 | 16424 | 4768 | 15749 | 7309 | 10904 | 2071 | 4194 | 15043 | 2025 | 10355 | 5253 |
| 18478 | 13347 | 18509 | 13344 | 18011 | 8209 | 8302 | 10221 | 16888 | 15948 | 1610 | 8147 |
| 11081 | 18398 | 17612 | 16052 | 2853 | 15154 | 15669 | 4108 | 413 | 8441 | 3479 | 1048 |
| 17549 | 9749 | 17291 | 14470 | 2008 | 13574 | 5988 | 3547 | 13320 | 12107 | 4540 | 6031 |
| 16301 | 6076 | 18372 | 10579 | 4109 | 11366 | 19231 | 11108 | 8482 | 5412 | 12150 | 14436 |
| 18176 | 674 | 6891 | 13730 | 8642 | 6095 | 3638 | 993 | 17479 | 11156 | 6014 | 4301 |
| 1954 | 10818 | 7039 | 15055 | 12418 | 10003 | 1924 | 13798 | 11179 | 15926 | 13552 | 9569 |
| 18113 | 9432 | 15846 | 14535 | 3145 | 12007 | 16132 | 12934 | 1538 | 16847 | 15583 | 6741 |
| 5534 | 14502 | 4641 | 7188 | 15672 | 4220 | 11219 | 12375 | 7172 | 3983 | 2230 | 6072 |
| 5854 | 17508 | 17766 | 18490 | 15885 | 4617 | 4368 | 16850 | 13940 | 3991 | 1557 | 11615 |
| 2624 | 18508 | 18457 | 10864 | 18750 | 6697 | 1128 | 12487 | 18977 | 5445 | 11199 | 10746 |
| 16932 | 5601 | 16270 | 559 | 5072 | 16012 | 1609 | 13543 | 18010 | 5876 | 18551 | 11710 |
| 16973 | 8611 | 17786 | 896 | 15150 | 6585 | 6520 | 13129 | 4965 | 18175 | 2832 | 10945 |
| 3423 | 17125 | 18855 | 16525 | 1748 | 5964 | 7611 | 14612 | 11300 | 17123 | 14105 | 1285 |
| 15156 | 11382 | 12956 | 17420 | 6607 | 6377 | 1873 | 5035 | 9405 | 13286 | 8729 | 9963 |
| 8621 | 9241 | 13064 | 2473 | 14203 | 19067 | 17080 | 10484 | 16852 | 9591 | 6583 | 5237 |
| 3885 | 11942 | 14024 | 5941 | 18484 | 18027 | 18467 | 18028 | 17973 | 17972 | 17970 | 17969 |
| 5302 | 16042 | 6762 | 8202 | 17109 | 9781 | 3057 | 12195 | 18035 | 14637 | 2924 | 5223 |
| 595 | 5651 | 5083 | 15409 | 17949 | 19109 | 11395 | 13131 | 5389 | 6525 | 18579 | 18245 |
| 18559 | 15802 | 6580 | 13199 | 10789 | 12935 | 13695 | 14236 | 5044 | 6962 | 15953 | 11413 |
| 2116 | 18736 | 3803 | 13906 | 7398 | 13922 | 18325 | 18513 | 18472 | 17557 | 328 | 15806 |
| 15935 | 7353 | 7349 | 7351 | 7348 | 7218 | 7214 | 7201 | 7224 | 7220 | 7200 | 2490 |
| 4329 | 7547 | 333 | 320 | 323 | 15058 | 19237 | 15045 | 15907 | 16396 | 16398 | 15912 |
| 15057 | 15636 | 16403 | 15079 | 15075 | 10637 | 12507 | 18489 | 363 | | | |
| 360:18341 | 18488 | 18007 | 16264 | 3371 | 18022 | 7638 | 13822 | 1141 | 15264 | 9022 | 8036 |
| 7857 | 2766 | 5222 | 7855 | 10380 | 9924 | 326 | 695 | 18994 | 17274 | 1593 | 18800 |
| 10188 | 3795 | 3794 | 974 | 7376 | 8980 | 2807 | 2826 | 15414 | 3221 | 11000 | 16877 |

(continued)

| SEQ ID NO: | homolog SEQ ID NOs | | | | | | | | | | |
|---|---|---|---|---|---|---|---|---|---|---|---|
| 358 | 11643 | 14101 | 17431 | 14104 | 11922 | 14106 | 14118 | 14103 | 11917 | 14117 | 11877 |
| 9156 | 2076 | 7599 | 18286 | 17450 | 10636 | 10883 | 19245 | 2706 | 17448 | 17445 | 13484 |
| 12363 | 8462 | 8465 | 1521 | 11661 | 18323 | 10495 | 18481 | 1858 | 17901 | 1025 | 15261 |
| 14337 | 11009 | 802 | 1489 | 9751 | 5535 | 19225 | 4741 | 8379 | 9986 | 5615 | 9570 |
| 18249 | 1579 | 18874 | 14957 | 2833 | 13097 | 11855 | 17444 | 2048 | 5863 | 16811 | 12374 |
| 13302 | 5440 | 14857 | 13516 | 10812 | 9419 | 6771 | 11768 | 12141 | 3543 | 6242 | 11404 |
| 18052 | 1963 | 2636 | 7150 | 2875 | 5500 | 15645 | 3024 | 19039 | 805 | 11538 | 2943 |
| 12915 | 17169 | 4471 | 17129 | 2420 | 9530 | 8029 | 15921 | 16041 | 11094 | 10747 | 13795 |
| 2144 | 17511 | 12731 | 6924 | 15147 | 18864 | 7598 | 2259 | 7950 | 18104 | 7123 | 13183 |
| 9498 | 651 | 5099 | 8353 | 13883 | 4389 | 2662 | 11940 | 19159 | 16364 | 4653 | 14754 |
| 12986 | 3439 | 9888 | 4998 | 8161 | 19192 | 17068 | 10691 | 934 | 606 | 769 | 8468 |
| 18297 | 1397 | 3167 | 10721 | 18909 | 18127 | 820 | 8767 | 11231 | 10209 | 18039 | 4769 |
| 4271 | 914 | 5454 | 17761 | 1436 | 16749 | 12983 | 7990 | 3461 | 11482 | 2488 | 19116 |
| 2605 | 15929 | 1132 | 11154 | 5499 | 16543 | 12081 | 4575 | 10781 | 15011 | 17427 | 14182 |
| 11494 | 4324 | 686 | 1033 | 15056 | 15910 | 7850 | 13576 | 15542 | 16151 | 16155 | 16095 |
| 13014 | 16069 | 3257 | 6401 | 17505 | 17504 | 17525 | 17524 | 2022 | 7377 | 432 | 16819 |
| 10332 | 2411 | 10331 | 2410 | 12098 | 12865 | 3223 | 4128 | 11048 | 1117 | 13770 | 13767 |
| 16781 | 16777 | 16779 | 16801 | 17741 | 16774 | 4860 | 15510 | 12777 | 12778 | 14071 | 14067 |
| 14053 | 14069 | 14091 | 14072 | 14075 | 8376 | 11001 | 9987 | 7853 | 5184 | 7393 | 7395 |
| 3219 | 3220 | 17517 | 3203 | 17996 | 3205 | 17513 | 10899 | 3204 | 18474 | 18023 | 600 |
| 7911 | 16191 | 13238 | 15975 | 4924 | 9729 | 6091 | 4812 | 4326 | 6820 | 13828 | 9933 |
| 4768 | 17784 | 16424 | 15749 | 7309 | 10904 | 2071 | 4194 | 15043 | 2025 | 10355 | 5253 |
| 13347 | 18509 | 13344 | 18011 | 8209 | 8302 | 10221 | 1048 | 15948 | 8147 | 11081 | 18398 |
| 17612 | 11108 | 1538 | 16052 | 2853 | 15154 | 15669 | 4108 | 413 | 16888 | 8441 | 3479 |
| 17549 | 9749 | 17291 | 14470 | 2008 | 13574 | 5988 | 3547 | 13320 | 12107 | 4540 | 6031 |
| 16301 | 6076 | 18372 | 10579 | 4109 | 11366 | 19231 | 8482 | 5412 | 12150 | 14436 | 18176 |
| 1610 | 674 | 6891 | 13730 | 8642 | 6095 | 3638 | 993 | 17479 | 11156 | 6014 | 4301 |
| 1954 | 10818 | 7039 | 15055 | 12418 | 10003 | 1924 | 13798 | 11179 | 15926 | 13552 | 9569 |
| 18113 | 9432 | 15846 | 14535 | 3145 | 12007 | 16132 | 12934 | 16847 | 15583 | 6741 | 5534 |
| 14502 | 4641 | 7188 | 15672 | 4220 | 11219 | 12375 | 7172 | 3983 | 2230 | 6072 | 559 |
| 5854 | 17508 | 17766 | 18490 | 15885 | 4617 | 4368 | 16850 | 13940 | 3991 | 1557 | 11615 |
| 2624 | 18508 | 18457 | 10864 | 18750 | 6697 | 1128 | 12487 | 18977 | 5445 | 11199 | 10746 |
| 16932 | 5601 | 16270 | 3423 | 5072 | 16012 | 1609 | 13543 | 18010 | 5876 | 18551 | 11710 |
| 16973 | 8611 | 17786 | 896 | 15150 | 6585 | 6520 | 13129 | 4965 | 18175 | 2832 | 16525 |
| 10945 | 17125 | 18855 | 1748 | 5964 | 7611 | 14612 | 11300 | 17123 | 14105 | 1285 | 15156 |
| 11382 | 12956 | 10484 | 17420 | 6607 | 6377 | 1873 | 5035 | 9405 | 13286 | 8729 | 9963 |
| 8621 | 9241 | 2473 | 13064 | 14203 | 19067 | 17080 | 16852 | 9591 | 6583 | 5237 | 3885 |
| 18484 | 18027 | 18467 | 18028 | 17973 | 17972 | 17970 | 17969 | 5302 | 16042 | 6762 | 8202 |
| 17109 | 9781 | 3057 | 12195 | 18035 | 14637 | 2924 | 5223 | 595 | 5651 | 5083 | 15409 |
| 17949 | 19109 | 11395 | 13131 | 5389 | 6525 | 18579 | 18245 | 18559 | 15802 | 6580 | 13199 |
| 10789 | 12935 | 13695 | 14236 | 5044 | 6962 | 15953 | 11413 | 2116 | 18736 | 3803 | 13906 |
| 7398 | 13922 | 18325 | 18513 | 18472 | 16404 | 327 | 5056 | 7353 | 7349 | 7351 | 7348 |
| 7218 | 7214 | 7201 | 7224 | 7200 | 7220 | 2490 | 2336 | 322 | 319 | 316 | 324 |
| 15058 | 19237 | 15907 | 15045 | 16396 | 16398 | 15912 | 15057 | 15636 | 16403 | 15079 | 15075 |
| 8658 | 18489 | 331 | | | | | | | | | |
| 361: 1702 | 3903 | 18678 | 18677 | 16723 | 3824 | 7338 | 18840 | 5965 | 12718 | 13789 | |
| 362: 16281 | 18488 | 18007 | 18022 | 3371 | 7061 | 7892 | 1141 | 15264 | 8036 | 7857 | 19191 |
| 7170 | 3442 | 7855 | 11765 | 326 | 2525 | 12433 | 5350 | 10188 | 3795 | 3794 | 974 |
| 7376 | 8980 | 2807 | 2826 | 15414 | 3221 | 11000 | 16877 | 358 | 11643 | 14101 | 17431 |
| 14104 | 11922 | 14106 | 14118 | 14103 | 11917 | 14117 | 11877 | 9156 | 15521 | 15519 | 15512 |

(continued)

| SEQ ID NO: | homolog SEQ ID NOs | | | | | | | | | | | |
|---|---|---|---|---|---|---|---|---|---|---|---|---|
| | 2076 | 7599 | 18286 | 17450 | 10636 | 10883 | 19245 | 2706 | 17448 | 17445 | 13484 | 12363 |
| | 8462 | 8465 | 1521 | 11661 | 18323 | 10495 | 18481 | 1858 | 17901 | 1025 | 15261 | 14337 |
| | 11009 | 802 | 1489 | 9751 | 5535 | 19225 | 4741 | 8379 | 9986 | 5615 | 9570 | 18249 |
| | 1579 | 18874 | 14957 | 2833 | 13097 | 11855 | 17444 | 2048 | 5863 | 16811 | 12374 | 13302 |
| | 5440 | 14857 | 13516 | 10812 | 9419 | 6771 | 11768 | 12141 | 3543 | 6242 | 11404 | 18052 |
| | 1963 | 2636 | 5500 | 7150 | 2875 | 15645 | 3024 | 19039 | 805 | 11538 | 2943 | 12915 |
| | 17169 | 4471 | 17129 | 2420 | 9530 | 8029 | 15921 | 16041 | 11094 | 10747 | 13795 | 2144 |
| | 17511 | 12731 | 6924 | 15147 | 18864 | 7598 | 2259 | 7950 | 18104 | 7123 | 13183 | 9498 |
| | 651 | 4653 | 5099 | 11940 | 8353 | 13883 | 4389 | 2662 | 8468 | 19159 | 16364 | 14754 |
| | 12986 | 3439 | 9888 | 4998 | 8161 | 19192 | 17068 | 10691 | 934 | 606 | 769 | 18297 |
| | 1397 | 3167 | 10721 | 18909 | 18127 | 820 | 8767 | 11231 | 10209 | 18039 | 4769 | 4271 |
| | 914 | 5454 | 17761 | 1436 | 16749 | 12983 | 7990 | 3461 | 11482 | 2488 | 2605 | 15929 |
| | 19116 | 1132 | 11154 | 5499 | 16543 | 12081 | 4575 | 10781 | 15011 | 17427 | 14182 | 11494 |
| | 4324 | 686 | 8119 | 16545 | 1033 | 15056 | 15910 | 7850 | 13576 | 15542 | 16151 | 16155 |
| | 16095 | 13014 | 16069 | 3257 | 6401 | 17505 | 17504 | 17525 | 17524 | 2022 | 7377 | 432 |
| | 16819 | 10332 | 2411 | 10331 | 2410 | 12098 | 12865 | 3223 | 4128 | 11048 | 1117 | 13770 |
| | 13767 | 16781 | 16777 | 16779 | 16801 | 17741 | 16774 | 4860 | 15510 | 12777 | 12778 | 14071 |
| | 14067 | 14053 | 14069 | 14091 | 14072 | 14075 | 8376 | 11001 | 9987 | 7853 | 5184 | 7393 |
| | 7395 | 3219 | 3220 | 17517 | 3203 | 17996 | 3205 | 17513 | 10899 | 3204 | 18474 | 18023 |
| | 600 | 7911 | 16191 | 15975 | 4924 | 13238 | 9729 | 6091 | 4326 | 6820 | 13828 | 4812 |
| | 9933 | 17784 | 16424 | 4768 | 15749 | 7309 | 10904 | 2071 | 4194 | 15043 | 2025 | 10355 |
| | 5253 | 18478 | 13347 | 18509 | 13344 | 18011 | 8209 | 8302 | 10221 | 17291 | 15948 | 17479 |
| | 2853 | 15154 | 15669 | 4108 | 17549 | 9749 | 14470 | 4540 | 18372 | 11366 | 19231 | 5412 |
| | 12150 | 14436 | 18176 | 13730 | 8642 | 3638 | 993 | 11156 | 6014 | 4301 | 1954 | 10818 |
| | 7039 | 10003 | 1924 | 16132 | 13798 | 15583 | 9569 | 13320 | 9432 | 15846 | 16301 | 14535 |
| | 8482 | 674 | 7188 | 12934 | 6741 | 1610 | 5534 | 7172 | 3983 | 2230 | 6072 | 15672 |
| | 5854 | 17508 | 18490 | 15055 | 4617 | 16847 | 413 | 3479 | 1048 | 13574 | 5988 | 6031 |
| | 10579 | 4109 | 6095 | 1557 | 11615 | 5445 | 18508 | 16052 | 16888 | 13552 | 11081 | 12107 |
| | 13940 | 12007 | 8147 | 12487 | 18977 | 17766 | 8441 | 2008 | 3547 | 6891 | 18398 | 5601 |
| | 17125 | 17612 | 11179 | 15926 | 18113 | 3145 | 16012 | 1609 | 18010 | 14502 | 4220 | 6076 |
| | 11108 | 12418 | 16973 | 8611 | 17786 | 18551 | 11710 | 1128 | 4641 | 1538 | 16850 | 15885 |
| | 4965 | 15150 | 6585 | 18750 | 6697 | 10746 | 11219 | 12375 | 13129 | 559 | 6520 | 4368 |
| | 2832 | 3991 | 13543 | 3423 | 2624 | 18457 | 10864 | 11199 | 16932 | 16270 | 5072 | 17123 |
| | 5876 | 14612 | 896 | 15156 | 16852 | 18175 | 10945 | 18855 | 16525 | 1748 | 5964 | 11300 |
| | 8621 | 7611 | 9963 | 9241 | 6607 | 5035 | 14105 | 1285 | 11382 | 13286 | 12956 | 17420 |
| | 6377 | 1873 | 17080 | 19067 | 9405 | 8729 | 13064 | 2473 | 14203 | 6583 | 10484 | 11942 |
| | 9591 | 5237 | 2321 | 3769 | 3885 | 7954 | 17997 | 14024 | 5941 | 9954 | 18484 | 18027 |
| | 18467 | 18028 | 17973 | 17972 | 17970 | 17969 | 15630 | 5302 | 6762 | 16042 | 17109 | 8202 |
| | 9781 | 3057 | 12195 | 14637 | 2924 | 18035 | 5223 | 15409 | 595 | 5651 | 5083 | 17949 |
| | 19109 | 11395 | 6580 | 10789 | 13695 | 15802 | 5389 | 18245 | 18559 | 7398 | 13922 | 18325 |
| | 18513 | 18472 | 328 | 327 | 7707 | 7353 | 7349 | 7351 | 7348 | 7218 | 7214 | 7201 |
| | 7224 | 7220 | 7200 | 2490 | 3607 | 15058 | 19237 | 15045 | 15907 | 16396 | 16398 | 15912 |
| | 15057 | 15636 | 16403 | 15079 | 15075 | 9155 | 18489 | 330 | | | | |
| 363: | 18274 | 18488 | 18007 | 11117 | 10258 | 9446 | 3371 | 18022 | 329 | 360 | 359 | 7341 |
| | 17634 | 11532 | 3678 | 17861 | 1141 | 11850 | 6626 | 1745 | 15264 | 16539 | 2664 | 8699 |
| | 5767 | 11011 | 8036 | 7857 | 1343 | 6839 | 8733 | 11515 | 14442 | 16737 | 6301 | 658 |
| | 5340 | 6383 | 3012 | 4309 | 4229 | 16075 | 2351 | 8932 | 12221 | 5354 | 6706 | 3213 |
| | 3896 | 17512 | 15668 | 15120 | 4825 | 18765 | 7855 | 4601 | 10991 | 2531 | 10828 | 17127 |
| | 1411 | 5701 | 15745 | 17917 | 4012 | 11414 | 8414 | 1948 | 325 | 8882 | 3926 | 7772 |
| | 18792 | 16498 | 13428 | 7045 | 7919 | 16773 | 3833 | 8372 | 5231 | 6402 | 8899 | 4814 |

(continued)

| SEQ ID NO: | homolog SEQ ID NOs | | | | | | | | | | |
|---|---|---|---|---|---|---|---|---|---|---|---|
| 12738 | 16127 | 326 | 12276 | 13535 | 12190 | 17785 | 10684 | 3455 | 14687 | 9274 | 12077 |
| 15788 | 10337 | 9859 | 10204 | 9466 | 15723 | 7916 | 18347 | 6113 | 15339 | 18049 | 14162 |
| 14158 | 7554 | 2228 | 5734 | 2183 | 1503 | 9273 | 13486 | 4699 | 18053 | 874 | 7758 |
| 7168 | 2911 | 16068 | 10075 | 5165 | 6610 | 8188 | 7505 | 5836 | 10110 | 3449 | 15698 |
| 17609 | 5486 | 10188 | 3795 | 3794 | 974 | 7376 | 8980 | 2807 | 2826 | 15414 | 3221 |
| 11000 | 16877 | 358 | 11643 | 16928 | 14101 | 17431 | 14104 | 11922 | 14106 | 14118 | 14103 |
| 11917 | 14117 | 11877 | 9156 | 15521 | 15519 | 15512 | 2076 | 7599 | 18286 | 16747 | 17450 |
| 10636 | 10883 | 19245 | 18626 | 2706 | 5629 | 17448 | 17445 | 13484 | 12611 | 12363 | 8462 |
| 8465 | 1521 | 11661 | 18323 | 10495 | 18481 | 1858 | 7476 | 18811 | 19045 | 17901 | 1025 |
| 15261 | 14337 | 11009 | 802 | 1489 | 9751 | 5535 | 19225 | 4741 | 8379 | 9986 | 5615 |
| 9570 | 18249 | 1579 | 18874 | 14957 | 2833 | 13097 | 11855 | 17444 | 2048 | 5863 | 16811 |
| 12374 | 13302 | 5440 | 14857 | 13516 | 10812 | 9419 | 6771 | 11768 | 12141 | 3543 | 6242 |
| 11404 | 18052 | 1963 | 2636 | 7150 | 2875 | 5500 | 15645 | 3024 | 19039 | 805 | 11538 |
| 2943 | 12915 | 17169 | 4471 | 17129 | 2420 | 9530 | 8029 | 15921 | 16041 | 11094 | 10747 |
| 13795 | 2144 | 17511 | 12731 | 1983 | 6924 | 15147 | 18864 | 7598 | 2259 | 7950 | 18104 |
| 7123 | 13183 | 9498 | 651 | 5099 | 8353 | 13883 | 4389 | 2662 | 11940 | 19159 | 16364 |
| 4653 | 14754 | 12986 | 3439 | 9888 | 4998 | 8161 | 19192 | 17068 | 10691 | 934 | 606 |
| 769 | 8468 | 18297 | 1397 | 3167 | 10721 | 18909 | 18127 | 10323 | 820 | 8767 | 11231 |
| 10209 | 18039 | 4769 | 4271 | 914 | 5454 | 17761 | 1436 | 16749 | 12983 | 10781 | 7990 |
| 3461 | 11482 | 2488 | 2605 | 15929 | 19116 | 1132 | 11154 | 5499 | 16543 | 12081 | 4575 |
| 15011 | 17427 | 14182 | 11494 | 4324 | 686 | 8119 | 16545 | 1033 | 15056 | 15910 | 7850 |
| 13576 | 15542 | 16151 | 16155 | 16095 | 13014 | 16069 | 3257 | 6401 | 17505 | 17504 | 17525 |
| 17524 | 2022 | 7377 | 432 | 16819 | 10332 | 2411 | 10331 | 2410 | 12098 | 12865 | 3223 |
| 4128 | 11048 | 1117 | 13770 | 13767 | 16781 | 16777 | 16779 | 16801 | 17741 | 16774 | 4860 |
| 15510 | 12777 | 12778 | 14071 | 14067 | 14053 | 14069 | 14091 | 14072 | 14075 | 8376 | 11001 |
| 9987 | 7853 | 5184 | 7393 | 7395 | 17909 | 3219 | 3220 | 17517 | 3203 | 17996 | 3205 |
| 17513 | 10899 | 3204 | 18474 | 18023 | 18329 | 600 | 6542 | 7911 | 16191 | 13238 | 15975 |
| 4924 | 9729 | 6091 | 4326 | 6820 | 13828 | 4812 | 9933 | 17784 | 16424 | 4768 | 15749 |
| 7309 | 10904 | 2071 | 4194 | 15043 | 2025 | 10355 | 5253 | 18478 | 13347 | 18509 | 13344 |
| 18011 | 4578 | 8209 | 8302 | 10221 | 15948 | 8147 | 11081 | 18398 | 17612 | 16052 | 2853 |
| 15154 | 15669 | 4108 | 413 | 16888 | 8441 | 3479 | 1048 | 17549 | 9749 | 17291 | 14470 |
| 2008 | 13574 | 5988 | 3547 | 13320 | 12107 | 4540 | 6031 | 16301 | 6076 | 18372 | 10579 |
| 4109 | 11366 | 19231 | 11108 | 8482 | 5412 | 12150 | 14436 | 18176 | 1610 | 674 | 6891 |
| 13730 | 8642 | 6095 | 3638 | 993 | 17479 | 11156 | 6014 | 4301 | 1954 | 10818 | 7039 |
| 15055 | 12418 | 10003 | 1924 | 13798 | 11179 | 15926 | 13552 | 9569 | 18113 | 9432 | 15846 |
| 14535 | 3145 | 12007 | 16132 | 12934 | 1538 | 16847 | 15583 | 6741 | 5534 | 14502 | 4641 |
| 7188 | 15672 | 4220 | 11219 | 12375 | 7172 | 3983 | 2230 | 6072 | 5854 | 17508 | 17766 |
| 18490 | 15885 | 4617 | 4368 | 16850 | 13940 | 3991 | 1557 | 11615 | 2624 | 18508 | 18457 |
| 10864 | 18750 | 6697 | 1128 | 12487 | 18977 | 5445 | 11199 | 10746 | 16932 | 5601 | 16270 |
| 559 | 5072 | 16012 | 1609 | 13543 | 18010 | 5876 | 18551 | 11710 | 16973 | 8611 | 17786 |
| 896 | 15150 | 6585 | 6520 | 13129 | 4965 | 18175 | 2832 | 10945 | 3423 | 17125 | 18855 |
| 16525 | 1748 | 5964 | 7611 | 14612 | 11300 | 17123 | 14105 | 1285 | 15156 | 11382 | 12956 |
| 17420 | 6607 | 6377 | 1873 | 5035 | 9405 | 13286 | 8729 | 9963 | 8621 | 9241 | 13064 |
| 2473 | 14203 | 19067 | 17080 | 10484 | 16852 | 9591 | 6583 | 5237 | 3885 | 11942 | 14024 |
| 5941 | 2321 | 18484 | 18027 | 18467 | 18028 | 17973 | 17972 | 17970 | 17969 | 11616 | 5302 |
| 16042 | 6762 | 8202 | 17109 | 9781 | 3057 | 12195 | 18035 | 14637 | 2924 | 5223 | 595 |
| 5651 | 5083 | 15409 | 17949 | 19109 | 11395 | 13131 | 5389 | 6525 | 18579 | 18245 | 18559 |
| 15802 | 6580 | 13199 | 10789 | 12935 | 13695 | 14236 | 5044 | 6962 | 15953 | 11413 | 2116 |
| 18736 | 3803 | 13250 | 13906 | 7398 | 13922 | 11708 | 9202 | 18325 | 18513 | 6519 | 18472 |
| 327 | 328 | 7929 | 7353 | 7349 | 7351 | 7348 | 7218 | 7214 | 7201 | 7224 | 7220 |

(continued)

| SEQ ID NO: | homolog SEQ ID NOs | | | | | | | | | | |
|---|---|---|---|---|---|---|---|---|---|---|---|
| 7200 | 17337 | 7741 | 2490 | 6801 | 3458 | 13727 | 18735 | 316 | 321 | 333 | 322 |
| 324 | 320 | 319 | 323 | 6750 | 15058 | 19237 | 15045 | 15907 | 16396 | 16398 | 15912 |
| 15057 | 15636 | 16403 | 15079 | 15075 | 18489 | 13580 | 7766 | | | | |
| 364: 8684 | 16027 | 13149 | 10126 | 8454 | 15932 | 11869 | 13702 | 13698 | 7664 | 8628 | 3039 |
| 3267 | | | | | | | | | | | |
| 365:17649 | 8270 | 3136 | 10804 | 6247 | 8834 | 17636 | 15016 | 8936 | 2194 | 13157 | 4323 |
| 8741 | 11308 | 472 | 19056 | | | | | | | | |
| 366: 5012 | 2906 | 8001 | 12356 | 6089 | 18788 | 4785 | 6186 | 6359 | 6711 | 3320 | 11092 |
| 367: 7896 | 15587 | 5766 | 14289 | 18000 | 13369 | 8046 | 16461 | 9247 | 13124 | 10208 | 15984 |
| 7503 | 11474 | 5470 | 765 | 11822 | | | | | | | |
| 368:18223 | 5598 | 10881 | 8595 | 15561 | 10514 | 9251 | 17180 | 10854 | 6055 | 12225 | 2422 |
| 8657 | 17250 | 3377 | 13990 | 4420 | 9030 | 19028 | 2248 | 7708 | 10035 | 12889 | 11370 |
| 19121 | 11952 | 3762 | 3651 | 7635 | 9473 | 17896 | 19118 | 6913 | 15466 | 3944 | 6131 |
| 3755 | 1161 | 13259 | 1151 | 5087 | 11439 | 18915 | 3175 | 5089 | 11438 | 16657 | 9710 |
| 10306 | 19085 | 1527 | 15920 | 12676 | 13514 | 14962 | 10018 | 13289 | 7013 | 8470 | 7160 |
| 10908 | 685 | 650 | 11493 | 4723 | 4184 | 18299 | 829 | 15453 | 12859 | 17424 | 9533 |
| 2244 | 2847 | 14721 | 11910 | 12477 | 7174 | 15879 | 5488 | 12702 | 9161 | 14497 | 12241 |
| 9336 | 7245 | 9646 | 15955 | 10132 | 17257 | 3161 | 18165 | 13393 | 18479 | 13438 | 1524 |
| 14655 | 11554 | 4462 | 7586 | 7930 | 14718 | 5571 | 17190 | 9164 | 19070 | 13526 | 2863 |
| 10779 | 7543 | 3731 | 10062 | 4442 | 2923 | 18088 | 2881 | 8943 | 3734 | 3058 | 4633 |
| 3900 | 3667 | 5575 | 4124 | 5363 | 995 | 2692 | 5332 | 5916 | 7378 | 1953 | 15812 |
| 2389 | 2390 | 2245 | 7361 | 10033 | 10128 | 18718 | 13416 | 17384 | 11063 | 18720 | 509 |
| 3241 | 6075 | 2361 | 541 | 15106 | 15987 | 2306 | 2730 | 906 | 2263 | 12377 | 11712 |
| 621 | 6011 | 6433 | 10564 | 10589 | 5120 | 18420 | 12406 | 14906 | 5170 | 16214 | 694 |
| 13162 | 6259 | 18759 | 5659 | 7744 | 4422 | 17687 | 665 | 9006 | 8004 | 15938 | 15486 |
| 12018 | 6903 | 5465 | 5202 | 3639 | 10189 | 4000 | 17403 | 4727 | 18570 | 18571 | 18591 |
| 13103 | 7276 | 8327 | 8749 | 14147 | 13677 | 8146 | 10195 | 4684 | 11527 | 15892 | 11819 |
| 15958 | 3517 | 15190 | 16361 | 11510 | | | | | | | |
| 369: 2084 | 15197 | 8662 | 8366 | 2930 | 6655 | 12876 | 8740 | 17571 | 13116 | | |
| 370: 1613 | 19204 | 11892 | 6151 | 11834 | 16271 | 7629 | | | | | |
| 371: 3593 | 6170 | 6205 | 17780 | 18378 | 1421 | 3053 | 15450 | 9886 | 15739 | 18891 | 6294 |
| 10793 | 15529 | 13666 | 11730 | 18928 | 3004 | 6197 | 13102 | 16177 | 13928 | 14868 | 12232 |
| 6033 | 7442 | 14783 | 15125 | 5741 | 864 | 13206 | 5210 | 12920 | 18629 | 12671 | 16466 |
| 2407 | 5884 | 6651 | 14574 | | | | | | | | |
| 372:15623 | 14801 | 953 | 10002 | 6238 | 10071 | 13756 | 11624 | 16541 | 14110 | 3858 | 4119 |
| 4412 | 10719 | | | | | | | | | | |
| 373: 4316 | 13909 | 16449 | 17769 | 15006 | 4781 | 10038 | 14140 | 13489 | 16035 | 4824 | 9107 |
| 6225 | 16014 | 8175 | 17809 | 3179 | 14740 | 16152 | 8688 | 16780 | 13019 | 17782 | 17245 |
| 374: 6174 | 6173 | 17263 | 5382 | 13230 | 416 | 13835 | 10352 | 12036 | 2106 | 2087 | 12205 |
| 12076 | 13351 | 5940 | 7910 | 11809 | 16730 | 15960 | 14680 | 8484 | 3972 | 4409 | 4808 |
| 18796 | 15783 | 12008 | 3864 | 440 | 18038 | 19246 | 18731 | 16299 | 4005 | 5505 | 5331 |
| 12153 | 14304 | 12102 | 17171 | | | | | | | | |
| 375:15552 | 2308 | 10821 | 4674 | 10575 | 4747 | 17033 | 5313 | 505 | 8735 | 1438 | 5247 |
| 4174 | 4173 | 10712 | 12685 | 676 | 14249 | 18327 | 4576 | 3363 | 13911 | 9752 | 7128 |
| 6923 | 5003 | 557 | | | | | | | | | |
| 376:12640 | 10761 | 17514 | 13509 | 16058 | 1234 | 7711 | 5310 | | | | |
| 377:15091 | 4251 | 7569 | 13412 | 9186 | 14005 | 11205 | 6753 | 5318 | 13512 | 5181 | 3332 |
| 4510 | 5730 | 19140 | 18482 | 2628 | 16322 | 6889 | 12710 | 13826 | 16117 | 1492 | 6630 |
| 14504 | 6975 | 3194 | 11115 | 1922 | 18860 | 18335 | 3969 | 18746 | 3485 | 12262 | 4545 |
| 3701 | 15340 | 14247 | 4624 | 9240 | 7559 | 17795 | 2280 | 18217 | 2121 | 14191 | 14648 |

| SEQ ID NO: | homolog SEQ ID NOs | | | | | | | | | | |
|---|---|---|---|---|---|---|---|---|---|---|---|
| 17689 | 18547 | 7154 | 2925 | 17348 | 10160 | 18995 | 18701 | 16015 | 2369 | 13208 | 13699 |
| 5625 | 2618 | 8394 | 17231 | 2934 | 1031 | 4588 | 931 | 5715 | 10557 | 13832 | 6308 |
| 799 | 3921 | 1420 | 3674 | 7819 | 11623 | 713 | 16019 | 7524 | 10823 | 6351 | 10511 |
| 18737 | 6199 | 7305 | 958 | 10010 | 12423 | 8451 | 14867 | 14792 | 1835 | 18830 | 9571 |
| 17441 | 12749 | 16458 | 2477 | 3430 | 14035 | 18847 | 18001 | 3771 | 1319 | 3705 | 18504 |
| 5474 | 4726 | 5270 | 785 | 18382 | 4164 | 8734 | 957 | 4755 | | | |
| 378: 7385 | 7389 | 4712 | 7216 | 7240 | 7425 | 7411 | 16142 | 3882 | 7263 | 7262 | 4838 |
| 7264 | 7267 | 7288 | 7269 | 7239 | 8013 | 6310 | 7299 | 7296 | 15830 | 4115 | 14488 |
| 4236 | 2148 | 12770 | 5634 | 8293 | 18656 | 9110 | 9130 | 5827 | 14078 | 13597 | 7511 |
| 7494 | 7198 | 11579 | 7426 | 7786 | 7194 | 7444 | 3270 | 15500 | 8339 | 1316 | 19032 |
| 6299 | 1170 | 8702 | 13733 | 10030 | 10478 | 10019 | 10089 | 4520 | 3432 | 18377 | 18380 |
| 8333 | 8284 | 8313 | 2075 | 6604 | 16849 | 5563 | 10245 | 3095 | 10445 | 11875 | 6960 |
| 10489 | 4952 | 7044 | 8314 | 17763 | 8310 | 12788 | 8318 | 7964 | 8340 | 8343 | 8346 |
| 1962 | 8847 | 2058 | 16080 | 3127 | 15015 | 17613 | 17633 | 2348 | 8003 | 7373 | 7372 |
| 15767 | 12500 | 16065 | 11929 | 8086 | 6387 | 14998 | 6461 | 11845 | 11762 | 11109 | 7190 |
| 17213 | 5075 | 5076 | 7367 | 7365 | 13195 | 7449 | 7491 | 7448 | 7535 | 11930 | 7290 |
| 7294 | 1927 | 1929 | 1975 | 1976 | 12927 | 8317 | | | | | |
| 379: 789 | 2899 | 9238 | 3272 | 5648 | 3579 | 15295 | 6275 | 14031 | 16259 | 5495 | 10963 |
| 5221 | 2572 | 18153 | 15647 | 4771 | 8787 | 11837 | 4871 | 6699 | 10308 | 17936 | 16583 |
| 14963 | 7534 | 12772 | 9430 | 13248 | 6068 | 13071 | | | | | |
| 380:11132 | 17164 | 10120 | 13780 | 18790 | 3980 | 6453 | 16881 | 5663 | 9535 | 19104 | 1529 |
| 18961 | 2182 | 9230 | 8958 | 10766 | 3066 | 14515 | 16060 | 4872 | 16197 | 5238 | 18266 |
| 381: 8677 | 2479 | 7211 | 7425 | 4341 | 4838 | 7350 | 7264 | 8013 | 7326 | 7209 | 7235 |
| 15830 | 5983 | 5979 | 5985 | 18656 | 7511 | 7198 | 15500 | 8339 | 627 | 6299 | 16863 |
| 8702 | 15217 | 15219 | 15220 | 12265 | 2585 | 12272 | 12270 | 12268 | 8753 | 3061 | 11536 |
| 11534 | 4520 | 3432 | 18952 | 18377 | 18380 | 8333 | 8284 | 8313 | 2075 | 4839 | 6604 |
| 5557 | 3612 | 4952 | 7044 | 3124 | 13964 | 13963 | 7964 | 8343 | 8346 | 16080 | 7195 |
| 8003 | 7373 | 16065 | 8853 | 11929 | 7949 | 7962 | 6461 | 3480 | 11762 | 11845 | 17213 |
| 13195 | 7449 | 12348 | 7448 | 7446 | 11930 | 6644 | 17544 | 1979 | 1976 | 688 | 12002 |
| 692 | 12927 | 12924 | 12921 | 8317 | | | | | | | |
| 382: 6843 | 6987 | 13364 | 15545 | 14423 | 4921 | 4285 | 2528 | 2557 | 18040 | 8350 | 9340 |
| 383:15954 | 6049 | 18779 | 18043 | 2249 | 15965 | | | | | | |
| 384: 7925 | 14352 | 11545 | 10615 | 16765 | 15479 | 8141 | 14076 | 15758 | 5411 | 12128 | 15947 |
| 3153 | 8503 | 16045 | 11187 | 3686 | 9965 | 1284 | 5392 | 16793 | 15607 | 5705 | 3059 |
| 7149 | | | | | | | | | | | |
| 385:12678 | 11761 | 19097 | 15082 | 10667 | 4225 | 11019 | 8039 | 8375 | 17367 | 9578 | 18072 |
| 386:10572 | 14694 | 5590 | 4876 | 3577 | 12056 | 17885 | 18628 | 17398 | 7894 | 7122 | 673 |
| 4475 | | | | | | | | | | | |
| 387:13459 | 5045 | 12999 | 2199 | 13901 | 17853 | 19179 | 15940 | 9881 | 12409 | 5036 | 14275 |
| 18503 | 8364 | 2168 | 8079 | 4928 | 2375 | 2053 | 17350 | 16572 | 10390 | 3007 | 7945 |
| 7762 | 641 | | | | | | | | | | |
| 388:16871 | 9761 | 11846 | 15784 | 18262 | 18446 | 17041 | 2127 | 8909 | 17081 | 8129 | 10993 |
| 5337 | 2955 | 10550 | 1632 | 4698 | 18308 | 13085 | 15485 | 13327 | 16105 | | |
| 389: 2476 | 3590 | 2914 | 12550 | 12552 | 7501 | 14570 | 15819 | 11167 | 4325 | 19077 | 6830 |
| 4556 | 10406 | 12930 | 5172 | 5060 | 12389 | 3569 | 16656 | 5168 | 9775 | 14074 | 17762 |
| 9431 | 2976 | 16817 | 11358 | 11360 | 2041 | 11375 | 10922 | 11355 | 11359 | 14965 | 10920 |
| 10918 | 3529 | 4796 | 11740 | 2289 | 15427 | 7180 | 9275 | 4129 | 7616 | 13151 | 18966 |
| 10689 | 14083 | 6228 | 2080 | 13696 | 5628 | 10009 | 411 | 10499 | 15596 | 8605 | 17387 |
| 390:13101 | 8214 | 18411 | 6355 | 17130 | 17131 | 17112 | 17106 | 11037 | 5175 | 5207 | 16634 |
| 16638 | 18236 | 18250 | 13007 | 12625 | 10843 | 10358 | 4002 | 905 | 7460 | 17283 | 10362 |

(continued)

| SEQ ID NO: | homolog SEQ ID NOs | | | | | | | | | |
|---|---|---|---|---|---|---|---|---|---|---|
| 10388 | 10845 | 13303 | 2314 | 12875 | 17641 | 7106 | 16084 | 4137 | 9491 | 4494 | 14185 |
| 14189 | 14160 | 13722 | 10262 | 9561 | 10261 | 2652 | 16419 | 14234 | 3867 | 3394 | 4122 |
| 16608 | 17323 | 18139 | 16832 | 11596 | 16537 | 14193 | 14190 | 14231 | 14212 | 17832 | 7504 |
| 18708 | 7840 | 16675 | 16237 | 7108 | 13984 | 2646 | 18664 | 14232 | 8358 | 12918 | 4473 |
| 4497 | 431 | 13345 | 11107 | 14988 | 10858 | 8415 | 10522 | 18463 | 2013 | 10896 | 13582 |
| 1453 | 8762 | 16443 | 14346 | 11683 | 2065 | 10233 | 10846 | 10363 | 10391 | 10393 | 14422 |
| 9935 | 17273 | 17249 | 8363 | 10121 | 14942 | | | | | | |
| 391: 7780 | 7778 | 5749 | 16030 | 8501 | 14291 | 14288 | 6980 | 8600 | 7808 | 14360 | 14094 |
| 2039 | 3393 | 16652 | 5033 | 5164 | 14882 | 16247 | 4249 | 12196 | 17478 | 5851 | 448 |
| 19005 | | | | | | | | | | | |
| 392: 9293 | 16557 | 723 | 6692 | 9622 | 2591 | 13628 | 6928 | 1592 | | | |
| 393: 5309 | 16676 | 1992 | 5415 | 17277 | 4097 | 6941 | 16134 | 3966 | 10723 | 12275 | 12413 |
| 9592 | | | | | | | | | | | |
| 394:10929 | 8533 | 15300 | 1943 | 2871 | 3355 | 8440 | 1643 | 4780 | 15402 | 10696 | 4627 |
| 2660 | 3426 | 1295 | 1614 | 466 | 18055 | 18870 | 5343 | 6172 | 13204 | 2509 | 14838 |
| 16696 | 14719 | 12186 | 9237 | 5229 | 14362 | 18820 | 11703 | 10441 | 9402 | 3427 | 6904 |
| 6394 | 15444 | 13539 | 480 | 4107 | 3143 | 11612 | 16378 | 9125 | 5638 | 5458 | 4628 |
| 12830 | 16118 | 11849 | 7565 | 14432 | | | | | | | |
| 395:17920 | 6614 | 14177 | 8433 | 18119 | 14743 | 1547 | 2798 | 4829 | 13098 | 1792 | 7185 |
| 5546 | 12686 | 17738 | 14286 | 17308 | 15203 | 7356 | 15424 | 8898 | 10366 | | |
| 396: 1612 | 16074 | 15966 | 6215 | 9512 | 6739 | 712 | 4754 | 9542 | 926 | 15250 | 17094 |
| 3886 | 1518 | 14141 | 3582 | 17743 | 2596 | 16577 | 7981 | 8476 | 11139 | 9991 | 7519 |
| 16523 | 4476 | 3159 | 2443 | | | | | | | | |
| 397: 6955 | 17338 | 4807 | 13366 | 13886 | 3990 | 12822 | 4206 | 11204 | 4966 | 12012 | 18854 |
| 18590 | 18505 | 5937 | 6734 | 2714 | 7052 | 1150 | 4851 | 7568 | 10870 | 5135 | 3950 |
| 2713 | 870 | 17666 | 8862 | 972 | 4428 | 1180 | 15407 | | | | |
| 398:10762 | 10763 | 17831 | 18710 | 4571 | 2491 | 18826 | 7281 | 7980 | 12839 | 7132 | 3515 |
| 11431 | 13508 | 15105 | 11150 | 12370 | 14765 | 7924 | 8625 | 10099 | 5689 | 10100 | 7454 |
| 10434 | 8719 | 3324 | 3759 | 1396 | 7985 | 1985 | | | | | |
| 399: 6493 | 10378 | 18309 | 8131 | 4746 | 2656 | 3568 | 15048 | 5685 | 9021 | 16845 | 8807 |
| 18908 | 2035 | 18616 | 19229 | 9844 | 10776 | 11514 | 15103 | 1336 | 4804 | 17204 | 14756 |
| 12584 | 18287 | 12312 | 5524 | 13410 | 6108 | 12653 | 16440 | 10889 | 4657 | 4508 | 11491 |
| 4103 | | | | | | | | | | | |
| 400:14710 | 10682 | 11646 | 10632 | 9092 | 7695 | 1867 | 15667 | 2172 | 14920 | 10057 | 4313 |
| 15838 | 6000 | 16168 | 13497 | 12464 | 7428 | 3227 | 15903 | 11817 | 6518 | 7230 | 15193 |
| 14479 | 15307 | 9534 | 8999 | 10201 | | | | | | | |
| 401: 4269 | 415 | 5196 | 1888 | 17473 | 5940 | 17913 | 2427 | 4192 | 894 | 2283 | 3972 |
| 4409 | 4808 | 15898 | 14757 | 16253 | 13957 | | | | | | |
| 402: 8349 | 2740 | 7671 | 12717 | 5139 | 12414 | 4134 | 7424 | 16056 | 14823 | | |
| 403: 1589 | 17604 | 12728 | 8019 | 2032 | 1121 | 5422 | 4393 | 814 | 3576 | 1329 | 18252 |
| 17534 | 7522 | 6755 | 8555 | 13532 | 14465 | 5342 | 14260 | 12366 | 13545 | 11833 | 15130 |
| 16303 | 3048 | 15435 | 12548 | 12465 | 9395 | 4550 | 15303 | 15548 | 16379 | 15934 | 3788 |
| 11207 | 10866 | 16567 | 9796 | 10597 | 2843 | 9814 | 6036 | 13084 | 15810 | 18073 | 12289 |
| 2610 | 8431 | 8133 | 10743 | 5277 | 5274 | 9812 | 9270 | 16062 | 1410 | 18832 | 3278 |
| 11379 | 7651 | 8619 | 4074 | 13579 | 11278 | 7552 | 3482 | 1921 | 3110 | 13040 | 3715 |
| 588 | 16215 | 14079 | 15855 | 15077 | 12165 | 4158 | 14545 | 7138 | 12317 | 14284 | 16674 |
| 7176 | 4557 | 4843 | 4827 | 14556 | 15959 | 12712 | 12693 | 12300 | 9895 | 9617 | 18976 |
| 3521 | 15031 | 1253 | 9850 | 1713 | 948 | 17470 | 9817 | 8246 | 14610 | 14409 | 4404 |
| 18149 | 18916 | 3122 | 6477 | 11641 | 5344 | 5193 | 15313 | 4566 | 4856 | 6382 | 6385 |
| 8287 | 13657 | 6526 | 12895 | 13000 | 17835 | 5551 | 4058 | 4427 | 6492 | 3925 | 15887 |

(continued)

| SEQ ID NO: | homolog SEQ ID NOs | | | | | | | | | | |
|---|---|---|---|---|---|---|---|---|---|---|---|
| 6882 | 13235 | 2054 | 11583 | 8492 | 7588 | 15092 | 977 | 3464 | 6674 | 11033 | 10561 |
| 12912 | 12978 | 14142 | 19136 | 951 | 13846 | 6528 | 11069 | 16860 | 10474 | 14237 | 10178 |
| 9206 | 9999 | 12994 | 1371 | 1369 | 18499 | 15946 | 14691 | 5796 | 15067 | 13591 | 14544 |
| 10116 | 9572 | 9076 | 3395 | 18068 | 9445 | 17137 | 10070 | 12598 | 12451 | 5291 | 8421 |
| 13493 | 12446 | 16898 | 3422 | 12469 | 9327 | 12751 | 1725 | 12393 | 11867 | 16709 | 6201 |
| 2489 | 19025 | 15355 | 12432 | 10704 | 10788 | 15175 | 13881 | 15050 | 11031 | 2284 | 16846 |
| 8929 | 13843 | 15877 | 12575 | 2501 | 3898 | 8155 | 8181 | 13467 | 7071 | 13380 | 18512 |
| 6767 | 11884 | 3330 | 15689 | 16649 | 4862 | 2400 | 11639 | 2206 | 6774 | 7277 | 10750 |
| 13838 | 5371 | 9923 | 6934 | 11974 | 17225 | 3561 | 17146 | 15437 | 5307 | 6795 | 8299 |
| 5491 | 4199 | 12086 | 13575 | 6650 | 12452 | 12843 | 7943 | 4725 | 8051 | 12281 | 12760 |
| 4561 | 2302 | 8823 | 7236 | 18407 | 18993 | 1395 | 18587 | 10428 | 1484 | 9297 | 4090 |
| 6052 | 746 | 5639 | 5240 | 11345 | 8048 | 13454 | 3013 | 3772 | 3177 | 2098 | 1828 |
| 5023 | 13427 | 15578 | 11178 | 5602 | 5276 | 14414 | 2819 | 615 | 9528 | 3937 | 5441 |
| 9527 | 18009 | 11254 | 11735 | 1651 | 10853 | 8300 | 12353 | 10928 | 8198 | 10913 | 11954 |
| 11949 | 16408 | 11950 | 8525 | 8047 | 17449 | 14589 | 10655 | 14519 | 9626 | 17814 | 7897 |
| 3029 | 4632 | 16072 | 1825 | 10344 | 16933 | 7125 | 16977 | 487 | 5370 | 1337 | 10164 |
| 7994 | 14863 | 4232 | 4289 | 11276 | 8180 | 14167 | 10838 | 18522 | 10765 | 13322 | 2506 |
| 18787 | 776 | 16529 | 6541 | 4678 | 5631 | 12943 | 17667 | 6970 | 19144 | 3198 | 7846 |
| 2492 | 15690 | 17586 | 13301 | 4379 | 2818 | 10177 | 3242 | 3316 | 949 | 9487 | 8259 |
| 11112 | 6256 | 10981 | 15481 | 11590 | 14273 | 5962 | 3698 | 13133 | 1978 | 10200 | 2281 |
| 15318 | 15337 | 15317 | 1960 | 10987 | 10959 | 10458 | 6605 | 1442 | 14285 | 16347 | 8163 |
| 9798 | 2999 | 6258 | 594 | 19224 | 11771 | 3948 | 319 | 18283 | 17226 | 10012 | 7302 |
| 16116 | 14915 | 6717 | 19232 | 2868 | 18598 | 8016 | 9887 | 12616 | | | |
| 404: 4607 | 2496 | 4440 | 11086 | 9902 | 13066 | 10299 | 13067 | 10058 | 7029 | 11937 | 11215 |
| 8917 | 18762 | 8922 | 6629 | 3169 | 4418 | 7255 | 17978 | 6392 | 4006 | 16582 | 4177 |
| 13299 | 18105 | 19050 | 1884 | 8982 | 18462 | 13743 | 10049 | 17020 | 1107 | 14164 | 17443 |
| 3211 | 807 | 9415 | 7769 | 2665 | 7716 | 9819 | 4793 | 8194 | 4487 | 9462 | 1406 |
| 8450 | 11140 | 4167 | 18203 | 3917 | 15866 | 2776 | 1567 | 9782 | 15174 | 12626 | 4681 |
| 12824 | 14018 | 4910 | 4659 | 2562 | 2122 | 2788 | 9880 | 2428 | 871 | 8389 | 7192 |
| 16613 | 2982 | 3929 | 11900 | 8750 | 5259 | 18517 | 14460 | 1370 | 18379 | 7537 | 11555 |
| 4777 | 12893 | 14616 | 15017 | 5702 | 12071 | 2926 | 2018 | 2392 | 18128 | 17823 | 17870 |
| 13449 | 12192 | 1385 | 8320 | 11784 | 8264 | 14559 | 5910 | 11792 | 8248 | 1361 | 17542 |
| 1038 | 17221 | 1250 | 12347 | 10203 | 15635 | 7181 | 7956 | 13405 | 1627 | 10660 | 3003 |
| 6686 | 18312 | 14977 | 2919 | 13737 | 1505 | 4646 | 9493 | 11913 | 9654 | 14413 | 16822 |
| 11895 | 12844 | 7146 | 17760 | 12372 | 14263 | 18841 | 10587 | 16428 | 18770 | 12264 | 16337 |
| 14633 | 11408 | 17098 | 2635 | 7437 | 18818 | 16147 | 17848 | 15393 | 9910 | 6063 | 5725 |
| 6571 | 5134 | 17464 | 10392 | 6472 | 17903 | | | | | | |
| 405: 3906 | 4734 | 13745 | 7402 | 14874 | 764 | | | | | | |
| 406: 5355 | 6984 | 4667 | 17305 | 11490 | 16367 | 8426 | 10381 | 1464 | 11818 | 624 | 7412 |
| 3333 | 9382 | 15475 | 11760 | 13337 | | | | | | | |
| 407:12506 | 19148 | 10381 | 2347 | 9031 | 1569 | | | | | | |
| 408: 1173 | 2332 | 4489 | | | | | | | | | |

## Example 3

This example illustrates the construction of a consensus amino acid sequence of homologous proteins of homologous genes that impart an enhanced trait.

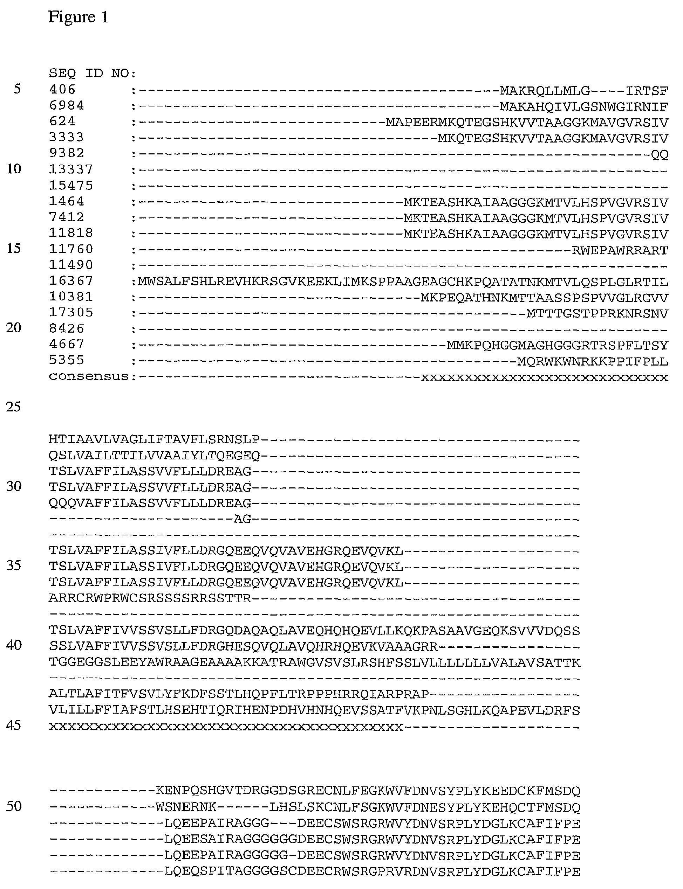

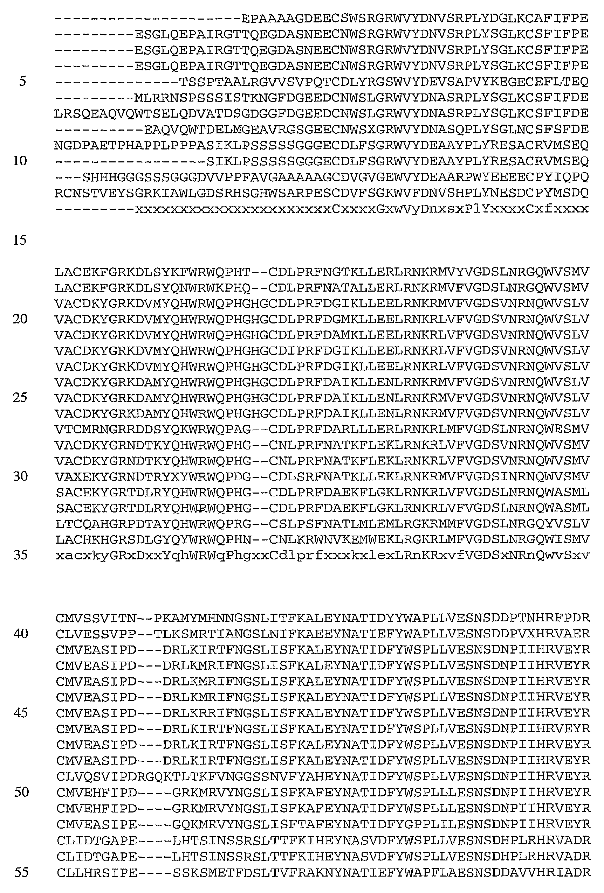

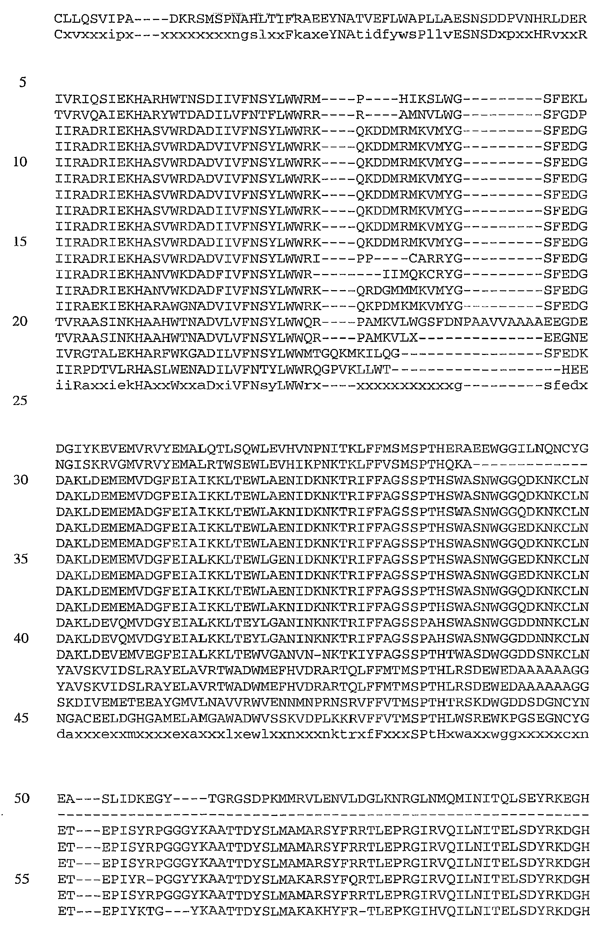

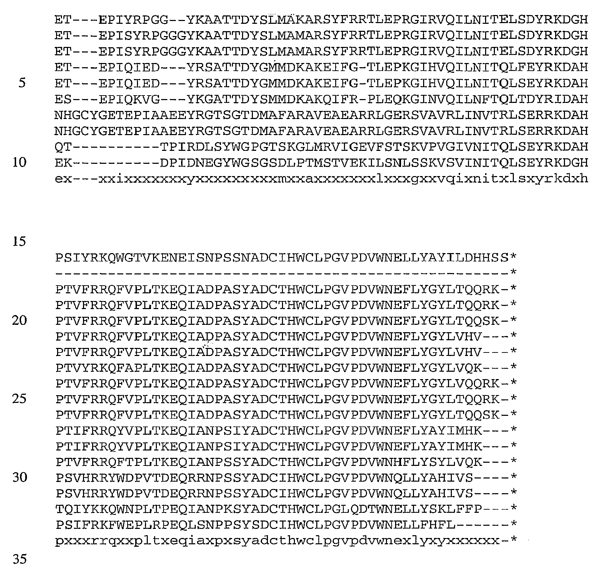

ClustalW program was selected for multiple sequence alignments of the amino acid sequence of SEQ ID NO: 406 and 17 homologs. Three major factors affecting the sequence alignments dramatically are (1) protein weight matrices; (2) gap open penalty; (3) gap extension penalty. Protein weight matrices available for ClustalW program include Blosum, Pam and Gonnet series. Those parameters with gap open penalty and gap extension penalty were extensively tested. On the basis of the test results, Blosum weight matrix, gap open penalty of 10 and gap extension penalty of 1 were chosen for multiple sequence alignment. Shown in Figure 1 are the sequences of SEQ ID NO: 406, its homo logs and the consensus sequence, as set forth in SEQ ID NO: 19248. The symbols for consensus sequence are (1) uppercase letters for 100% identity in all positions of multiple sequence alignment output; (2) lowercase letters for >=70% identity; symbol; (3) "X" indicated <70% identity; (4) dashes "-" meaning that gaps were in >=70% sequences.

The consensus amino acid sequence can be used to identify DNA corresponding to the full scope of this invention that is useful in providing transgenic plants, for example corn and soybean plants with enhanced agronomic traits, for example improved nitrogen use efficiency, improved yield, improved water use efficiency and/or improved growth under cold stress, due to the expression in the plants of DNA encoding a protein with amino acid sequence identical to the consensus amino acid sequence.

**Example 4**

This example illustrates the identification of amino acid domain by Pfam analysis.

The amino acid sequence of the expressed proteins that were shown to be associated with an enhanced trait were analyzed for Pfam protein family against the current Pfam collection of multiple sequence alignments and hidden Markov models using the HMMER software in the appended computer listing. The Pfam protein families for the proteins of SEQ ID NO: 205 through 408 are shown in Table 16. The Hidden Markov model databases for the identified patent families are also in the appended computer listing allowing identification of other homologous proteins and their cognate encoding DNA to enable the full breadth of the invention for a person of ordinary skill in the art. Certain proteins are identified by a single Pfam domain and others by multiple Pfam domains. For instance, the protein with amino acids of SEQ ID NO: 214 is characterized by two Pfam domains, i.e. "C1_4" and "Ssl1". See also the protein with amino acids of SEQ ID NO:222 which is characterized by two copies of the Pfam domain "MatE". In Table 16 "score" is the gathering score for the Hidden Markov Model of the domain which exceeds the gathering cutoff reported in Table 17.

Table 16

| PEP SEQ ID NO | Pfam domain name | begin | stop | score | E-value |
|---|---|---|---|---|---|
| 206 | zf-A20 | 10 | 34 | 35 | 2.40E-07 |
| 206 | zf-AN 1 | 104 | 144 | 64.5 | 3.10E-16 |
| 207 | Cyclin_N | 33 | 168 | 12.1 | 0.00023 |
| 208 | Gpi16 | 9 | 603 | 1272 | 0 |
| 212 | BSD | 100 | 167 | 44.7 | 2.80E-10 |
| 212 | BSD | 179 | 244 | 67.5 | 3.90E-17 |
| 213 | Catalase | 18 | 401 | 977.7 | 3.80E-291 |
| 214 | Ssl1 | 22 | 277 | 646.3 | 2.20E-191 |
| 214 | C1_4 | 361 | 409 | 101.1 | 2.90E-27 |
| 215 | Bromodomain | 119 | 208 | 105.8 | 1.10E-28 |
| 216 | Pkinase | 75 | 343 | -9.1 | 1.60E-07 |
| 217 | PTR2 | 133 | 544 | 197.2 | 3.50E-56 |
| 218 | FTCD_N | 5 | 195 | 377.6 | 1.70E-110 |
| 219 | Abhydrolase_1 | 50 | 272 | 30.4 | 3.00E-06 |
| 220 | HhH-GPD | 172 | 317 | 88.2 | 2.30E-23 |
| 222 | MatE | 40 | 200 | 121.9 | 1.60E-33 |
| 222 | MatE | 261 | 433 | 96 | 1.00E-25 |
| 224 | ubiquitin | 48 | 120 | 24.7 | 0.00029 |
| 224 | BAG | 138 | 219 | 94.2 | 3.50E-25 |
| 225 | Lipase_GDSL | 37 | 365 | 370.9 | 1.80E-108 |

(continued)

| PEP SEQ ID NO | Pfam domain name | begin | stop | score | E-value |
|---|---|---|---|---|---|
| 226 | DUF231 | 280 | 449 | 234.8 | 1.60E-67 |
| 227 | Aldedh | 16 | 485 | 842 | 2.80E-250 |
| 228 | RRM_1 | 110 | 181 | 79.9 | 7.10E-21 |
| 228 | RRM_1 | 214 | 284 | 87.8 | 2.90E-23 |
| 229 | GATase_2 | 2 | 162 | 10.5 | 7.60E-12 |
| 229 | Asn_synthase | 211 | 478 | 335.9 | 6.10E-98 |
| 230 | zf-C2H2 | 273 | 295 | 30.6 | 5.00E-06 |
| 231 | HLH | 62 | 113 | 39 | 1.50E-08 |
| 232 | PP2C | 39 | 323 | 106.9 | 5.20E-29 |
| 233 | zf-C3HC4 | 96 | 137 | 33.4 | 7.10E-07 |
| 234 | AP2 | 83 | 146 | 144.5 | 2.60E-40 |
| 235 | Homeobox | 84 | 145 | 72.6 | 1.10E-18 |
| 236 | zf-C3HC4 | 87 | 129 | 31.8 | 2.10E-06 |
| 237 | Acyl_transf_1 | 52 | 354 | -13.2 | 6.40E-11 |
| 238 | Aldo_ket_red | 5 | 292 | 465.6 | 5.50E-137 |
| 239 | adh_short | 13 | 179 | 55.1 | 2.00E-13 |
| 240 | HMG_CoA_synt | 5 | 453 | 992.9 | 1.00E-295 |
| 241 | SRF-TF | 9 | 59 | 121.8 | 1.80E-33 |
| 241 | K-box | 75 | 175 | 148.8 | 1.20E-41 |
| 242 | Ribosomal_S8e | 1 | 237 | 327.1 | 2.70E-95 |
| 243 | zf-C3HC4 | 103 | 144 | 37.8 | 3.30E-08 |
| 244 | zf-C2H2 | 4 | 26 | 22.3 | 0.0015 |
| 244 | zf-C2H2 | 204 | 226 | 22.4 | 0.0014 |
| 244 | zf-C2H2 | 236 | 258 | 20.6 | 0.005 |
| 245 | Aa_trans | 45 | 439 | 358 | 1.30E-104 |
| 246 | LEA_4 | 71 | 141 | 15.6 | 0.05 |
| 247 | p450 | 66 | 499 | 209.4 | 7.60E-60 |
| 248 | p450 | 40 | 503 | 289.8 | 4.70E-84 |
| 249 | Phi_1 | 25 | 278 | 515 | 7.40E-152 |
| 250 | zf-B_box | 3 | 47 | 56.2 | 9.50E-14 |
| 251 | PP2C | 84 | 348 | 267.9 | 1.80E-77 |
| 254 | Radical_SAM | 171 | 337 | 77.3 | 4.40E-20 |
| 255 | Pkinase | 25 | 283 | 174.5 | 2.30E-49 |
| 256 | adh_short | 6 | 182 | 42.9 | 9.80E-10 |
| 257 | Skp1_POZ | 4 | 64 | 105.1 | 1.80E-28 |
| 257 | Skp1 | 112 | 190 | 173 | 6.70E-49 |
| 258 | DPBB_1 | 73 | 151 | 142.6 | 9.30E-40 |
| 258 | Pollen_allerg_1 | 162 | 239 | 163.9 | 3.70E-46 |

(continued)

| PEP SEQ ID NO | Pfam domain name | begin | stop | score | E-value |
|---|---|---|---|---|---|
| 259 | LRRNT_2 | 18 | 58 | 40.7 | 4.40E-09 |
| 259 | LRR_1 | 86 | 108 | 12.3 | 1.6 |
| 259 | LRR_1 | 109 | 133 | 15.7 | 0.15 |
| 259 | LRR_1 | 134 | 157 | 17.4 | 0.047 |
| 259 | LRR_1 | 158 | 177 | 8.3 | 11 |
| 259 | LRR_1 | 179 | 202 | 12.5 | 1.3 |
| 259 | Pkinase | 334 | 601 | 3.8 | 2.90E-08 |
| 260 | Methyltransf_11 | 135 | 225 | 25.7 | 0.00015 |
| 261 | Ubie_methyltran | 34 | 287 | 368.5 | 9.10E-108 |
| 261 | Methyltransf_11 | 88 | 205 | 70.4 | 5.00E-18 |
| 261 | Methyltransf_12 | 88 | 203 | 38.6 | 1.90E-08 |
| 262 | MtN3_slv | 11 | 100 | 69.4 | 9.90E-18 |
| 262 | MtN3_slv | 135 | 221 | 122.5 | 1.10E-33 |
| 263 | Cys_Met_Meta_PP | 88 | 460 | 768.4 | 3.90E-228 |
| 263 | Beta_elim_lyase | 129 | 376 | -107.7 | 0.0016 |
| 264 | PAR1 | 1 | 181 | 469.5 | 3.60E-138 |
| 265 | Chal_sti_synt_C | 350 | 493 | 13.4 | 0.00012 |
| 266 | Cyclin_N | 43 | 195 | 113.1 | 7.30E-31 |
| 267 | Pkinase | 21 | 418 | 193.4 | 5.00E-55 |
| 268 | Brix | 96 | 271 | 216.4 | 5.60E-62 |
| 269 | tRNA-synt_2b | 81 | 250 | 166.9 | 4.70E-47 |
| 269 | HGTP_anticodon | 402 | 486 | 17.1 | 0.00088 |
| 270 | Proteasome | 38 | 233 | 81.4 | 2.50E-21 |
| 271 | Ammonium_transp | 19 | 423 | 597.5 | 1.10E-176 |
| 272 | AUX_IAA | 8 | 204 | 332.3 | 7.20E-97 |
| 273 | Lectin_legB | 25 | 215 | -14.8 | 1.60E-09 |
| 273 | Lectin_legA | 236 | 279 | 31 | 3.70E-06 |
| 273 | Pkinase | 355 | 624 | 179.3 | 8.50E-51 |
| 273 | Pkinase_Tyr | 355 | 624 | 116.1 | 9.20E-32 |
| 274 | PGK | 2 | 395 | 675.6 | 3.30E-200 |
| 275 | Sugar_tr | 27 | 491 | 416.7 | 2.90E-122 |
| 275 | MFS_1 | 31 | 467 | 75.7 | 1.30E-19 |
| 276 | Hpt | 30 | 112 | 54.9 | 2.30E-13 |
| 278 | RRN3 | 37 | 620 | 1128.7 | 0 |
| 279 | Sugar_tr | 89 | 546 | 595 | 6.00E-176 |
| 279 | MFS_1 | 93 | 505 | 100.6 | 4.10E-27 |
| 280 | AP2 | 25 | 88 | 133.6 | 4.70E-37 |
| 281 | Pkinase | 4 | 205 | 28 | 1.20E-09 |

(continued)

| PEP SEQ ID NO | Pfam domain name | begin | stop | score | E-value |
|---|---|---|---|---|---|
| 282 | ADH_N | 33 | 119 | 53.8 | 5.30E-13 |
| 282 | ADH_zinc_N | 155 | 318 | 43.1 | 8.50E-10 |
| 283 | ADH_N | 34 | 149 | 129.2 | 1.00E-35 |
| 283 | ADH_zinc_N | 180 | 315 | 119.1 | 1.10E-32 |
| 284 | ADH_N | 40 | 165 | 112.8 | 8.90E-31 |
| 284 | ADH_zinc_N | 196 | 338 | 125.2 | 1.60E-34 |
| 285 | ADH_N | 43 | 173 | 105.4 | 1.50E-28 |
| 285 | ADH_zinc_N | 204 | 348 | 112.5 | 1.10E-30 |
| 286 | Mov34 | 23 | 133 | 136.1 | 8.40E-38 |
| 287 | RRM_1 | 36 | 107 | 101.5 | 2.20E-27 |
| 288 | RRM_1 | 36 | 107 | 73 | 8.20E-19 |
| 289 | FA_desaturase | 54 | 269 | 135 | 1.80E-37 |
| 290 | Ras | 15 | 176 | 336.7 | 3.50E-98 |
| 291 | SAC3_GANP | 24 | 209 | 128.9 | 1.20E-35 |
| 292 | RNA_pol_L | 6 | 83 | 75.5 | 1.50E-19 |
| 293 | GSHPx | 8 | 117 | 234.4 | 2.20E-67 |
| 294 | FKBP_C | 115 | 214 | 127.7 | 3.00E-35 |
| 295 | Ras | 10 | 171 | 339 | 7.10E-99 |
| 296 | UQ_con | 15 | 148 | 81.4 | 2.50E-21 |
| 297 | Proteasome | 28 | 215 | 246.5 | 4.90E-71 |
| 298 | Ras | 14 | 175 | 317.4 | 2.20E-92 |
| 299 | Ldh_1_N | 83 | 226 | 247.5 | 2.40E-71 |
| 299 | Ldh_1_C | 228 | 394 | 199.8 | 5.50E-57 |
| 300 | Ras | 8 | 209 | 221.5 | 1.60E-63 |
| 301 | ThiF | 30 | 167 | -12.5 | 3.50E-05 |
| 303 | Sterol_desat | 10 | 215 | 128.3 | 1.90E-35 |
| 304 | Sterol_desat | 12 | 227 | 195.1 | 1.50E-55 |
| 306 | Enolase_N | 3 | 133 | 227.6 | 2.40E-65 |
| 306 | Enolase_C | 138 | 424 | 567.5 | 1.10E-167 |
| 307 | NTP_transferase | 4 | 267 | 89.5 | 9.20E-24 |
| 308 | NAD_binding_2 | 16 | 185 | -39.1 | 1.10E-05 |
| 308 | NAD_Gly3P_dh_N | 17 | 154 | -24.6 | 0.00015 |
| 308 | F420_oxidored | 18 | 265 | 341.2 | 1.60E-99 |
| 309 | F420_oxidored | 4 | 255 | 278.2 | 1.40E-80 |
| 310 | ADH_N | 27 | 145 | 115.5 | 1.40E-31 |
| 310 | ADH_zinc_N | 175 | 318 | 138.1 | 2.10E-38 |
| 311 | Aminotran_1_2 | 44 | 399 | 178.8 | 1.20E-50 |
| 312 | Aldedh | 11 | 476 | 903.9 | 6.50E-269 |

(continued)

| PEP SEQ ID NO | Pfam domain name | begin | stop | score | E-value |
|---|---|---|---|---|---|
| 313 | NTP_transferase | 5 | 269 | 52.4 | 1.40E-13 |
| 314 | PFK | 6 | 281 | 515.1 | 7.10E-152 |
| 315 | NTP_transferase | 10 | 288 | 20.8 | 2.90E-11 |
| 316 | Aminotran_1_2 | 114 | 480 | 492.5 | 4.40E-145 |
| 317 | NTP_transferase | 94 | 349 | 346.3 | 4.40E-101 |
| 318 | Aldedh | 13 | 475 | 734.7 | 5.60E-218 |
| 319 | Aminotran_3 | 113 | 448 | 471.8 | 7.60E-139 |
| 320 | Cys_Met_Meta_PP | 117 | 395 | -276.6 | 0.0042 |
| 320 | Aminotran_1_2 | 118 | 471 | 184.5 | 2.30E-52 |
| 321 | Aminotran_1_2 | 121 | 483 | 125.6 | 1.20E-34 |
| 322 | DAO | 214 | 489 | -32.3 | 0.0011 |
| 322 | Pyr_redox_2 | 214 | 474 | 69.5 | 9.70E-18 |
| 322 | Pyr_redox | 343 | 433 | 55.7 | 1.40E-13 |
| 323 | PGM_PMM_I | 127 | 272 | 140.6 | 3.80E-39 |
| 323 | PGM_PMM_II | 297 | 407 | 69.6 | 8.70E-18 |
| 323 | PGM_PMM_III | 408 | 528 | 105.7 | 1.20E-28 |
| 323 | PGM_PMM_IV | 543 | 632 | 55.6 | 1.50E-13 |
| 324 | Aminotran_3 | 192 | 522 | 634.1 | 1.00E-187 |
| 325 | Biotin_lipoyl | 91 | 164 | 82.6 | 1.10E-21 |
| 325 | E3_binding | 198 | 234 | 74.5 | 3.00E-19 |
| 325 | 2-oxoacid_dh | 262 | 493 | 486.5 | 2.70E-143 |
| 326 | Biotin_lipoyl | 91 | 164 | 79.5 | 9.50E-21 |
| 326 | E3_binding | 198 | 234 | 76.7 | 6.40E-20 |
| 326 | 2-oxoacid_dh | 261 | 492 | 483.1 | 2.90E-142 |
| 327 | Transaldolase | 101 | 401 | 580 | 2.00E-171 |
| 328 | Biotin_lipoyl | 92 | 165 | 88.9 | 1.40E-23 |
| 328 | E3_binding | 201 | 237 | 69 | 1.40E-17 |
| 328 | 2-oxoacid_dh | 261 | 491 | 478.4 | 7.90E-141 |
| 329 | cNMP_binding | 103 | 191 | 73.7 | 5.30E-19 |
| 330 | H10933_like | 90 | 415 | -239.1 | 0.0006 |
| 330 | FAD_binding_2 | 91 | 401 | -113.1 | 0.0016 |
| 330 | GIDA | 91 | 420 | -208 | 0.00018 |
| 330 | DAO | 91 | 353 | -32.7 | 0.0012 |
| 330 | Pyr_redox_2 | 91 | 399 | 237.8 | 2.10E-68 |
| 330 | Pyr_redox | 261 | 354 | 100.9 | 3.30E-27 |
| 330 | Pyr_redox_dim | 428 | 537 | 164.9 | 1.90E-46 |
| 331 | GIDA | 94 | 435 | -211.8 | 0.00032 |
| 331 | Pyr_redox_2 | 94 | 413 | 217.1 | 3.40E-62 |

(continued)

| PEP SEQ ID NO | Pfam domain name | begin | stop | score | E-value |
|---|---|---|---|---|---|
| 331 | Pyr_redox | 271 | 368 | 107.5 | 3.60E-29 |
| 331 | Pyr_redox_dim | 443 | 552 | 193.7 | 3.90E-55 |
| 332 | FAD_binding_2 | 99 | 410 | -122.5 | 0.0042 |
| 332 | Pyr_redox_2 | 99 | 409 | 275 | 1.30E-79 |
| 332 | Pyr_redox | 266 | 362 | 114.7 | 2.30E-31 |
| 332 | Pyr_redox_dim | 437 | 546 | 202.7 | 7.40E-58 |
| 333 | Biotin_lipoyl | 92 | 165 | 81.4 | 2.40E-21 |
| 333 | E3_binding | 202 | 238 | 68.7 | 1.60E-17 |
| 333 | 2-oxoacid_dh | 261 | 491 | 485.1 | 7.30E-143 |
| 334 | DUF6 | 117 | 242 | 57.9 | 3.00E-14 |
| 334 | DUF250 | 251 | 397 | 196.5 | 5.70E-56 |
| 335 | Alpha-amylase | 61 | 489 | -62.8 | 4.70E-06 |
| 336 | GAF | 141 | 304 | 86.6 | 6.80E-23 |
| 336 | Phytochrome | 315 | 501 | 33 | 8.10E-07 |
| 336 | HWE_HK | 520 | 600 | 115.8 | 1.10E-31 |
| 336 | Response_reg | 732 | 848 | 36.6 | 7.80E-08 |
| 337 | NIR_SIR_ferr | 69 | 137 | 83 | 8.50E-22 |
| 337 | NIR_SIR | 169 | 332 | 206.4 | 5.80E-59 |
| 337 | NIR_SIR_ferr | 348 | 419 | 75.4 | 1.50E-19 |
| 338 | Fer4 | 9 | 32 | 9.4 | 0.011 |
| 338 | Fer4 | 177 | 202 | 9.1 | 0.012 |
| 339 | PGI | 52 | 541 | 1099 | 0 |
| 340 | Molybdop_Fe4S4 | 2 | 68 | 80.6 | 4.30E-21 |
| 340 | Molybdopterin | 70 | 501 | 390.7 | 1.90E-114 |
| 340 | Molydop_binding | 627 | 738 | 174.9 | 1.80E-49 |
| 341 | PGI | 55 | 545 | 751.6 | 4.50E-223 |
| 342 | Iso_dh | 28 | 469 | 379.1 | 6.10E-111 |
| 343 | NIR_SIR_ferr | 78 | 147 | 71.9 | 1.80E-18 |
| 343 | NIR_SIR | 179 | 338 | 199.5 | 6.80E-57 |
| 343 | NIR_SIR_ferr | 354 | 425 | 67.3 | 4.50E-17 |
| 344 | DUF783 | 27 | 236 | 348.6 | 9.00E-102 |
| 346 | PA | 43 | 144 | 89.4 | 9.50E-24 |
| 346 | zf-C3HC4 | 232 | 273 | 34.7 | 3.00E-07 |
| 347 | Peptidase_C26 | 13 | 248 | 158.9 | 1.10E-44 |
| 348 | SURF5 | 17 | 143 | 252 | 1.10E-72 |
| 349 | DUF962 | 7 | 171 | 329.8 | 4.20E-96 |
| 350 | Cyclin_N | 191 | 318 | 198.5 | 1.40E-56 |
| 350 | Cyclin_C | 320 | 447 | 173.7 | 4.00E-49 |

(continued)

| PEP SEQ ID NO | Pfam domain name | begin | stop | score | E-value |
|---|---|---|---|---|---|
| 351 | WRKY | 237 | 297 | 137.6 | 2.90E-38 |
| 352 | SBDS | 6 | 244 | 261.6 | 1.40E-75 |
| 353 | DUF167 | 38 | 112 | 82.3 | 1.40E-21 |
| 355 | zf-CCCH | 2 | 25 | 30.5 | 5.40E-06 |
| 356 | DIM1 | 5 | 139 | 78.9 | 1.40E-20 |
| 358 | Rieske | 100 | 198 | 68.5 | 1.90E-17 |
| 359 | Fe_bilin_red | 112 | 333 | 416.6 | 3.20E-122 |
| 360 | Radical_SAM | 123 | 282 | 47.3 | 4.70E-11 |
| 361 | LEA_4 | 107 | 180 | 55.3 | 1.80E-13 |
| 363 | Pribosyltran | 92 | 236 | 146.3 | 7.30E-41 |
| 364 | Rhodanese | 254 | 367 | 102 | 1.60E-27 |
| 367 | DRMBL | 248 | 368 | 129 | 1.20E-35 |
| 368 | MIP | 30 | 251 | 276.9 | 3.60E-80 |
| 369 | EBP | 23 | 219 | 362.2 | 7.50E-106 |
| 371 | CcmH | 1 | 139 | 16.6 | 6.30E-09 |
| 373 | CTP_transf_2 | 30 | 170 | 48.4 | 2.20E-11 |
| 374 | AP2 | 46 | 109 | 145.7 | 1.10E-40 |
| 375 | HLH | 48 | 97 | 52.3 | 1.40E-12 |
| 376 | Myb_DNA-binding | 59 | 104 | 58.1 | 2.50E-14 |
| 377 | Ham1p_like | 12 | 188 | 153.2 | 6.10E-43 |
| 378 | SRF-TF | 9 | 59 | 112.2 | 1.40E-30 |
| 378 | K-box | 74 | 174 | 76.9 | 5.80E-20 |
| 379 | Response_reg | 10 | 152 | 75.9 | 1.20E-19 |
| 380 | Nuc_sug_transp | 105 | 341 | 17.6 | 1.50E-14 |
| 381 | SRF-TF | 9 | 59 | 105.7 | 1.20E-28 |
| 381 | K-box | 70 | 167 | 6.3 | 0.00011 |
| 382 | E2F_TDP | 16 | 81 | 111.2 | 2.60E-30 |
| 382 | E2F_TDP | 151 | 231 | 128 | 2.30E-35 |
| 384 | RNA_pol_Rpb6 | 61 | 114 | 86.8 | 5.90E-23 |
| 386 | Myb_DNA-binding | 45 | 96 | 41.3 | 2.90E-09 |
| 387 | zf-C3HC4 | 83 | 122 | 41.3 | 2.90E-09 |
| 388 | zf-C3HC4 | 42 | 86 | 35.1 | 2.20E-07 |
| 389 | AUX_IAA | 11 | 234 | 381.5 | 1.10E-111 |
| 390 | Myb_DNA-binding | 14 | 61 | 49.1 | 1.30E-11 |
| 390 | Myb_DNA-binding | 67 | 112 | 38.4 | 2.10E-08 |
| 391 | AP2 | 49 | 121 | 137.7 | 2.80E-38 |
| 391 | AP2 | 151 | 215 | 101.8 | 1.80E-27 |
| 392 | TCP | 56 | 241 | 161.1 | 2.60E-45 |

(continued)

| PEP SEQ ID NO | Pfam domain name | begin | stop | score | E-value |
|---|---|---|---|---|---|
| 393 | zf-C3HC4 | 383 | 424 | 43.1 | 8.30E-10 |
| 394 | SET | 109 | 238 | 182.9 | 7.10E-52 |
| 395 | zf-C2H2 | 36 | 59 | 18 | 0.031 |
| 396 | zf-C3HC4 | 206 | 246 | 34.5 | 3.40E-07 |
| 397 | Myb_DNA-binding | 26 | 75 | 34.3 | 3.70E-07 |
| 397 | Myb_DNA-binding | 134 | 181 | 53.7 | 5.30E-13 |
| 398 | bZIP_1 | 171 | 234 | 26.9 | 6.50E-05 |
| 398 | bZIP_2 | 171 | 221 | 25.3 | 0.00019 |
| 399 | zf-C3HC4 | 111 | 152 | 37.5 | 4.10E-08 |
| 400 | WD40 | 15 | 51 | 16.6 | 0.079 |
| 400 | WD40 | 59 | 95 | 23.3 | 0.00075 |
| 400 | WD40 | 210 | 246 | 16.4 | 0.093 |
| 400 | WD40 | 329 | 366 | 21.2 | 0.0033 |
| 401 | AP2 | 15 | 80 | 89.1 | 1.20E-23 |
| 402 | Arm | 35 | 75 | 34.2 | 4.00E-07 |
| 402 | Arm | 297 | 337 | 21.5 | 0.0027 |
| 403 | Aminotran_3 | 42 | 384 | 494.7 | 9.80E-146 |
| 404 | iPGM_N | 3 | 383 | 716.6 | 1.60E-212 |
| 404 | Metalloenzyme | 393 | 512 | 147.2 | 3.80E-41 |
| 405 | DUF231 | 239 | 408 | 227.7 | 2.30E-65 |
| 406 | DUF231 | 231 | 404 | 334.6 | 1.50E-97 |
| 407 | DUF231 | 237 | 405 | 336.4 | 4.40E-98 |
| 408 | NPH3 | 204 | 452 | 364.6 | 1.40E-106 |

Table 17

| Pfam domain name | accession number | gathering cutoff | domain description |
|---|---|---|---|
| 2-oxoacid_dh | PF00198.12 | -112 | 2-oxoacid dehydrogenases acyltransferase (catalytic domain) |
| ADH_N | PF08240.1 | -14.5 | Alcohol dehydrogenase GroES-like domain |
| ADH_zinc_N | PF00107.15 | 23.8 | Zinc-binding dehydrogenase |
| AP2 | PF00847.9 | 0 | AP2 domain |
| AUX_IAA | PF02309.6 | -83 | AUX/IAA family |
| Aa_trans | PF01490.7 | -128.4 | Transmembrane amino acid transporter protein |
| Abhydrolase_1 | PF00561.9 | 5.5 | alpha/beta hydrolase fold |
| Acyl_transf_1 | PF00698.10 | -120 | Acyl transferase domain |
| Aldedh | PF00171.11 | -295 | Aldehyde dehydrogenase family |
| Aldo_ket_red | PF00248.10 | -97 | Aldo/keto reductase family |

(continued)

| Pfam domain name | accession number | gathering cutoff | domain description |
|---|---|---|---|
| Alpha-amylase | PF00128.11 | -93 | Alpha amylase, catalytic domain |
| Aminotran_1_2 | PF00155.9 | -57.5 | Aminotransferase class I and II |
| Aminotran_3 | PF00202.10 | -207.6 | Aminotransferase class-III |
| Ammonium_transp | PF00909.10 | -144 | Ammonium Transporter Family |
| Arm | PF00514.11 | 40.1 | Armadillo/beta-catenin-like repeat |
| Asn_synthase | PF00733.10 | -52.8 | Asparagine synthase |
| BAG | PF02179.5 | 25 | BAG domain |
| BSD | PF03909.6 | 25 | BSD domain |
| Beta_elim_lyase | PF01212.10 | -114.4 | Beta-eliminating lyase |
| Biotin_lipoyl | PF00364.11 | -2.3 | Biotin-requiring enzyme |
| Brix | PF04427.7 | 11.4 | Brix domain |
| Bromodomain | PF00439.13 | 8.9 | Bromodomain |
| C1_4 | PF07975.1 | 25 | TFIIH C1-like domain |
| CTP_trans_2 | PF01467.15 | -11.8 | Cytidylyltransferase |
| Catalase | PF00199.8 | -229 | Catalase |
| CcmH | PF03918.4 | -30.8 | Cytochrome C biogenesis protein |
| Chal_sti_synt_C | PF02797.5 | -6.1 | Chalcone and stilbene synthases, C-terminal domain |
| Cyclin_C | PF02984.7 | -13 | Cyclin, C-terminal domain |
| Cyclin_N | PF00134.12 | -14.7 | Cyclin, N-terminal domain |
| Cys_Met_Meta_PP | PF01053.9 | -278.4 | Cys/Met metabolism PLP-dependent enzyme |
| DAO | PF01266.11 | -36.5 | FAD dependent oxidoreductase |
| DIM1 | PF02966.6 | 25 | Mitosis protein DIM1 |
| DPBB_1 | PF03330.7 | 30 | Rare lipoprotein A (RlpA)-like double-psi beta-barrel |
| DRMBL | PF07522.3 | 25 | DNA repair metallo-beta-lactamase |
| DUF167 | PF02594.6 | 25 | Uncharacterized ACR, YggU family COG 1872 |
| DUF231 | PF03005.5 | -58 | Arabidopsis proteins of unknown function |
| DUF250 | PF03151.6 | 125 | Domain of unknown function, DUF250 |
| DUF6 | PF00892.9 | 30 | Integral membrane protein DUF6 |
| DUF783 | PF05615.2 | 25 | Protein of unknown function (DUF783) |
| DUF962 | PF06127.1 | 25 | Protein of unknown function (DUF962) |
| E2F_TDP | PF02319.9 | 17 | E2F/DP family winged-helix DNA-binding domain |
| E3_binding | PF02817.6 | 10 | e3 binding domain |
| EBP | PF05241.1 | 25 | Emopamil binding protein |
| Enolase_C | PF00113.11 | -34 | Enolase, C-terminal TIM barrel domain |
| Enolase_N | PF03952.5 | -4 | Enolase, N-terminal domain |
| F420_oxidored | PF03807.5 | -34.5 | NADP oxidoreductase coenzyme F420-dependent |
| FAD_binding_2 | PF00890.13 | -124.8 | FAD binding domain |

(continued)

| Pfam domain name | accession number | gathering cutoff | domain description |
|---|---|---|---|
| FA_desaturase | PF00487.13 | -46 | Fatty acid desaturase |
| FKBP_C | PF00254.16 | -7.6 | FKBP-type peptidyl-prolyl cis-trans isomerase |
| FTCD_N | PF07837.2 | -67.7 | Formiminotransferase domain, N-terminal subdomain |
| Fe_bilin_red | PF05996.2 | 25 | Ferredoxin-dependent bilin reductase |
| Fer4 | PF00037.14 | 8 | 4Fe-4S binding domain |
| GAF | PF01590.14 | 23 | GAF domain |
| GATase_2 | PF00310.10 | -106.2 | Glutamine amidotransferases class-II |
| GIDA | PF01134.11 | -226.7 | Glucose inhibited division protein A |
| GSHPx | PF00255.9 | -16 | Glutathione peroxidase |
| Gpi16 | PF04113.3 | -207.8 | Gpi16 subunit, GPI transamidase component |
| HGTP_anticodon | PF03129.9 | -2 | Anticodon binding domain |
| HI0933_like | PF03486.4 | -255.8 | HI0933-like protein |
| HLH | PF00010.15 | 8.2 | Helix-loop-helix DNA-binding domain |
| HMG_CoA_synt | PF01154.7 | -230 | Hydroxymethylglutaryl-coenzyme A synthase |
| HWE_HK | PF07536.4 | 25 | HWE histidine kinase |
| Ham1p_like | PF01725.6 | -46 | Ham1 family |
| HhH-GPD | PF00730.13 | 13.5 | HhH-GPD superfamily base excision DNA repair protein |
| Homeobox | PF00046.17 | -4.1 | Homeobox domain |
| Hpt | PF01627.11 | 25 | Hpt domain |
| Iso_dh | PF00180.9 | -97 | Isocitrate/isopropylmalate dehydrogenase |
| K-box | PF01486.7 | 0 | K-box region |
| LEA_4 | PF02987.6 | 25 | Late embryogenesis abundant protein |
| LRRNT_2 | PF08263.1 | 18.6 | Leucine rich repeat N-terminal domain |
| LRR_1 | PF00560.20 | 19 | Leucine Rich Repeat |
| Ldh_1_C | PF02866.6 | -13 | lactate/malate dehydrogenase, alpha/beta C-terminal domain |
| Ldh_1_N | PF00056.11 | -31.3 | lactate/malate dehydrogenase, NAD binding domain |
| Lectin_legA | PF00138.7 | 19 | Legume lectins alpha domain |
| Lectin_legB | PF00139.9 | -77 | Legume lectins beta domain |
| Lipase_GDSL | PF00657.11 | 10.9 | GDSL-like Lipase/Acylhydrolase |
| MFS_1 | PF07690.4 | 23.5 | Major Facilitator Superfamily |
| MIP | PF00230.8 | -62 | Major intrinsic protein |
| MatE | PF01554.8 | 59.6 | MatE |
| Metalloenzyme | PF01676.7 | -14.4 | Metalloenzyme superfamily |
| Methyltransf_11 | PF08241.1 | 17.1 | Methyltransferase domain |
| Methyltransf_12 | PF08242.1 | 21.4 | Methyltransferase domain |
| Molybdop_Fe4S4 | PF04879.5 | 13.6 | Molybdopterin oxidoreductase Fe4S4 domain |

(continued)

| Pfam domain name | accession number | gathering cutoff | domain description |
|---|---|---|---|
| Molybdopterin | PF00384.11 | -50 | Molybdopterin oxidoreductase |
| Molydop_binding | PF01568.10 | 1.1 | Molydopterin dinucleotide binding domain |
| Mov34 | PF01398.10 | -4 | Mov34/MPN/PAD-1 family |
| MtN3_slv | PF03083.5 | -0.8 | MtN3/saliva family |
| Myb_DNA-binding | PF00249.18 | 19.1 | Myb-like DNA-binding domain |
| NAD_Gly3P_dh_N | PF01210.12 | -44 | NAD-dependent glycerol-3-phosphate dehydrogenase N-terminus |
| NAD_binding_2 | PF03446.4 | -63.5 | NAD binding domain of 6-phosphogluconate dehydrogenase |
| NIR_SIR | PF01077.10 | -25 | Nitrite and sulphite reductase 4Fe-4S domain |
| NIR_SIR_ferr | PF03460.5 | 20 | reductase ferredoxin-like half domain |
| NPH3 | PF03000.4 | 25 | NPH3 family |
| NTP_transferase | PF00483.12 | -90.5 | Nucleotidyl transferase |
| Nuc_sug_transp | PF04142.5 | -92.4 | Nucleotide-sugar transporter |
| PA | PF02225.10 | 13 | PA domain |
| PAR1 | PF06521.1 | 25 | PAR1 protein |
| PFK | PF00365.9 | -132 | Phosphofructokinase |
| PGI | PF00342.8 | -168.9 | Phosphoglucose isomerase |
| PGK | PF00162.8 | -95.1 | Phosphoglycerate kinase |
| PGM_PMM_I | PF02878.5 | -37.5 | Phosphoglucomutase/phosphomannomutase, alpha/beta/alpha domain I |
| PGM_PMM_II | PF02879.5 | -20 | Phosphoglucomutase/phosphomannomutase, alpha/beta/alpha domain II |
| PGM_PMM_III | PF02880.5 | -11 | Phosphoglucomutase/phosphomannomutase, alpha/beta/alpha domain III |
| PGM_PMM_IV | PF00408.9 | -6 | Phosphoglucomutase/phosphomannomutase, C-terminal domain |
| PP2C | PF00481.10 | -44 | Protein phosphatase 2C |
| PTR2 | PF00854.11 | -50 | POT family |
| Peptidase_C26 | PF07722.2 | 25 | Peptidase C26 |
| Phi_1 | PF04674.2 | 25 | Phosphate-induced protein 1 conserved region |
| Phytochrome | PF00360.9 | 11 | Phytochrome region |
| Pkinase | PF00069.14 | -70.8 | Protein kinase domain |
| Pkinase_Tyr | PF07714.4 | 65 | Protein tyrosine kinase |
| Pollen_allerg_1 | PF01357.10 | 17.2 | Pollen allergen |
| Pribosyltran | PF00156.14 | 2 | Phosphoribosyl transferase domain |
| Proteasome | PF00227.14 | -36.7 | Proteasome A-type and B-type |
| Pyr_redox | PF00070.16 | 5 | Pyridine nucleotide-disulphide oxidoreductase |
| Pyr_redox_2 | PF07992.2 | -20 | Pyridine nucleotide-disulphide oxidoreductase |

(continued)

| Pfam domain name | accession number | gathering cutoff | domain description |
|---|---|---|---|
| Pyr_redox_dim | PF02852.11 | -13 | Pyridine nucleotide-disulphide oxidoreductase, dimerisation domain |
| RNA_pol_L | PF01193.11 | 16.9 | polymerase Rpb3/Rpb11 dimerisation domain |
| RNA_pol_Rpb6 | PF01192.12 | 25 | RNA polymerase Rpb6 |
| RRM_1 | PF00076.10 | 15.2 | RNA recognition motif. (a.k.a. RRM, RBD, or RNP domain) |
| RRN3 | PF05327.1 | 25 | RNA polymerase I specific transcription initiation factor RRN3 |
| Radical_SAM | PF04055.8 | 8.4 | Radical SAM superfamily |
| Ras | PF00071.11 | 18 | Ras family |
| Response_reg | PF00072.11 | -14.4 | Response regulator receiver domain |
| Rhodanese | PF00581.9 | 25 | Rhodanese-like domain |
| Ribosomal_S8e | PF01201.11 | 25 | Ribosomal protein S8e |
| Rieske | PF00355.15 | -7 | Rieske [2Fe-2S] domain |
| SAC3_GANP | PF03399.5 | -15.2 | SAC3/GANP/Nin1/mts3/eIF-3 p25 family |
| SBDS | PF01172.7 | -78 | Shwachman-Bodian-Diamond syndrome (SBDS) proteins |
| SET | PF00856.16 | 15.8 | SET domain |
| SRF-TF | PF00319.8 | 11 | SRF-type transcription factor (DNA-binding and dimerisation domain) |
| SURF5 | PF06179.2 | 25 | Surfeit locus protein 5 |
| Skp1 | PF01466.8 | -2 | Skp1 family, dimerisation domain |
| Skp1_POZ | PF03931.4 | 14.9 | Skp1 family, tetramerisation domain |
| Ssl1 | PF04056.4 | -151.8 | Ssl1-like |
| Sterol_desat | PF01598.7 | -13 | Sterol desaturase |
| Sugar_tr | PF00083.12 | -85 | Sugar (and other) transporter |
| TCP | PF03634.3 | -38 | TCP family transcription factor |
| ThiF | PF00899.10 | -38.4 | ThiF family |
| Transaldolase | PF00923.8 | -49 | Transaldolase |
| UQ_con | PF00179.15 | -30 | Ubiquitin-conjugating enzyme |
| Ubie_methyltran | PF01209.8 | -117 | ubiE/COQ5 methyltransferase family |
| WD40 | PF00400.19 | 21.4 | WD domain, G-beta repeat |
| WRKY | PF03106.5 | 25 | WRKY DNA -binding domain |
| adh_short | PF00106.13 | -46.6 | short chain dehydrogenase |
| bZIP_1 | PF00170.10 | 16.5 | bZIP transcription factor |
| bZIP_2 | PF07716.4 | 15 | Basic region leucine zipper |
| cNMP_binding | PF00027.17 | 20.6 | Cyclic nucleotide-binding domain |
| iPGM_N | PF06415.3 | -263.4 | BPG-independent PGAM N-terminus (iPGM_N) |
| p450 | PF00067.11 | -105 | Cytochrome P450 |
| tRNA-synt_2b | PF00587.14 | -40.5 | tRNA synthetase class II core domain (G, H, P, S and T) |

(continued)

| Pfam domain name | accession number | gathering cutoff | domain description |
|---|---|---|---|
| Ubiquitin | PF00240.12 | 19.4 | Ubiquitin family |
| zf-A20 | PF01754.6 | 25 | A20-like zinc finger |
| zf-AN 1 | PF01428.6 | 15 | AN1-like Zinc finger |
| zf-B_box | PF00643.13 | 11.1 | B-box zinc finger |
| zf-C2H2 | PF00096.14 | 19 | Zinc finger, C2H2 type |
| zf-C3HC4 | PF00097.12 | 16.9 | Zinc finger, C3HC4 type (RING finger) |
| zf-CCCH | PF00642.14 | 10.7 | Zinc finger C-x8-C-x5-C-x3-H type (and similar) |

**Example 5**

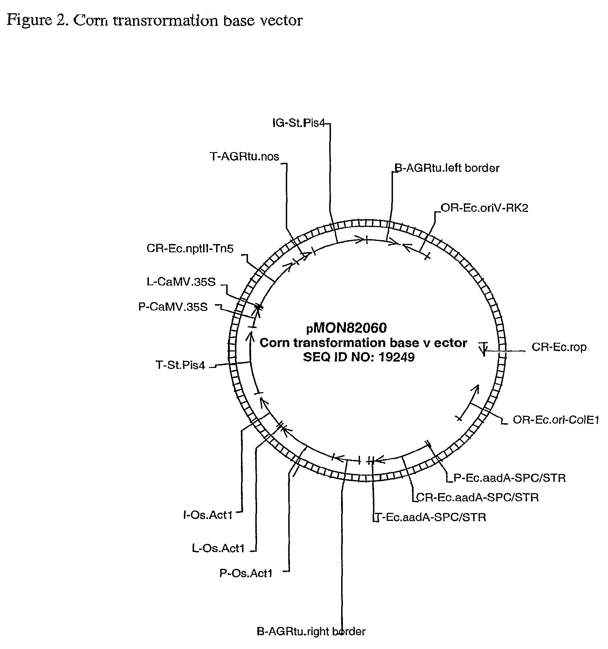

This example illustrates the construction of plasmids for transferring recombinant DNA into plant cells which can be regenerated into transgenic crop plants of this invention. Primers for PCR amplification of protein coding nucleotides of recombinant DNA are designed at or near the start and stop codons of the coding sequence, in order to eliminate most of the 5' and 3' untranslated regions. DNA of interest, i.e. each DNA identified in Table 1 and the DNA for the identified homologous genes, are cloned and amplified by PCR prior to insertion into the insertion site the base vector.
Elements of an exemplary common expression vector, pMON82060 are illustrated in Table 18. The exemplary base vector which is especially useful for corn transformation is illustrated in Figure 2 and assembled using technology known in the art. The DNA of interest are inserted in a expression vector at the insertion site between the intron1 of rice act 1 gene and the termination sequence of PinII gene.

Table 18. pMON82060

| function | name | annotation | Coordinates of SEQ ID NO: 19249 |
|---|---|---|---|
| Agro transformation | B-AGRtu.right border | Agro right border sequence, essential for transfer of T-DNA. | 5235-5591 |
| Gene of | P-Os.Act1 | Promoter from the rice actin gene act1. | 5609-7009 |
| interest plant expression cassette | L-Os.Act1 | Leader (first exon) from the rice actin 1 gene. | |
| | I-Os.Act1 | First intron and flanking UTR exon sequences from the rice actin 1 gene | |
| | insertion site | | |
| | T-St.Pis4 | The 3'non-translated region of the potato proteinase inhibitor II gene which functions to direct polyadenylation of the mRNA | 7084-8026 |
| Plant selectable marker expression cassette | P-CaMV.35S | CaMV 35S promoter | |
| | L-CaMV.35S | 5' UTR from the 35S RNA of CaMV | 8075-8398 |
| | CR-Ec.nptII-Tn5 | nptII selectable marker that confers resistance to neomycin and kanamycin | 8432-9226 |
| | T-AGRtu.nos | A 3'non-translated region of the nopaline synthase gene of Agrobacterium tumefaciens Ti plasmid which functions to direct polyadenylation of the mRNA. | 9255-9507 |
| Agro transformation | B-AGRtu.left border | Agro left border sequence, essential for transfer of T-DNA. | 39-480 |

(continued)

| function | name | annotation | Coordinates of SEQ ID NO: 19249 |
|---|---|---|---|
| Maintenance in E. coli | OR-Ec.oriV-RK2 | The vegetative origin of replication from plasmid RK2. | 567-963 |
| | CR-Ec.rop | Coding region for repressor of primer from the ColE1 plasmid. Expression of this gene product interferes with primer binding at the origin of replication, keeping plasmid copy number low. | 2472-2663 |
| | OR-Ec.ori-ColE1 | The minimal origin of replication from the E. coli plasmid ColE1. | 3091-3679 |
| | P-Ec.aadA-SPC/STR | promoter for Tn7 adenylyltransferase (AAD(3")) | 4210-4251 |
| | CR-Ec.aadA-SPC/STR | Coding region for Tn7 adenylyltransferase (AAD(3")) conferring spectinomycin and streptomycin resistance. | 4252-5040 |
| | T-Ec.aadA-SPC/STR | 3' UTR from the Tn7 adenylyltransferase (AAD(3")) gene of E. coli. | 5041-5098 |

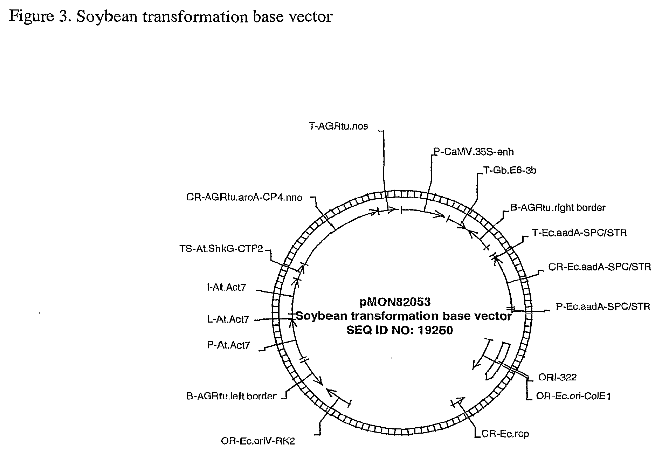

Plasmids for use in transformation of soybean are also prepared. Elements of an exemplary common expression vector plasmid pMON82053 are shown in Table 19 below. This exemplary soybean transformation base vector illustrated in Figure 3 was assembled using the technology known in the art. DNA of interest, i.e. each DNA identified in Table 1 and the DNA for the identified homologous genes, are cloned and amplified by PCR prior to insertion into the insertion site the base vector at the insertion site between the enhanced 35S CaMV promoter and the termination sequence of cotton E6 gene.

**Table 19. pMON82053**

| function | name | annotation | Coordinates of SEQ ID NO: 19250 |
|---|---|---|---|
| Agro transforamtion | B-AGRtu.left border | Agro left border sequence, essential for transfer of T-DNA. | 6144-6585 |
| Plant selectable marker expression cassette | P-At.Act7 | Promoter from the arabidopsis actin 7 gene | 6624-7861 |
| | L-At.Act7 | 5'UTR of Arabidopsis Act7 gene | |
| | I-At.Act7 | Intron from the Arabidopsis actin7 gene | |
| | TS-At.ShkG-CTP2 | Transit peptide region of Arabidopsis EPSPS | 7864-8091 |
| | CR-AGRtu.aroA-CP4.nno_At | Synthetic CP4 coding region with dicot preferred codon usage. | 8092-9459 |
| | T-AGRtu.nos | A 3'non-translated region of the nopaline synthase gene of Agrobacterium tumefaciens Ti plasmid which functions to direct polyadenylation of the mRNA. | 9466-9718 |
| Gene of interest expression cassette | P-CaMV.35S-enh | Promoter for 35S RNA from CaMV containing a duplication of the -90 to - 350 region. | 1-613 |
| | insertion site | | |
| | T-Gb.E6-3b | 3' untranslated region from the fiber protein E6 gene of sea-island cotton; | 688-1002 |

(continued)

| function | name | annotation | Coordinates of SEQ ID NO: 19250 |
|---|---|---|---|
| Agro transformation | B-AGRtu.right border | Agro right border sequence, essential for transfer of T-DNA. | 1033-1389 |
| Maintenance in E. coli | OR-Ec.oriV-RK2 | The vegetative origin of replication from plasmid RK2. | 5661-6057 |
| | CR-Ec.rop | Coding region for repressor of primer from the ColE1 plasmid. Expression of this gene product interferes with primer binding at the origin of replication, keeping plasmid copy number low. | 3961-4152 |
| | OR-Ec.ori-ColE1 | The minimal origin of replication from the E. coli plasmid ColE1. | 2945-3533 |
| | P-Ec.aadA-SPC/STR | romoter for Tn7 adenylyltransferase (AAD(3")) | 2373-2414 |
| | CR-Ec.aadA-SPC/STR | Coding region for Tn7 adenylyltransferase (AAD(3")) conferring spectinomycin and streptomycin resistance. | 1584-2372 |
| | T-Ec.aadA-SPC/STR | 3' UTR from the Tn7 adenylyltransferase (AAD(3")) gene of E. coli. | 1526-1583 |

## Example 5

This example illustrates monocot plant transformation useful in producing the transgenic plant cells of this invention by transformation of corn. Corn plants of a readily transformable line are grown in the greenhouse and ears harvested when the embryos are 1.5 to 2.0 mm in length. Ears are surface sterilized by spraying or soaking the ears in 80% ethanol, followed by air drying. Immature embryos are isolated from individual kernels on surface sterilized ears. Prior to inoculation of maize cells, *Agrobacterium* cells are grown overnight at room temperature. Immature maize embryos are inoculated with *Agrobacterium* shortly after excision, and incubated at room temperature with *Agrobacterium* for 5-20 minutes. Immature embryos are then co-cultured with *Agrobacterium* for 1 to 3 days at 23°C in the dark. Co-cultured embryos are transferred to selection media and cultured for approximately two weeks to allow embryogenic callus to develop. Embryogenic callus is transferred to culture medium containing 100 mg/L paromomycin and subcultured at about two week intervals. Transformants are recovered 6 to 8 weeks after initiation of selection.

Plasmid vectors are prepared essentially as described in Example 5 for transforming into corn each of the DNA of interest, i.e. each DNA identified in Table 1 and the DNA for the identified homologous genes, by *Agrobacterium*-mediated transformation.

For *Agrobacterium*-mediated transformation of maize callus, immature embryos are cultured for approximately 8-21 days after excision to allow callus to develop. Callus is then incubated for about 30 minutes at room temperature with the *Agrobacterium* suspension, followed by removal of the liquid by aspiration. The callus and *Agrobacterium* are co-cultured without selection for 3-6 days followed by selection on paromomycin for approximately 6 weeks, with biweekly transfers to fresh media, and paromomycin resistant callus identified as containing the recombinant DNA in an expression cassette.

To regenerate transgenic corn plants trangenic callus resulting from transformation is placed on media to initiate shoot development in plantlets which are transferred to potting soil for initial growth in a growth chamber at 26 degrees C followed by a mist bench before transplanting to 5 inch pots where plants are grown to maturity. The plants are self fertilized and seed is harvested for screening as seed, seedlings or progeny R2 plants or hybrids, e.g. for yield trials in the screens indicated above. Populations of transgenic plants and seeds produced form transgenic plant cells from each transgenic event are screened as described in Example 7 below to identify the members of the population having the enhanced trait.

## Example 6.

This example illustrates dicot plant transformation useful in producing the transgenic plant cells of this invention by transformation of soybean plants. For Agrobacterium mediated transformation, soybean seeds are germinated overnight and the meristem explants excised. The meristems and the explants are placed in a wounding vessel. Soybean explants and induced Agrobacterium cells from a strain containing plasmid DNA with the gene of interest cassette and a plant selectable marker cassette are mixed no later than 14 hours from the time of initiation of seed germination and wounded using sonication. Following wounding, explants are placed in co-culture for 2-5 days at which point they are transferred to selection media for 6-8 weeks to allow selection and growth of transgenic shoots. Trait positive shoots are harvested approximately 6-8 weeks post bombardment and placed into selective rooting media for 2-3 weeks. Shoots producing roots are transferred to the greenhouse and potted in soil. Shoots that remain healthy on selection, but do not produce roots are transferred to non-selective rooting media for an additional two weeks. Roots from any shoots that produce roots off selection are tested for expression of the plant selectable marker before they are transferred to the greenhouse and potted in soil. Populations of transgenic plants and seeds produced form transgenic plant cells from each transgenic event are screened as described in Example 7 below to identify the members of the population having the enhanced trait.

**Example 7.**

This example illustrates identification of plant cells of the invention by screening derived plants and seeds for enhanced trait. Many transgenic events which survive to fertile transgenic plants that produce seeds and progeny plants will not exhibit an enhanced agronomic trait. Populations of transgenic seed and plants prepared in Examples 5 and 6 are screened to identify those transgenic events providing transgenic plant cells with recombinant DNA imparting an enhanced trait. Each population is screened for nitrogen use efficiency, increased yield, enhanced water use efficiency, enhanced tolerance to cold and heat, enhanced level of oil and protein in seed using assays described below. Plant cells having recombinant DNA with each of the genes identified in Table 1 and the identified homologs are identified in plants and seeds with at least one of the enhanced traits.

**Selection for enhanced Nitrogen Use Efficiency**

The physiological efficacy of transgenic corn plants (tested as hybrids) can be tested for nitrogen use efficiency (NUE) traits in a high-throughput nitrogen (N) selection method. The collected data are compared to the measurements from wildtype controls using a statistical model to determine if the changes are due to the transgene. Raw data were analyzed by SAS software. Results shown herein are the comparison of transgenic plants relative to the wildtype controls.

(1) Media Preparation for Planting a NUE Protocol

Planting materials used: Metro Mix 200 (vendor: Hummert) Cat. # 10-0325, Scotts Micro Max Nutrients (vendor: Hummert) Cat. # 07-6330, OS 4 1/3" x 3 7/8" pots (vendor: Hummert) Cat. # 16-1415, OS trays (vendor: Hummert) Cat. # 16-1515, Hoagland's macronutrients solution, Plastic 5" stakes (vendor: Hummert) yellow Cat. # 49-1569, white Cat. # 49-1505, Labels with numbers indicating material contained in pots. Fill 500 pots to rim with Metro Mix 200 to a weight of ~140g/pot. Pots are filled uniformly by using a balancer. Add 0.4g of Micro Max nutrients to each pot. Stir ingredients with spatula to a depth of 3 inches while preventing material loss.

(2) Planting a NUE selection in the Greenhouse

(a) Seed Germination - Each pot is lightly atered twice using reverse osmosis purified water. The first watering is scheduled to occur just before planting; and the second watering, after the seed has been planted in the pot. Ten Seeds of each entry (1 seed per pot) are planted to select eight healthy uniform seedlings. Additional wild type controls are planted for use as border rows. Alternatively, 15 seeds of each entry (1 seed per pot) are planted to select 12 healthy uniform seedlings (this larger number of plantings is used for the second, or confirmation, planting). Place pots on each of the 12 shelves in the Conviron growth chamber for seven days. This is done to allow more uniform germination and early seedling growth. The following growth chamber settings are 25° C/day and 22° C/night, 14 hours light and ten hours dark, humidity ~ 80%, and light intensity ~350 $\mu$mol/m$^2$/s (at pot level). Watering is done via capillary matting similar to greenhouse benches with duration of ten minutes three times a day.
(b) Seedling transfer - After seven days, the best eight or 12 seedlings for the first or confirmation pass runs, respectively, are chosen and transferred to greenhouse benches. The pots are spaced eight inches apart (center to center) and are positioned on the benches using the spacing patterns printed on the capillary matting. The Vattex matting creates a 384-position grid, randomizing all range, row combinations. Additional pots of controls are placed along the outside of the experimental block to reduce border effects.
Plants are allowed to grow for 28 days under the low N run or for 23 days under the high N run. The macronutrients are dispensed in the form of a macronutrient solution (see composition below) containing precise amounts of N added (2mM $NH_4NO_3$ for limiting N selection and 20mM $NH_4NO_3$ for high N selection runs). Each pot is manually dispensed 100ml of nutrient solution three times a week on alternate days starting at eight and ten days after planting for high N and low N runs, respectively. On the day of nutrient application, two 20 min waterings at 05:00 and 13:00 are skipped. The vattex matting should be changed every third run to avoid N accumulation and buildup of root matter. Table 7 shows the amount of nutrients in the nutrient solution for either the low or high nitrogen selection.

Table 22

| | 2mM $NH_4NO_3$ (Low Nitrogen Growth Condition, Low N) | 20mM $NH_4NO_3$ (high Nitrogen Growth Condition, High N) |
|---|---|---|
| Nutrient Stock | mL/L | mL/L |
| 1 M $NH_4NO_3$ | 2 | 20 |
| 1 M $KH_2PO_4$ | 0.5 | 0.5 |
| 1 M $MgSO_4.7H_2O$ | 2 | 2 |
| 1 M $CaCl_2$ | 2.5 | 2.5 |
| 1 M $K_2SO_4$ | 1 | 1 |
| Note: Adjust pH to 5.6 with HCl or KOH | | |

(c) Harvest Measurements and Data Collection - After 28 days of plant growth for low N runs and 23 days of plant growth for high N runs, the following measurements are taken (phenocodes in parentheses): total shoot fresh mass (g) (SFM) measured by Sartorius electronic balance, V6 leaf chlorophyll measured by Minolta SPAD meter (relative units) (LC), V6 leaf area ($cm^2$) (LA) measured by a Li-Cor leaf area meter, V6 leaf fresh mass (g) (LFM) measured by Sartorius electronic balance, and V6 leaf dry mass (g) (LDM) measured by Sartorius electronic balance. Raw data were analyzed by SAS software. Results shown are the comparison of transgenic plants relative to the wildtype controls.

To take a leaf reading, samples were excised from the V6 leaf. Since chlorophyll meter readings of corn leaves are affected by the part of the leaf and the position of the leaf on the plant that is sampled, SPAD meter readings were done on leaf six of the plants. Three measurements per leaf were taken, of which the first reading was taken from a point one-half the distance between the leaf tip and the collar and halfway from the leaf margin to the midrib while two were taken toward the leaf tip. The measurements were restricted in the area from 1/2 to 3/4 of the total length of the leaf (from the base) with approximately equal spacing between them. The average of the three measurements was taken from the SPAD machine.

Leaf fresh mass is recorded for an excised V6 leaf, the leaf is placed into a paper bag. The paper bags containing the leaves are then placed into a forced air oven at 80° C for 3 days. After 3 days, the paper bags are removed from the oven and the leaf dry mass measurements are taken.

From the collected data, two derived measurements are made: (1)Leaf chlorophyll area (LCA), which is a product of V6 relative chlorophyll content and its leaf area (relative units). Leaf chlorophyll area= leaf chlorophyll X leaf area. This parameter gives an indication of the spread of chlorophyll over the entire leaf area; (2)specific leaf area (LSA) is calculated as the ratio of V6 leaf area to its dry mass ($cm^2$/g dry mass), a parameter also recognized as a measure of NUE.

Nitrogen use field efficacy assay

Level I. Transgenic plants provided by the present invention are planted in field without any nitrogen source being applied. Transgenic plants and control plants are grouped by genotype and construct with controls arranged randomly within genotype blocks. Each type of transgenic plants are tested by 3 replications and across 5 locations. Nitrogen levels in the fields are analyzed in early April pre-planting by collecting 30 sample soil cores from 0-24" and 24 to 48" soil layer. Soil samples are analyzed for nitrate-nitrogen, phosphorus(P), Potassium(K), organic matter and pH to provide baseline values. P, K and micronutrients are applied based upon soil test recommendations.

Level II. Transgenic plants provided by the present invention are planted in field with three levels of nitrogen (N) fertilizer being applied, i.e. low level (0 N), medium level (80 lb/ac) and high level (180 lb/ac). Liquid 28% or 32% UAN (Urea, Ammonium Nitrogen) are used as the N source and apply by broadcast boom and incorporate with a field cultivator with rear rolling basket in the same direction as intended crop rows. Although there is no N applied to the 0 N treatment the soil should still be disturbed in the same fashion as the treated area. Transgenic plants and control plants are grouped by genotype and construct with controls arranged randomly within genotype blocks. Each type of transgenic plants is tested by 3 replications and across 4 locations. Nitrogen levels in the fields are analyzed in early April pre-planting by collecting 30 sample soil cores from 0-24" and 24 to 48" soil layer. Soil samples are analyzed for nitrate-nitrogen, phosphorus(P), Potassium(K), organic matter and pH to provide baseline values. P, K and micronutrients are applied based upon soil test recommendations.

**Selection for increased yield**

Many transgenic plants of this invention exhibit improved yield as compared to a control plant. Improved yield can result from enhanced seed sink potential, i.e. the number and size of endosperm cells or kernels and/or enhanced sink strength, i.e. the rate of starch biosynthesis. Sink potential can be established very early during kernel development, as endosperm cell number and size are determined within the first few days after pollination.

Much of the increase in corn yield of the past several decades has resulted from an increase in planting density. During that period, corn yield has been increasing at a rate of 2.1 bushels/acre/year, but the planting density has increased at a rate of 250 plants/acre/year. A characteristic of modem hybrid corn is the ability of these varieties to be planted at high density. Many studies have shown that a higher than current planting density should result in more biomass production, but current germplasm does not perform. well at these higher densities. One approach to increasing yield is to increase harvest index (HI), the proportion of biomass that is allocated to the kernel compared to total biomass, in high density plantings. Effective yield selection of enhanced yielding transgenic corn events uses hybrid progeny of the transgenic event over multiple locations with plants grown under optimal production management practices, and maximum pest control. A useful target for improved yield is a 5% to 10% increase in yield as compared to yield produced by plants grown from seed for a control plant. Selection methods may be applied in multiple and diverse geographic locations, for example up to 16 or more locations, over one or more planting seasons, for example at least two planting seasons to statistically distinguish yield improvement from natural environmental effects. It is to plant multiple transgenic plants, positive and negative control plants, and pollinator plants in standard plots, for example 2 row plots, 20 feet long by 5 feet wide with 30 inches distance between rows and a 3 foot alley between ranges. Transgenic events can be grouped by recombinant DNA constructs with groups randomly placed in the field. A pollinator plot of a high quality corn line is planted for every two plots to allow open pollination when using male sterile transgenic events. A useful planting density is about 30,000 plants/acre. High planting density is greater than 30,000 plants/acre, preferably about 40,000 plants/acre, more preferably about 42,000 plants/acre, most preferably about 45,000 plants/acre. Transgenic corn plants and soybean plants with each recombinant DNA construct prepared in Examples 5 and 6 are identified as exhibiting at least 5% yield increase as compared to control plants.

**Selection for enhanced water use efficiency (WUE)**

Described in this example is a high-throughput method for greenhouse selection of transgenic corn plants to wild type corn plants (tested as inbreds or hybrids) for water use efficiency. This selection process imposes 3 drought/re-water cycles on plants over a total period of 15 days after an initial stress free growth period of 11 days. Each cycle consists of 5 days, with no water being applied for the first four days and a water quenching on the 5th day of the cycle. The primary phenotypes analyzed by the selection method are the changes in plant growth rate as determined by height and biomass during a vegetative drought treatment. The hydration status of the shoot tissues following the drought is also measured. The plant height are measured at three time points. The first is taken just prior to the onset drought when the plant is 11 days old, which is the shoot initial height (SIH). The plant height is also measured halfway throughout the drought/re-water regimen, on day 18 after planting, to give rise to the shoot mid-drought height (SMH). Upon the completion of the final drought cycle on day 26 after planting, the shoot portion of the plant is harvested and measured for a final height, which is the shoot wilt height (SWH) and also measured for shoot wilted biomass (SWM). The shoot is placed in water at 40 degree Celsius in the dark. Three days later, the shoot is weighted to give rise to the shoot turgid weight (STM). After drying in an oven for four days, the shoots are weighted for shoot dry biomass (SDM). The shoot average height (SAH) is the mean plant height across the 3 height measurements. The procedure described above may be adjusted for +/- ~one day for each step given the situation.

To correct for slight differences between plants, a size corrected growth value is derived from SIH and SWH. This is the Relative Growth Rate (RGR). Relative Growth Rate (RGR) is calculated for each shoot using the formula [RGR% = (SWH-SIH)/((SWH+SIH)/2)* 100]. Relative water content (RWC) is a measurement of how much (%) of the plant was water at harvest. Water Content (RWC) is calculated for each shoot using the formula [RWC% = (SWM-SDM)/(STM-SDM)*100]. Fully watered corn plants of this age run around 98% RWC.

**Selection for Growth Under Cold Stress**

(1) Cold germination assay - Three sets of seeds are used for the assay. The first set consists of positive transgenic events (F1 hybrid) where the genes of the present invention are expressed in the seed. The second seed set is nontransgenic, wild-type negative control made from the same genotype as the transgenic events. The third set consisted of two cold tolerant and one cold sensitive commercial check lines of corn. All seeds are treated with a fungicide " Captan" (MAESTRO® 80DF Fungicide, Arvesta Corporation, San Francisco, CA, USA). 0.43 mL Captan is applied per 45 g of corn seeds by mixing it well and drying the fungicide prior to the experiment.

Corn kernels are placed embryo side down on blotter paper within an individual cell (8.9 x 8.9 cm) of a germination tray (54 x 36 cm). Ten seeds from an event are placed into one cell of the germination tray. Each tray can hold 21 transgenic events and 3 replicates of wildtype (LH244SDms+LH59), which is randomized in a complete block design. For every event there are five replications (five trays). The trays are placed at 9.7C for 24 days (no light) in a Convrion growth chamber *(Conviron Model PGV36, Controlled Environments, Winnipeg, Canada).* Two hundred and fifty millilters of deionized water are added to each germination tray. Germination counts are taken 10th, 11th, 12th, 13th, 14th, 17th, 19th, 21st, and 24th day after start date of the experiment. Seeds are considered germinated if the emerged radicle size is1 cm. From the germination counts germination index is calculated.

*The germination index is calculated as per:*

$$Germination\ index = (\Sigma\ ([T+1-n_i]*[P_i-P_{i-1}]))/T$$

Where T is the total number of days for which the germination assay is performed. The number of days after planting is defined by n. "i" indicated the number of times the germination had been counted, including the current day. P is the percentage of seeds germinated during any given rating. Statistical differences are calculated between transgenic events and wild type control. After statistical analysis, the events that show a statistical significance at the p level of less than 0.1 relative to wild-type controls will advance to a secondary cold selection. The secondary cold screen is conducted in the same manner of the primary selection only increasing the number of repetitions to ten. Statistical analysis of the data from the secondary selection is conducted to identify the events that show a statistical significance at the p level of less than 0.05 relative to wild-type controls.

(2) Cold Shock assay - The experimental set-up for the cold shock assay is the same as described in the above cold germination assay except seeds were grown in potted media for the cold shock assay.

The desired numbers of 2.5" square plastic pots are placed on flats (n=32, 4x8). Pots were filled with Metro Mix 200 soil-less media containing 19:6:12 fertilizer (6 lbs/cubic yard) (Metro Mix, Pots and Flat are obtained from Hummert International, Earth City, MO). After planting seeds, pots are placed in a growth chamber set at 23° C, relative humidity of 65% with 12 hour day and night photoperiod (300 uE/m2-min). Planted seeds are watered for 20 minute every other day by sub-irrigation and flats were rotated every third day in a growth chamber for growing corn seedlings.

On the 10th day after planting the transgenic positive and wild-type negative (WT) plants are positioned in flats in an alternating pattern. Chlorophyll fluorescence of plants is measured on the 10th day during the dark period of growth by using a PAM-2000 portable fluorometer as per the manufacturer's instructions (Walz, Germany). After chlorophyll measurements, leaf samples from each event are collected for confirming the expression of genes of the present invention. For expression analysis six V1 leaf tips from each selection are randomly harvested. The flats are moved to a growth chamber set at 5° C. All other conditions such as humidity, day/night cycle and light intensity are held constant in the growth chamber. The flats are subirrigated every day after transfer to the cold temperature. On the 4th day chlorophyll fluorescence is measured. Plants are transferred to normal growth conditions after six days of cold shock treatment and allowed to recover for the next three days. During this recovery period the length of the V3 leaf is measured on the 1st and 3rd days. After two days of recovery V2 leaf damage is determined visually by estimating percent of green V2 leaf.

Statistical differences in V3 leaf growth, V2 leaf necrosis and fluorescence during preshock and cold shock can be used for estimation of cold shock damage on corn plants. (3) Early seedling growth assay - Three sets of seeds are used for the experiment. The first set consists of positive transgenic events (F1 hybrid) where the genes of the present invention are expressed in the seed. The second seed set is nontransgenic, wild-type negative control made from the same genotype as the transgenic events. The third seed set consists of two cold tolerant and two cold sensitive commercial check lines of corn. All seeds are treated with a fungicide " Captan", (3a,4,7,a-tetrahydro-2-[(trichloromethly)thio]-1H-isoindole-1,3(2H)-dione, Drex Chemical Co. Memphis, TN). Captan (0.43 mL) was applied per 45 g of corn seeds by mixing it well and drying the fungicide prior to the experiment.

Seeds are grown in germination paper for the early seedling growth assay. Three 12"x18" pieces of germination paper (Anchor Paper #SD7606) are used for each entry in the test (three repetitions per transgenic event). The papers are wetted in a solution of 0.5% $KNO_3$ and 0.1 % Thyram.

For each paper fifteen seeds are placed on the line evenly spaced down the length of the paper. The fifteen seeds are positioned on the paper such that the radical would grow downward, for example longer distance to the paper's edge. The wet paper is rolled up starting from one of the short ends. The paper is rolled evenly and tight enough to hold the seeds in place. The roll is secured into place with two large paper clips, one at the top and one at the bottom. The rolls are incubated in a growth chamber at 23° C for three days in a randomized complete block design within an appropriate container. The chamber is set for 65% humidity with no light cycle. For the cold stress treatment the rolls are then incubated in a growth chamber at 12° C for twelve days. The chamber is set for 65% humidity with no light cycle.

After the cold treatment the germination papers are unrolled and the seeds that did not germinate are discarded. The lengths of the radicle and coleoptile for each seed are measured through an automated imaging program that automatically collects and processes the images. The imaging program automatically measures the shoot length, root length, and whole seedling length of every individual seedling and then calculates the average of each roll.

After statistical analysis, the events that show a statistical significance at the p level of less than 0.1 relative to wild-type controls will advance to a secondary cold selection. The secondary cold selection is conducted in the same manner of the primary selection only increasing the number of repetitions to five. Statistical analysis of the data from the secondary selection is conducted to identify the events that show a statistical significance at the p level of less than 0.05 relative to wild-type controls.

4. Cold field efficacy trial

This example sets forth a cold field efficacy trial to identify gene constructs that confer enhanced cold vigor at germination and early seedling growth under early spring planting field conditions in conventional-till and simulated no-till environments. Seeds are planted into the ground around two weeks before local farmers are beginning to plant corn so that a significant cold stress is exerted onto the crop, named as cold treatment. Seeds also are planted under local optimal planting conditions such that the crop has little or no exposure to cold condition, named as normal treatment. The cold field efficacy trials are carried out in five locations, including Glyndon MN, Mason MI, Monmouth IL, Dayton IA, Mystic CT. At each location, seeds are planted under both cold and normal conditions with 3 repetitions per treatment, 20 kernels per row and single row per plot. Seeds are planted 1.5 to 2 inch deep into soil to avoid muddy conditions. Two temperature monitors are set up at each location to monitor both air and soil temperature daily.

Seed emergence is defined as the point when the growing shoot breaks the soil surface. The number of emerged seedling in each plot is counted everyday from the day the earliest plot begins to emerge until no significant changes in emergence occur. In addition, for each planting date, the latest date when emergence is 0 in all plots is also recorded. Seedling vigor is also rated at V3-V4 stage before the average of corn plant height reaches 10 inches, with 1=excellent early growth, 5=Average growth and 9=poor growth. Days to 50% emergence, maximum percent emergence and seedling vigor are calculated using SAS software for the data within each location or across all locations.

**Screens for transgenic plant seeds with increased protein and/or oil levels**

This example sets forth a high-throughput selection for identifying plant seeds with improvement in seed composition using the Infratec 1200 series Grain Analyzer, which is a near-infrared transmittance spectrometer used to determine the composition of a bulk seed sample. Near infrared analysis is a non-destructive, high-throughput method that can analyze multiple traits in a single sample scan.. An NIR calibration for the analytes of interest is used to predict the values of an unknown sample. The NIR spectrum is obtained for the sample and compared to the calibration using a complex chemometric software package that provides a predicted values as well as information on how well the sample fits in the calibration.

Infratec Model 1221, 1225, or 1227 with transport module by Foss North America is used with cuvette, item # 1000-4033, Foss North America or for small samples with small cell cuvette, Foss standard cuvette modified by Leon Girard Co. Corn and soy check samples of varying composition maintained in check cell cuvettes are supplied by Leon Girard Co. NIT collection software is provided by Maximum Consulting Inc.Software. Calculations are performed automatically by the software. Seed samples are received in packets or containers with barcode labels from the customer. The seed is poured into the cuvettes and analyzed as received.

Table 26.

| Typical sample(s): | Whole grain corn and soybean seeds |
|---|---|
| Analytical time to run method: | Less than 0.75 min per sample |
| Total elapsed time per run: | 1.5 minute per sample |
| Typical and minimum sample size: | Corn typical: 50cc; minimum 30cc Soybean typical: 50cc; minimum 5cc |

(continued)

| Typical analytical range: | Determined in part by the specific calibration. |
|---|---|
| | Corn - moisture 5-15%, oil 5-20%, protein 5-30%, starch 50-75%, and density 1.0-1.3%. |
| | Soybean - moisture 5-15%, oil 15-25%, and protein 35-50%. |

**Example 8.**

This example describes recombinant DNA constructs of the invention, useful for suppressing the expression of a protein identified by Pfam, Catalase, Bromdomain, FTCD_N, MatE, DPBB_1, tRNA-synt_2b, Sugar_tr and MFS_1, DUF6 and DUF250, LEA_4, MIP or DUF231, in a corn or soybean plant, by expressing a sense and an anti-sense fragment of the native DNA encoding the protein, essentially as described in U.S. Patent Application Serial No. 11/303,745, incorporated herein by reference. Specific gene suppression constructs are targeted to the native gene in corn and soybean plants that are homologs of the genes encoding the protein with an amino acid sequence of SEQ ID NO:213, 215, 218, 222, 258, 269, 275, 334, 361, 368, and 407.

The constructs include a promoter operably linked to DNA that transcribes to RNA that forms a double stranded RNA in transgenic plant cells for suppressing expression of the protein to provided the enhanced trait in the corn and soybean plants. Populations of transgenic plants and seeds derived from the plant cells are screened to identify those plants exhibiting the enhanced traits associated with suppression of those genes.

A plant cell with stably integrated, recombinant DNA comprising a promoter that is functional in plant cells and that is operably linked to DNA from a plant, bacteria or yeast that encodes a protein having at least one domain of amino acids in a sequence that exceeds the Pfam gathering cutoff of 16.9 for amino acid sequence alignment with a protein domain family identified by Pfam name, RNA_pol_L; wherein said plant cell is selected from a population of plant cells with said recombinant DNA by screening plants that are regenerated from plant cells in said population and that express said protein for an enhanced trait as compared to control plants that do not have said recombinant DNA; and wherein said enhanced trait is selected from group of enhanced traits consisting of enhanced water use efficiency, enhanced cold tolerance, increased yield, enhanced nitrogen use efficiency, enhanced seed protein and enhanced seed oil. A plant cell with stably integrated, recombinant DNA comprising a promoter that is functional in plant cells and that is operably linked to DNA from a plant, bacteria or yeast that encodes a protein having at least one domain of amino acids in a sequence that exceeds the Pfam gathering cutoff for amino acid sequence alignment with a protein domain family identified by a Pfam name in the group of Pfam names consisting of 2-oxoacid_dh, ADH_N, ADH_zinc_N, AP2, AUX_IAA, Aa_trans, Abhydrolase_1, Acyl_transf_1, Aldedh, Aldo_ket_red, Alpha-amylase, Aminotran_1_2, Aminotran_3, Ammonium_transp, Arm, Asn_synthase, BAG, BSD, Beta_elim_lyase, Biotin_lipoyl, Brix, Bromodomain, C1_4, CTP_transf_2, Catalase, CcmH, Chal_sti_synt_C, Cyclin_C, Cyclin_N, Cys_Met_Meta_PP, DAO, DIM1, DPBB_1, DRMBL, DUF167, DUF231, DUF250, DUF6, DUF783, DUF962, E2F_TDP, E3_binding, EBP, Enolase_C, Enolase_N, F420_oxidored, FAD_binding_2, FA_desaturase, FKBP_C, FTCD_N, Fe_bilin_red, Fer4, GAF, GATase_2, GIDA, GSHPx, Gpi16, HGTP_anticodon, HI0933_like, HLH, HMG_CoA_synt, HWE_HK, Ham1p_like, HhH-GPD, Homeobox, Hpt, Iso_dh, K-box, LEA 4, LRRNT_2, LRR_1, Ldh_1_C, Ldh_1_N, Lectin_legA, Lectin_legB, Lipase_GDSL, MFS_1, MIP, MatE, Metalloenzyme, Methyltransf_11, Methyltransf_12, Molybdop_Fe4S4, Molybdopterin, Molydop_binding, Mov34, MtN3_slv, Myb_DNA-binding, NAD_Gly3P_dh_N, NAD_binding_2, NIR_SIR, NIR_SIR_ferr, NPH3, NTP_transferase, Nuc_sug_transp, PA, PAR1, PFK, PGI, PGK, PGM_PMM_I, PGM_PMM_II, PGM_PMM_III, PGM_PMM_IV, PP2C, PTR2, Peptidase_C26, Phi_1, Phytochrome, Pkinase, Pkinase_Tyr, Pollen_allerg_1, Pribosyltran, Proteasome, Pyr_redox, Pyr_redox_2, Pyr_redox_dim, RNA_pol_L, RNA_pol_Rpb6, RRM_1, RRN3, Radical_SAM, Ras, Response_reg, Rhodanese, Ribosomal_S8e, Rieske, SAC3_GANP, SBDS, SET, SRF-TF, SURF5, Skp1, Skp1_POZ, Ssl1, Sterol_desat, Sugar_tr, TCP, ThiF, Transaldolase, UQ_con, Ubie_methyltran, WD40, WRKY, adh_short, bZIP_1, bZIP_2, cNMP_binding, iPGM_N, p450, tRNA-synt_2b, ubiquitin, zf-A20, zf-AN1, zf-B_box, zf-C2H2, zf-C3HC4, zf-CCCH wherein the Pfam gathering cuttoff for said protein domain families is stated in Table 16 ; wherein said plant cell is selected from a population of plant cells with said recombinant DNA by screening plants that are regenerated from plant cells in said population and that express said protein for an enhanced trait as compared to control plants that do not have said recombinant DNA; and wherein said enhanced trait is selected from group of enhanced traits consisting of enhanced water use efficiency, enhanced cold tolerance, enhanced heat tolerance, enhanced resistance to salt exposure, enhanced shade tolerance, increased yield, enhanced nitrogen use efficiency, enhanced seed protein and enhanced seed oil.

A plant cell as defined above, wherein said protein has an amino acid sequence with at least 90% identity to a consensus amino acid sequence in the group of consensus amino acid sequences consisting of the consensus amino acid sequence constructed for SEQ ID NO: 205 and homologs thereof listed in Table 2 through the consensus amino acid sequence constructed for SEQ ID NO:408 and homologs thereof listed in Table 2.

A plant cell as defined above, wherein said protein is selected from the group of proteins identified in Table 1.

A plant cell as defined above, further comprising DNA expressing a protein that provides tolerance from exposure to an herbicide applied at levels that are lethal to a wild type of said plant cell.

A plant cell as defined above, wherein the agent of said herbicide is a glyphosate, dicamba, or glufosinate compound.

A transgenic plant comprising a plurality of the plant cells of as defined above.

A transgenic plant as defined above, which is homozygous for said recombinant DNA.

A transgenic seed comprising a pluarility of the plant cell as defined above.

A transgenic seed as defined above from a corn, soybean, cotton, canola, alfalfa, wheat or rice plant.

Non-natural, transgenic corn seed as defined above, wherein said seed can produce corn plants that are resistant to disease from the Mal de Rio Cuarto virus or the Puccina sorghi fungus or both.

A transgenic pollen grain comprising a haploid derivative of a plant cell as defined above.

A method for manufacturing non-natural, transgenic seed that can be used to produce a crop of transgenic plants with an enhanced trait resulting from expression of stably-integrated, recombinant DNA comprising a promoter that is (a) functional in plant cells and (b) is operably linked to DNA from a plant, bacteria or yeast that encodes a protein having at least one domain of amino acids in a sequence that exceeds the Pfam gathering cutoff for amino acid sequence alignment with a protein domain family identified by a Pfam name in the group of Pfam names consisting of 2-oxoacid_dh, ADH_N, ADH_zinc_N, AP2, AUX_IAA, Aa_trans, Abhydrolase_1, Acyl_transf_1, Aldedh, Aldo_ket_red, Alpha-amylase, Aminotran_1_2, Aminotran_3, Ammonium_transp, Arm, Asn_synthase, BAG, BSD, Beta_elim_lyase, Biotin_lipoyl, Brix, Bromodomain, C1_4, CTP_transf_2, Catalase, CcmH, Chal_sti_synt_C, Cyclin_C, Cyclin_N, Cys_Met_Meta_PP, DAO, DIM1, DPBB_1, DRMBL, DUF167, DUF231, DUF250, DUF6, DUF783, DUF962, E2F_TDP, E3_binding, EBP, Enolase_C, Enolase_N, F420_oxidored, FAD_binding_2, FA_desaturase, FKBP_C, FTCD_N, Fe_bilin_red, Fer4, GAF, GATase_2, GIDA, GSHPx, Gpi16, HGTP_anticodon, HI0933_like, HLH, HMG_CoA_synt, HWE_HK, Ham1p_like, HhH-GPD, Homeobox, Hpt, Iso_dh, K-box, LEA_4, LRRNT_2, LRR_1, Ldh_1_C, Ldh_1_N, Lectin_legA, Lectin_legB, Lipase_GDSL, MFS_1, MIP, MatE, Metalloenzyme, Methyltransf_11, Methyltransf_12, Molybdop_Fe4S4, Molybdopterin, Molydop_binding, Mov34, MtN3_slv, Myb_DNA-binding, NAD_Gly3P_dh_N, NAD_binding_2, NIR_SIR, NIR_SIR_ferr, NPH3, NTP_transferase, Nuc_sug_transp, PA, PAR1, PFK, PGI, PGK, PGM_PMM_I, PGM_PMM_II, PGM_PMM_III, PGM_PMM_IV, PP2C, PTR2, Peptidase_C26, Phi_1, Phytochrome, Pkinase, Pkinase_Tyr, Pollen_allerg_1, Pribosyltran, Proteasome, Pyr_redox, Pyr_redox_2, Pyr_redox_dim, RNA_pol_L, RNA_pol_Rpb6, RRM_1, RRN3, Radical_SAM, Ras, Response_reg, Rhodanese, Ribosomal_S8e, Rieske, SAC3_GANP, SBDS, SET, SRF-TF, SURF5, Skp1, Skp1_POZ, Ssl1, Sterol_desat, Sugar_tr, TCP, ThiF, Transaldolase, UQ_con, Ubie_methyltran, WD40, WRKY, adh_short, bZIP_1, bZIP_2, cNMP_binding, iPGM_N, p450, tRNA-synt_2b, ubiquitin, zf-A20, zf-AN1, zf-B_box, zf-C2H2, zf-C3HC4, zf-CCCH; wherein the gathering cutoff for said protein domain families is stated in Table 16; and wherein said enhanced trait is selected from the group of enhanced traits consisting of enhanced traits consisting of enhanced water use efficiency, enhanced cold tolerance, enhanced heat tolerance, enhanced resistance to salt exposure, enhanced shade tolerance, increased yield, enhanced nitrogen use efficiency, enhanced seed protein and enhanced seed oil, said method for manufacturing said seed comprising:

(a) screening a population of plants for said enhanced trait and said recombinant DNA, wherein individual plants in said population can exhibit said trait at a level less than, essentially the same as or greater than the level that said trait is exhibited in control plants which do not express the recombinant DNA,
(b) selecting from said population one or more plants that exhibit the trait at a level greater than the level that said trait is exhibited in control plants,
(c) verifying that said recombinant DNA is stably integrated in said selected plants,
(d) analyzing tissue of a selected plant to determine the production of a protein having the function of a protein encoded by nucleotides in a sequence of one of SEQ ID NO:205-408; and
(e) collecting seed from a selected plant.

A method as defined above, wherein plants in said population further comprise DNA expressing a protein that provides tolerance to exposure to an herbicide applied at levels that are lethal to wild type plant cells, and wherein said selecting is effected by treating said population with said herbicide.

A method as defined above, wherein said herbicide comprises a glyphosate, dicamba, or glufosinate compound.

A method as defined above, wherein said selecting is effected by identifying plants with said enhanced trait.

A method as defined above, wherein said seed is corn, soybean, cotton, alfalfa, wheat or rice seed.

A method of producing hybrid corn seed comprising:

(a) acquiring hybrid corn seed from a herbicide tolerant corn plant which also has stably-integrated, recombinant DNA comprising a promoter that is (a) functional in plant cells and (b) is operably linked to DNA that encodes a protein having at least one domain of amino acids in a sequence that exceeds the Pfam gathering cutoff for amino acid sequence alignment with a protein domain family identified by a Pfam name in the group of Pfam names consisting of 2-oxoacid_dh, ADH_N, ADH_zinc_N, AP2, AUX_IAA, Aa_trans, Abhydrolase_1, Acyl_transf_1, Aldedh, Aldo_ket_red, Alpha-amylase, Aminotran_1_2, Aminotran_3, Ammonium transp, Arm, Asn_synthase, BAG, BSD, Beta_elim_lyase, Biotin_lipoyl, Brix, Bromodomain, C1_4, CTP_transf_2, Catalase, CcmH, Chal_sti_synt_C, Cyclin_C, Cyclin_N, Cys_Met_Meta_PP, DAO, DIM1, DPBB_1, DRMBL, DUF167, DUF231, DUF250, DUF6, DUF783, DUF962, E2F_TDP, E3_binding, EBP, Enolase_C, Enolase_N, F420_oxidored, FAD_binding_2, FA_desaturase, FKBP_C, FTCD_N, Fe_bilin_red, Fer4, GAF, GATase_2, GIDA, GSHPx, Gpi16, HGTP_anticodon, HI0933_like, HLH, HMG_CoA_synt, HWE_HK, Ham1p_like, HhH-GPD, Homeobox, Hpt, Iso_dh, K-box, LEA_4, LRRNT_2, LRR_1, Ldh_1_C, Ldh_1_N, Lectin_legA, Lectin_legB, Lipase_GDSL, MFS_1, MIP, MatE, Metalloenzyme, Methyltransf_11, Methyltransf_12, Molybdop_Fe4S4, Molybdopterin, Molydop_binding, Mov34, MtN3_slv, Myb_DNA-binding, NAD_Gly3P_dh_N, NAD_binding_2, NIR_SIR, NIR_SIR_ferr, NPH3, NTP_transferase, Nuc_sug_transp, PA, PAR1, PFK, PGI, PGK, PGM_PMM_I, PGM_PMM_II, PGM_PMM_III, PGM_PMM_IV, PP2C, PTR2, Peptidase_C26, Phi_1, Phytochrome, Pkinase, Pkinase_Tyr, Pollen_allerg_1, Pribosyltran, Proteasome, Pyr_redox, Pyr_redox_2, Pyr_redox_dim, RNA_pol_L, RNA_pol_Rpb6, RRM_1, RRN3, Radical_SAM, Ras, Response_reg, Rhodanese, Ribosomal_S8e, Rieske, SAC3_GANP, SBDS, SET, SRF-TF, SURF5, Skp1, Skp1_POZ, Ssl1, Sterol_desat, Sugar_tr, TCP, ThiF, Transaldolase, UQ_con, Ubie_methyltran, WD40, WRKY, adh_short, bZIP_1, bZIP_2, cNMP_binding, iPGM_N, p450, tRNA-synt_2b, ubiquitin, zf-A20, zf-AN1, zf-B_box, zf-C2H2, zf-C3HC4, zf-CCCH; wherein the gathering cuttoff for said protein domain families is stated in Table 16;

(b) producing corn plants from said hybrid corn seed, wherein a fraction of the plants produced from said hybrid corn seed is homozygous for said recombinant DNA, a fraction of the plants produced from said hybrid corn seed is hemizygous for said recombinant DNA, and a fraction of the plants produced from said hybrid corn seed has none of said recombinant DNA;

(c) selecting corn plants which are homozygous and hemizygous for said recombinant DNA by treating with an herbicide;

(d) collecting seed from herbicide-treated-surviving corn plants and planting said seed to produce further progeny corn plants;

(e) repeating steps (c) and (d) at least once to produce an inbred corn line;

(f) crossing said inbred corn line with a second corn line to produce hybrid seed.

The method of selecting a plant comprising cells as defined above, wherein an immunoreactive antibody is used to detect the presence of said protein in seed or plant tissue.

Anti-counterfeit milled seed having, as an indication of origin, a plant cell as defined above,.

A method of growing a corn, cotton or soybean crop without irrigation water comprising planting seed having plant cells as defined above, which are selected for enhanced water use efficiency.

A method as defined above comprising providing up to 300 millimeters of ground water during the production of said crop.

A method of growing a corn, cotton or soybean crop without added nitrogen fertilizer comprising planting seed having plant cells as defined above, which are selected for enhanced nitrogen use efficiency.

A plant cell with stably integrated, recombinant DNA that is to suppress the level of an endogenous protein having at least one domain of amino acids in a sequence that exceeds that Pfam gathering cutoff for amino acid sequence alignment with a protein domain family identified by Pfam name in the group of Pfam names consisting of Catalase, Bromodomain, FTCD_N, MatE, DPBB_1, Pollen_allerg_1, tRNA-synt_2b, HGTP_anticodon, Sugar_tr, MFS_1, DUF6, DUF250, LEA_4, MIP, and DUF231 wherein the Pfam gathering cutoff for said protein domain families is stated in Table 16; wherein said plant cells is selected from a population of plant cells with said recombinant DNA by screening plants that are regenerated from plant cells in said population and that the level of said protein is suppressed for an enhanced trait as compared to control plants that do not have said recombinant DNA; and wherein said enhanced trait is selected from the group of enhanced traits consisting of enhanced water use efficiency, enhanced cold tolerance, enhanced heat tolerance, enhanced resistance to salt exposure, enhanced shade tolerance, increased yield, enhanced nitrogen use efficiency, enhanced seed protein and enhanced seed oil.

**Claims**

1. A plant cell with stably integrated, recombinant DNA comprising a promoter that is functional in plant cells and that is operably linked to DNA from a plant, bacteria or yeast that encodes a protein having an amino acid sequence with at least 90% identity to a sequence of SEQ ID NO:220, wherein the plant cell is selected from a population of plant cells with the recombinant DNA by screening plants that are regenerated from plant cells in the population and that express the protein for an enhanced trait as compared to control plants that do not have the recombinant DNA, wherein the enhanced trait is selected from enhanced resistance to salt exposure, enhanced heat tolerance, enhanced cold tolerance, and increased yield.

2. The plant cell of claim 1 further comprising DNA expressing a protein that provides tolerance from exposure to an herbicide applied at levels that are lethal to a wild type of the plant cell, preferably the agent of the herbicide is a glyphosate, dicamba, or glufosinate compound.

3. A transgenic plant or a transgenic seed comprising a plurality of the plant cells of claim 1.

4. The transgenic plant of claim 3 which is homozygous for the recombinant DNA.

5. A transgenic seed of claim 3 which is from a corn, soybean, cotton, canola, alfalfa, wheat or rice plant.

6. Non-natural, transgenic corn seed of claim 7, wherein the seed can produce corn plants that are resistant to disease from the Mal de Rio Cuarto virus or the Puccina sorghi fungus or both.

7. A transgenic pollen grain comprising a haploid derivative of a plant cell of claim 1.

8. A method for manufacturing non-natural, transgenic seed that can be used to produce a crop of transgenic plants with an enhanced trait resulting from expression of stably-integrated, recombinant DNA comprising a promoter that is (a) functional in plant cells and (b) is operably linked to DNA from a plant, bacteria or yeast that encodes a protein having an amino acid sequence with at least 90% identity to a sequence of SEQ ID NO:220, and wherein the enhanced trait is enhanced cold tolerance, enhanced heat tolerance, enhanced resistance to salt exposure or enhanced yield, and the method comprising:

   (a) screening a population of plants for the enhanced trait and the recombinant DNA, wherein individual plants in the population can exhibit the trait at a level less than, essentially the same as or greater than the level that the trait is exhibited in control plants which do not express the recombinant DNA,
   (b) selecting from the population one or more plants that exhibit the trait at a level greater than the level that the trait is exhibited in control plants,
   c) verifying that the recombinant DNA is stably integrated in the selected plants,
   d) analyzing tissue of a selected plant to determine the production of a protein having the function of a protein encoded by nucleotides in the sequence of SEQ ID NO 220; and
   e) collecting seed from a selected plant.

9. The method of claim 8, wherein

   i) plants in the population further comprise DNA expressing a protein that provides tolerance to exposure to an herbicide applied at levels that are lethal to wild type plant cells, and wherein the selecting is effected by treating the population with the herbicide, preferably the herbicide comprises a glyphosate, dicamba, or glufosinate compound, or the seed is corn, soybean, cotton, alfalfa, wheat or rice seed; or
   ii) the selecting is effected by identifying plants with the enhanced trait.

10. A method of producing hybrid corn seed comprising:

   a) acquiring hybrid corn seed from a herbicide tolerant corn plant which also has stably-integrated, recombinant DNA comprising a promoter that is (a) functional in plant cells and (b) is operably linked to DNA that encodes a protein having an amino acid sequence with at least 90% identity to a sequence of one of SEQ ID NO:220;
   (b) producing corn plants from the hybrid corn seed, wherein a fraction of the plants produced from the hybrid corn seed is homozygous for the recombinant DNA, a fraction of the plants produced from the hybrid corn seed is hemizygous for the recombinant DNA, and a fraction of the plants produced from the hybrid corn seed has none of the recombinant DNA;
   (c) selecting corn plants which are homozygous and hemizygous for the recombinant DNA by treating with an herbicide;
   d) collecting seed from herbicide-treated-surviving corn plants and planting the seed to produce further progeny corn plants;

e) repeating steps (c) and (d) at least once to produce an inbred corn line;

f) crossing the inbred corn line with a second corn line to produce hybrid seed.

**11.** A method of selecting a plant comprising cells of claim 1 wherein an immunoreactive antibody is used to detect the presence of the protein in seed or plant tissue.

**12.** Anti-counterfeit milled seed having, as an indication of origin, a plant cell of claim 1.

**13.** A method of growing a corn, cotton or soybean crop without irrigation water comprising planting seed having plant cells of claim 1 which are selected for enhanced water use efficiency.

**14.** A method of claim 13 comprising providing up to 300 millimeters of ground water during the production of the crop.

**15.** A method of growing a corn, cotton or soybean crop without added nitrogen fertilizer comprising planting seed having plant cells of claim 1 which are selected for enhanced nitrogen use efficiency.

## Figure 1

```
SEQ ID NO:
406      :-----------------------------------------------MAKRQLLMLG----IRTSF
6984     :----------------------------------------------MAKAHQIVLGSNWGIRNIF
624      :-------------------------------MAPEERMKQTEGSHKVVTAAGGKMAVGVRSIV
3333     :----------------------------------MKQTEGSHKVVTAAGGKMAVGVRSIV
9382     :----------------------------------------------------------QQ
13337    :------------------------------------------------------------
15475    :------------------------------------------------------------
1464     :---------------------------------MKTEASHKAIAAGGGKMTVLHSPVGVRSIV
7412     :---------------------------------MKTEASHKAIAAGGGKMTVLHSPVGVRSIV
11818    :---------------------------------MKTEASHKAIAAGGGKMTVLHSPVGVRSIV
11760    :-------------------------------------------------RWEPAWRRART
11490    :------------------------------------------------------------
16367    :MWSALFSHLREVHKRSGVKEEKLIMKSPPAAGEAGCHKPQATATNKMTVLQSPLGLRTIL
10381    :------------------------------------MKPEQATHNKMTTAASSPSPVVGLRGVV
17305    :---------------------------------------------MTTTGSTPPRKNRSNV
8426     :------------------------------------------------------------
4667     :------------------------------------MMKPQHGGMAGHGGGRTRSPFLTSY
5355     :-----------------------------------------------MQRWKWNRKKPPIFPLL
consensus:---------------------------------------xxxxxxxxxxxxxxxxxxxxxxxxxxxxxx
```

```
HTIAAVLVAGLIFTAVFLSRNSLP-----------------------------------
QSLVAILTTILVVAAIYLTQEGEQ-----------------------------------
TSLVAFFILASSVVFLLLDREAG------------------------------------
TSLVAFFILASSVVFLLLDREAG------------------------------------
QQQVAFFILASSVVFLLLDREAG------------------------------------
-----------------------AG---------------------------------
----------------------------------------------------------
TSLVAFFILASSIVFLLDRGQEEQVQVAVEHGRQEVQVKL------------------
TSLVAFFILASSIVFLLDRGQEEQVQVAVEHGRQEVQVKL------------------
TSLVAFFILASSIVFLLDRGQEEQVQVAVEHGRQEVQVKL------------------
ARRCRWPRWCSRSSSSRRSSTTR-----------------------------------
----------------------------------------------------------
TSLVAFFIVVSSVSLLFDRGQDAQAQLAVEQHQHQEVLLKQKPASAAVGEQKSVVVDQSS
SSLVAFFIVVSSVSLLFDRGHESQVQLAVQHRHQEVKVAAAGRR--------------
TGGEGGSLEEYAWRAAGEAAAAKKATRAWGVSVSLRSHFSSLVLLLLLLLVALAVSATTK
----------------------------------------------------------
ALTLAFITFVSVLYFKDFSSTLHQPFLTRPPPHRRQIARPRAP---------------
VLILLFFIAFSTLHSEHTIQRIHENPDHVHNHQEVSSATFVKPNLSGHLKQAPEVLDRFS
xxxxxxxxxxxxxxxxxxxxxxxxxxxxxxxxxxxxxxxxxxxxxxxx-----------------
```

```
----------KENPQSHGVTDRGGDSGRECNLFEGKWVFDNVSYPLYKEEDCKFMSDQ
-----------WSNERNK------LHSLSKCNLFSGKWVFDNESYPLYKEHQCTFMSDQ
------------LQEEPAIRAGGG---DEECSWSRGRWVYDNVSRPLYDGLKCAFIFPE
-----------LQEESAIRAGGGGGGDEECSWSRGRWVYDNVSRPLYDGLKCAFIFPE
------------LQEEPAIRAGGGGG-DEECSWSRGRWVYDNVSRPLYDGLKCAFIFPE
------------LQEQSPITAGGGGSCDEECRWSRGPRVRDNVSRPLYDGLKCAFIFPE
```

```
------------------------EPAAAAGDEECSWSRGRWVYDNVSRPLYDGLKCAFIFPE
----------ESGLQEPAIRGTTQEGDASNEECNWSRGRWVYDNVSRPLYSGLKCSFIFPE
----------ESGLQEPAIRGTTQEGDASNEECNWSRGRWVYDNVSRPLYSGLKCSFIFPE
----------ESGLQEPAIRGTTQEGDASNEECNWSRGRWVYDNVSRPLYSGLKCSFIFPE
---------------TSSPTAALRGVVSVPQTCDLYRGSWVYDEVSAPVYKEGECEFLTEQ
---------MLRRNSPSSSISTKNGFDGEEDCNWSLGRWVYDNASRPLYSGLKCSFIFDE
LRSQEAQVQWTSELQDVATDSGDGGFDGEEDCNWSLGRWVYDNASRPLYSGLKCSFIFDE
----------EAQVQWTDELMGEAVRGSGEECNWSXGRWVYDNASQPLYSGLNCSFSFDE
NGDPAETPHAPPLPPPASIKLPSSSSSGGGECDLFSGRWVYDEAAYPLYRESACRVMSEQ
-----------------SIKLPSSSSSGGGECDLFSGRWVYDEAAYPLYRESACRVMSEQ
---SHHHGGGSSSGGGDVVPPPFAVGAAAAAGCDVGVGEWWVYDEAARPWYEEEECPYIQPQ
RCNSTVEYSGRKIAWLGDSRHSGHWSARPESCDVFSGKWVFDNVSHPLYNESDCPYMSDQ
----------xxxxxxxxxxxxxxxxxxxxxxxxCxxxxGxwVyDnxsxPlYxxxxCxfxxxx
```

```
LACEKFGRKDLSYKFWRWQPHT--CDLPRFNGTKLLERLRNKRMVYVGDSLNRGQWVSMV
LACEKFGRKDLSYQNWRWKPHQ--CDLPRFNATALLERLRNKRMVFVGDSLNRGQWVSMV
VACDKYGRKDVMYQHWRWQPHGHGCDLPRFDGIKLLEELRNKRMVFVGDSVNRNQWVSLV
VACDKYGRKDVMYQHWRWQPHGHGCDLPRFDGMKLLEELRNKRLVFVGDSVNRNQWVSLV
VACDKYGRKDVMYQHWRWQPHGHGCDLPRFDAMKLLEELRNKRLVFVGDSVNRNQWVSLV
VACDKYGRKDVMYQHWRWQPHGHGCDIPRFDGIKLLEELRNKRLVFVGDSVNRNQWVSLV
VACDKYGRKDVMYQHWRWQPHGHGCDLPRFDGIKLLEELRNKRLVFVGDSVNRNQWVSLV
VACDKYGRKDAMYQHWRWQPHGHGCDLPRFDAIKLLENLRNKRMVFVGDSVNRNQWVSLV
VACDKYGRKDAMYQHWRWQPHGHGCDLPRFDAIKLLENLRNKRMVFVGDSVNRNQWVSLV
VACDKYGRKDAMYQHWRWQPHGHGCDLPRFDAIKLLENLRNKRMVFVGDSVNRNQWVSLV
VTCMRNGRRDDSYQKWRWQPAG--CDLPRFDARLLLERLRNKRLMFVGDSLNRNQWESMV
VACDKYGRNDTKYQHWRWQPHG--CNLPRFNATKFLEKLRNKRLVFVGDSVNRNQWVSMV
VACDKYGRNDTKYQHWRWQPHG--CNLPRFNATKFLEKLRNKRLVFVGDSVNRNQWVSMV
VAXEKYGRNDTRYXYWRWQPDG--CDLSRFNATKLLEKLRNKRMVFVGDSINRNQWVSMV
SACEKYGRTDLRYQHWRWQPHG--CDLPRFDAEKFLGKLRNKRLVFVGDSLNRNQWASML
SACEKYGRTDLRYQHWRWQPHG--CDLPRFDAEKFLGKLRNKRLVFVGDSLNRNQWASML
LTCQAHGRPDTAYQHWRWQPRG--CSLPSFNATLMLEMLRGKRMMFVGDSLNRGQYVSLV
LACHKHGRSDLGYQYWRWQPHN--CNLKRWNVKEMWEKLRGKRLMFVGDSLNRGQWISMV
xacxkyGRxDxxYqhWRWqPhgxxCdlprfxxxkxlexLRnKRxvfVGDSxNRnQwvSxv
```

```
CMVSSVITN--PKAMYMHNNGSNLITFKALEYNATIDYYWAPLLVESNSDDPTNHRFPDR
CLVESSVPP--TLKSMRTIANGSLNIFKAEEYNATIEFYWAPLLVESNSDDPVXHRVAER
CMVEASIPD---DRLKIRTFNGSLISFKALEYNATIDFYWSPLLVESNSDNPIIHRVEYR
CMVEASIPD---DRLKMRIFNGSLISFKALEYNATIDFYWSPLLVESNSDNPIIHRVEYR
CMVEASIPD---DRLKMRIFNGSLISFKALEYNATIDFYWSPLLVESNSDNPIIHRVEYR
CMVEASIPD---DRLKMRIFNGSLISFKALEYNATIDFYWSPLLVESNSDNPIIHRVEYR
CMVEASIPD---DRLKRRIFNGSLISFKALEYNATIDFYWSPLLVESNSDNPIIHRVEYR
CMVEASIPD---DRLKIRTFNGSLISFKALEYNATIDFYWSPLLVESNSDNPIIHRVEYR
CMVEASIPD---DRLKIRTFNGSLISFKALEYNATIDFYWSPLLVESNSDNPIIHRVEYR
CMVEASIPD---DRLKIRTFNGSLISFKALEYNATIDFYWSPLLVESNSDNPIIHRVEYR
CLVQSVIPDRGQKTLTKFVNGGSSNVFYAHEYNATIDFYWSPLLVESNSDNPIIHRVEYR
CMVEHFIPD----GRKMRVYNGSLISFKAFEYNATIDFYWSPLLLESNSDNPIIHRVEYR
CMVEHFIPD----GRKMRVYNGSLISFKAFEYNATIDFYWSPLLLESNSDNPIIHRVEYR
CMVEASIPE----GQKMRVYNGSLISFTAFEYNATIDFYGPPLILESNSDNPIIHRVEYR
CLIDTGAPE----LHTSINSSRSLTTFKIHEYNASVDFYWSPLLVESNSDHPLRHRVADR
CLIDTGAPE----LHTSINSSRSLTTFKIHEYNASVDFYWSPLLVESNSDHPLRHRVADR
CLLHRSIPE----SSKSMETFDSLTVFRAKNYNATIEFYWAPFLAESNSDDAVVHRIADR
```

```
CLLQSVIPA----DKRSMSPNAHLTIFRAEEYNATVEFLWAPLLAESNSDDPVNHRLDER
Cxvxxxipx---xxxxxxxxngslxxFkaxeYNAtidfywsPllvESNSDxpxxHRvxxR


IVRIQSIEKHARHWTNSDIIVFNSYLWWRM----P----HIKSLWG---------SFEKL
TVRVQAIEKHARYWTDADILVFNTFLWWRR----R----AMNVLWG---------SFGDP
IIRADRIEKHASVWRDADIIVFNSYLWWRK----QKDDMRMKVMYG---------SFEDG
IIRADRIEKHASVWRDADVIVFNSYLWWRK----QKDDMRMKVMYG---------SFEDG
IIRADRIEKHASVWRDADVIVFNSYLWWRK----QKDDMRMKVMYG---------SFEDG
IIRADRIEKHASVWRDADVIVFNSYLWWRK----QKDDMRMKVMYG---------SFEDG
IIRADRIEKHASVWRDADVIVFNSYLWWRK----QKDDMRMKVMYG---------SFEDG
IIRADRIEKHASVWRDADIIVFNSYLWWRK----QKDDMRMKVMYG---------SFEDG
IIRADRIEKHASVWRDADIIVFNSYLWWRK----QKDDMRMKVMYG---------SFEDG
IIRADRIEKHASVWRDADIIVFNSYLWWRK----QKDDMRMKVMYG--------SFEDG
IIRADRIEKHASVWRDADVIVFNSYLWWRI----PP----CARRYG---------SFEDG
IIRADRIEKHANVWKDADFIVFNSYLWWR--------IIMQKCRYG---------SFEDG
IIRADRIEKHANVWKDADFIVFNSYLWWRK----QRDGMMMKVMYG---------SFEDG
IIRAEKIEKHARAWGNADVIVFNSYLWWRK----QKPDMKMKVMYG---------SFEDG
TVRAASINKHAAHWTNADVLVFNSYLWWQR----PAMKVLWGSFDNPAAVVAAAAEEGDE
TVRAASINKHAAHWTNADVLVFNSYLWWQR----PAMKVLX--------------EEGNE
IVRGTALEKHARFWKGADILVFNSYLWWMTGQKMKILQG---------------SFEDK
IIRPDTVLRHASLWENADILVFNTYLWWRQGPVKLLWT-----------------HEE
iiRaxxiekHAxxWxxaDxiVFNsyLWWrx----xxxxxxxxxxxg---------sfedx


DGIYKEVEMVRVYEMALQTLSQWLEVHVNPNITKLFFMSMSPTHERAEEWGGILNQNCYG
NGISKRVGMVRVYEMALRTWSEWLEVHIKPNKTKLFFVSMSPTHQKA-------------
DAKLDEMEMVDGFEIAIKKLTEWLAENIDKNKTRIFFAGSSPTHSWASNWGGQDKNKCLN
DAKLDEMEMVDGFEIAIKKLTEWLAENIDKNKTRIFFAGSSPTHSWASNWGGQDKNKCLN
DAKLDEMEMADGFEIAIKKLTEWLAKNIDKNKTRIFFAGSSPTHSWASNWGGQDKNKCLN
DAKLDEMEMADGFEIAIKKLTEWLAENIDKNKTRIFFAGSSPTHSWASNWGGEDKNKCLN
DAKLDEMEMADGFEIAIKKLTEWLAENIDKNKTRIFFAGSSPTHSWASNWGGQDKNKCLN
DAKLDEMEMVDGFEIALKKLTEWLGENIDKNKTRIFFAGSSPTHSWASNWGGEDKNKCLN
DAKLDEMEMADGFEIAIKKLTEWLAENIDKNKTRIFFAGSSPTHSWASNWGGEDKNKCLN
DAKLDEMEMVDGFEIAIKKLTEWLAENIDKNKTRIFFAGSSPTHSWASNWGGQDKNKCLN
DAKLDEMEMADGFEIAIKKLTEWLAKNIDKNKTRIFFAGSSPTHSWASNWGGQDKNKCLN
DAKLDEVQMVDGYEIALKKLTEYLGANINKNKTRIFFAGSSPAHSWASNWGGDDNNKCLN
DAKLDEVQMVDGYEIALKKLTEYLGANINKNKTRIFFAGSSPAHSWASNWGGDDNNKCLN
DAKLDEVEMVEGFEIALKKLTEWVGANVN-NKTKIYFAGSSPTHTWASDWGGDDSNKCLN
YAVSKVIDSLRAYELAVRTWADWMEFHVDRARTQLFFMTMSPTHLRSDEWEDAAAAAAGG
YAVSKVIDSLRAYELAVRTWADWMEFHVDRARTQLFFMTMSPTHLRSDEWEDAAAAAAGG
SKDIVEMETEEAYGMVLNAVVRWVENNMNPRNSRVFFVTMSPTHTRSKDWGDDSDGNCYN
NGACEELDGHGAMELAMGAWADWVSSKVDPLKKRVFFVTMSPTHLWSREWKPGSEGNCYG
daxxxexxmxxxxexaxxxlxewlxxnxxxnktrxfFxxxSPtHxwaxxwggxxxxxcxn


EA---SLIDKEGY----TGRGSDPKMMRVLENVLDGLKNRGLNMQMINITQLSEYRKEGH
------------------------------------------------------------
ET---EPISYRPGGGYKAATTDYSLMAMARSYFRRTLEPRGIRVQILNITELSDYRKDGH
ET---EPISYRPGGGYKAATTDYSLMAMARSYFRRTLEPRGIRVQILNITELSDYRKDGH
ET---EPISYRPGGGYKAATTDYSLMAMARSYFRRTLEPRGIRVQILNITELSDYRKDGH
ET---EPIYR-PGGYYKAATTDYSLMAKARSYFQRTLEPRGIRVQILNITELSDYRKDGH
ET---EPISYRPGGGYKAATTDYSLMAMARSYFRRTLEPRGIRVQILNITELSDYRKDGH
ET---EPIYKTG---YKAATTDYSLMAKAKHYFR-TLEPKGIHVQILNITELSDYRKDGH
```

```
ET---EPIYRPGG--YKAATTDYSLMAKARSYFRRTLEPRGIRVQILNITELSDYRKDGH
ET---EPISYRPGGGYKAATTDYSLMAMARSYFRRTLEPRGIRVQILNITELSDYRKDGH
ET---EPISYRPGGGYKAATTDYSLMAMARSYFRRTLEPRGIRVQILNITELSDYRKDGH
ET---EPIQIED---YRSATTDYGMMDKAKEIFG-TLEPKGIHVQILNITQLFEYRKDAH
ET---EPIQIED---YRSATTDYGMMDKAKEIFG-TLEPKGIHVQILNITQLSEYRKDAH
ES---EPIQKVG---YKGATTDYSMMDKAKQIFR-PLEQKGINVQILNFTQLTDYRIDAH
NHGCYGETEPIAAEEYRGTSGTDMAFARAVEAEARRLGERSVAVRLINVTRLSERRKDAH
NHGCYGETEPIAAEEYRGTSGTDMAFARAVEAEARRLGERSVAVRLINVTRLSERRKDAH
QT----------TPIRDLSYWGPGTSKGLMRVIGEVFSTSKVPVGIVNITQLSEYRKDAH
EK----------DPIDNEGYWGSGSDLPTMSTVEKILSNLSSKVSVINITQLSEYRKDGH
ex---xxixxxxxxyxxxxxxxxxmxxaxxxxxxxlxxxgxxvqixnitxlsxyrkdxh
```

```
PSIYRKQWGTVKENEISNPSSNADCIHWCLPGVPDVWNELLYAYILDHHSS*
--------------------------------------------------*
PTVFRRQFVPLTKEQIADPASYADCTHWCLPGVPDVWNEFLYGYLTQQRK-*
PTVFRRQFVPLTKEQIADPASYADCTHWCLPGVPDVWNEFLYGYLTQQRK-*
PTVFRRQFVPLTKEQIADPASYADCTHWCLPGVPDVWNEFLYGYLTQQSK-*
PTVFRRQFVPLTKEQIADPASYADCTHWCLPGVPDVWNEFLYGYLVHV---*
PTVFRRQFVPLTKEQIADPASYADCTHWCLPGVPDVWNEFLYGYLVHV---*
PTVYRKQFAPLTKEQIADPASYADCTHWCLPGVPDVWNEFLYGYLVQK---*
PTVFRRQFVPLTKEQIADPASYADCTHWCLPGVPDVWNEFLYGYLVQQRK-*
PTVFRRQFVPLTKEQIADPASYADCTHWCLPGVPDVWNEFLYGYLTQQRK-*
PTVFRRQFVPLTKEQIADPASYADCTHWCLPGVPDVWNEFLYGYLTQQSK-*
PTIFRRQYVPLTKEQIANPSIYADCTHWCLPGVPDVWNEFLYAYIMHK---*
PTIFRRQYVPLTKEQIANPSIYADCTHWCLPGVPDVWNEFLYAYIMHK---*
PTVFRRQFTPLTKEQIANPSSYADCTHWCLPGVPDVWNHFLYSYLVQK---*
PSVHRRYWDPVTDEQRRNPSSYADCIHWCLPGVPDVWNQLLYAHIVS----*
PSVHRRYWDPVTDEQRRNPSSYADCIHWCLPGVPDVWNQLLYAHIVS----*
TQIYKKQWNPLTPEQIANPKSYADCTHWCLPGLQDTWNELLYSKLFFP---*
PSIFRKFWEPLRPEQLSNPPSYSDCIHWCLPGVPDVWNELLFHFL------*
pxxxrrqxxpltxeqiaxpxsyadcthwclpgvpdvwnexlyxyxxxxx-*
```

Figure 2. Corn transformation base vector

Figure 3. Soybean transformation base vector

**Europäisches Patentamt**
**European Patent Office**
**Office européen des brevets**

# EUROPEAN SEARCH REPORT

**Application Number**

EP 16 15 1745

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE Geneseq [Online]<br><br>17 October 2000 (2000-10-17),<br>"Arabidopsis thaliana DNA fragment SEQ ID NO: 33257.",<br>XP002757481,<br>retrieved from EBI accession no. GSN:AAC41820<br>Database accession no. AAC41820<br>* the whole document * | 1-5,7 | INV.<br>C12N5/04<br>C12N15/82<br>C12N15/90<br>A01H5/00<br>C12N9/10<br>C12N9/24 |
| X | -& EP 1 033 405 A2 (CERES INC [US])<br>6 September 2000 (2000-09-06)<br>* Seq. ID No. 33257; claims 1-4, 10-18, 23, 29-34; pages 327-329 *<br>----- | 1-5,7,<br>10-12 | |
| X | DATABASE EMBL [Online]<br><br>22 March 2001 (2001-03-22),<br>"Arabidopsis thaliana putative 3-methyladenine DNA glycosylase (At1g75230) mRNA, complete cds.",<br>XP002757482,<br>retrieved from EBI accession no. EM_STD:AF360213<br>Database accession no. AF360213<br>* the whole document *<br>----- | 1-5,7 | |
| | | | **TECHNICAL FIELDS SEARCHED (IPC)**<br><br>C12N |
| A | Anne Santerre ET AL: "Cloning of a 3-methyladenine-DNA glycosylase from Arabidopsis thaliana (DNA repair)",<br>PNAS, Vol. 91, No. 6,<br>15 March 1994 (1994-03-15), pages 2240-2244, XP055271320,<br>Retrieved from the Internet:<br>URL:http://www.pnas.org/content/91/6/2240.full.pdf<br>[retrieved on 2016-05-10]<br>* abstract; pages 2243 and 2244 *<br>----- | 1-15 | |

-/--

The present search report has been drawn up for all claims

| Place of search | Date of completion of the search | Examiner |
|---|---|---|
| Munich | 10 May 2016 | Kurz, Birgit |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 16 15 1745

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ZONGRANG LIU ET AL: "Arabidopsis UVH3 gene is a homolog of the Saccharomyces cerevisiae RAD2 and human XPG DNA repair genes", THE PLANT JOURNAL, vol. 26, no. 3, 1 May 2001 (2001-05-01), pages 329-338, XP055271303, GB ISSN: 0960-7412, DOI: 10.1046/j.1365-313X.2001.01031.x * page 330 * ----- | 1-15 | |

TECHNICAL FIELDS
SEARCHED (IPC)

The present search report has been drawn up for all claims

| Place of search | Date of completion of the search | Examiner |
|---|---|---|
| Munich | 10 May 2016 | Kurz, Birgit |

EPO FORM 1503 03.82 (P04C01)

## ANNEX TO THE EUROPEAN SEARCH REPORT
## ON EUROPEAN PATENT APPLICATION NO.

EP 16 15 1745

This annex lists the patent family members relating to the patent documents cited in the above-mentioned European search report.
The members are as contained in the European Patent Office EDP file on
The European Patent Office is in no way liable for these particulars which are merely given for the purpose of information.

10-05-2016

| Patent document cited in search report | | Publication date | Patent family member(s) | Publication date |
|---|---|---|---|---|
| EP 1033405 | A2 | 06-09-2000 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 60642717 B
- US 20030101479 A, Cheikh
- US 5188642 A
- US 5728925 A
- US 4196265 A
- US 5858742 A
- US 5322938 A
- US 5378619 A
- US 6437217 B
- US 5641876 A
- US 6426446 B
- US 6429362 B
- US 6232526 B
- US 6177611 B
- US 6433252 B
- US 6429357 B
- US 5837848 A
- US 6084089 A
- US 6294714 B
- US 6140078 A
- US 6252138 B
- US 6175060 B
- US 20020192813 A1
- US 078972 A
- US 757089 A
- US 739565 A
- US 10739565 B
- US 5420034 A
- US 5107065 A, Shewmaker
- US 5759829 A, Shewmaker
- US 5283184 A, Jorgensen
- US 5231020 A, Jorgensen
- EP 0426195 A1, Goldbach
- WO 9401550 A, Agrawal
- WO 9805770 A, Werner
- US 20030175965 A1, Lowe
- US 20020048814 A1
- US 20030018993 A1, Gutterson
- US 20030036197 A1, Glassman
- US 6326193 B
- WO 9953050 A, Waterhouse
- WO 9949029 A, Graham
- US 465800 A
- US 6506559 B, Fire
- US 393347 A, Shewmaker
- US 6448473 B, Mitsky
- US 5015580 A
- US 5550318 A
- US 5538880 A
- US 5914451 A
- US 6160208 A
- US 6399861 B
- US 6153812 A
- US 5159135 A
- US 5824877 A
- US 5591616 A
- US 6384301 B
- US 4959317 A
- US 5527695 A
- US 6194636 B
- US 5633435 A
- US 5780708 A
- US 6118047 A
- US 20030106096 A1
- US 20020112260 A1
- US 5034322 A
- US 5776760 A
- US 6107549 A
- US 6376754 B
- US 5250515 A
- US 5880275 A
- US 6506599 B
- US 5986175 A
- US 20030150017 A1
- US 303745 A

### Non-patent literature cited in the description

- **S.R. EDDY.** Profile Hidden Markov Models. *Bioinformatics,* 1998, vol. 14, 755-763
- Antibodies: A Laboratory Manual. Cold Spring Harbor Laboratory, 1988
- *Goding,* 1986, 60-61
- **RUSSELL et al.** *Transgenic Res.,* 1997, vol. 6 (2), 157-166
- **BELANGER et al.** *Genetics,* 1991, vol. 129, 863-872
- **STACY et al.** *PlantMol Biol.,* 1996, vol. 31 (6), 1205-1216
- **FISCHHOFF et al.** *Plant Mol Biol.,* 1992, vol. 20, 81-93

- **TANIGUCHI et al.** *Plant Cell Physiol.,* 2000, vol. 41 (1), 42-48
- **REDENBAUGH et al.** Safety Assessment of Genetically Engineered Flavr Savr™ Tomato. CRC Press, Inc, 1992
- **JORGENSEN et al.** *Mol. Gen. Genet.,* 1987, vol. 207, 471-477
- **STAM et al.** *The Plant Journal,* 1997, vol. 12 (1), 63-82
- **SIJEN et al.** *The Plant Cell,* 1996, vol. 8, 2277-2294
- **METTE et al.** *The EMBO Journal,* 1999, vol. 18 (1), 241-148
- **METTE et al.** *The EMBO Journal,* 2000, vol. 19 (19), 5194-5201, 148
- **PILON-SMITS et al.** *Plant Physiol.,* 1995, vol. 107, 125-130
- **BETHTOLD et al.** *Methods Mol. Biol.,* 1998, vol. 82, 259-66
- **BOYES et al.** *The Plant Cell,* 2001, vol. 13, 1499-1510
- **BOYES, D.C.** *The Plant Cell,* 2001, vol. 13, 1499, , 1510